# EUROPEAN PATENT APPLICATION

(11) **EP 4 596 544 A1**
(43) Date of publication of application: **06.08.2025**
(21) Application number: 23859530.0
(22) Date of filing: 19.10.2023
(51) Int. Cl.: C07D 471/04, A61K 31/4375, A61K 31/675, A61P 35/00, A61P 3/00, A61P 25/00

(54) **EP300/CBP REGULATOR, AND PREPARATION METHOD THEREFOR AND USE THEREOF**

(30) Priority: 29.08.2022 CN 202211043600; 13.09.2022 CN 202211112301; 13.09.2022 US 202263375444 P; 18.08.2023 CN 202311049072
(71) Applicant: Miracure Biotechnology Limited, Haidian, Beijing 100192 (CN)
(72) Inventor: LI, Xiaolin, Beijing 100192 (CN); QI, Longwu, Beijing 100192 (CN); XU, Shusen, Beijing 100192 (CN); BIE, Jianbo, Beijing 100192 (CN)
(74) Representative: Gille Hrabal Partnerschaftsgesellschaft mbB Patentanwälte
(86) International application number: PCT/CN2023/125452
(87) International publication number: WO 2024/046504

(57) **Abstract**

The present disclosure relates to an EP300/CBP modulator as shown in formula I, and a preparation method therefor and the use thereof. The structure of formula I is as follows.

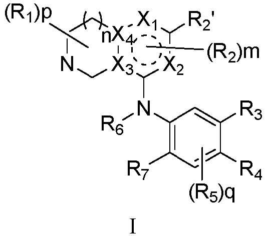

## Description

The present disclosure claims:
priority to a prior application with the application No. 2022110436007 entitled "EP300/CBP INHIBITOR, PREPARATION METHOD THEREFOR AND USE THEREOF" and filed with China National Intellectual Property Administration on Aug. 29, 2022;
priority to a prior application with the application No. 2022111123014 entitled "EP300/CBP INHIBITOR, PREPARATION METHOD THEREFOR AND USE THEREOF" and filed with China National Intellectual Property Administration on Sept. 13, 2022;
priority to a prior application with the U.S. Provisional Application No. 63/375,444 entitled "EP300/CBP INHIBITOR, PREPARATION METHOD THEREFOR AND USE THEREOF" on Sept. 13, 2022; and
priority to a prior application with the application No. 2023110490720 entitled "EP300/CBP MODULATOR, PREPARATION METHOD THEREFOR AND USE THEREOF" and filed with China National Intellectual Property Administration on Aug. 18, 2023;
the contents of which are incorporated herein by reference in their entirety

### TECHNICAL FIELD

The present disclosure belongs to the field of pharmaceuticals, and particularly relates to an EP300/CBP modulator, a preparation method therefor and use thereof.

### BACKGROUND

Histone acetyltransferases (HATs) and histone deacetylases (HDACs) can affect histone acetylation. Recruitment and normal functioning of HATs and HDACs are key regulatory steps for gene expression and the cell cycle. Functional defects of these enzymes may lead to various diseases including tumors. The E1A binding protein EP300 (EP300) and its closely related analog cAMP response element binding protein (CBP) binding protein are extensively expressed lysine residue acetyltransferases that are capable of transferring acetyl groups from acetyl-CoA to ε-N-acetyl lysine to acetylate conserved lysine residues in histones.

EP300/CBP is also capable of acetylating non-histone proteins and, together with transcription factors, forming transcription complexes as transcriptional coactivators to regulate gene expression as nuclear receptors and other transcription factors do. EP300 and CBP are protein molecules with multiple functional domains that regulate the interactions between many proteins. EP300 and CBP, as transcription coactivators, are involved in the regulation of target gene expression by binding to different transcription factors. Changes in protein expression affect many fundamental functions of cells, including proliferation, the cell cycle, cell differentiation, DNA damage responses, etc., and thus the phenotype of the cells, and play an important role in the development and progression of many tumors. Current research shows that EP300/CBP is highly expressed and activated in various tumors, and that EP300/CBP is closely associated with various tumor diseases and is a target that holds great promise of being applied to the treatment of tumors. Therefore, EP300/CBP modulators are of increasing interest to researchers.

The bromodomain of EP300/CBP is a domain of about 110 amino acids capable of recognizing acetylated lysine residues. Bromodomains, as "readers" of lysine acetylation, are responsible for transducing the signal carried by acetylated lysine residues and translating the signal into normal or abnormal phenotypes. The bromodomain of EP300/CBP has a wide range of functions ranging from histone acetyltransferase activity and chromatin remodeling to mediating transcriptional coactivation. There is now much evidence that bromodomains play a major role in the malignant progression of tumors. Modulators for the characteristic bromodomain of EP300/CBP are of great developmental value and clinical significance in a variety of tumors.

However, a small number of modulators for the characteristic bromodomain of EP300/CBP that have been reported are mostly at early stages of development or early clinical trials and have the disadvantages of having relatively low activity and being not safe enough and the like and thus are far from meeting the clinical requirements. Therefore, it is of great significance to develop more EP300/CBP modulators of novel structures with higher selectivity and better modulatory activity for the treatment of a variety of EP300/CBP-associated tumors.

### SUMMARY

To solve the problems in the prior art, the present disclosure provides in a first aspect a compound represented by formula I and a racemate, a stereoisomer, a tautomer, an isotopic derivative, a nitrogen oxide, a solvate, a polymorph, a metabolite, an ester, a prodrug or a pharmaceutically acceptable salt thereof: 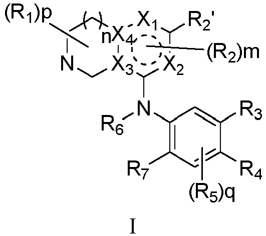 wherein:
n = 0, 1 or 2;
m = 0, 1, 2 or 3;
p = 0, 1, 2, 3, 4 or 5;
q = 0, 1 or 2;
X₁, X₂, X₃ and X₄ are each independently selected from C and N;
each R₁ is the same or different and is independently selected from oxo (=O), halogen, CN, NH₂, COOH, OH, and the following groups that are unsubstituted or optionally substituted with 1, 2 or more R₁a: C₁₋₁₂ alkyl, C₁₋₁₂ alkoxy, and -(C=O)R;
the R is selected from C₁₋₁₂ alkyl, C₁₋₁₂ alkoxy, NH₂-, C₁₋₁₂ alkyl-NH-, N,N-di C₁₋₁₂ alkylamino, and C₃₋₁₂ cycloalkyl; each R₁a is the same or different and is independently selected from oxo (=O), halogen, CN, NH₂, COOH, OH, C₁₋₁₂ alkyl, and C₁₋₁₂ alkoxy;
each R₂ is the same or different and is independently selected from oxo (=O), halogen, CN, NH₂, COOH, OH, C₁₋₁₂ alkyl, and C₁₋₁₂ alkoxy;
R₂' is selected from H, halogen, CN, NH₂, COOH, OH, and the following groups that are unsubstituted or optionally substituted with 1, 2 or more R₂a: C₆₋₁₄ aryl, 5- to 14-membered heteroaryl, 3- to 14-membered heterocyclyl, C₃₋₁₂ cycloalkyl, and C₁₋₁₂ alkyl;
each R₂a is the same or different and is independently selected from H, oxo (=O), halogen, COOH, OH, and the following groups that are unsubstituted or optionally substituted with 1, 2 or more R₂b: NH₂, C₁₋₁₂ alkyl, C₁₋₁₂ alkoxy, C₁₋₁₂ alkylthio, C₁₋₁₂ alkyl-NH-, N,N-di C₁₋₁₂ alkylamino, C₁₋₁₂ alkyl C(=O)-, C₁₋₁₂ alkoxy C(=O)-, C₁₋₁₂ alkyl-NHC(=O)-, N,N-di C₁₋₁₂ alkylaminocarbonyl, C₆₋₁₄ aryl, 5- to 14-membered heteroaryl, 3- to 14-membered heterocyclyl, C₃₋₁₂ cycloalkyl, C₆₋₁₄ aryl C(=O)-, 5- to 14-membered heteroaryl C(=O)-, 3- to 14-membered heterocyclyl C(=O)-, C₃₋₁₂ cycloalkyl C(=O)-, C₁₋₁₂ alkyl S(=O)₂-, C₁₋₁₂ alkyl S(=O)-, C₁₋₁₂ alkyl S(=O)(=NH)-, C₃₋₁₂ cycloalkyl S(=O)₂-, C₁₋₁₂ alkyl-C(=O)-NH-, C₁₋₁₂ alkyl-S(=O)₂-NH-;
each R₂b is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino;
R₃ is selected from H, halogen, CN, NH₂, COOH, OH, and the following groups that are unsubstituted or optionally substituted with 1, 2 or more R₃a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), N,N-di C₁₋₁₂ alkylaminocarbonyl, C₆₋₁₄ aryl, 5- to 14-membered heteroaryl, 3- to 14-membered heterocyclyl, and C₃₋₁₂ cycloalkyl;
each R₃a is the same or different and is independently selected from oxo (=O), halogen, CN, NH₂, COOH, OH, and the following groups that are unsubstituted or optionally substituted with 1, 2 or more R₃b: C₁₋₁₂ alkyl, C₁₋₁₂ alkoxy, C₁₋₁₂ alkyl-NH-, N,N-di C₁₋₁₂alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
each R₃b is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino;
R₄ is selected from H, halogen, CN, NH₂, COOH, OH, and the following groups that are unsubstituted or optionally substituted with 1, 2 or more R₄a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), N,N-di C₁₋₁₂ alkylaminocarbonyl, C₆₋₁₄ aryl, 5- to 14-membered heteroaryl, 3- to 14-membered heterocyclyl, and C₃₋₁₂ cycloalkyl;
each R₄a is the same or different and is independently selected from oxo (=O), halogen, CN, NH₂, COOH, OH, and the following groups that are unsubstituted or optionally substituted with 1, 2 or more R₄b: C₁₋₁₂ alkyl, C₁₋₁₂ alkoxy, C₁₋₁₂ alkyl-NH-, N,N-di C₁₋₁₂alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
each R₄b is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino;
each R₅ is the same or different and is independently selected from oxo (=O), halogen, CN, NH₂, COOH, OH, and the following groups that are unsubstituted or optionally substituted with 1, 2 or more R₅a: C₁₋₁₂ alkyl, and C₁₋₁₂ alkoxy;
each R₅a is the same or different and is independently selected from oxo (=O), halogen, CN, NH₂, COOH, OH, C₁₋₁₂ alkyl, and C₁₋₁₂ alkoxy;
R₆ is selected from H, halogen, CN, NH₂, COOH, OH, and the following groups that are unsubstituted or optionally substituted with 1, 2 or more R₆a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), and N,N-di C₁₋₁₂ alkylaminocarbonyl;
each R₆a is the same or different and is independently selected from oxo (=O), halogen, CN, NH₂, COOH, OH, C₁₋₁₂ alkyl, C₁₋₁₂ alkoxy, C₁₋₁₂ alkyl-NH-, N,N-di C₁₋₁₂ alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
R₇ is selected from H, halogen, CN, NH₂, COOH, OH, and the following groups that are unsubstituted or optionally substituted with 1, 2 or more R₇a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), and N,N-di C₁₋₁₂ alkylaminocarbonyl;
each R₇a is the same or different and is independently selected from oxo (=O), halogen, CN, NH₂, COOH, OH, C₁₋₁₂ alkyl, C₁₋₁₂ alkoxy, C₁₋₁₂ alkyl-NH-, N,N-di C₁₋₁₂ alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
or together R₆ and R₇ form the following group that is unsubstituted or optionally substituted with 1, 2 or more Rsa: 5- to 14-membered heteroaryl, or 3- to 14-membered heterocyclyl, wherein the 5- to 14-membered heteroaryl or the 3- to 14-membered heterocyclyl contains at least one N atom;
each Rsa is the same or different and is independently selected from oxo (=O), halogen, CN, NH₂, COOH, OH, and the following groups that are unsubstituted or optionally substituted with 1, 2 or more Rsb: C₁₋₁₂ alkyl, C₁₋₁₂ alkoxy, C₁₋₁₂ alkyl-NH-, N,N-di C₁₋₁₂alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
each Rsb is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino.

In some embodiments, X₁, X₂, X₃ and X₄ are all selected from C.

In some embodiments, X₁ and X₂ are N; X₃ and X₄ are C.

In some embodiments,

In some embodiments, in formula I, the moiety 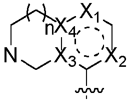 is selected from the following structures: 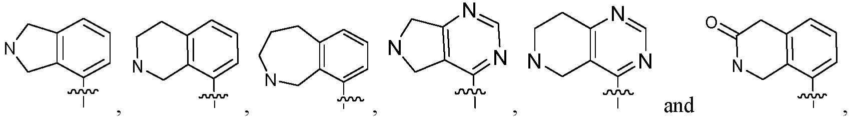 and the structures comprise R₁ and R₂ substituents as defined above.

In some embodiments, each R₁ is the same or different and is independently selected from oxo (=O), C₁₋₆ alkyl, C₁₋₆ alkoxy, and -(C=O)R, wherein the R is selected from C₁₋₆ alkyl, C₁₋₆ alkoxy, NH₂-, C₁₋₆ alkyl-NH-, and C₃₋₆ cycloalkyl.

In some embodiments, R₁ is a substituent at the ring N atom, illustratively, for example, 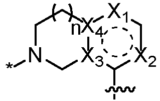 (* indicates the substitution position for R₁).

In some embodiments, R₁ is -(C=O)CH₃.

In some embodiments, each R₁ is the same or different and is independently selected from =O, CH₃, -C(=O)CH₃, - C(=O)OCH₃, -C(=O)OCH₂CH₃, CH₃NHC(=O)-, CH₃CH₂NHC(=O)-, NH₂C(=O)-, and cyclopropyl(=O)-.

In some embodiments, R₂' is selected from H, Cl, and C₆₋₁₀ aryl, 5- to 9-membered heteroaryl, 3- to 12-membered heterocyclyl, C₃₋₈ cycloalkyl and C₁₋₃ alkyl that are unsubstituted or optionally substituted with 1, 2 or more R₂a, wherein the heterocyclyl contains 1-4 (1, 2, 3 or 4) heteroatoms selected from N, O and S.

In some embodiments, R₂' is selected from H, C, and the following structures that are unsubstituted or optionally substituted with 1, 2 or more R₂a:

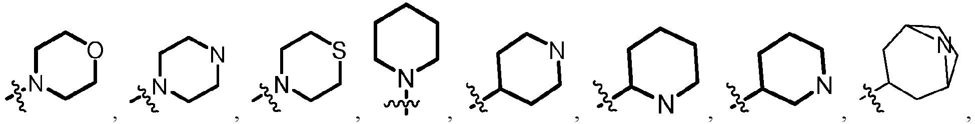

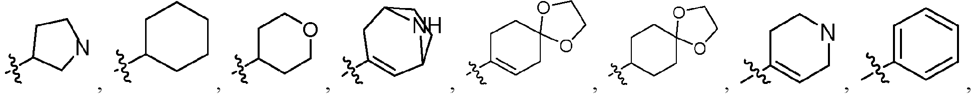

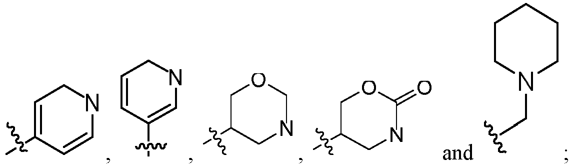

In some embodiments, R₂' is selected from the following structures:

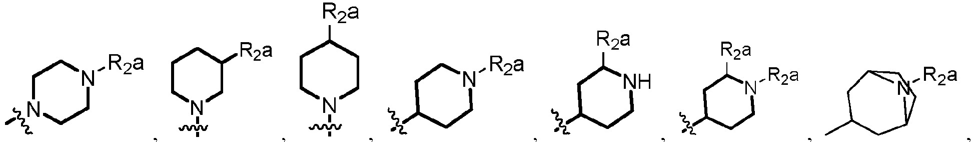

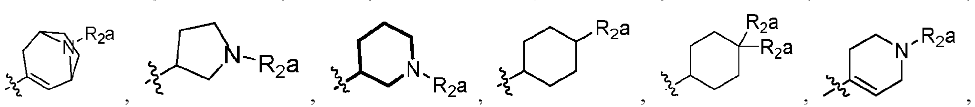

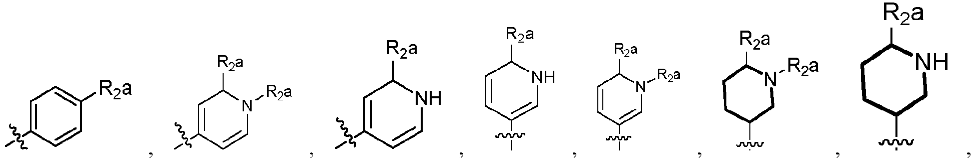

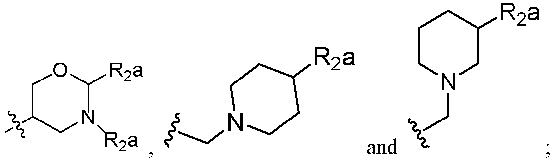

In some embodiments, R₂a is selected from H, halogen (such as F), methyl, ethyl, oxo (=O), methoxy, OH, COOH, 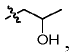 CH₃C(=O)-, -CH₂CHF₂, -CH₂CF₃, -Cbz, -Boc, amino, methylamino, dimethylamino, methylthio,

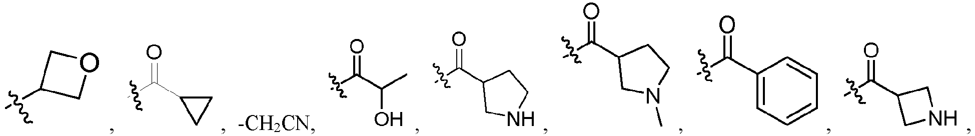

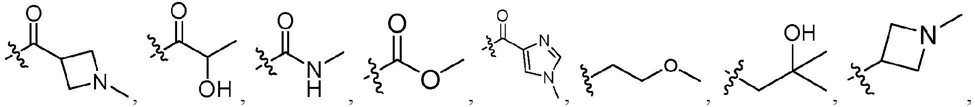

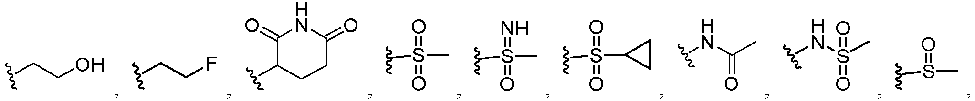

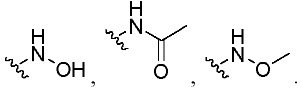

In some embodiments, R₂' is selected from the following structures: H, Cl,

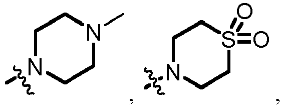

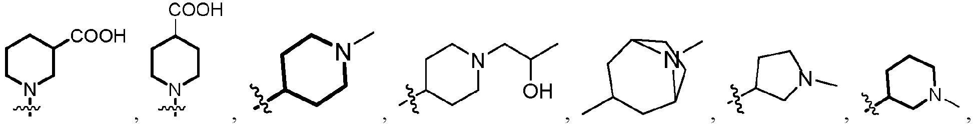

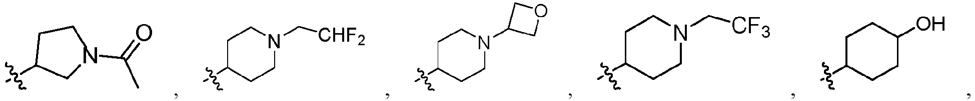

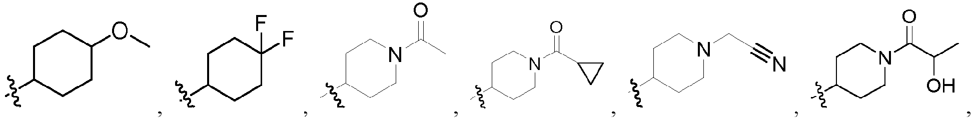

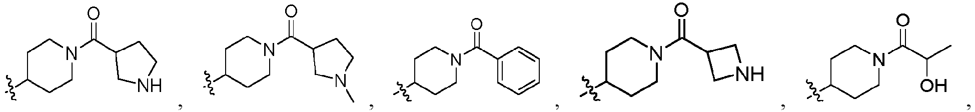

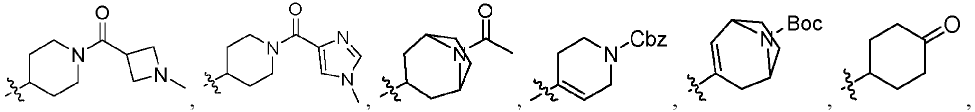

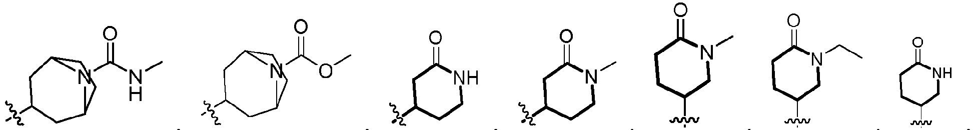

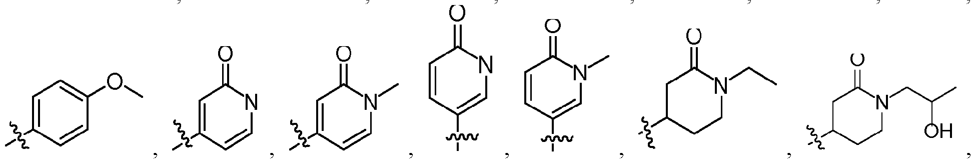

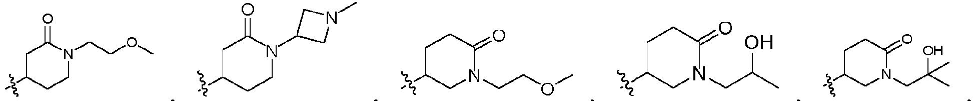

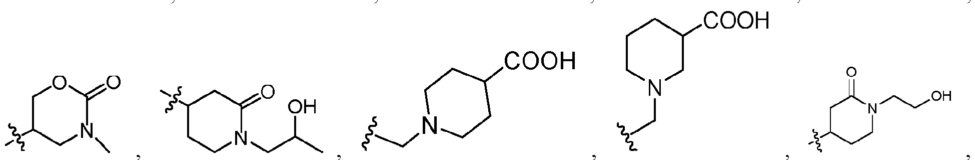

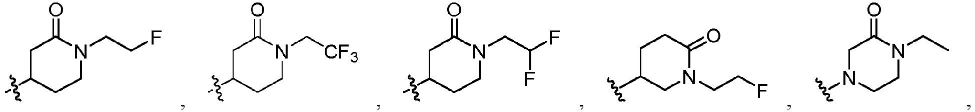

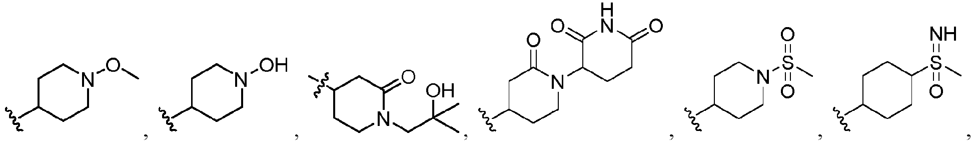

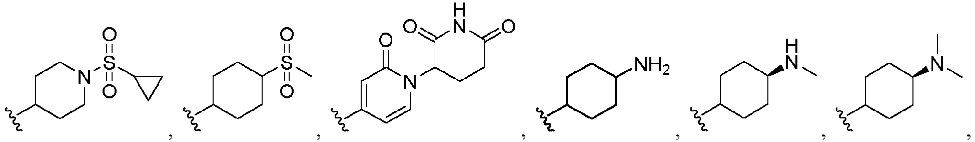

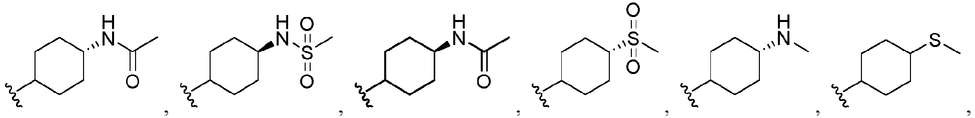

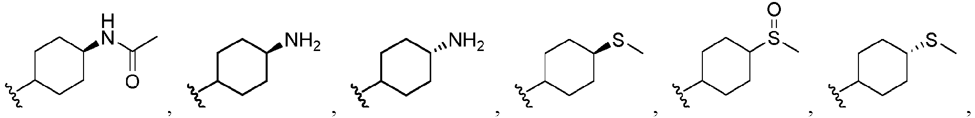

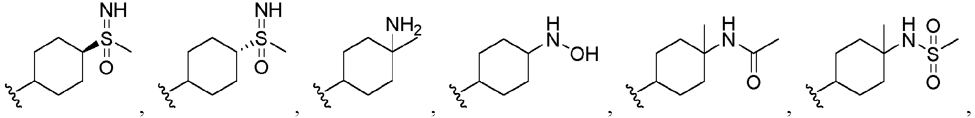

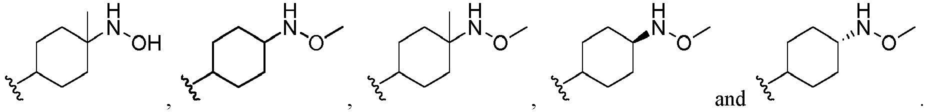

In some embodiments, R₃ is selected from H, and the following groups that are unsubstituted or optionally substituted with 1, 2 or more R₃a: C₁₋₆ alkyl, C₁₋₆ alkoxy, and C₃₋₈ cycloalkyl, wherein each R₃a is the same or different and is independently selected from halogen, CN, NH₂, COOH, and OH.

In some embodiments, R₃ is selected from H, -CHF₂, and

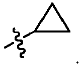

In some embodiments, R₄ is selected from 5- to 6-membered heteroaryl (e.g., pyrazolyl) that is unsubstituted or optionally substituted with 1, 2 or more R₄a, wherein each R₄a is the same or different and is independently selected from the following groups that are unsubstituted or optionally substituted with 1, 2 or more R₄b: C₁₋₆ alkyl, C₁₋₆ alkoxy, and N,N-di C₁₋₆ alkylaminocarbonyl, wherein each R₄b is the same or different and is independently selected from oxo (=O), halogen, CN, NH₂, COOH, OH, C₁₋₆ alkyl, and C₁₋₆ alkoxy.

In some embodiments, R₄ is selected from

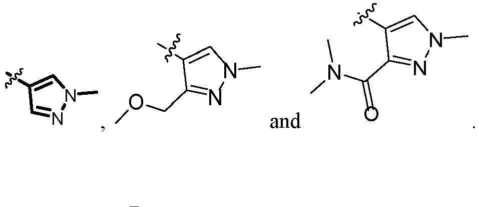

In some embodiments, each R₅ is the same or different and is independently selected from H, halogen, C₁₋₆ alkyl, and C₁₋₆ alkoxy.

In some embodiments, R₆ is selected from H, halogen, C₁₋₆ alkyl, and C₁₋₆ alkoxy.

In some embodiments, R₆ is selected from H.

In some embodiments, R₇ is selected from H, halogen, C₁₋₆ alkyl, and C₁₋₆ alkoxy.

In some embodiments, R₇ is selected from F.

In some embodiments, together R₆ and R₇ form the following groups that are unsubstituted or optionally substituted with 1, 2 or more Rsa: 5- to 6-membered heteroaryl, and 5- to 6-membered heterocyclyl.

In some embodiments, together R₆ and R₇ form the following structure that is unsubstituted or optionally substituted with 1, 2 or more Rsa:

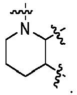

In some embodiments, the formula I is further selected from the following structure represented by formula I-1: 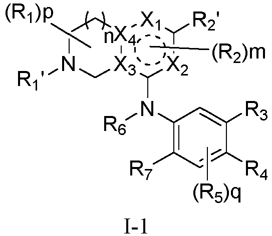
wherein, X₁, X₂, X₃, X₄, R₁, R₂, R₂', R₃, R₄, R₅, R₆, R₇, R, n, m, p and q are as defined in the compound of formula (I);
R₁' has the definition of R₁.

In some embodiments, R₁' is selected from oxo (=O), C₁₋₆ alkyl, C₁₋₆ alkoxy, and -(C=O)R, wherein the R is selected from C₁₋₆ alkyl, C₁₋₆ alkoxy, NH₂-, C₁₋₆ alkyl-NH-, and C₃₋₆ cycloalkyl.

In some embodiments, R₁' is selected from H, CH₃, -C(=O)CH₃, -C(=O)OCH₃, -C(=O)OCH₂CH₃, - C(=O)OCH(CH₃)₃, CH₃NHC(=O)-, CH₃CH₂NHC(=O)-, NH₂C(=O)-, and cyclopropyl-C(=O)-.

In some embodiments, R₁' is -(C=O)CH₃.

In some embodiments, the formula I is further selected from the following structures represented by formula II, formula III and formula IV:

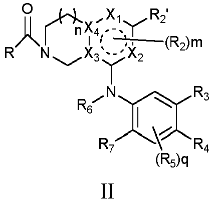

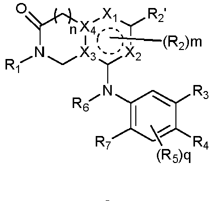

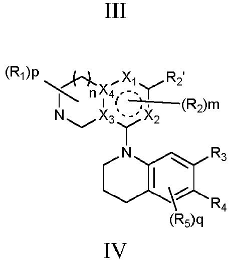

In the formula II, formula III and formula IV, X₁, X₂, X₃, X₄, R₁, R₂, R₂', R₃, R₄, R₅, R₆, R₇, R, n, m, p and q are as defined in the compound represented by formula (I).

In some embodiments, the formula I is further selected from the following structures represented by formula IIa and formula IIIa:

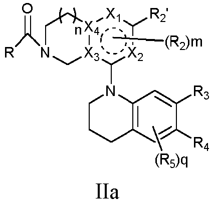

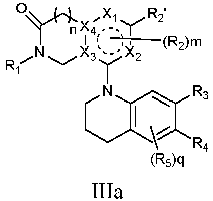

In the formula IIa and formula IIIa, X₁, X₂, X₃, X₄, R₁, R₂, R₂', R₃, R₄, R₅, R, n, m, p and q are as defined in the compound represented by formula (I).

In some embodiments, exemplary specific compounds of the compound represented by formula (I) are as follows:

| Compound ID | Structural formula | Compound ID | Structural formula |
|---|---|---|---|
| M001001 | 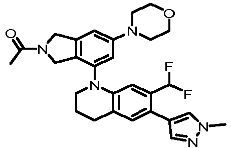 | M001191 | 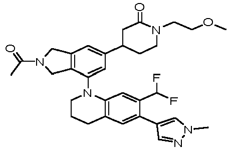 |
| M001002 | 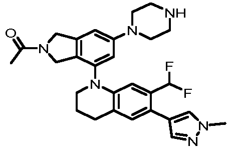 | M001192 | 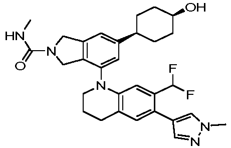 |
| M001003 | 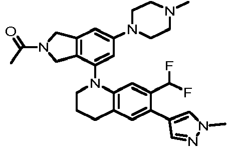 | M001193 | 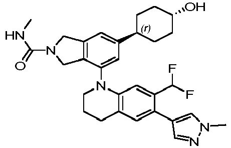 |
| M001004 | 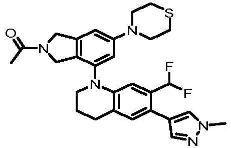 | M001194 | 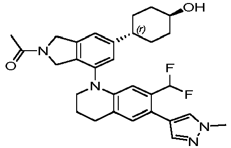 |
| M001006 | 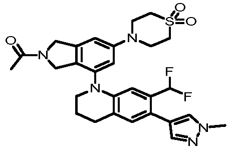 | M001195 | 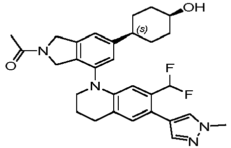 |
| M001014 | 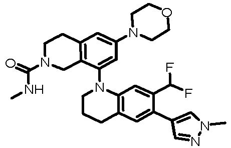 | M001196 | 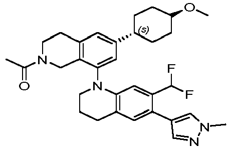 |
| M001015 | 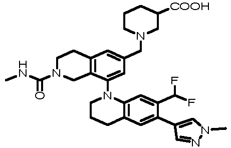 | M001197 | 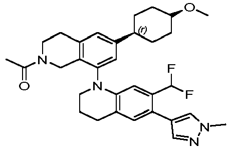 |
| M001016 | 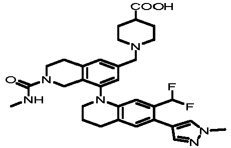 | M001198 | 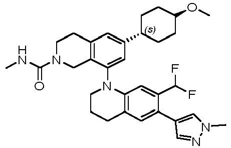 |
| M001017 | 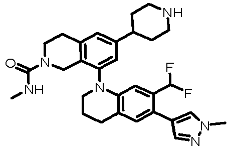 | M001199 | 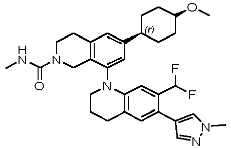 |
| M001018 | 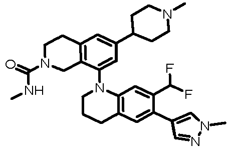 | M001200 | 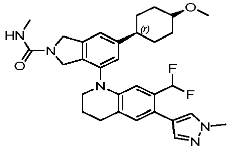 |
| M001019 | 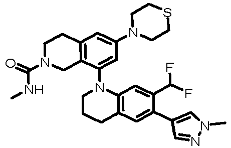 | M001201 | 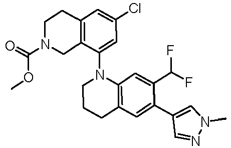 |
| M001021 | 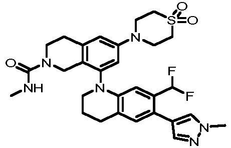 | M001202 | 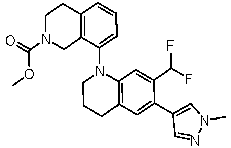 |
| M001024 | 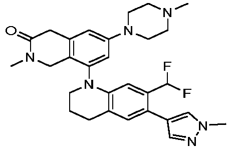 | M001203 | 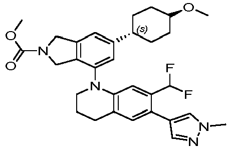 |
| M001027 | 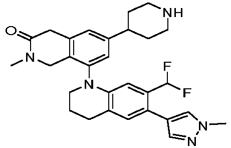 | M001204 | 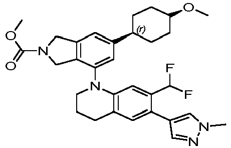 |
| M001028 | 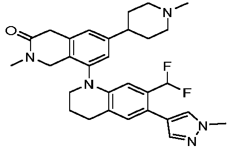 | M001206 | 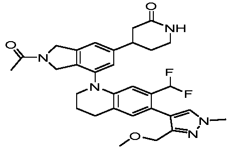 |
| M001030 | 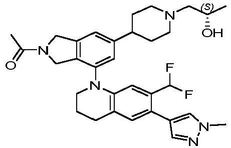 | M001207 | 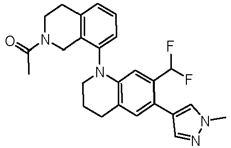 |
| M001032 | 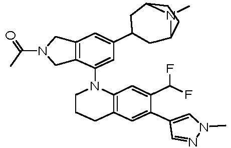 | M001208 | 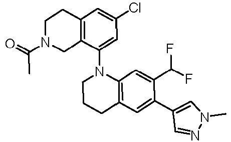 |
| M001033 | 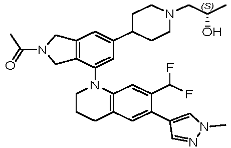 | M001209 | 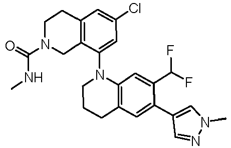 |
| M001035 | 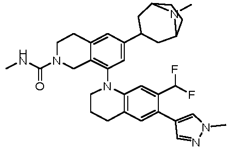 | M001210 | 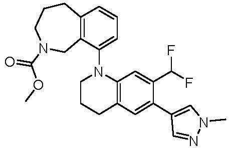 |
| M001040 | 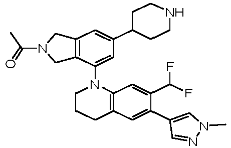 | M001211 | 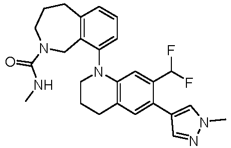 |
| M001041 | 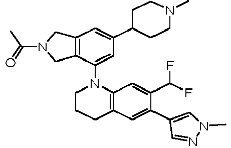 | M001212 | 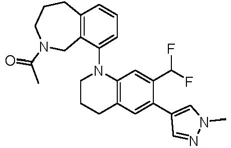 |
| M001042 | 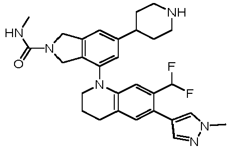 | M001213 | 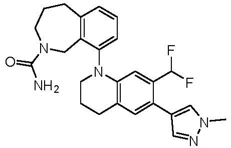 |
| M001043 | 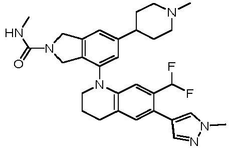 | M001214 | 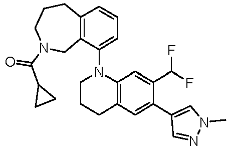 |
| M001044 | 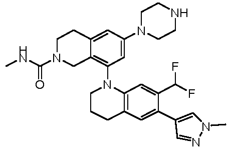 | M001215 | 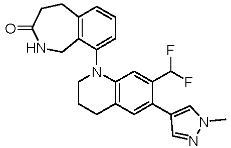 |
| M001045 | 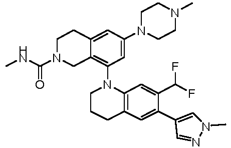 | M001217 | 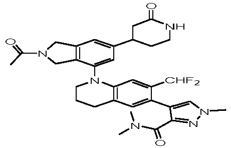 |
| M001046 | 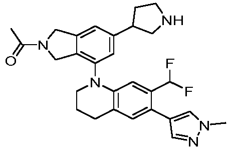 | M001218 | 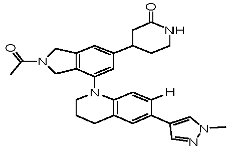 |
| M001047 | 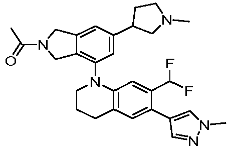 | M001219 | 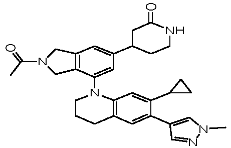 |
| M001048 | 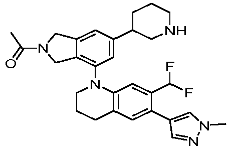 | M001221 | 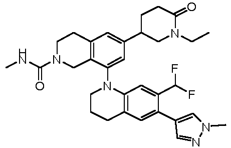 |
| M001049 | 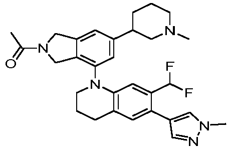 | M001222 | 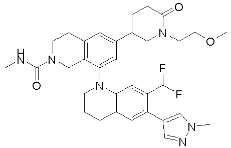 |
| M001050 | 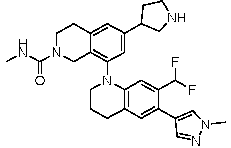 | M001223 | 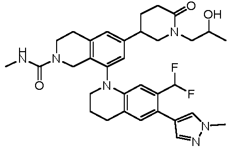 |
| M001051 | 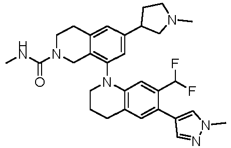 | M001224 | 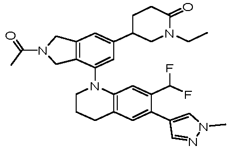 |
| M001052 | 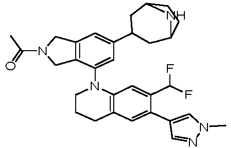 | M001225 | 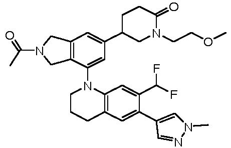 |
| M001053 | 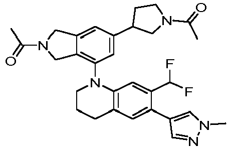 | M001226 | 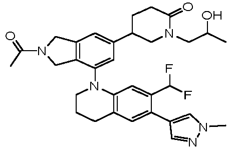 |
| M001054 | 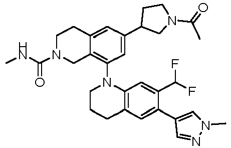 | M001227 | 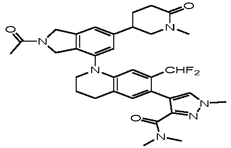 |
| M001055 | 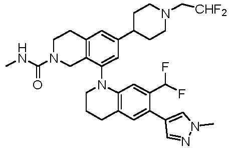 | M001228 | 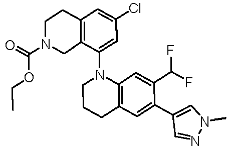 |
| M001056 | 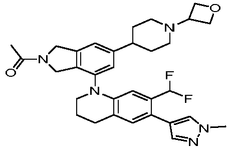 | M001229 | 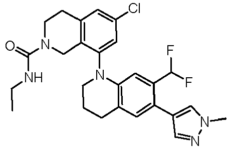 |
| M001057 | 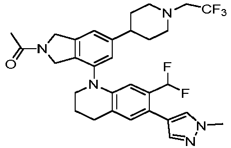 | M001230 | 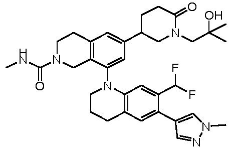 |
| M001058 | 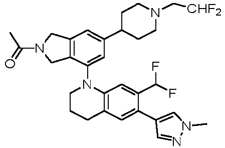 | M001231 | 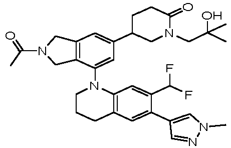 |
| M001059 | 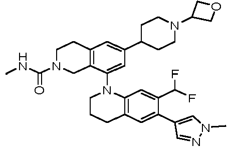 | M001232 | 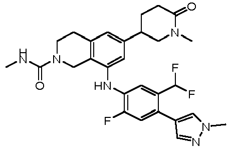 |
| M001060 | 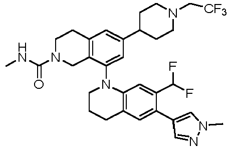 | M001233 | 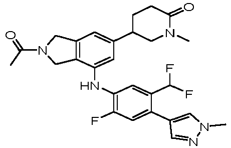 |
| M001061 | 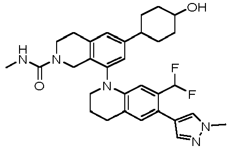 | M001234 | 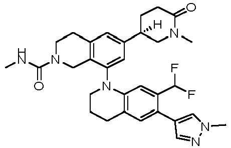 |
| M001062 | 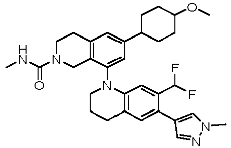 | M001235 | 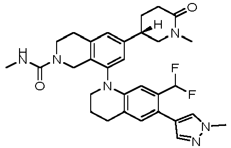 |
| M001063 | 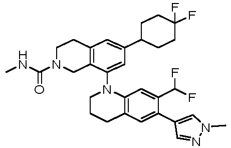 | M001236 | 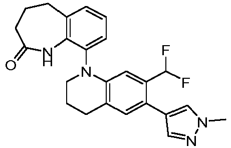 |
| M001064 | 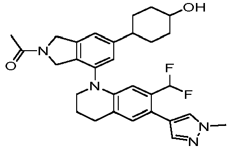 | M001237 | 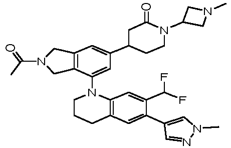 |
| M001065 | 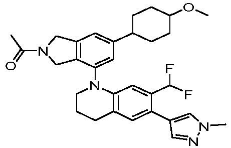 | M001238 | 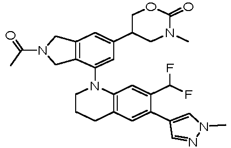 |
| M001066 | 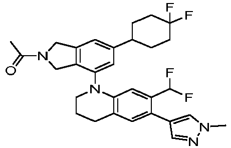 | M001239 | 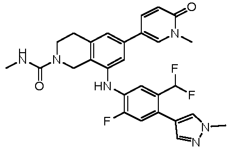 |
| M001067 | 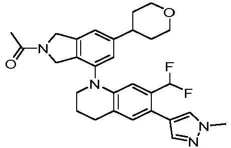 | M001240 | 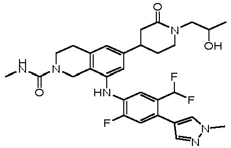 |
| M001068 | 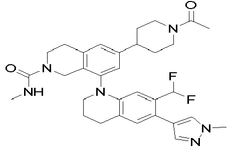 | M001241 | 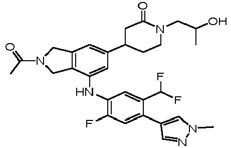 |
| M001069 | 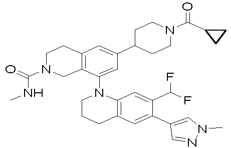 | M001242 | 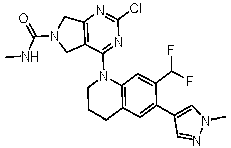 |
| M001070 | 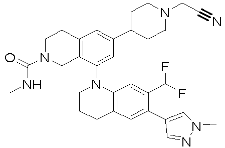 | M001243 | 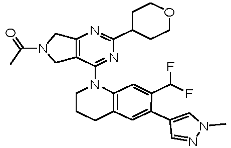 |
| M001071 | 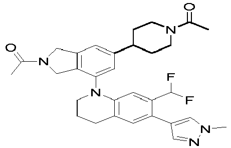 | M001244 | 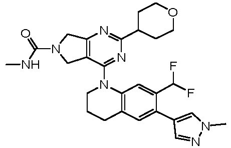 |
| M001072 | 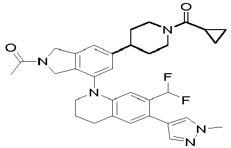 | M001245 | 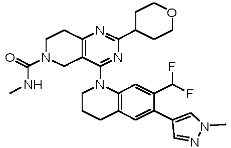 |
| M001073 | 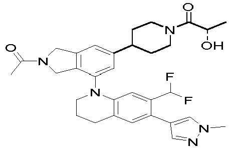 | M001246 | 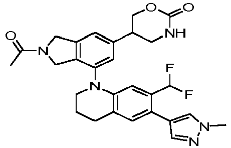 |
| M001074 | 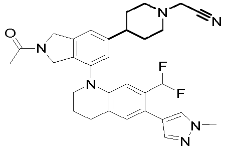 | M001247 | 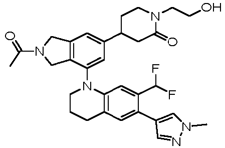 |
| M001075 | 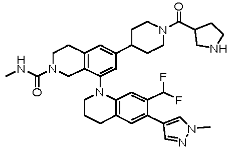 | M001248 | 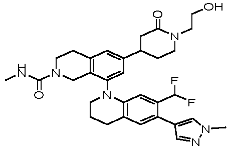 |
| M001076 | 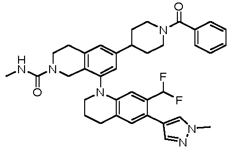 | M001249 | 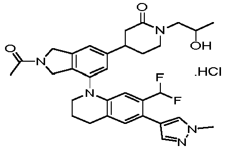 |
| M001079 | 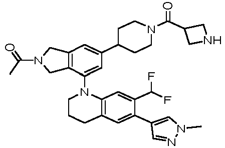 | M001250 | 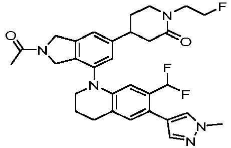 |
| M001080 | 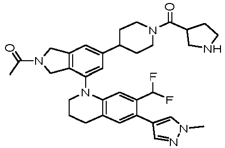 | M001251 | 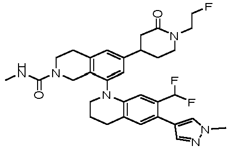 |
| M001081 | 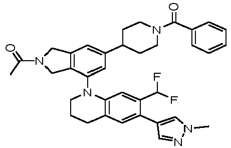 | M001252 | 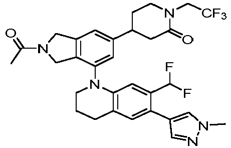 |
| M001082 | 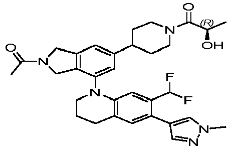 | M001253 | 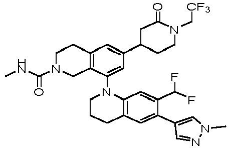 |
| M001083 | 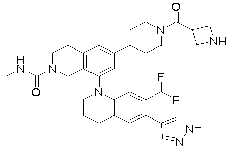 | M001254 | 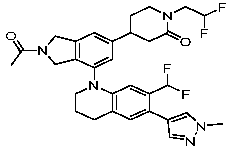 |
| M001084 | 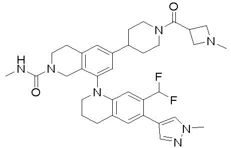 | M001255 | 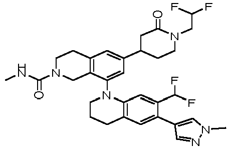 |
| M001085 | 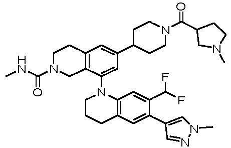 | M001256 | 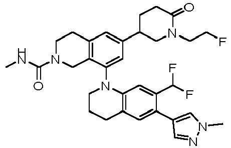 |
| M001086 | 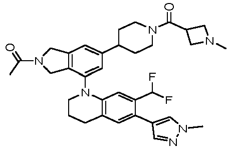 | M001257 | 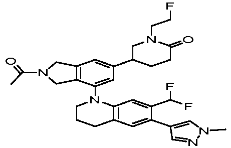 |
| M001087 | 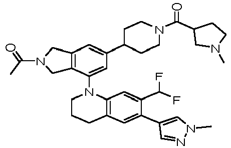 | M001258 | 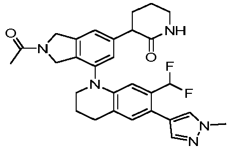 |
| M001088 | 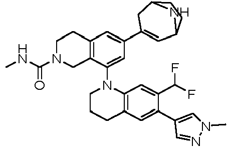 | M001260 | 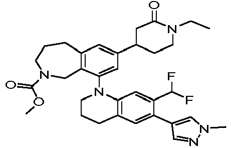 |
| M001089 | 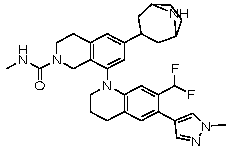 | M001261 | 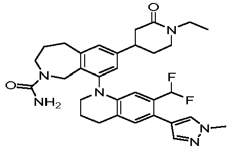 |
| M001090 | 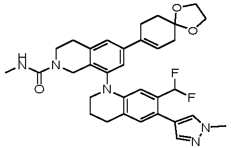 | M001262 | 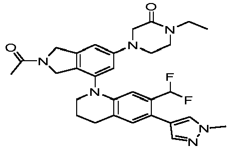 |
| M001092 | 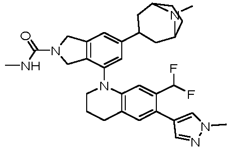 | M001263 | 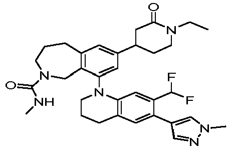 |
| M001093 | 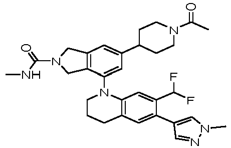 | M001264 | 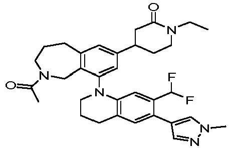 |
| M001094 | 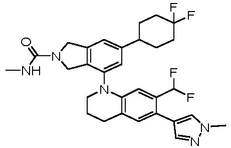 | M001268 | 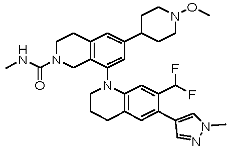 |
| M001095 | 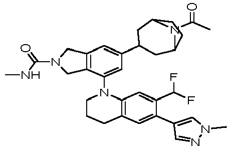 | M001269 | 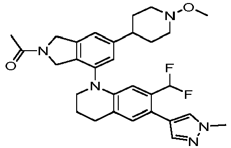 |
| M001096 | 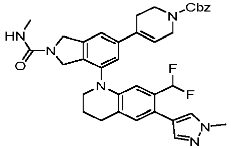 | M001270 | 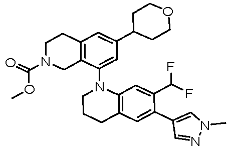 |
| M001097 | 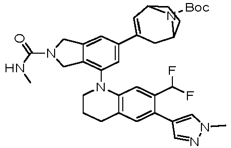 | M001271 | 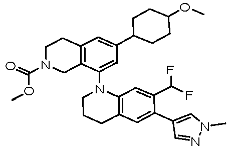 |
| M001098 | 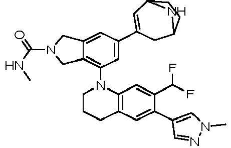 | M001272 | 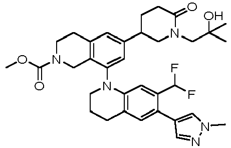 |
| M001099 | 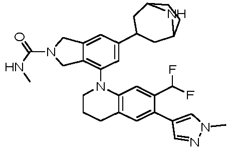 | M001274 | 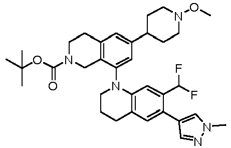 |
| M001101 | 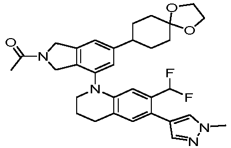 | M001275 | 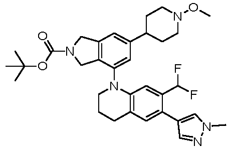 |
| M001102 | 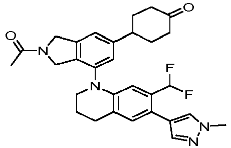 | M001276 | 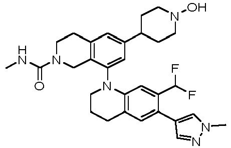 |
| M001103 | 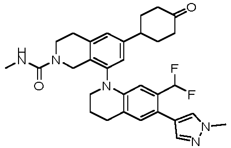 | M001277 | 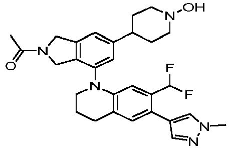 |
| M001116 | 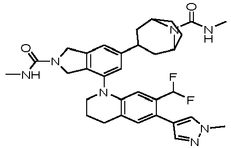 | M001280 | 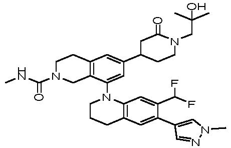 |
| M001117 | 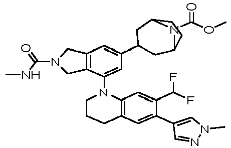 | M001281 | 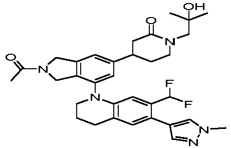 |
| M001118 | 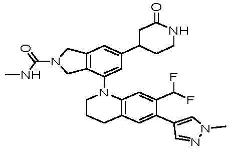 | M001287 | 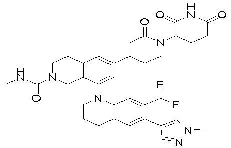 |
| M001119 | 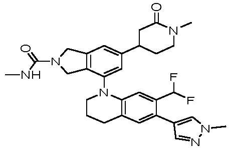 | M001288 | 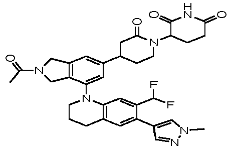 |
| M001120 | 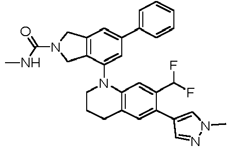 | M001289 | 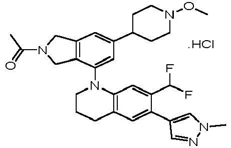 |
| M001121 | 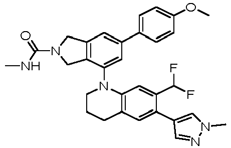 | M001296 | 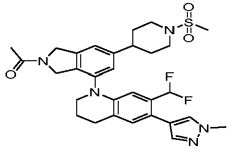 |
| M001122 | 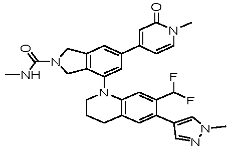 | M001297 | 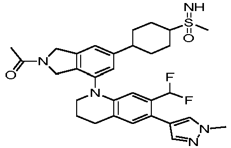 |
| M001123 | 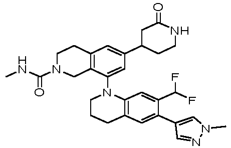 | M001298 | 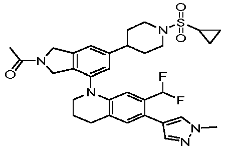 |
| M001124 | 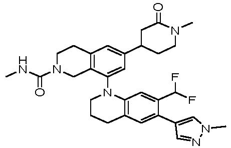 | M001299 | 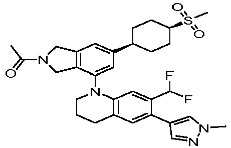 |
| M001125 | 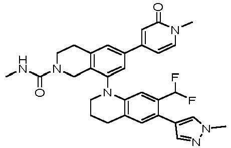 | M001300 | 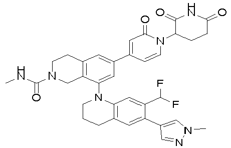 |
| M001126 | 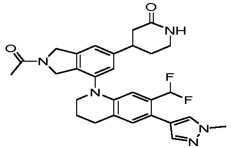 | M001301 | 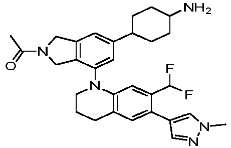 |
| M001127 | 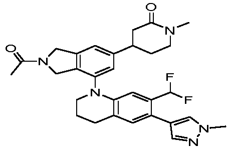 | M001302 | 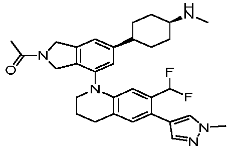 |
| M001128 | 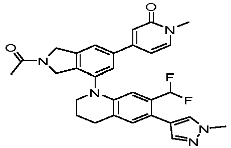 | M001303 | 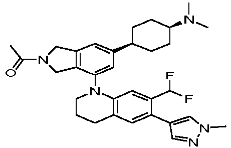 |
| M001129 | 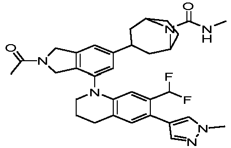 | M001304 | 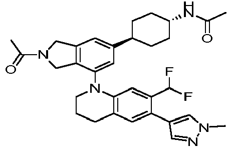 |
| M001132 | 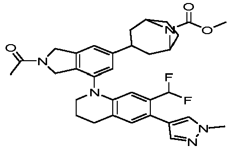 | M001305 | 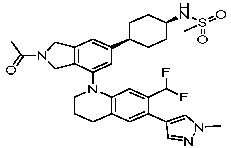 |
| M001137 | 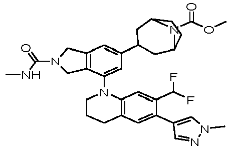 | M001306 | 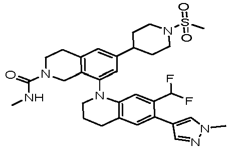 |
| M001142 | 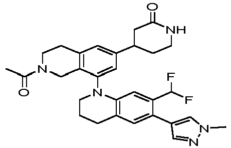 | M001307 | 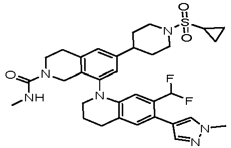 |
| M001143 | 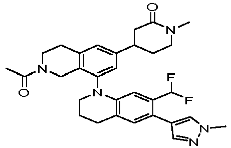 | M001308 | 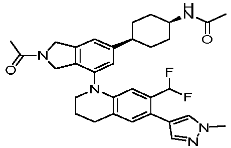 |
| M001144 | 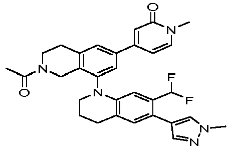 | M001309 | 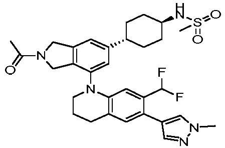 |
| M001153 | 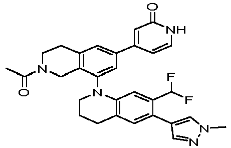 | M001310 | 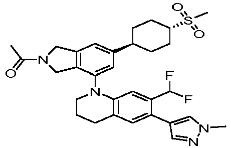 |
| M001154 | 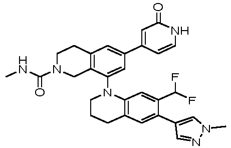 | M001311 | 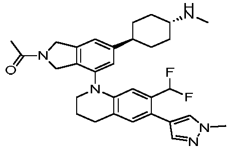 |
| M001155 | 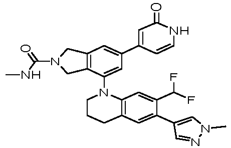 | M001312 | 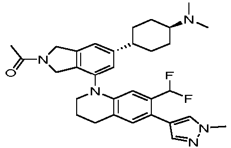 |
| M001156 | 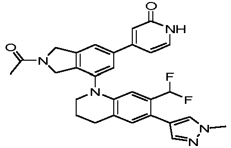 | M001313 | 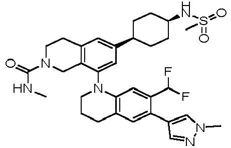 |
| M001161 | 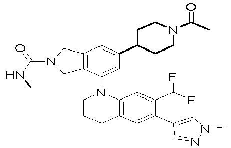 | M001314 | 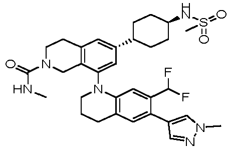 |
| M001162 | 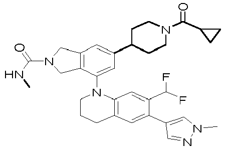 | M001315 | 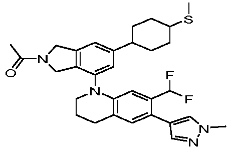 |
| M001163 | 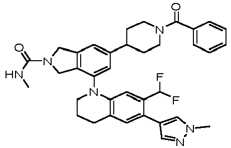 | M001316 | 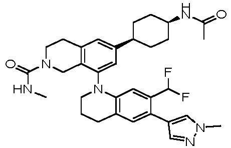 |
| M001164 | 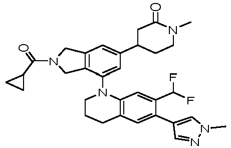 | M001317 | 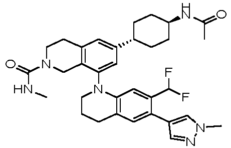 |
| M001166 | 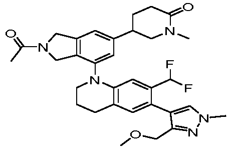 | M001318 | 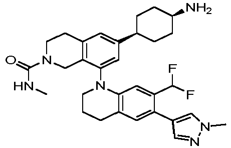 |
| M001167 | 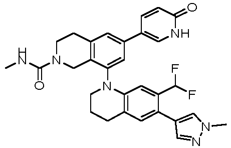 | M001319 | 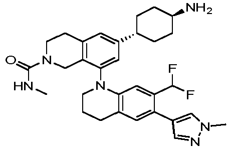 |
| M001168 | 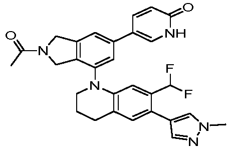 | M001320 | 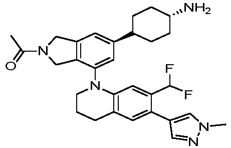 |
| M001169 | 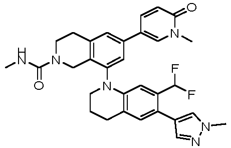 | M001321 | 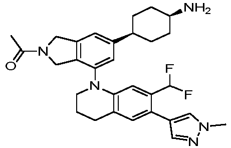 |
| M001170 | 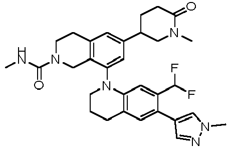 | M001322 | 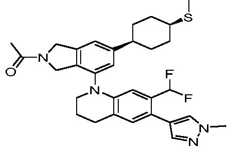 |
| M001171 | 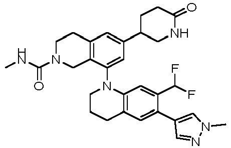 | M001323 | 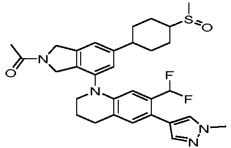 |
| M001172 | 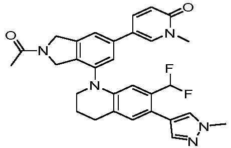 | M001324 | 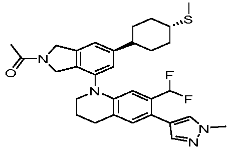 |
| M001173 | 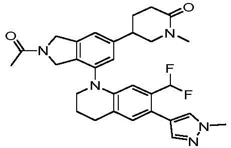 | M001327 | 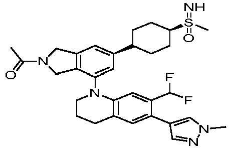 |
| M001174 | 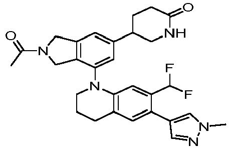 | M001328 | 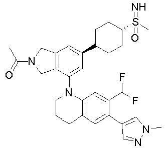 |
| M0011175 | 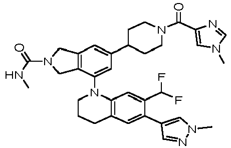 | M001329 | 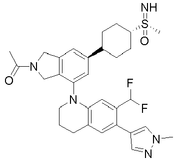 |
| M001176 | 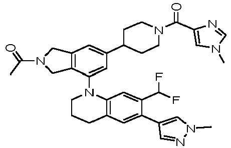 | M001330 | 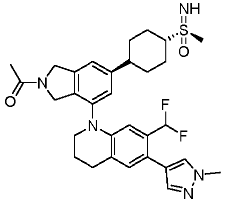 |
| M001178 | 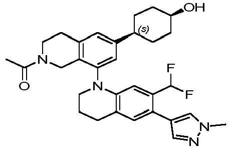 | M001331 | 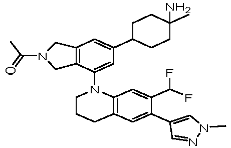 |
| M001179 | 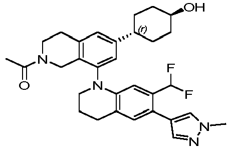 | M001332 | 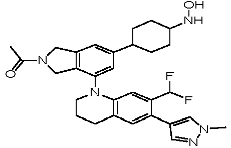 |
| M001180 | 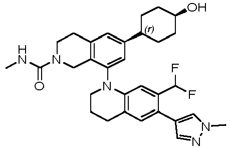 | M001333 | 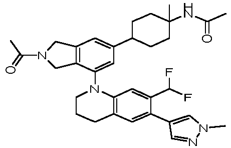 |
| M001181 | 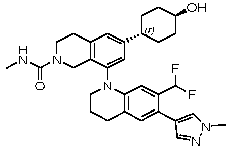 | M001334 | 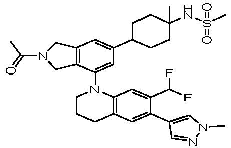 |
| M001184 | 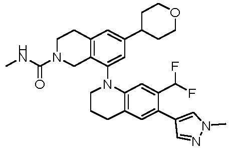 | M001335 | 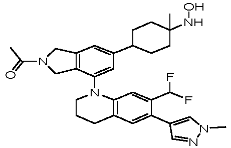 |
| M001186 | 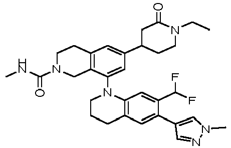 | M001336 | 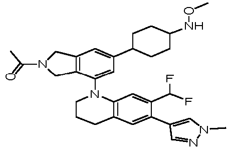 |
| M001187 | 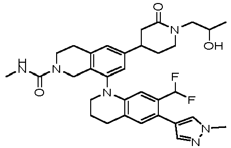 | M001337 | 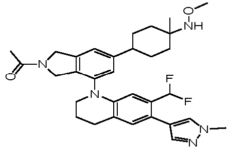 |
| M001188 | 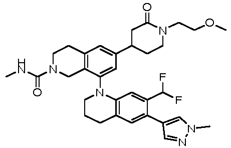 | M001340 | 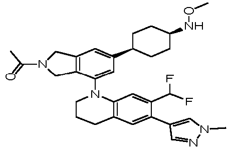 |
| M001189 | 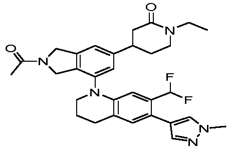 | M001341 | 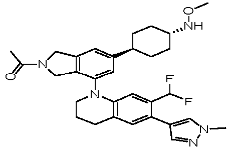 |
| M001190 | 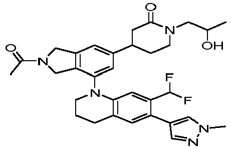 | | |

In yet another aspect, the present disclosure also provides a pharmaceutical composition comprising the compound represented by formula I (including formulas II, III, IV, IIa, and IIIa) and the racemate, the stereoisomer, the tautomer, the isotopic derivative, the nitrogen oxide, the solvate, the polymorph, the metabolite, the ester, the prodrug or the pharmaceutically acceptable salt thereof.

In yet another aspect, the present disclosure also provides use of the compound represented by formula I (including formulas II, III, IV, IIa, and IIIa) and the racemate, the stereoisomer, the tautomer, the isotopic derivative, the nitrogen oxide, the solvate, the polymorph, the metabolite, the ester, the prodrug or the pharmaceutically acceptable salt thereof and the pharmaceutical composition comprising the same for manufacturing a medicament for the prevention and/or treatment of a CBP and/or EP300-mediated disease or condition.

In yet another aspect, the present disclosure also provides a method for preventing and/or treating a CBP and/or EP300-mediated disease or condition comprising administering to a patient in need thereof a therapeutically effective amount of the compound represented by formula I (including formulas II, III, IV, IIa, and IIIa) and the racemate, the stereoisomer, the tautomer, the isotopic derivative, the nitrogen oxide, the solvate, the polymorph, the metabolite, the ester, the prodrug or the pharmaceutically acceptable salt thereof, and the pharmaceutical composition comprising the same.

The CBP and/or EP300-mediated disease or condition of the present disclosure is selected from cancer, cardiac diseases, metabolic diseases, inflammatory diseases, fibrotic diseases, and viral infections. The cancer includes, but is not limited to, prostate cancer, breast cancer, bladder cancer, lung cancer, melanoma, colorectal cancer, gastric cancer, ovarian cancer, cervical cancer, bladder cancer, laryngeal cancer, multiple myeloma, liver cancer, lymphoma, leukemia, etc. The prostate cancer may be, for example, castration-resistant prostate cancer (CRPC). The lung cancer may be, for example, non-small cell lung cancer or small cell lung cancer. The lymphoma may be selected from non-Hodgkin lymphoma, diffuse large B cell lymphoma, etc.

The compound of the present disclosure can be used in combination with other drugs or other therapies.

In some embodiments, the compound described above in the present disclosure is used in combination with radiotherapy.

In some embodiments, the present disclosure also provides the use of the compound in combination with immune modulators for the treatment of CBP and/or EP300 mediated diseases or related conditions (such as multiple myeloma).

In some embodiments, the immune modulator includes tumor necrosis factor; interferon α,β and γ; IL-2 and other cytokines; F42K and other cytokine analogues; or MIP-1, MIP-1β, MCP-1, RANTES, and other chemokines.

In some embodiments, the compound described above in the present disclosure is used in combination with a second therapeutic agent, wherein the second therapeutic agent may be selected from drugs conventionally used for the treatment of cancer, cardiac diseases, metabolic diseases, inflammatory diseases, fibrotic diseases and viral infections. In some embodiments, the categories of the second therapeutic agent may include androgen receptor antagonists such as enzalutamide, CYP17A1 inhibitors (17α-hydroxylase/C17,20 lyase) such as abiraterone, and cytotoxic chemotherapeutic agents such as docetaxel; the categories of therapeutic agents for treating lung cancer include cytotoxic chemotherapeutic agents such as cisplatin, carboplatin and docetaxel; the categories of therapeutic agents for treating bladder cancer include cytotoxic chemotherapeutic agents such as gemcitabine, cisplatin or immunotherapy such as the Bacillus Calmette-Guérin vaccine (BCG). The categories of the second therapeutic agent may also be selected from immune checkpoint inhibitors such as pembrolizumab, nivolumab, atezolizumab and ipilimumab, PARP (poly(ADP ribose)polymerase) inhibitors such as olaparib, and CDK4/6 (cyclin-dependent kinases 4 and 6) inhibitors. In some embodiments, the second therapeutic agent may be selected from drugs for use in combination with KRAS inhibitors and the like for the treatment of various tumors such as lung cancer, rectal cancer, pancreatic cancer, liver cancer, hematologic tumors, etc.

Accordingly, the present disclosure provides a composition for combination therapy comprising the compound represented by formula I (including formulas II, III, IV, IIa, and IIIa) and the racemate, the stereoisomer, the tautomer, the isotopic derivative, the nitrogen oxide, the solvate, the polymorph, the metabolite, the ester, the prodrug or the pharmaceutically acceptable salt thereof, and the second therapeutic agent.

The present disclosure also provides a method for preventing and/or treating a CBP and/or EP300-mediated disease or condition through combination therapy comprising administering to a patient in need thereof a therapeutically effective amount of the compound represented by formula I (including formulas II, III, IV, IIa, and IIIa) and the racemate, the stereoisomer, the tautomer, the isotopic derivative, the nitrogen oxide, the solvate, the polymorph, the metabolite, the ester, the prodrug or the pharmaceutically acceptable salt thereof, and the second therapeutic agent.

The pharmaceutically acceptable salts of the compounds of the present disclosure may be inorganic or organic salts. If these compounds have basic centers, they can form acid addition salts; if these compounds have acidic centers, they can form base addition salts; if these compounds contain both acidic centers (e.g., carboxyl) and basic centers (e.g., amino), they can also form internal salts.

The compounds of the present disclosure may be present in specific geometric or stereoisomeric forms, for example, cis and trans isomers, (-)- and (+)-enantiomers, (*R*)- and (*S*)-enantiomers, diastereoisomers, (*D*)-isomers, (*L*)-isomers, racemic mixtures and other mixtures, as well as an enantiomer- or diastereoisomer-enriched mixture, all of which are encompassed within the scope of the present disclosure. Substituents such as alkyl may have an additional asymmetric carbon atom. All these isomers and mixtures thereof are encompassed within the scope of the present disclosure.

The compounds and intermediates of the present disclosure may also be present in different tautomeric forms, and all such forms are included within the scope of the present disclosure. "Tautomers" refer to structural isomers of different energies that can interconvert via a low energy barrier. For example, proton tautomers (also referred to as proton transfer tautomers) include interconversion via proton migration, such as keto-enol isomerization, imine-enamine isomerization, and lactam-lactim isomerization. All tautomeric forms of all the compounds in the present disclosure fall within the scope of the present disclosure. The name of a compound named in a single way does not exclude any tautomers.

The present disclosure also includes some isotopically-labeled compounds of the present disclosure which are identical to the structures described herein except that one or more atoms are replaced with atoms having atomic masses or mass numbers different from the atomic masses or mass numbers usually found in nature. Examples of isotopes that can be incorporated into the compounds of the present disclosure include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively, and the like. All isotopic variations of the compounds of the present disclosure, whether radioactive or not, are encompassed within the scope of the present disclosure.

Unless otherwise stated, when a position is specifically designated as deuterium (D), that position is understood to be deuterium with the abundance that is at least 1000 times greater than the natural abundance of deuterium (which is 0.015%) (i.e., at least 10% deuterium incorporation). In the exemplary compounds, the deuterium with the abundance that is greater than the natural abundance of deuterium may be deuterium with at least 1000 times greater abundance, deuterium with at least 2000 times greater abundance, deuterium with at least 3000 times greater abundance, deuterium with at least 4000 times greater abundance, deuterium with at least 5000 times greater abundance, deuterium with at least 6000 times greater abundance, or deuterium with higher abundance. Each available hydrogen atom linked to carbon atoms may be independently replaced with a deuterium atom. Those skilled in the art are able to synthesize the compounds in deuterated form by referring to the relevant literature. Commercially available deuterated starting materials can be used in preparing the compounds in deuterated form, or they can be synthesized using conventional techniques using deuterated reagents including, but not limited to, deuterated boranes, tri-deuterated boranes in tetrahydrofuran, deuterated lithium aluminum hydrides, deuterated iodoethanes, deuterated iodomethanes, and the like.

The term "modulator" as used in the present disclosure refers to a compound that alters (i.e., increases or decreases) the activity of a target biomolecule, such as an enzyme. In general, the modulator will be a small molecule.

The term "modulation" refers to the ability of a compound to increase or decrease the functions and/or expression of a target (such as EP300 or CBP). Such functions may include transcriptional regulatory activity and/or binding. Modulation may be performed *in vitro* or *in vivo.* Modulation, as described herein, includes the direct or indirect inhibition, antagonism, partial antagonism, degradation, activation, agonism or partial agonism of a function or characteristic associated with EP300 or CBP, and/or the direct or indirect upregulation or downregulation of the expression of EP300 or CBP. In another embodiment, the modulation is direct. Inhibitors or antagonists or degraders are compounds that, e.g., bind to, partially or totally block stimulation, decrease, prevent, inhibit, delay activation, inactivate, desensitize, or downregulate signal transduction. Activators or agonists are compounds that, e.g., bind to, stimulate, increase, open, activate, facilitate, enhance activation, activate, sensitize or upregulate signal transduction. Thus, "EP300&CBP modulators" can further encompass "EP300&CBP inhibitors", "EP300&CBP degraders", and "EP300&CBP agonists".

The term "CBP and/or EP300-mediated disease or condition" as used herein refers to a disease or condition in which the biological function of EP300, CBP, or both EP300 and CBP affects the development and/or course of the disease or condition, and/or in which the modulation of EP300, CBP, or both EP300 and CBP alters the development, course and/or symptoms. An EP300 or CBP-mediated disease or condition includes a disease or condition for which EP300 inhibition, CBP inhibition, or both EP300 and CBP inhibition provides a therapeutic benefit, e.g. wherein treatment with EP300 or CBP inhibitors, including the compounds described herein, provides a therapeutic benefit to the subject suffering from or at risk of the disease or condition. An EP300 or CBP-mediated disease or condition is intended to include a cancer that harbors loss of function mutations in CBP or EP300, or a cancer in which the activation of EP300 or CBP is present. An EP300 or CBP-mediated disease or condition is also intended to include a cancer that expresses the androgen receptor.

As used herein, "therapeutically effective amount" refers to the amount of the active compound or drug that causes a biological or medical response that researchers, veterinarians, physicians, other clinicians, etc., are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition.(i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms). In the context of a drug or pharmacologically active agent, "therapeutically effective amount" refers to an amount of the drug or medicament that is sufficient to achieve the desired effects without producing toxic effects. The effective amount varies from person to person, depending on the age and general condition of the subject and also on the particular active substance. The suitable effective amount in a case can be determined by those skilled in the art through routine tests.

### Beneficial Effects

The present disclosure provides a novel compound having a structure represented by formula (I), which has good EP300/CBP modulatory activity (such as inhibitory activity) and pharmaceutical prospects.

### Definitions and Description

Unless otherwise stated, the terms used in the specification and claims have the following meanings.

The term "alkyl" refers to a saturated aliphatic hydrocarbon group which is a linear or branched group containing 1 to 12 carbon atoms (e.g., 1, 2, 3, 4, 5, 6, 7, 8, 9, 10, 11, and 12), preferably an alkyl group containing 1 to 6 carbon atoms (C₁₋₆ alkyl). Non-limiting examples of alkyl include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof and the like. Alkyl may be substituted or unsubstituted. The term "alkoxy" refers to -O-(alkyl), wherein the alkyl is as defined herein. Non-limiting examples of alkoxy include: methoxy, ethoxy, propoxy and butoxy. Alkoxy may be substituted or unsubstituted.

The term "cycloalkyl" refers to a saturated or partially unsaturated monocyclic or polycyclic hydrocarbon substituent, and the cycloalkyl ring preferably contains 3 to 12 or 3 to 8 (e.g., 3, 4, 5, 6, 7, and 8) carbon atoms, and more preferably contains 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl groups include spiro, fused and bridged cycloalkyl groups.

The term "spirocycloalkyl" refers to a polycyclic group in which a carbon atom (referred to as spiro atom) is shared between monocyclic rings in the system, which may contain one or more double bonds. The spirocycloalkyl is preferably 6- to 12-membered, and is more preferably 7- to 10-membered (e.g., 7-, 8-, 9- or 10-membered). According to the number of spiro atoms shared between rings, spirocycloalkyl groups are classified into monospirocycloalkyl groups, bispirocycloalkyl groups and polyspirocycloalkyl groups. The monospirocycloalkyl groups and bispirocycloalkyl groups are preferred. 3-membered/5-membered, 3-membered/6-membered, 4-membered/4-membered, 4-membered/5-membered, 4-membered/6-membered, 5-membered/5-membered or 5-membered/6-membered monospirocycloalkyl groups are more preferred. Non-limiting examples of spirocycloalkyl include:

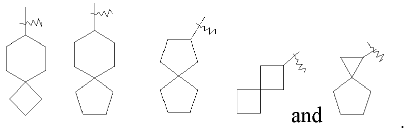

The term "fused cycloalkyl" refers to an all-carbon polycyclic group in which each ring shares a pair of adjacent carbon atoms with the other rings in the system, wherein one or more of the rings may contain one or more double bonds. The fused cycloalkyl is preferably 6- to 12-membered, and is more preferably 7- to 10-membered (e.g., 7-, 8-, 9- or 10-membered). According to the number of constituent rings, fused cycloalkyl groups can be classified into bicyclic, tricyclic, tetracyclic and polycyclic fused cycloalkyl groups. The bicyclic and tricyclic fused cycloalkyl groups are preferred, and 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered bicycloalkyl groups are more preferred. Non-limiting examples of fused cycloalkyl include:

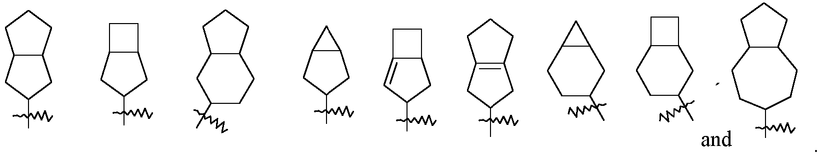

The term "bridged cycloalkyl" refers to an all-carbon polycyclic group in which any two rings share two carbon atoms that are not directly linked to each other, which may contain one or more double bonds. The bridged cycloalkyl is preferably 6- to 12-membered, and is more preferably 7- to 10-membered (e.g., 7-, 8-, 9- or 10-membered). According to the number of constituent rings, bridged cycloalkyl groups may be classified into bicyclic, tricyclic, tetracyclic and polycyclic bridged cycloalkyl groups. The bicyclic, tricyclic and tetracyclic bridged cycloalkyl groups are preferred, and the bicyclic and tricyclic bridged cycloalkyl groups are more preferred. Non-limiting examples of bridged cycloalkyl include:

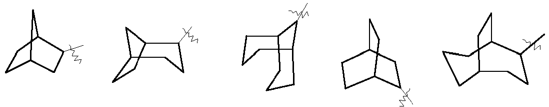

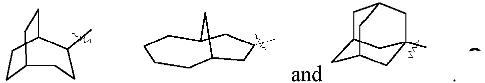

The cycloalkyl rings include those in which the cycloalkyl as described herein (including monocyclic, spiro, fused and bridged ones) is fused to an aryl, heteroaryl or heterocycloalkyl ring, and the ring linked to a parent structure is the cycloalkyl; non-limiting examples include 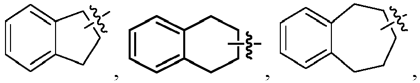 etc., and 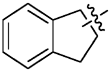 and 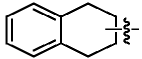 are preferred. The cycloalkyl may be substituted or unsubstituted.

The term "heterocyclyl" refers to a saturated or partially unsaturated monocyclic or polycyclic substituent containing 3 to 14 ring atoms, one or more of which is heteroatoms selected from nitrogen, oxygen and sulfur; the sulfur atoms may be optionally oxidized (that is, to form a sulfoxide or sulfone), but the ring moiety -O-O-, -O-S- or -S-S- is not included, and the remaining ring atoms are carbon atoms. Preferably, the heterocyclyl contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably, the heterocyclyl contains 3 to 8 (e.g., 3, 4, 5, 6, 7 and 8) ring atoms, of which 1 to 3 (e.g., 1, 2 and 3) are heteroatoms; more preferably, the heterocyclyl contains 3 to 6 ring atoms, of which 1 to 3 are heteroatoms; most preferably, the heterocyclyl contains 5 or 6 ring atoms, of which 1 to 3 are heteroatoms. Non-limiting examples of monocyclic heterocyclyl include pyrrolidinyl, tetrahydropyranyl, 1,2,3,6-tetrahydropyridinyl, piperidyl, piperazinyl, morpholinyl, thiomorpholinyl, homopiperazinyl, etc. Polycyclic heterocyclyl includes spiroheterocyclyl, fused heterocyclyl, and bridged heterocyclyl.

The term "spiroheterocyclyl" refers to a polycyclic heterocyclic group in which an atom (referred to as spiro atom) is shared between monocyclic rings in the system, of which one or more ring atoms are heteroatoms selected from nitrogen, oxygen and sulfur; the sulfur atoms may be optionally oxidized (that is, to form a sulfoxide or sulfone), and the remaining ring atoms are carbon atoms. The spiroheterocyclyl may contain one or more double bonds. The spiroheterocyclyl is preferably 6- to 14-membered, and is more preferably 7- to 10-membered (e.g., 7-, 8-, 9- or 10-membered). According to the number of spiro atoms shared between rings, spiroheterocyclyl groups are classified into monospiroheterocyclyl groups, bispiroheterocyclyl groups and polyspiroheterocyclyl groups. The monospiroheterocyclyl groups and bispiroheterocyclyl groups are preferred. 3-membered/5-membered, 3-membered/6-membered, 4-membered/4-membered, 4-membered/5-membered, 4-membered/6-membered, 5-membered/5-membered or 5-membered/6-membered monospiroheterocyclyl groups are more preferred. Non-limiting examples of spiroheterocyclyl include:

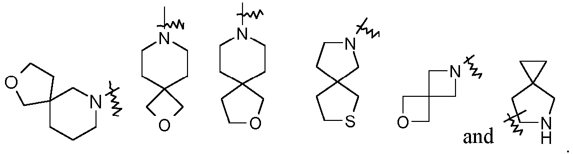

The term "fused heterocyclyl" refers to a polycyclic heterocyclic group in which a pair of adjacent atoms are shared between each ring and another ring in the system, of which one or more rings may contain one or more double bonds and one or more ring atoms are heteroatoms selected from nitrogen, oxygen and sulfur; the sulfur atoms may be optionally oxidized (that is, to form a sulfoxide or sulfone), and the remaining ring atoms are carbon atoms. The fused heterocyclyl is preferably 6- to 14-membered, and is more preferably 7- to 10-membered (e.g., 7-, 8-, 9- or 10-membered). According to the number of constituent rings, fused heterocyclyl groups can be classified into bicyclic, tricyclic, tetracyclic and polycyclic fused heterocyclyl groups. The bicyclic and tricyclic fused heterocyclyl groups are preferred, and 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered bicyclic fused heterocyclyl groups are more preferred. Non-limiting examples of fused heterocyclyl include:

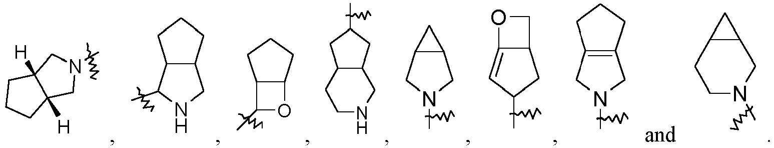

The term "bridged heterocyclyl" refers to a polycyclic heterocyclic group in which any two rings share two atoms that are not directly linked to each other, which may contain one or more double bonds, and one or more ring atoms are heteroatoms selected from nitrogen, oxygen and sulfur; the sulfur atoms may be optionally oxidized (that is, to form a sulfoxide or sulfone), and the remaining ring atoms are carbon atoms. The bridged heterocyclyl is preferably 6- to 14-membered, and is more preferably 7- to 10-membered (e.g., 7-, 8-, 9- or 10-membered). According to the number of constituent rings, bridged heterocyclyl groups may be classified into bicyclic, tricyclic, tetracyclic and polycyclic bridged heterocyclyl groups. The bicyclic, tricyclic and tetracyclic bridged heterocyclyl groups are preferred, and the bicyclic and tricyclic bridged heterocyclyl groups are more preferred. Non-limiting examples of bridged heterocyclyl groups include:

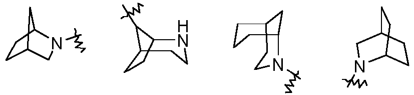

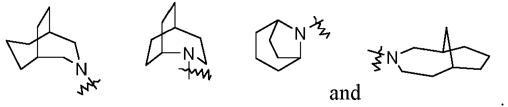

The heterocyclyl rings include those in which the heterocyclyl as described herein (including monocyclic, spiro, fused and bridged ones) is fused to an aryl, heteroaryl or cycloalkyl ring, and the ring linked to a parent structure is the heterocyclyl; its non-limiting examples include: 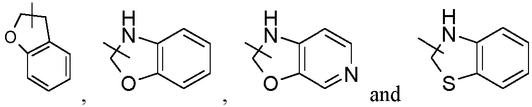 and the like. The heterocyclyl may be substituted or unsubstituted.

The term "aryl" refers to a 6- to 14-membered all-carbon monocyclic or fused polycyclic (fused polycyclic rings are rings that share a pair of adjacent carbon atoms) group with a conjugated π-electron system, which is preferably 6-to 10-membered, e.g., phenyl and naphthyl. The aryl rings include those in which the aryl ring as described herein is fused to a heteroaryl, heterocyclyl or cycloalkyl ring, and the ring linked to a parent structure is the aryl ring; its non-limiting examples include: 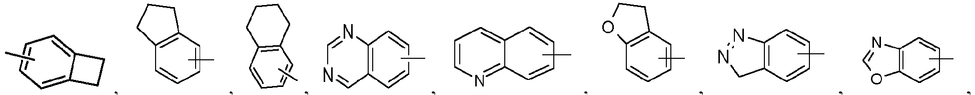 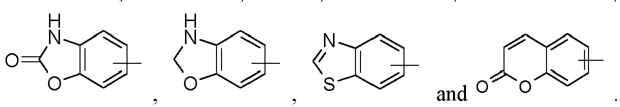 The aryl may be substituted or unsubstituted. The term "heteroaryl" refers to a heteroaromatic system containing 1 to 4 (e.g., 1, 2, 3 and 4) heteroatoms and 5 to 14 ring atoms, wherein the heteroatoms are selected from oxygen, sulfur and nitrogen. The heteroaryl is preferably 5- to 10-membered (e.g., 5-, 6-, 7-, 8-, 9- or 10-membered), and is more preferably 5- or 6-membered, e.g., furanyl, thienyl, pyridinyl, pyrrolyl, N-alkylpyrrolyl, pyrimidinyl, pyrazinyl, pyridazinyl, imidazolyl, pyrazolyl, triazolyl, tetrazolyl, etc. The heteroaryl ring includes those in which the heteroaryl as described herein is fused to an aryl, heterocyclyl or cycloalkyl ring, and the ring linked to a parent structure is the heteroaryl ring; its non-limiting examples include: 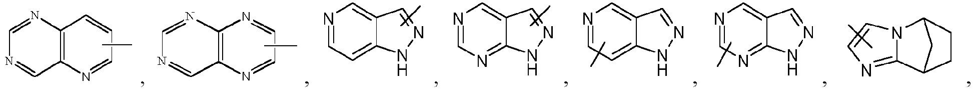 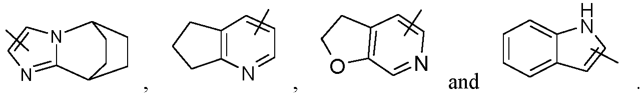 The heteroaryl may be substituted or unsubstituted.

It will be understood by those skilled in the art that the cycloalkyl, heterocyclyl, aryl, heteroaryl, etc. include residues derived from removing one hydrogen atom from a parent ring atom, or residues derived from removing two hydrogen atoms from the same ring atom or two different ring atoms of the parent structure, i.e., "divalent cycloalkyl", "divalent heterocyclyl", "arylene", and "heteroarylene".

The term "halogen" refers to F, Cl, Br or I.

"Optional" or "optionally" means that the subsequently described event or circumstance may, but not necessarily, occur, and the description includes instances where the event or circumstance occurs or does not occur. For example, "heterocycloalkyl optionally substituted with alkyl" means that the alkyl may, but not necessarily, be present, and the description includes the case where the heterocycloalkyl is substituted with the alkyl and the case where the heterocycloalkyl is not substituted with the alkyl.

The term "more" includes 3, 4, 5 and more.

### DETAILED DESCRIPTION

The technical solutions of the present disclosure will be further explained in detail by the description of the following specific examples. It should be understood that the following examples are merely exemplary illustrations and explanations of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared using known methods.

### Example 1

### Synthesis of 7-(difluoromethyl)-6-(1-methyl-1H-pyrazol-4-yl)-1,2,3,4-tetrahydroquinoline:

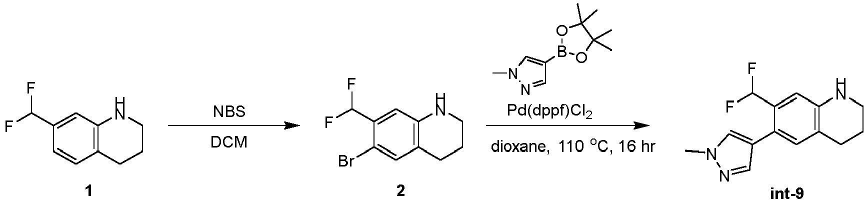

### Synthesis of 6-bromo-7-(difluoromethyl)-1,2,3,4-tetrahydroquinoline:

To a solution of 7-(difluoromethyl)-1,2,3,4-tetrahydroquinoline (18 g, 98.2 mmol, 1 eq) in DCM (180 mL) at 0 °C was added NBS (17.5 g, 98.2 mmol, 1 eq). The mixture was stirred at 25 °C for 16 h. The reaction mixture was quenched by adding ice H₂O (200 mL), extracted with DCM (150 mL × 2), dried over Na₂SO₄, filtered, and concentrated under reduced pressure. The residue was purified by column chromatography (petroleum ether/ethyl acetate = 0 to 10%) to give compound 2 (16 g, 61.1 mmol, 62.1% yield) as a yellow solid.

MS (ESI): C10H11F2N; found 262.6 (M+H)⁺.

¹H NMR (400MHz, CDCl₃) δ = 7.18 (s, 1H), 7.04 (s, 1H), 6.63 (s, 1H), 4.09 - 3.82 (m, 1H), 3.28 - 3.18 (m, 2H), 2.66 (br t, J=6.3 Hz, 2H), 1.88 - 1.79 (m, 2H)

### Synthesis of 7-(difluoromethyl)-6-(1-methyl-1H-pyrazol-4-yl)-1,2,3,4-tetrahydroquinoline:

A mixture of 6-bromo-7-(difluoromethyl)-1,2,3,4-tetrahydroquinoline (11 g, 41.9 mmol, 1 eq), 1-methyl-4-(4,4,5,5-tetramethyl-1,3,2-dioxabenzofuran-2-yl)pyrazole (8.7 g, 41-9 mmol, 1 eq) and cyclopentyl(diphenyl)phosphine, palladium dichloride and a mixture of iron (3.0 g, 4.2 mmol, 0.1 eq), K₂CO₃ (11.6 g, 83.9 mmol, 2 eq) in dioxane (80 mL) and H₂O (20 mL) were stirred under N₂ at 110 °C for 12 h. The reaction mixture was concentrated underreduced pressure. The residue was purified by column chromatography (petroleum ether/ethyl acetate = 0 to 40%) to give int-9 (10 g, 37.9 mmol, 90.5% yield) as a brown solid.

MS (ESI): C14H15F2N3; found 263.9 (M+H)⁺.

¹H NMR (400MHz, DMSO-d6) δ = 7.68 (s, 1H), 7.43 (s, 1H), 6.90 (s, 1H), 6.73 (s, 1H), 6.01 (s, 1H), 3.84 (s, 3H), 3.24 - 3.14 (m, 2H), 2.68 (br t, J=6.0 Hz, 2H), 1.79 (quin, J=5.9 Hz, 2H)

### Example 2

### Synthesis of 1-(6-chloro-4-(7-(difluoromethyl)-6-(1-methyl-1H-pyrazol-4-yl)-3,4-dihydroquinolin-1(2H)-yl)isoindol-2-yl)ethan-1-one:

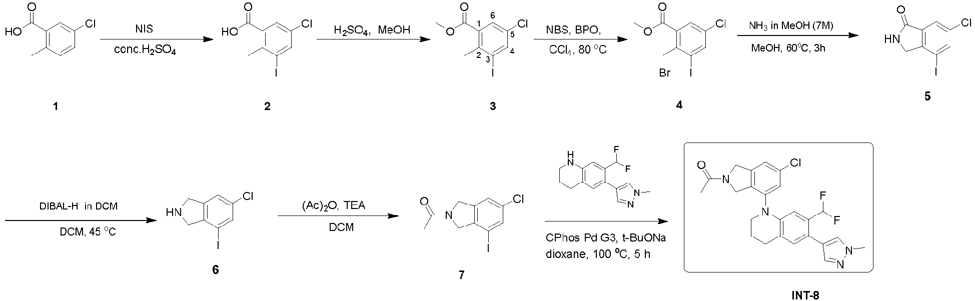

### Synthesis of 5-chloro-3-iodo-2-methylbenzoic acid:

To a solution of compound 1 (50 g, 293.1 mmol, 1 eq) in concentrated sulfuric acid (350 mL) at 0 °C was added dropwise a solution of NIS (76 g, 331 mmol, 98% purity, 1.13 eq) in sulfuric acid (30 mL). The mixture was stirred at 25 °C for 16 h. The reaction mixture was poured into ice-cold water (500 mL), and the resulting mixture was stirred for 30 min and then filtered to give compound 2 (68.5 g, 231 mmol, 78.8% yield) as a brown solid.

MS (ESI): C₈H₆ClIO₂; found 294.8 (M-H)⁺.

### Synthesis of methyl 5-chloro-3-iodo-2-methylbenzoate:

To a solution of compound 2 (68.5 g, 231 mmol, 1 eq) in MeOH (350 mL) was added concentrated H₂SO₄ (12 mL). The mixture was stirred at 60 °C for 16 h. The reaction mixture was concentrated under reduced pressure. The residue was diluted with DCM (300 mL), and the combined organic phases were washed with aqueous NaHCO₃ (200 mL) and brine (200 mL), dried over anhydrous Na₂SO₄, filtered and concentrated under vacuum to give compound 3 (58.5 g, 188.4 mmol, 81.5% yield) as a yellow oil.

¹H NMR (400MHz, CHLOROFORM-d) δ = 7.98 (d, J=2.0 Hz, 1H), 7.76 (d, J=1.8 Hz, 1H), 3.92 (s, 4H), 2.64 (s, 3H).

### Synthesis of methyl 2-(bromomethyl)-5-chloro-3-iodobenzoate:

To a solution of methyl 5-chloro-3-iodo-2-methylbenzoate (55 g, 177.12 mmol, 1 eq) in CCl₄ (350 mL) were added NBS (33 g, 185.41 mmol, 1.05 eq) and BPO (4.5 g, 18.58 mmol, 1e-1 eq). The mixture was stirred at 80 °C for 17 h. The reaction mixture was filtered and concentrated under reduced pressure. The residue was purified by column chromatography (petroleum ether/ethyl acetate 100/0 to 10/1) to give compound 4 (40 g, 103.1 mmol, 57% yield) as a colorless oil.

¹H NMR (400MHz, CHLOROFORM-d) δ = 7.97 (d, J=2.2 Hz, 1H), 7.84 (d, J=2.2 Hz, 1H), 5.01 (s, 2H), 3.89 (s, 3H).

### Synthesis of methyl 2-(bromomethyl)-5-chloro-3-iodobenzoate:

Compound 4 (40 g, 102.5 mmol, 1 eq) was added to a solution of NH₃/MeOH (150 mL). The mixture was stirred at 60 °C for 2.5 h. The reaction mixture was concentrated under reduced pressure to remove MeOH. The residue was washed 3 times with EA (150 mL) to give compound 5 (20.6 g, 70.4 mmol, 68.5% yield) as a white solid.

MS (ESI): C₈H₆ClINO; found 295.8 (M+H)⁺.

¹H NMR (400MHz, DMSO-d6) δ = 8.96 (br s, 1H), 8.09 (s, 1H), 7.71 (s, 1H), 4.18 (s, 2H)

### Synthesis of 6-chloro-4-iodoisoindole:

To a solution of compound 5 (17 g, 57.92 mmol, 1 eq) in DCM (150 mL) at 0 °C was added dropwise DIBALH (1 M, 202.73 mL, 3.5 eq) under nitrogen. The mixture was stirred at 45 °C for 54 h. The reaction mixture was quenched by adding 100 mL of 15% NaOH at 0 °C, and then filtered and washed with EtOAc. The filtrate was washed with brine (200 mL), dried over Na₂SO₄, filtered and concentrated under reduced pressure. The residue was purified by silica gel flash chromatography (silica gel flash column, 0-10% MeOH/DCM as eluent) to give compound 6 (5.5 g, 19.6 mmol, 33.9%) as a brown solid.

¹H NMR (400MHz, DMSO-d6) δ = 7.64 (s, 1H), 7.37 (s, 1H), 4.20 (s, 2H), 3.93 (s, 2H).

### Synthesis of 1-(6-chloro-4-iodoisoindol-2-yl)ethan-1-one:

To a solution of compound 6 (4.5 g, 16.1 mmol, 1 eq) and TEA (4.1 g, 40.3 mmol, 5.6 mL, 2.5 eq) in DCM (80 mL) at 0 °C was added dropwise acetic anhydride (2.46 g, 24.1 mmol, 2.26 mL, 1.5 eq) under N₂. The mixture was stirred at 25 °C for 16 h. The residue was diluted with H₂O (100 mL) and extracted with DCM (40 mL × 2). The combined organic layers were washed with NaCl (40 mL × 2), dried over Na₂SO₄, filtered, and concentrated under reduced pressure. The residue was purified by silica gel flash chromatography (0-100% ethyl acetate/petroleum ether gradient eluent) to give compound 7 (3.25 g, 8.2 mmol, 51% yield, 70% purity) as a brown solid.

MS (ESI): C₁₀H₉ClINO; found 322.3 (M+H)⁺.

### Synthesis of 1-(6-chloro-4-(7-(difluoromethyl)-6-(1-methyl-1H-pyrazol-4-yl)-3,4-dihydroquinolin-1(2H)-yl)isoindol-2-yl)ethan-1-one:

Compound 7 (1.15 g, 3.58 mmol, 1 eq), 7-(difluoromethyl)-6-(1-methyl-1H-pyrazol-4-yl)-1,2,3,4-tetrahydroquinoline (941.6 mg, 3.58 mmol, 1 eq), NaOBu-t (859.2 mg, 8.94 mmol, 2.5 eq) and Cphos-Pd G3 (288.4 mg, 357.6 µmol, 0.1 eq) were placed in dioxane (10 mL) in a microwave tube. The sealed tube was heated under microwaves at 110 °C for 50 min. The reaction mixture was diluted with H₂O (20 mL) and extracted with EtOAc (20 mL × 3). The combined organic layers were washed with brine (50 mL), dried over Na₂SO₄, filtered, and concentrated under reduced pressure. The residue was purified by silica gel flash chromatography to give compound 8 (700 mg, 1.53 mmol, 42.8% yield) as a brown solid.

MS (ESI): C₂₄H₂₃ClF₂N₄O; found 457.0 (M+H)⁺.

5,6-Fused cyclic (isoindole) compounds can be synthesized by following this method.

### Example 3

### Synthesis of M001014 (8-(7-(difluoromethyl)-6-(1-methyl-1H-pyrazol-4-yl)-3,4-dihydroquinolin-1(2H)-yl)-N-methyl-6-morpholine-3,4-dihydroisoquinoline-2(1H)-carboxamide):

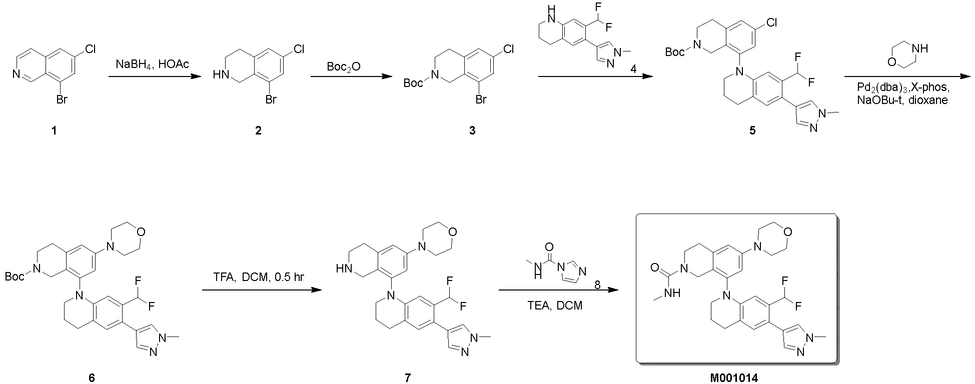

### Synthesis of 8-bromo-6-chloro-1,2,3,4-tetrahydroisoquinoline:

A solution of 8-bromo-6-chloroisoquinoline (8.6 g, 35.46 mmol) in AcOH (80 mL) was cooled to 0 °C, and then NaBH₄ (2.68 g, 70.93 mmol) was added in batches. The mixture was stirred at 25 °C for 0.5 h. The mixture was poured into H₂O to quench the reaction, and then the pH was adjusted to 8 with NH₄Cl. The mixture was extracted with EtOAc (200 mL) to give compound 2 (8.48 g, 34.40 mmol, 97.0% yield, crude) as a yellow solid, and the product was confirmed by HNMR.

¹H NMR (400 MHz, CD₃Cl) δ 7.42 (s, 1 H), 7.09 (s, 1 H), 4.00 (s, 2H), 3.15-3.18(m, 2H), 2.85-2.88 (m, 2H).

### Synthesis of tert-butyl 8-bromo-6-chloro-3,4-dihydroisoquinoline-2(1H)-carboxylate:

To a solution of 8-bromo-6-chloro-1,2,3,4-tetrahydroisoquinoline (8.48 g, 34.40 mmol) in DCM (100 mL) were added TEA (6.96 g, 68.79 mmol) and Boc₂O (11.26 g, 51.60 mmol). The mixture was stirred at 25 °C for 12 h. The mixture was washed with brine (50 mL × 2) to give a crude product. The crude product was then purified by silica gel column chromatography (with petroleum ether/ethyl acetate from 100% to 70%) to give compound 3 (6.7 g, 19.33 mmol, 56.2% yield) as a yellow oil, and the product was confirmed by nuclear magnetic resonance.

¹H NMR (400 MHz, CD₃Cl) δ 7.42 (s, 1 H), 7.10 (s, 1 H), 4.49 (s, 2H), 3.61-3.63(m, 2H), 2.80-2.82 (m, 2H), 1.50 (s, 9H).

### Synthesis of tert-butyl 6-chloro-8-(7-(difluoromethyl)-6-(1-methyl-1H-pyrazol-4-yl)-3,4-dihydroquinolin-1(2H)-yl)-3,4-dihydroxyisoquinoline-2(1H)-carboxylate:

A mixture of tert-butyl 8-bromo-6-chloro-3,4-dihydroisoquinoline-2(1H)-carboxylate (29.3 g, 84.52 mmol, 29.3 mg, 84.52 mmol), 7-(difluoromethyl)-6-(1-methyl-1H-pyrazol-4-yl)-1.2,3.3,4-tetrahydroquinoline (13.35 g, 50.71 mmol), t-BuONa (24.37 g, 253.57 mmol), CPHOS PD G3 (6.82 g, 8.45 mmol) and dioxane (300 mL) was degassed and purged with N₂ three times and then stirred under N₂ at 100 °C for 2 h. The mixture was extracted with EtOAc (200 mL) to give a crude product. The crude product was then purified by silica gel column chromatography (with petroleum ether/ethyl acetate from 100% to 70%) to give compound 5 (22.6 g, 42.72 mmol, 50.5%) as a yellow oil.

LCMS (ESI+): m/z 529.3 (M+H)⁺, Rt: 2.38 min.

### Synthesis of tert-butyl 8-(7-(difluoromethyl)-6-(1-methyl-1H-pyrazol-4-yl)-3,4-dihydroquinolin-1(2H)-yl)-6-morpholine-3,4-dihydroisoquinoline-2(1H)-carboxylate:

tert-Butyl 6-chloro-8-(7-(difluoromethyl)-6-(1-methyl-1H-pyrazol-4-yl)-3,4-dihydroquinolin-1(2H)-yl)-3,4-dihydroxyisoquinoline-2(1H)-carboxylate (200 mg, 378.06 µmol), morpholine (164.68 mg, 1.89 mmol), XPhos (36.05 mg, 75.61 µmol), Pd₂(dba)3 (34.62 mg, 37.81 µmol) and t-BuONa (72.67 mg, 756.12 µmol) were dissolved in dioxane (5 mL), and the mixture was degassed and purged with N₂ three times and then stirred under N₂ at 100 °C for 2 h. The mixture was extracted with EtOAc (10 mL) and washed with brine (5 mL × 2). The residue was purified by silica gel column chromatography (with petroleum ether/ethyl acetate from 100% to 35%) to give compound 6 (70 mg, 118.34 µmol, 31.3%) as a yellow gum, and the product was examined by LCMS.

LCMS (ESI+): m/z 580.4 (M+H)⁺, Rt: 1.77 min.

### Synthesis of 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)morpholine:

To a solution of tert-butyl 8-(7-(difluoromethyl)-6-(1-methyl-1H-pyrazol-4-yl)-3,4-dihydroquinolin-1(2H)-yl)-6-morpholine-3,4-dihydroisoquinoline-2(1H)-carboxylate (60 mg, 103.51 µmol) in DCM (5 mL) was added TFA (27.01 mmol, 2 mL). The mixture was stirred at 25 °C for 0.5 h. The mixture was concentrated to give compound 7 (60 mg, 101.08 µmol, 97.7% yield, crude TFA salt) as a yellow solid.

LCMS (ESI+): m/z 480.2 (M+H)⁺, Rt: 1.12 min.

### Synthesis of 8-(7-(difluoromethyl)-6-(1-methyl-1H-pyrazol-4-yl)-3,4-dihydroquinolin-1(2H)-yl)-N-methyl-6-morpholine-3,4-dihydroisoquinoline-2(1H)-carboxamide:

To a solution of 4-(8-(7-(7-(difluoromethyl)-6-(1-methyl-1H-pyrazol-4-yl)-3,4-dihydroquinolin-1(2H)-1,2,3,4-tetrahydroisoquinolin-6-yl)morpholine (60 mg, 101.08 µmol, TFA) in DCM (2 mL) were added TEA (30.68 mg, 303.24 µmol) and methylcarbamate (11.34 mg, 121.30 µmol), and the mixture was stirred at 55 °C for 16 h. The mixture was extracted with DCM (10 mL) and washed with brine (5 mL). The organic layer was dried over Na₂SO₄, filtered, and concentrated to give a crude product, and the crude product was then purified by prep-HPLC (FA) to give M001014 (28.12 mg, 52.40 µmol, 51.8% yield) as a gray solid.

LCMS (ESI+): m/z 559.4 (M+Na)⁺, Rt: 1.75 min.

¹H NMR (400MHz, CD₃Cl) δ 7.53 (s, 1H), 7.40 (s, 1H), 7.05 (s, 1H), 6.67 (d, J = 3.9Hz, 2H), 6.62 - 6.24 (m, 2H), 4.46 (d, J = 15.9 Hz, 1H), 4.36 - 4.27 (m, 1H), 4.16 -4.06 (m, 1H), 3.95 (s, 3H), 3.85 (t, J = 4.8 Hz, 4H), 3.78 - 3.68 (m, 1H), 3.63 - 3.43(m, 3H), 3.20 - 3.09 (m, 4H), 3.01 - 2.83 (m, 4H), 2.79 (d, J = 4.6 Hz, 3H), 2.24 -2.03 (m, 2H). 6,6-Fused cyclic (dihydroisoquinoline) compounds can be synthesized by following this method.

### Example 4

### Synthesis of 8-(7-(difluoromethyl)-6-(1-methyl-1H-pyrazol-4-yl)-3,4-dihydroquinolin-1(2H)-yl)-N-methyl-6-(1-methyl-2-oxopiperidin-4-yl)-3,4-dihydroisoquinoline-2(1H)-carboxamide (M001124):

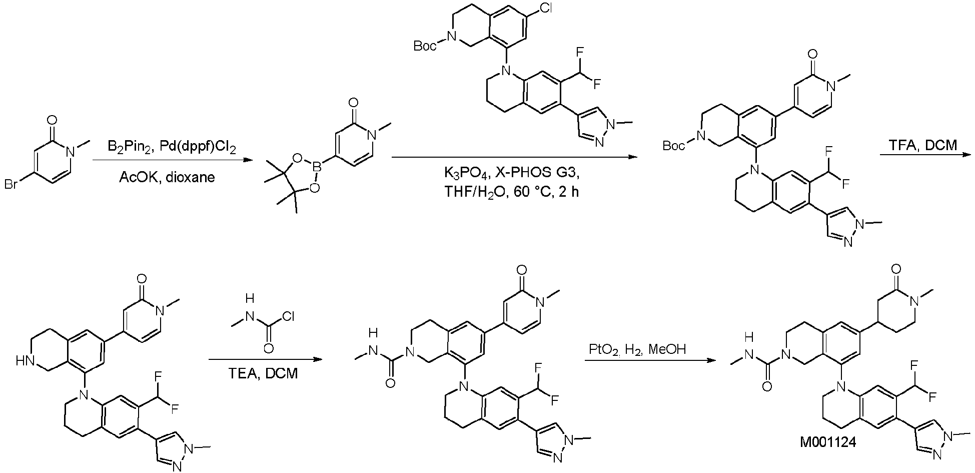

### Synthesis of 1-methyl-4-(4,4,5,5-tetramethyl-1,3,2-dioxaborane-2-yl) pyridin-2(1H)-one:

To a solution of 4-bromo-1-methylpyridin-2-one (500 mg, 2.66 mmol) in 1,4-dioxane (10.0 mL) were added bis(pinacolato)diboron (1350 mg, 5.31 mmol), AcOK (783 mg, 7.97 mmol) and Pd(dppf)Cl₂ (113 mg, 0.132 mmol). The reaction solution was heated to 100 °C and stirred at that temperature under N₂ for 2 h. The mixture was cooled to 25 °C and concentrated under reduced pressure. The residue was purified by silica gel chromatography eluting with MeOH/DCM (with MeOH from 0 to 10%) in 15 min to give 1-methyl-4-(4,4,5,5-tetramethyl-1,3,2-dioxabenzofuran-2-yl) pyridin-2(1H)-one (500 mg, 62% yield) as a yellow oil.

### Synthesis of tert-butyl 8-(7-(difluoromethyl)-6-(1-methyl-1H-pyrazol-4-yl)-3,4-dihydroquinolin-1(2H)-yl)-6-(1-methyl-2-oxo-1,2-dihydropyridin-4-yl)-3,3-dihydroisoquinoline-2(1H)-carboxylate:

To a solution of tert-butyl 6-chloro-8-(7-(difluoromethyl)-6-(1-methyl-1H-pyrazol-4-yl)-3,4-dihydroquinolin-1(2H)-yl)-3,4-dihydroxyisoquinoline-2(1H)-carboxylate (450 mg, 0.849 mmol) in THF (10.0 mL) and H₂O (1.0 mL) were added 1-methyl-4-(4,4,5,5,4-tetramethyl-1,3,2-dioxacyclopentanol-2(2H)-1 (400), potassium triphosphate (360 mg, 1.69 mmol) and X-phosphorus G3 (72.0 mg, 0.084 mmol). The reaction solution was heated to 60 °C and stirred at that temperature under N₂ for 3 h. The mixture was cooled to 25 °C and concentrated under reduced pressure. The residue was purified by silica gel chromatography eluting with MeOH/DCM (with MeOH from 0 to 5%) in 10 min to afford tert-butyl 8-(7-(difluoromethyl)-6-(1-methyl-1H-pyrazolyl-pyrazol-4-yl)-3,4-dihydroquinolin-1(2H)-yl)-3,4-dihydroxyquinoline-2(2H)-6-(2-methyl-2-oxo-1,2-dihydropyridine-4-carboxylate (460 mg, 85% yield) as a yellow oil.

### Synthesis of 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-1-methylpyridin-2(1H)-one:

To a solution of tert-butyl 8-(7-(difluoromethyl)-6-(1-methyl-1H-pyrazol-4-yl)-3,4-dihydroquinolin-1(2H)-yl)-6-(2-methyl-2-oxo-1,2-dihydropyridin-4-yl)-3,4-dihydroisoquinoline-2(1H)-carboxylate (450 mg, 0.746 mmol) in DCM (10.0 mL) was added TFA (3.0 mL). The solution was stirred at 25 °C for 1 h. The mixture was concentrated under reduced pressure. The residue was directly used in the next step without purification.

### Synthesis of 8-(7-(difluoromethyl)-6-(1-methyl-1H-pyrazol-4-yl)-3,4-dihydroquinolin-1(2H)-yl)-N-methyl-6-(1-methyl-2-oxo-1,2-dihydropyridin-4-yl)-3-tetrahydroisoquinoline-2(1H)-carboxamide:

To a solution of 4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-1-methylpyridin-2(1H)-one (450 mg, 0.897 mmol) in DCM (10.0 mL) were added Et₃N (454 mg, 4.486 mmol) and N-methylcarbamoyl chloride (252 mg, 2.69 mmol). The reaction solution was stirred at 25 °C for 1 h. The mixture was concentrated under reduced pressure. The residue was purified by Prep HPLC (with CH₃CN from 35% to 65% in 7 min) to afford 8-(7-(difluoromethyl)-6-(1-methyl-1H-pyrazol-4-yl)-3,4-dihydroquinolin-1(2H)-yl)-N-methyl-6-(1-methyl-2-oxo-1,2-dihydropyridin-4-yl)-3,4-dihydroisoquinoline-2(1H)-carboxamide (320 mg, 64% yield) as a yellow solid.

### Synthesis of 8-(7-(difluoromethyl)-6-(1-methyl-1H-pyrazol-4-yl)-3,4-dihydroquinolin-1(2H)-yl)-N-methyl-6-(1-methyl-2-oxopiperidin-4-yl)-3,4-dihydroisoquinoline-2(1H)-carboxamide:

To a solution of 8-(7-(difluoromethyl)-6-(1-methyl-1H-pyrazol-4-yl)-3,4-dihydroquinolin-1(2H)-yl)-N-methyl-6-(1-methyl-2-oxo-1,2-dihydropyridin-4-yl)-3,4-dihydroisoquinoline-2(1H)-carboxamide (200 mg, 0.358 mmol) in MeOH (10.0 mL) was added PtO₂ (81.0 mg, 0.358 mmol). The solution was stirred under H₂ at 25 °C for 12 h. The mixture was filtered to remove solids and concentrated. The residue was purified by Prep HPLC (with CH₃CN from 25% to 50% in 8 min) to afford 8-(7-(difluoromethyl)-6-(1-methyl-1H-pyrazol-4-yl)-3,4-dihydroquinolin-1(2H)-yl)-N-methyl-6-(1-methyl-2-oxopiperidin-4-yl)-3,4-dihydroisoquinoline-2(1H)-carboxamide (90.0 mg, 41% yield) as a yellow solid.

¹H NMR (400 MHz, DMSO-d6) δ 7.72 (s, 1H), 7.47 (s, 1H), 7.06 (m, 3H), 6.70 (m, 1H), 6.48 (d, J = 4.0 Hz, 1H), 6.17 (s, 1H), 4.43 (d, J = 16.0 Hz, 1H), 4.16-4.08 (m, 1H), 3.86 (s, 3H), 3.59-3.41 (m, 4H), 3.28-3.22 (m, 1H), 3.05 (s, 1H), 2.81 (m, 7H), 2.54 (d, J = 4.0 Hz, 3H), 2.43-2.30 (m, 2H), 2.18-1.81 (m, 5H).

### Example 5

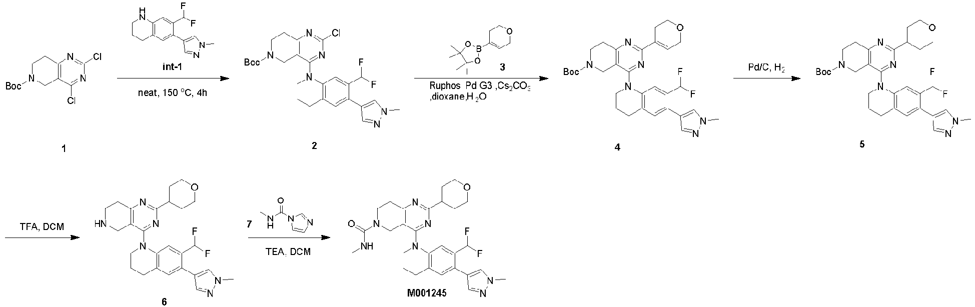

To a mixture of tert-butyl 2,4-dichloro-7,8-dihydropyrido[4,3-d]pyrimidine-6(5H)-carboxylate (compound 1, 432 mg, 1.64 mmol, 1.0 eq), 7-(difluoromethyl)-6-(1-methyl-1H-pyrazol-4-yl)-1,2,3,4-tetrahydroquinoline (compound int-1, 1.0 g, 3.29 mmol, 2.0 eq) at 20 °C was added DIEA (637 mg, 4.93 mmol, 858 µL, 3.0 eq). The mixture was stirred at 150 °C for 4 h. LC-MS showed compound 1 was consumed completely. The resulting mixture was extracted with ethyl acetate (100 mL), washed with brine (10 mL × 3), dried over Na₂SO₄ (25 g), and concentrated. The residue was purified by prep-HPLC (Phenomenex luna C₁₈ 100 × 40 mm × 3 µm; mobile phase: [water-ACN]; B%: 40%-60%, 12 min) to give tert-butyl 2-chloro-4-(7-(difluoromethyl)-6-(1-methyl-1H-pyrazol-4-yl)-3,4-dihydroquinolin-1(2H)-yl)-7,8-dihydropyrido[4,3-d]pyrimidine-6(5H)-carboxylate (compound 2, 100 mg, crude) as a yellow oil. The product was confirmed by H NMR.

To a mixture of compound 2 (100 mg, 188 µmol, 1.0 eq) and 2-(3,6-dihydro-2H-pyran-4-yl)-4,4,5-5-tetramethyl-1,3,2-dioxaborane (compound 3, 79.1 mg, 376 µmol, 2.0 eq) in dioxane (4.0 mL) and H₂O (1.0 mL) at 90 °C were added Ruphos Pd G3 (31.5 mg, 37.6 µmol, 0.2 eq) and Cs₂CO₃ (184 mg, 564 µmol, 3.0 eq) in one portion. The mixture was heated to 90 °C and stirred for 16 h. LC-MS showed 32% of the desired compound was detected. The reaction solution was concentrated under reduced pressure. The residue was purified by silica gel flash chromatography (ISCO^{®}; 25 g SepaFlash^{®} silica gel flash column, 20-30% ethyl acetate/petroleum ether gradient eluent @ 50 mL/min). TLC (petroleum ether:ethyl acetate = 3:1, Rf = 0.48). tert-Butyl 4-(7-(difluoromethyl)-6-(1-methyl-1H-pyrazol-4-yl)-3,4-dihydroquinolin-1(2H)-yl)-2-(3,6-dihydro-2H-pyran-4-yl)-7,8-dihydropyrido[4,3-d]pyrimidine-6(5H)-carboxylate (compound 4, 50.0 mg, 86.4 µmol, 45.9% yield) was obtained as a yellow oil.

To a mixture of compound 4 (50.0 mg, 86.4 µmol, 1.0 eq) in THF (5.0 mL) at 20 °C were added H₂ and Pd/C (20.0 mg, 10% purity) (15 psi) in one portion. The mixture was heated to 20 °C and stirred for 16 h. LC-MS showed 46.5% of the desired compound was detected. The reaction solution was concentrated under reduced pressure to give tert-butyl 4-(7-(difluoromethyl)-6-(1-methyl-1H-pyrazol-4-yl)-3,4-dihydroquinolin-1(2H)-yl)-2-(tetrahydro-2H-pyran-4-yl)-7,8-dihydropyrido[4,3-d]pyrimidine-6(SH)-carboxylate (compound 5, 50.0 mg, crude) as a yellow oil.

To compound 5 (50.0 mg, 86.1 µmol, 1.0 eq) in DCM (3.0 mL) at 20 °C was added TFA (85.6 mg, 751 µmol, 55.6 µL, 8.72 eq) in one portion under N₂. The mixture was stirred at 20 °C for 16 h. LC-MS showed compound 5 was consumed completely. The reaction mixture was partitioned between water (20.0 mL) and ethyl acetate (20.0 mL). The organic layer was dried over Na₂SO₄ and concentrated to give the desired product. 4-(7-(Difluoromethyl)-6-(1-methyl-1H-pyrazol-4-yl)-3,4-dihydroquinolin-1(2H)-yl)-2-(tetrahydro-2H-pyran-4-yl)-5,6,7,8-tetrahydropyrido[4,3-d]pyrimidine (compound 6, 50.0 mg, crude) was obtained as a yellow oil.

To a mixture of N-methyl-1H-imidazole-1-carboxamide (compound 7, 26.0 mg, 208 µmol, 2.0 eq) in DCM (5.0 mL) at 20 °C were added TEA (63.1 mg, 624 µmol, 86.8 µL, 6.0 eq) and compound 6 (50.0 mg, 104 µmol, 1.0 eq) in one portion under N₂. The mixture was stirred at 20 °C for 16 h. LC-MS showed compound 6 was consumed completely. The reaction mixture was partitioned between water (20.0 mL) and ethyl acetate (20.0 mL). The organic layer was dried over Na₂SO₄ and concentrated to give the desired product. The residue was purified by preparative HPLC column: PhenomenexGemini-NXC18 75 × 30 mm × 3 µm; mobile phase: [water (0.05% NH₃H₂ + 10 mM NH₄HCO₃)-ACN]; B%: 22%-62%, 11 min. 4-(7-(Difluoromethyl)-6-(1-methyl-1H-pyrazol-4-yl)-3,4-dihydroquinolin-1(2H)-yl)-N-methyl-2-(tetrahydro-2H-pyran-4-yl)-7,8-dihydropyrido[4,3-d]pyrimidine-6(5H)-carboxamide (compound M001245, 34 mg, 62.4 µmol, 60.0% yield, 98.7% purity) was obtained as a yellow oil. M001245:
¹H NMR (400 MHz, DMSO-d6) *δ* 7.74 (s, 1H), 7.60 (s, 1H), 7.30 (s, 1H), 6.84-6.82 (m, 1H), 6.82-6.53 (m, 1H), 4.62 (s, 1H), 4.07 (s, 3H), 4.04 (d, *J* = 2.0 Hz, 1H), 3.97 (s, 3H), 3.92 (t, *J* = 6.0 Hz, 2H), 3.68 (t, *J* = 6.4 Hz, 2H), 3.58 (dt, *J* = 2.4, 11.8 Hz, 2H), 3.06-2.90 (m, 5H), 2.68 (s, 3H), 2.18-2.10 (m, 2H), 2.07-1.96 (m, 2H), 1.94-1.87 (m, 2H).

### Example 6

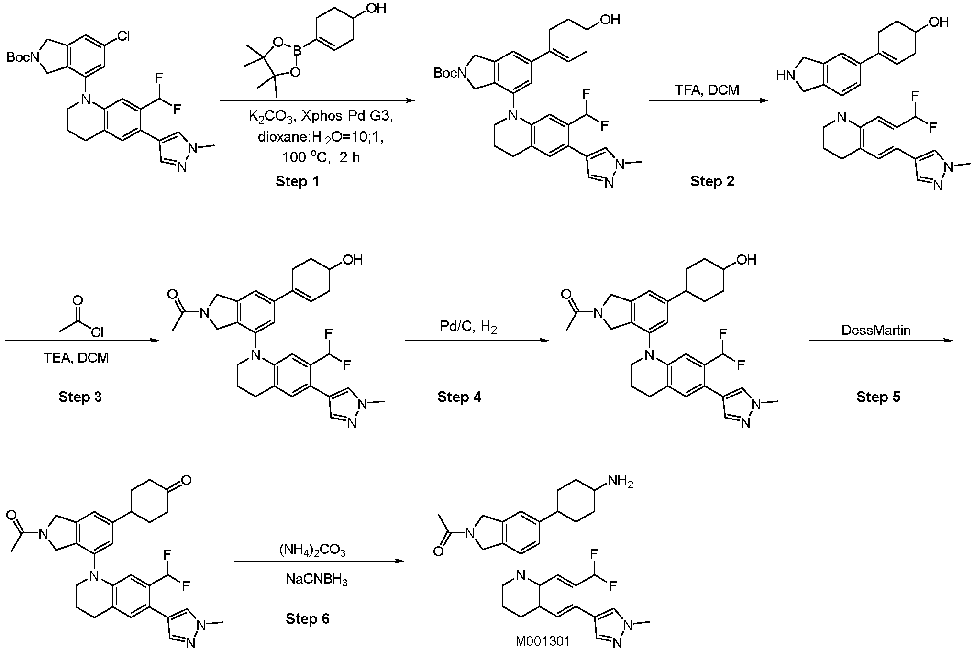

To a solution of tert-butyl 6-chloro-4-(7-(difluoromethyl)-6-(1-methyl-1H-pyrazol-4-yl)-3,4-dihydroquinolin-1(2H)-yl)isoindoline-2-carboxylate (700 mg, 1.36 mmol) in dioxane (5.0 mL) and H₂O (0.5 mL) were added 4-(4,4,5,5-tetramethyl-1,3,2-dioxaborolan-2-yl)cyclohex-3-en-1-ol (609.2 mg, 2.72 mmol), K₂CO₃ (563.6 mg, 4.08 mmol) and XPhos Pd G3 (114.9 mg, 0.14 mmol). The mixture was then stirred at 100 °C for 2 h. LCMS showed the starting materials were consumed and the desired product was detected. The residue was purified by flash column chromatography eluting with EtOAc in PE from 0 to 50% and then by flash column chromatography eluting with MeOH in DCM from 0 to 5% to give tert-butyl 4-(7-(difluoromethyl)-6-(1-methyl-1H-pyrazol-4-yl)-3,4-dihydroquinolin-1(2H)-yl)-6-(4-hydroxycyclohex-1-en-1-yl)isoindoline-2-carboxylate as a yellow solid.

To a solution of tert-butyl 4-(7-(difluoromethyl)-6-(1-methyl-1H-pyrazol-4-yl)-3,4-dihydroquinolin-1(2H)-yl)-6-(4-hydroxycyclohex-1-en-1-yl)isoindoline-2-carboxylate (700 mg, 1.21 mmol) in DCM (3.0 mL) was added TFA (1.0 mL). The mixture was concentrated under reduced pressure to give 4-(7-(7-(difluoromethyl)-6-(1-methyl-1H-pyrazol-4-yl)-3,4-dihydroquinolin-1(2H)-yl)isoindolin-5-yl)cyclohex-3-en-1-ol (600 mg, 98% yield) as a yellow solid.

To a solution of 4-(7-(7-(difluoromethyl)-6-(1-methyl-1H-pyrazol-4-yl)-3,4-dihydroquinolin-1(2H)-yl)isoindolin-5-yl)cyclohex-3-en-1-ol (600 mg, 1.3 mmol) in DCM (4.0 mL) were added 25% TEA (382.2 mg, 3.8 mmol) and acetyl chloride (197.7 mg, 2.5 mmol). Water (50 mL) was added to quench the reaction, and the mixture was extracted with EtOAc (50 mL × 3). The combined organic layers were washed with brine (50 mL × 3) and dried over anhydrous Na₂SO₄. The residue was purified by flash column chromatography eluting with EtOAc in PE from 0 to 50% and then by flash column chromatography eluting with methanol in DCM from 0 to 5% to give 1-(4-(7-(difluoromethyl)-6-(1-methyl-1H-pyrazol-4-yl)-3,4-dihydroquinolin-1(2H)-yl)-6-(4-hydroxycyclohex-1-en-1-yl)isoindolin-2-yl)ethan-1-one (600 mg, 92% yield) as a yellow solid.

To a solution of 1-(4-(7-(difluoromethyl)-6-(1-methyl-1H-pyrazol-4-yl)-3,4-dihydroquinolin-1(2H)-yl)-6-(4-hydroxycyclohex-1-en-1-yl)isoindolin-2-yl)ethan-1-one (600 mg, 1.2 mmol) in THF (4.0 mL) at 25 °C was added Pd/C (60% in oil) (123.1 mg, 1.2 mol) under H₂ atmosphere (15 PSI). After filtration, the filtrate was concentrated under reduced pressure to give 1-(4-(7-(difluoromethyl)-6-(1-methyl-1H-pyrazol-4-yl)-3,4-dihydroquinolin-1(2H)-yl)-6-(4-hydroxycyclohexyl)isoindolin-2-yl)ethan-1-one (600 mg, 99% yield) as a yellow solid.

To a solution of 1-(4-(7-(difluoromethyl)-6-(1-methyl-1H-pyrazol-4-yl)-3,4-dihydroquinolin-1(2H)-yl)-6-(4-hydroxycyclohexyl)isoindolin-2-yl)ethan-1-one (130 mg, 0.25 mmol) in DCM (2.0 mL) at 25 °C was added DessMartin (158.9 mg, 0.37 mmol). The mixture was then stirred at 25 °C for 2 h. The mixture was quenched by adding saturated aqueous NaHCO₃ (20 mL) and extracted with DCM (3 × 20 mL). The combined organic layers were dried over Na₂SO₄. After filtration, the filtrate was purified by flash column chromatography eluting with EtOAc in PE from 0% to 50% and then by flash column chromatography eluting with methanol in dichloromethane from 0% to 5% to give 4-(2-acetyl-7-(7-(difluoromethyl)-6-(1-methyl-1H-pyrazol-4-yl)-3,4-dihydroquinolin-1(2H)-yl)isoindolin-5-yl)cyclohexan-1-one (100 mg, 77% yield) as a white solid.

To a solution of 4-(2-acetyl-7-(7-(difluoromethyl)-6-(1-methyl-1H-pyrazol-4-yl)-3,4-dihydroquinolin-1(2H)-yl)isoindolin-5-yl)cyclohexan-1-one (200 mg, 0.39 mmol) in MeOH (3.0 mL) at 25 °C was added ammonium carbonate (74.1 mg, 0.77 mmol). The mixture was stirred at 25 °C for 1 h. NaCNBH₃ (48.5 mg, 0.77 mmol) was then added to the mixture. The mixture was stirred at 25 °C for 1 h. LCMS showed the starting materials were consumed and the desired product was detected. The crude product was purified by preparative HPLC eluting with CH₃CN in 0.1% FA/water from 38% to 45% in 8 min to give 1-(6-(4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one (30.4 mg, 15% yield) as a white solid.

### M001301:

¹H NMR (400 MHz, DMSO-*d₆*) δ 8.40 (s, 1H), 7.74 (s, 1H), 7.49 (s, 1H), 7.16-7.10 (m, 2H), 6.74 (t, *J* = 55.2 Hz, 1H), 6.40 (d, *J* = 28.0 Hz, 1H), 4.85 (s, 1H), 4.64 (s, 1H), 4.60 (s, 1H), 4.28 (s, 1H), 3.86 (s, 3H), 3.57-3.48 (m, 4H), 2.98-2.94 (m, 1H), 2.87-2.80 (m, 2H), 2.64-2.53 (m, 1H), 2.47-2.42 (m, 1H), 2.19-1.96 (m, 6H), 1.87-1.81 (s, 2H), 1.62-1.47 (m, 2H), 1.43-1.36 (m, 2H).

M001301 was further used as a starting material to prepare M001320 and M001321.

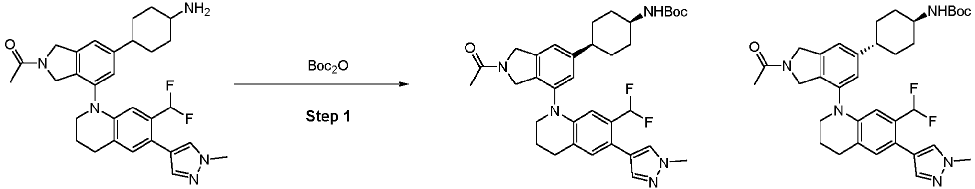

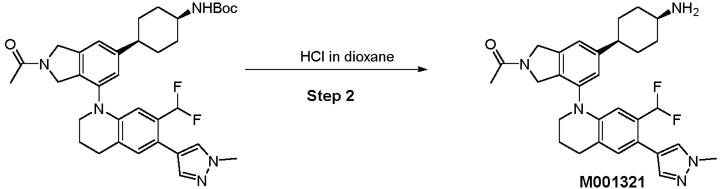

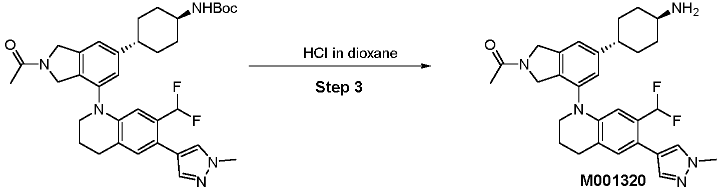

To a solution of 1-(6-(4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one (215 mg, 0.414 mmol) and Boc₂O (181 mg, 0.828 mol) in DCM (6 mL) at 25 °C was added TEA (41.9 mg, 1.24 mmol). The reaction mixture was stirred at 25 °C under N₂ for 1 h. The mixture was concentrated under reduced pressure. The residue was purified by SFC (Daicel CHIRALCEL IB-N, 250 mm × 30 mm I.D., 10 µm; mobile phase: CO2/MeOH [0.2% NH3 (7 M MeOH solution)] = 50/40; 80 g/min; 35 °C) to give tert-butyl ((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (37.2 mg, 14.5% yield) and tert-butyl ((1r,4r)-4-(2-acetyl-7-(7-(difluoromethyl)-6-(1-methyl-1H-pyrazol-4-yl)-3,4-dihydroquinolin-1 (2H)-yl)isoindolin-5-yl)cyclohexyl)carbamate (105.3 mg, 41%) as white solids.

To a solution of tert-butyl (1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (37.2 mg, 0.0601 mmol) in dioxane (5 mL) at 25 °C was added HCl in dioxane (4 N, 5 mL). The reaction mixture was stirred at 25 °C for 1 h. The solution in water was dried by lyophilization to give 1-(6-((1s,4s)-4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one hydrochloride (28.5 mg, 84%) as a white solid.

To a solution of tert-butyl (1r,4r)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)carbamate (105.3 mg, 0.170 mmol) in dioxane (5 mL) at 25 °C was added HCl in dioxane (4 N, 5 mL). The reaction mixture was stirred at 25 °C for 1 h. The solution in water was dried by lyophilization to give 1-(6-((1r,4r)-4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one hydrochloride (93.2 mg, 98% yield) as a white solid.
M001320:
   ¹H NMR (400 MHz, DMSO-*d₆*) δ 7.98 (s, 3H), 7.75 (s, 1H), 7.49 (s, 1H), 7.19-7.10 (m, 3H), 6.74 (t, *J* = 55.2 Hz, 1H), 6.45-6.35 (m, 1H), 4.92-7.80 (m, 1H), 4.71-4.50 (m, 2H), 4.36-4.20 (m, 1H), 3.86 (s, 3H), 3.56-3.49 (m, 2H), 3.14-3.00 (m, 1H), 2.93-2.83 (m, 2H), 2.61-2.52 (m, 1H), 2.07-1.96 (m, 7H), 1.92-1.80 (m, 2H), 1.62-1.40 (m, 4H).
M001321:
   ¹H NMR (400 MHz, DMSO-*d₆*) δ 7.99 (s, 3H), 7.75 (s, 1H), 7.49 (s, 1H), 7.28-7.18 (m, 2H), 7.13 (s, 1H), 6.74 (t, *J* = 55.2 Hz, 1H), 6.46-6.34 (m, 1H), 4.92-4.81 (m, 1H), 4.74-4.52 (m, 2H), 4.45-4.22 (m, 1H), 3.86 (s, 3H), 3.62-3.51 (m, 2H), 3.45-3.42 (m, 1H), 2.92-2.84 (m, 2H), 2.65-2.55 (m, 1H), 2.05-1.98 (m, 5H), 1.89-1.72 (m, 6H), 1.69-1.58 (m, 2H).

### Example 7

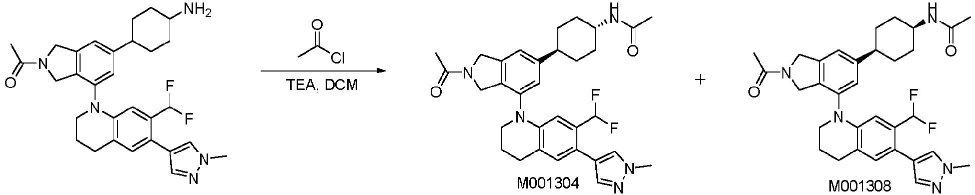

To a solution of 1-(6-(4-aminocyclohexyl)-4-(7-(difluoromethyl)-6-(1-methyl-1H-pyrazol-4-yl)-3,4-dihydroquinolin-1(2H)-yl)isoindolin-2-yl)ethan-1-one (60 mg, 0.12 mmol) in DCM (2.0 mL) and THF (1.0 mL) at 0 °C were added TEA (23.4 mg, 0.23 mmol) and acetyl chloride (13.6 mg, 0.17 mmol). The mixture was stirred at 25 °C for 0.5 h. LCMS showed the starting materials were consumed and the desired product was detected. The crude product was purified by preparative HPLC eluting with CH₃CN in H₂O (0.1% NH₃) from 33% to 43% in 7 min to give N-((1s,4s)-4-(2-acetyl-7-(7-(difluoromethyl)-6-(1-methyl-1H-pyrazol-4-yl)-3,4-dihydroquinolin-1(2H)-yl)isoindolin-5-yl)cyclohexyl)acetamide (2.8 mg, 4% yield) and N-((1r,4r)-4(2-acetyl-7-(7-[difluoromethyl]-6-(1-ethyl-1H-pyrazol-4-yl)-3,4-dihydroquinolin-1(2H)-yl)isoindolin-5-yl)cyclohexyl)acetamide (11.7 mg, 18% yield) as white solids.
M001304
   ¹H NMR (400 MHz, DMSO-*d₆*) δ 7.76-7.73 (m, 2H), 7.49 (s, 1H), 7.17 (s, 1H), 7.15-7.07 (m, 2H), 6.74 (t, *J =* 55.1 Hz, 1H), 6.40 (d, *J* = 28.8 Hz, 1H), 4.85 (s, 1H), 4.64 (s, 1H), 4.61 (s, 1H), 4.28 (s, 1H), 3.86 (s, 3H), 3.58-3.52 (m, 3H), 2.90-2.86 (m, 2H), 2.60-2.53 (m, 1H), 2.10-1.96 (m, 5H), 1.91-1.81 (m, 4H), 1.79-1.77 (m, 3H), 1.59-1.49 (m, 2H), 1.32-1.24 (m, 2H).
M001308
   ¹H NMR (400 MHz, DMSO-*d₆*) δ 7.83 (d, *J* = 8.0 Hz, 1H), 7.74 (s, 1H), 7.49 (s, 1H), 7.19 (d, *J* = 11.2 Hz, 1H), 7.17-7.08 (m, 2H), 6.74 (t, *J* = 55.2 Hz, 1H), 6.40 (d, *J* = 27.2 Hz, 1H), 4.86 (s, 1H), 4.66 (s, 1H), 4.62 (s, 1H), 4.29 (s, 1H), 4.00-3.96 (m, 1H), 3.86 (s, 3H), 3.67-3.48 (m, 2H), 2.90-2.87 (m, 2H), 2.63-2.54 (m, 1H), 2.13-1.92 (m, 5H), 1.85 -1.83 (m, 3H), 1.82-1.76 (m, 2H), 1.69-1.65 (m, 2H), 1.61-1.56 (m, 4H).

### Example 8

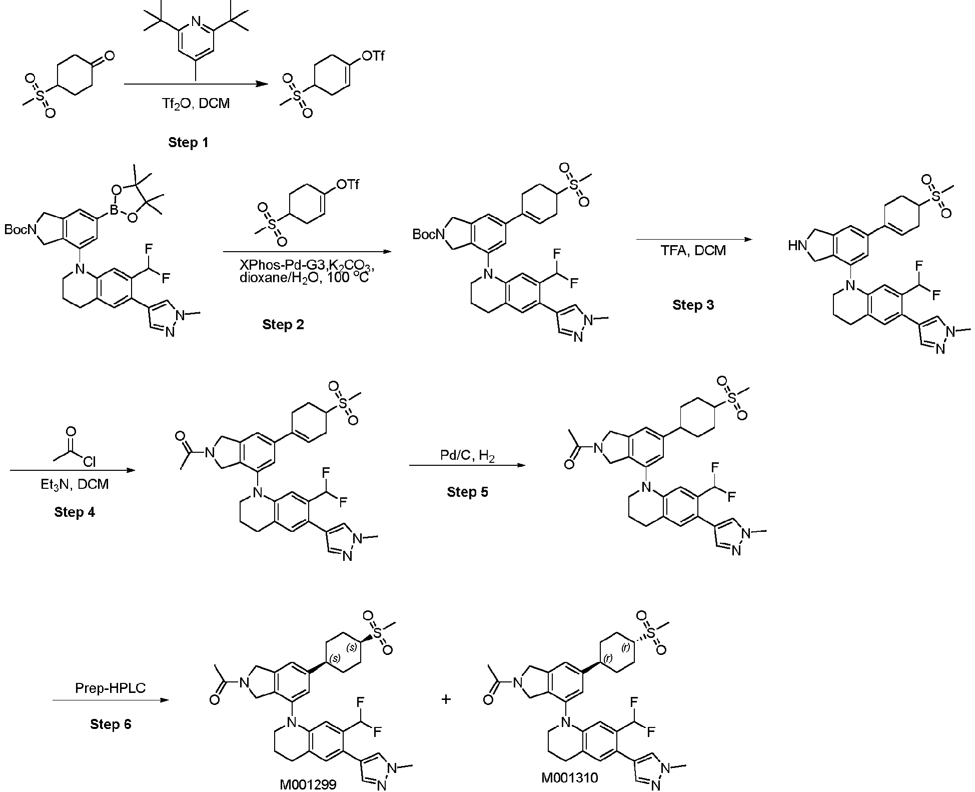

To a solution of 4-methanesulfonylcyclohexan-1-one (0.6 g, 0.0034 mol) in DCM (10 mL) were added Tf₂O (1.92 g, 0.0068 mol) and 2,6-di-tert-butyl-4-methylpyridine (1.54 g, 0.00748 mol). The reaction mixture was stirred at 40 °C for 16 h. TLC showed the desired product was formed. The reaction mixture was diluted with DCM, washed with HCl (1 N) and extracted with DCM. The organic layer was dried over anhydrous Na₂SO₄, filtered and concentrated under vacuum. The residue was purified by flash column chromatography eluting with DCM in PE from 0% to 100% to give 4-methanesulfonylcyclohex-1-en-1-yl trifluoromethanesulfonate (490 mg, 47%) as a white solid.

To a solution of tert-butyl 4-[7-(difluoromethyl)-6-(1-methylpyrazol-4-yl)-3,4-dihydro-2H-quinolin-1-yl]-6-(4,4,5,5-tetramethyl-1,3,2-dioxaborolan-2-yl)-1,3-dihydroisoindole-2-carboxylate (325 mg, 0.5359 mmol) in 1,4-dioxane (6 mL) and H₂O (1 mL) were added 4-methanesulfonylcyclohex-1-en-1-yl trifluoromethanesulfonate (165 mg, 0.5359 mmol), XPhos Pd G3 (45 mg, 0.05359 mmol) and K₂CO₃ (2 mg, 1.6077 mmol). The reaction solution was heated to 100 °C and stirred at that temperature under N₂ for 4 h. LCMS showed the desired product was formed. The mixture was cooled to 25 °C and concentrated under reduced pressure. The residue was purified by silica gel chromatography eluting with MeOH in DCM from 0% to 5% to give tert-butyl 4-(7-(difluoromethyl)-6-(1-methyl-1H-pyrazol-4-yl)-3,4-dihydroquinolin-1(2H)-yl)-6-(4-(methylsulfonyl)cyclohex-1-en-1-yl)isoindoline-2-carboxylate (225 mg, 66%) as a yellow oil.

To a solution of tert-butyl 4-(7-(difluoromethyl)-6-(1-methyl-1H-pyrazol-4-yl)-3,4-dihydroquinolin-1(2H)-yl)-6-(4-(methylsulfonyl)cyclohex-1-en-1-yl)isoindoline-2-carboxylate (225 mg, 0.3522 mmol) in DCM (3 mL) was added TFA (1 mL). The reaction mixture was stirred at 25 °C for 30 min. LCMS showed the desired product was formed. The solvent was removed under reduced pressure, and 7-(difluoromethyl)-6-(1-methyl-1H-pyrazol-4-yl)-1-(6-(4-(methylsulfonyl)cyclohex-1-en-1-yl)isoindolin-4-yl)-1,2,3,4-tetrahydroquinoline (185 mg, crude) was obtained as a yellow oil.

To a solution of 7-(difluoromethyl)-6-(1-methyl-1H-pyrazol-4-yl)-1-(6-(4-(methylsulfonyl)cyclohex-1-en-1-yl)isoindolin-4-yl)-1,2,3,4-tetrahydroquinoline (185 mg, 0.3434 mmol) in DCM (1 mL) were added TEA (695 mg, 6.868 mmol) and acetyl chloride (270 mg, 3.434 mmol). The reaction mixture was stirred at 25 °C for 50 min. LCMS showed the desired product was formed. The solvent was removed under reduced pressure, and the residue was purified by silica gel chromatography eluting with MeOH in DCM from 0% to 10% to give 1-(4-(7-(difluoromethyl)-6-(1-methyl-1H-pyrazol-4-yl)-3,4-dihydroquinolin-1(2H)-yl)-6-(4-(methylsulfonyl)cyclohex-1-en-1-yl)isoindolin-2-yl)ethan-1-one (90 mg, 45%) as a yellow solid.

To a solution of 1-(4-(7-(difluoromethyl)-6-(1-methyl-1H-pyrazol-4-yl)-3,4-dihydroquinolin-1(2H)-yl)-6-(4-(methylsulfonyl)cyclohex-1-en-1-yl)isoindolin-2-yl)ethan-1-one (90 mg, 0.1550 mmol) in MeOH (30 mL) was added 10% Pd/C (49 mg, 0.0465 mmol). The reaction mixture was stirred at 25 °C for 16 h. LCMS showed the desired product was formed. The reaction mixture was filtered and concentrated under vacuum, and the residue was purified by Pre-HPLC (chromatographic column: -Xbridge-C₁₈ 150 × 19 × 19 mm, 5 µm; mobile phase: ACN-H₂O (0.1% FA)) to give 1-(4-(7-(difluoromethyl)-6-(1-methyl-1H-pyrazol-4-yl)-3,4-dihydroquinolin-1(2H)-yl)-6-(4-(methylsulfonyl)cyclohexyl)isoindolin-2-yl)ethan-1-one (30 mg, 33%).

1-(4-(7-(Difluoromethyl)-6-(1-methyl-1H-pyrazol-4-yl)-3,4-dihydroquinolin-1(2H)-yl)-6-(4-(methylsulfonyl)cyclohexyl)isoindolin-2-yl)ethan-1-one (30 mg, 0.0515 mmol) was purified by high performance liquid chromatography (Daicel CHIRALCEL IB-N, 250 mm × i.d. 30 mm, 10 µm; mobile phase: CO₂/MeOH [0.2% NH₃(7 M MeOH solution)] = 50/40; 80 g/min; 35 °C) to give 1-(4-(7-(difluoromethyl)-6-(1-methyl-1H-pyrazol-4-yl)-3,4-dihydroquinolin-1(2H)-yl)-6-((1s,4s)4-(methylsulfonyl)cyclohexyl)isoindolin-2-yl)ethan-1-one (6.4 mg, 21%) as a yellow solid, and 1-(4-(7-(difluoromethyl)-6-(1-methyl-1H-pyrazol-4-yl)-3,4-dihydroquinolin-1(2H)-yl)-6-((1r,4r)4-(methylsulfonyl)cyclohexyl)isoindolin-2-yl)ethan-1-one.
M001299:
   ¹H NMR (400 MHz, DMSO-*d₆*) δ 7.75 (s, 1H), 7.49 (s, 1H), 7.13 (t, *J* = 12.8 Hz, 3H), 6.88 (s, 0.25H), 6.74 (s, 0.5H), 6.60 (s, 0.25H), 6.44-6.37 (m, 1H), 4.87-4.25 (m, 4H), 3.86 (s, 3H), 3.55 (d, *J =* 4.8 Hz, 2H), 3.11 (s, 1H), 2.93 (d, *J* = 4.4 Hz, 3H), 2.88 (s, 2H), 2.61 (s, 1H), 2.18 (s, 2H), 2.04-1.94 (m, 7H), 1.60-1.50 (m, 4H).
M001310:
   ¹H NMR (400 MHz, DMSO-*d₆*) δ 7.75 (s, 1H), 7.49 (s, 1H), 7.17-7.06 (m, 3H), 6.87 (s, 0.25H), 6.74 (s, 0.5H), 6.60 (s, 0.25H), 6.44-6.37 (m, 1H), 4.87-4.29 (m, 4H), 3.86 (s, 3H), 3.55 (d, *J* = 4.8 Hz, 2H), 3.31-3.26 (m, 1H), 2.97 (d, *J* = 4.8 Hz, 3H), 2.88 (s, 2H), 2.82-2.72 (m, 1H), 2.15 (d, *J =* 12.8 Hz,2H), 2.09-1.97 (m, 7H), 1.91-1.83 (m, 2H), 1.73-1.67 (m, 2H).

### Example 9

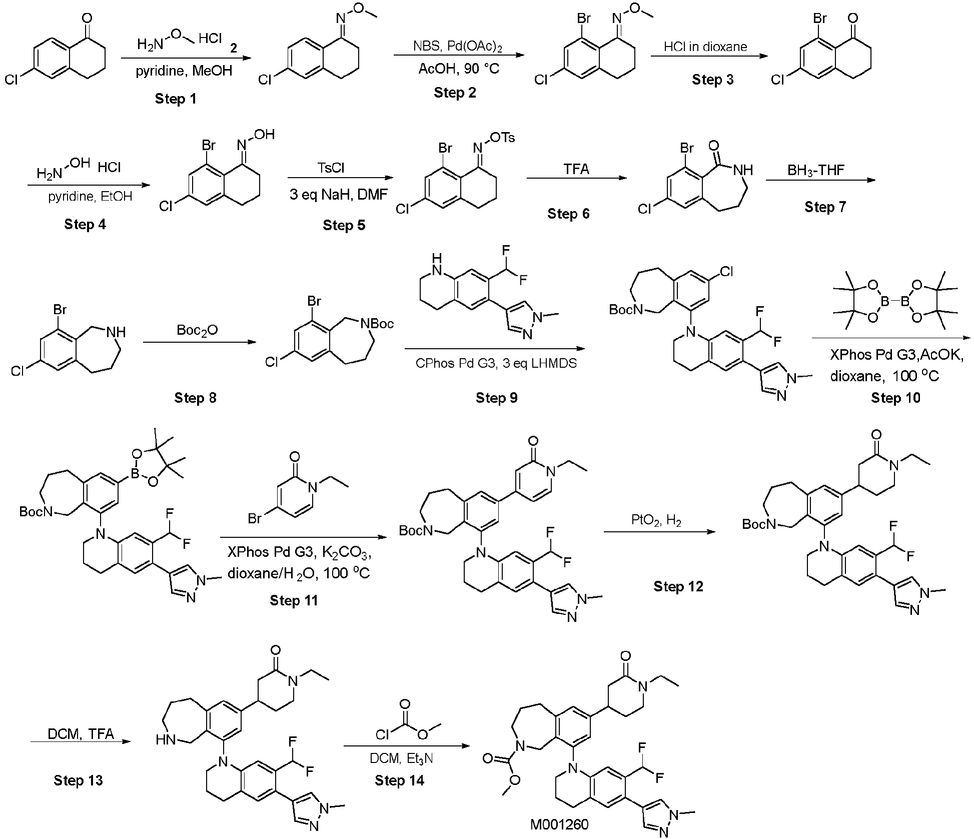

To a solution of 6-chloro-3,4-dihydro-2H-naphthalen-1-one (5.00 g, 0.027 mol) in MeOH (50.0 mL) were added pyridine (2.63 g, 0.033 mol) and O-methylhydroxylamine hydrochloride (2.78 g, 0.03 mol). The reaction solution was stirred at 25 °C under N₂ for 12 h. The mixture was concentrated under reduced pressure. The residue was purified by silica gel chromatography eluting with EA/PE (with EA from 0 to 5%) in 10 min to give (E)-6-chloro-3,4-dihydronaphthalen-1(2H)-one O-methyl oxime (5.60 g, 92% yield) as a white oil.

To a solution of (E)-6-chloro-3,4-dihydronaphthalen-1(2H)-one O-methyl oxime (5.6 g, 0.026 mol) in AcOH (50.0 mL) were added N-bromosuccinimide (4.75 g, 0.026 mol) and Pd(OAc)2 (0.60 g, 0.003 mol). The reaction solution was stirred at 90 °C under microwave irradiation for 0.5 h. The mixture was concentrated, and the residue was washed with NaHCO₃ solution (100 mL) and extracted with DCM (60 mL × 2). The combined organic layers were washed with brine (50 mL) and dried over Na₂SO₄, filtered and concentrated to dryness. The residue was purified by silica gel chromatography eluting with EA/PE (with EA from 0 to 5%) in 10 min to give (E)-8-bromo-6-chloro-3,4-dihydronaphthalen-1(2H)-one O-methyl oxime (6.50 g, 80% yield) as a yellow oil.

A solution of (E)-8-bromo-6-chloro-3,4-dihydronaphthalen-1(2H)-one O-methyl oxime (6.50 g, 0.022 mol) in 1,4-dioxane (15.0 mL) and 6 N HCl (70.0 mL) was stirred at 120 °C for 2 h under microwave irradiation. The mixture was concentrated, and the residue was washed with NaHCO₃ solution (100 mL) and extracted with DCM (60 mL × 2). The combined organic layers were washed with brine (50 mL) and dried over Na₂SO₄, filtered and concentrated to dryness. The residue was purified by silica gel chromatography eluting with EA/PE (with EA from 0 to 5%) in 10 min to give 8-bromo-6-chloro-3,4-dihydronaphthalen-1(2H)-one (5.10 g, 83% yield) as a yellow oil.

To a solution of 8-bromo-6-chloro-3,4-dihydronaphthalen-1(2H)-one (5.10 g, 0.019 mol) in EtOH (50.0 mL) and pyridine (10.0 mL) was added hydroxylamine hydrochloride (2.05 g, 0.029 mol). The reaction solution was stirred at 100 °C under microwave irradiation for 1 h. The mixture was filtered, and the residue was collected to give (E)-8-bromo-6-chloro-3,4-dihydronaphthalen-1(2H)-one oxime (4.60 g, 81% yield) as a white solid.

To a solution of (E)-8-bromo-6-chloro-3,4-dihydronaphthalen-1(2H)-one oxime (4.60 g, 0.016 mol) in DMF (50.0 mL) at 0 °C was added NaH (2.02 g, 0.050 mol). The whole mixture was then stirred at 0 °C for 0.5 h, and 4-methylbenzenesulfonyl chloride (3.84 g, 0.020 mol) was added. The mixture was stirred at 25 °C for 0.5 h. The mixture was poured into water, and the whole mixture was extracted with DCM (100 mL × 2). The combined organic phases were washed with brine (50 mL). The combined organic phases were dried over sodium sulfate and filtered. The filtrate was concentrated under reduced pressure. The residue was purified by silica gel chromatography eluting with THF in PE from 0% to 10% to give (E)-8-bromo-6-chloro-3,4-dihydronaphthalen-1(2H)-one O-tosyl oxime (2.30 g, 27% yield) as a yellow solid.

A solution of (E)-8-bromo-6-chloro-3,4-dihydronaphthalen-1(2H)-one O-tosyl oxime (2.30 g, 0.005 mol) in TFA (30.0 mL) was stirred at 25 °C for 1 h. The mixture was concentrated under reduced pressure. The residue was purified by silica gel chromatography eluting with THF/PE (with THF from 0 to 50%) in 15 min to give 9-bromo-7-chloro-2,3,4,5-tetrahydro-1H-benzo[c]azepin-1-one (0.91 g, 57% yield) as a yellow oil.

To a solution of BH₃ (1 M THF, 66.3 mL) at 25 °C was added 9-bromo-7-chloro-2,3,4,5-tetrahydro-1H-benzo[c]azepin-1-one (910 mg, 3.32 mmol) . The reaction mixture was heated to 65 °C and stirred at 65 °C under N₂ for 25 h. The reaction mixture was cooled to ambient temperature and quenched with MeOH until bubbling ceased. Then 4 N aqueous HCl was added, and the mixture was heated at 80 °C for 1 h. The mixture was cooled to room temperature. The reaction mixture was extracted with ethyl acetate (50 mL × 2). 5 N KOH was added to the aqueous phase to adjust the pH to 8, and the aqueous phase was extracted with DCM (50 mL × 2). The mixture was concentrated under reduced pressure to give 9-bromo-7-chloro-2,3,4,5-tetrahydro-1H-2-benzazepine (850 mg, 89% yield) as a yellow oil.

To a solution of 9-bromo-7-chloro-2,3,4,5-tetrahydro-1H-2-benzazepine (850 mg, 3.26 mmol) in THF (20.0 mL) and H₂O (10.0 mL) at 25 °C were added NaHCO₃ (822 mg, 9.79 mmol) and di-tert-butyl dicarbonate (783 mg, 3.59 mmol). The reaction mixture was stirred at 25 °C for 16 h. The mixture was extracted with ethyl acetate (20 mL × 2). The combined organic phases were dried over sodium sulfate and filtered. The filtrate was concentrated under reduced pressure. The residue was purified by silica gel chromatography eluting with EtOAc in petroleum from 0% to 5% to give tert-butyl 9-bromo-7-chloro-1,3,4,5-tetrahydro-2-benzazepine-2-carboxylate (1000 mg, 77% yield) as a colorless oil.

To a solution of tert-butyl 9-bromo-7-chloro-1,3,4,5-tetrahydro-2-benzazepine-2-carboxylate (300 mg, 0.831 mmol) in 1,4-dioxane (10.0 mL) were added 7-(difluoromethyl)-6-(1-methylpyrazol-4-yl)-1,2,3,4-tetrahydroquinoline (219 mg, 0.831 mmol), Cphos-Pd G3 (67.0 mg, 0.083 mmol) and LHMDS (418 mg, 2.495 mmol). The reaction solution was heated to 100 °C and stirred under N₂ for 12 h. The mixture was cooled to 25 °C and concentrated under reduced pressure. The residue was purified by silica gel chromatography eluting with MeOH/DCM (with MeOH from 0 to 10%) in 10 min to give tert-butyl 7-chloro-9-(7-(difluoromethyl)-6-(1-methyl-1H-pyrazol-4-yl)-3,4-dihydroquinolin-1(2H)-yl)-1,3,4,5-tetrahydro-2H-benzo[c]azepine-2-carboxylate (220 mg, 22% yield) as a yellow solid.

To a solution of tert-butyl 7-chloro-9-(7-(difluoromethyl)-6-(1-methyl-1H-pyrazol-4-yl)-3,4-dihydroquinolin-1(2H)-yl)-1,3,4,4,5-tetrahydro-2H-benzo[c]azepine-2-carboxylate (200 mg, 0.368 mmol) in 1,4-dioxane (10.0 mL) were added 4,4,5,5-tetramethyl-2-(4,4,5,5-tetramethyl-1,3,2-dioxaborolan-2-yl)-1,3,2-dioxaborolane (187 mg, 0736 mmol), AcOK (72.0 mg, 0.736 mmol) and XPhos Pd G3 (31.0 mg, 0.036 mmol). The reaction solution was heated to 100 °C and stirred under N₂ for 2 h. The mixture was cooled to 25 °C and concentrated under reduced pressure to give tert-butyl 9-(7-(difluoromethyl)-6-(1-methyl-1H-pyrazol-4-yl)-3,4-dihydroquinolin-1(2H)-yl)-7-(4,4,5,5-tetramethyl-1,3,2-dioxaboran-2-yl)-1,3,4,5-tetrahydro-2H-benzo[c]azepine-2-carboxylate (300 mg, 64% yield) as a black solid.

To a solution of tert-butyl 9-(7-(difluoromethyl)-6-(1-methyl-1H-pyrazol-4-yl)-3,4-dihydroquinoline-1(2H)-1-(4,4,4,5,5-tetramethyl-1,3,3,4,5-tetramethyl-1,3,4,5-tetrahydro-2H-benzo[c]azepine-2-carboxylate (200 mg, 0.315 mmol) in 1,4-dioxane (6.0 mL) and H₂O (1.1.0 mL) were added 4-bromo-1-ethylpyridin-2-one (191 mg, 0.945 mmol), K₂CO₃ (87.0 mg, 0.630 mmol) and XPhos Pd G3 (27.0 mg, 0.031 mmol). The reaction solution was heated to 100 °C and stirred under N₂ for 3 h. The mixture was cooled to 25 °C and concentrated under reduced pressure. The residue was purified by silica gel chromatography eluting with MeOH/DCM (with MeOH from 0 to 5%) in 10 min to give tert-butyl 9-(7-(difluoromethyl)-6-(1-methyl-1H-pyrazol-4-yl)-3,4-dihydroquinolin-1(2H)-yl)-7-(1-ethyl-2-oxo-1,2-dihydropyridin-4-yl)-1,3,4,5-tetrahydro-2H-benzo[c]aza-2-carboxylate as a yellow solid.

To a solution of tert-butyl 9-(7-(difluoromethyl)-6-(1-methyl-1H-pyrazol-4-yl)-3,4-dihydroquinolin-1(2H)-yl)-7-(1-ethyl-2-oxo-1,2-dihydropyridin-4-yl)-1,3,4,5-tetrahydro-2H-benzo[c]azepine-2-carboxylate (120 mg, 0.190 mmol) in MeOH (10.0 mL) was added PtO₂ (43.0 mg, 0 mmol). The solution was stirred at 25 °C under H₂ for 12 h. The mixture was filtered to remove solids and concentrated. The residue was purified by silica gel chromatography eluting with MeOH/DCM (with MeOH from 0 to 5%) in 10 min to give tert-butyl 9-(7-(difluoromethyl)-6-(1-methyl-1H-pyrazol-4-yl)-3,4-dihydroquinolin-1(2H)-yl)-7-(1-ethyl-2-oxopiperidin-4-yl)-1,3,4,5-tetrahydro-2H-benzo[c]azepine-2-carboxylate (114 mg, 90% yield) as a yellow solid.

A solution of tert-butyl 9-(7-(difluoromethyl)-6-(1-methyl-1H-pyrazol-4-yl)-3,4-dihydroquinolin-1(2H)-yl)-7-(1-ethyl-2-oxopiperidin-4-yl)-1,3,4,5-tetrahydro-2H-benzo[c]azepine-2-carboxylate (114 mg, 0.179 mmol) in DCM (5.0 mL) and TFA (2.0 mL) was stirred at 25 °C for 1 h. The mixture was concentrated under reduced pressure to give 4-(9-(7-(difluoromethyl)-6-(1-methyl-1H-pyrazol-4-yl)-3,4-dihydroquinolin-1(2H)-yl)-2,3,4,5-tetrahydro-1H-benzo[c]azepin-7-yl)-1-ethylpiperidin-2-one (90.0 mg, 84% yield) as a yellow oil.

To a solution of 4-(9-(7-(difluoromethyl)-6-(1-methyl-1H-pyrazol-4-yl)-3,4-dihydroquinolin-1(2H)-yl)-2,3,4,5-tetrahydro-1H-benzo[c]azepin-7-yl)-1-ethylpiperidin-2-one (25.0 mg, 0.046 mmol) in DCM (5.0 mL) were added Et3N (14.0 mg, 0.0 mg, 0.140 mmol) and chloro(methoxy)methanone (13.0 mg, 0.140 mmol). The reaction solution was stirred at 25 °C for 1 h. The mixture was concentrated under reduced pressure, and the residue was purified by preparative HPLC (with CH₃CN from 50% to 80% in 8 min) to give methyl 9-(7-(difluoromethyl)-6-(1-methyl-1H-pyrazol-4-yl)-3,4-dihydroquinolin-1(2H)-yl)-7-(1-ethyl-2-oxopiperidin-4-yl)-1,3,4,5-tetrahydro-2H-benzo[c]azepine-2-carboxylate (4.4 mg, 16% yield) as a white solid.

### M001260:

¹H NMR (400 MHz, DMSO-*d₆*) δ 7.71 (s, 1H), 7.47 (s, 1H), 7.09 (d, *J* = 8.0 Hz, 2H), 7.02 (d, *J* = 8.0 Hz, 1H), 6.86-6.53 (m, 1H), 6.09 (d, *J =* 48.0 Hz, 1H), 4.85-3.91 (m, 2H), 3.85 (s, 3H), 3.69 (s, 1H), 3.54 (d, *J* = 28.0 Hz, 3H), 3.39-3.34 (m, 2H), 3.31-3.19 (m, 5H), 3.02-2.88 (m, 5H), 2.49-2.30 (m, 2H), 2.16-1.98 (m, 3H), 1.91-1.58 (m, 3H), 1.03-0.96 (m, 3H).

### Example 10

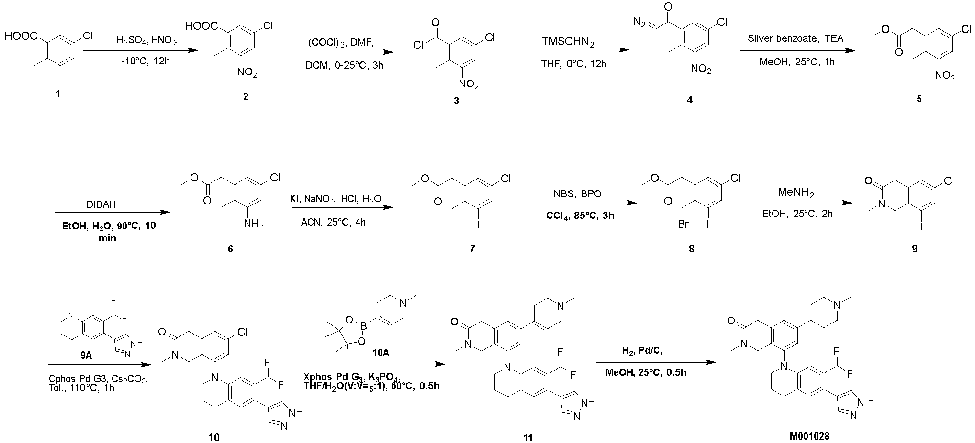

5-Chloro-2-methylbenzoic acid (20 g, 117.24 mmol) was added to cooled concentrated H₂SO₄ (135 mL) at -10 °C. After 10 min of stirring, a mixed solution of concentrated HNO3 (16.47 g, 261.44 mmol) and concentrated H₂SO₄ (22 mL) was added dropwise at -10 °C. The mixture was stirred at -10 °C for 12 h and poured into ice-cold water. The solid precipitated was collected by filtration and washed with water until the pH of the filtrate was about 7, and it was dried under vacuum to give 5-chloro-2-methyl-3-nitro-benzoic acid (15.5 g, crude) as an off-white solid.

To a solution of 5-chloro-2-methyl-3-nitro-benzoic acid (15.5 g, 71.90 mmol) in DCM (150 mL) at 0 °C were added dropwise oxalyl chloride (18.25 g, 143.79 mmol) and DMF (525.49 mg, 7.19 mmol). The mixture was stirred at 25 °C for 3 h. The reaction was concentrated under vacuum to give 5-chloro-2-methyl-3-nitro-benzoyl chloride (15.8 g, 67.51 mmol, crude) as a yellow solid.

To a solution of 5-chloro-2-methyl-3-nitro-benzoyl chloride (15.8 g, 67.51 mmol) in THF (150 mL) at 0 °C was added dropwise diazomethyl(trimethyl)silane (2 M, 135.02 mL) under N₂. The mixture was stirred at 25 °C for 12 h. The reaction was concentrated under vacuum to give 1-(5-chloro-2-methyl-3-nitro-phenyl)-2-diazo-ethanone (16 g, crude) as a yellow gum.

To a solution of TEA (33.78 g, 3.87 mmol) and silver benzoate (9.17 g, 40.06 mmol) in MeOH (60 mL) at 0 °C with stirring, 1-(5-chloro-2-methyl-3-nitro-phenyl)-2-diazo-ethanone (16 g, 66.77 mmol) dissolved in MeOH was added dropwise. The reaction mixture was stirred at 25 °C for 1 h. The mixture was washed with saturated aqueous HCl (1 M) (500 mL) and then extracted with EA (600 mL × 3). The organic layer was dried over anhydrous Na₂SO₄, filtered and concentrated under reduced pressure to give a residue. The crude product was purified by FCC (ISCO^{®}; 40 g SepaFlash^{®} silica column, 0-10% ethyl acetate/petroleum ether gradient eluent @ 50 mL/min), petroleum ether/ethyl acetate = 3:1, Rf = 0.7) to give methyl 2-(5-chloro-2-methyl-3-nitro-phenyl)acetate (12.1 g, 49.66 mmol, 74.37% yield) as a yellow solid.

To a solution of methyl 2-(5-chloro-2-methyl-3-nitro-phenyl)acetate (12.1 g, 49.66 mmol) in EtOH (100 mL) at 90 °C was added DIBAH (25.94 g, 148.99 mmol) dissolved in H₂O (30 mL). The mixture was stirred at 90 °C for 10 min. The mixture was washed with saturated H₂O (400 mL) and then extracted with EA (600 mL × 3). The organic layer was dried over anhydrous Na₂SO₄, filtered and concentrated under reduced pressure to give a residue. The crude product was purified by FCC (ISCO^{®}; 40 g SepaFlash^{®} silica column, 0-20% ethyl acetate/petroleum ether gradient eluent @ 40 mL/min), petroleum ether/ethyl acetate = 3:1, Rf = 0.4) to give methyl 2-(3-amino-5-chloro-2-methylphenyl)acetate (3.2 g, 14.98 mmol, 30.16% yield) as a white solid.

A solution of NaNO₂ (1.14 g, 16.47 mmol) in H₂O (15 mL) was added to a solution of methyl 2-(3-amino-5-chloro-2-methylphenyl)acetate (3.2 g, 14.98 mmol) in HCl (2 M, 14.98 mL), and ACN (10 mL) was cooled to below 5 °C. The mixture was stirred at 0 °C for 45 min and then poured dropwise into a solution of KI (3.73 g, 22.47 mmol) in H₂O (15 mL). The resulting mixture was warmed to 25 °C and stirred for 4 h. The mixture was poured into 150 mL of water and extracted with EA (300 mL × 3), and the combined organic layers were concentrated to give a crude product. The crude product was purified by FCC (ISCO^{®}; 20 g SepaFlash^{®} silica column, 0-10% ethyl acetate/petroleum ether gradient eluent 40 mL/min). TLC: petroleum:ethyl acetate = 3:1, Rf = 0.8) to give methyl 2-(5-chloro-3-iodo-2-methyl-phenyl)acetate (3.8 g, 11.71 mmol, 78.18% yield) as a white solid.

A mixture of methyl 2-(5-chloro-3-iodo-2-methyl-phenyl)acetate (3.8 g, 11.71 mmol), NBS (2.50 g, 14.05 mmol) in CCl4 (38 mL) was stirred at 0 °C. BPO (567.24 mg, 2.34 mmol) was then added to the mixture. The residue was degassed and purged with N₂ three times, and then the mixture was stirred at 85 °C under N₂ atmosphere for 3 h. The mixture was poured into water (500 mL) and extracted with DCM (500 mL × 3), and the combined organic layers were concentrated to give a crude product. The crude product was purified by FCC (ISCO^{®}; 40 g SepaFlash^{®} silica column, 0-10% ethyl acetate/petroleum ether gradient eluent 40 mL/min) to give methyl 2-[2-(bromomethyl)-5-chloro-3-iodo-phenyl]acetate (4.1 g, 10.16 mmol, 86.79% yield) as an off-white liquid.

To a solution of methyl 2-[2-(bromomethyl)-5-chloro-3-iodo-phenyl]acetate (4.1 g, 10.16 mmol) in EtOH (5 mL) at 25 °C was added MeNH₂ (10.52 g, 101.63 mmol). The mixture was stirred at 25 °C for 2 h. After the reaction was concentrated under vacuum, the crude product was purified by FCC (ISCO^{®}; 40 g SepaFlash^{®} silica column, 0-30% ethyl acetate/petroleum ether gradient eluent 40 mL/min). TLC: petroleum ether:ethyl acetate = 0:1, Rf = 0.5) to give 6-chloro-8-iodo-2-methyl-1,4-dihydroisoquinolin-3-one (2.32 g, 7.22 mmol, 71.00% yield) as an off-white solid.

6-Chloro-8-iodo-2-methyl-1,4-dihydroisoquinolin-3-one (500 mg, 1.56 mmol), 7-(difluoromethyl)-6-(1-methylpyrazol-4-yl)-1,2,3,4-tetrahydroquinoline (409.41 mg, 1.56 mmol), Cs₂CO₃ (1.52 g, 4.67 mmol) and toluene (5 mL) were degassed and purged with N₂ three times, then Cphos Pd G3 (125.39 mg, 0.156 mmol) was added, and the mixture was stirred at 110 °C under N₂ for 1 h. The reaction was concentrated under vacuum. The mixture was poured into 50 mL of water and extracted with EA (100 mL × 3), and the combined organic layers were concentrated to give a crude product. The residue was purified by silica gel flash chromatography (ISCO^{®}; 12 g SepaFlash^{®} silica column; eluent: 0-5% MeOH/DCM gradient @ 30 mL/min) to give 6-chloro-8-[7-(difluoromethyl)-6-(1-methylpyrazol-4-yl)-3,4-dihydro-2H-quinolin-1-yl]-2-methyl-1,4-dihydroisoquinoline-3-one (160 mg, 0.350 mmol, 22.52% yield) as a yellow oil.

A solution of 6-chloro-8-[7-(difluoromethyl)-6-(1-methylpyrazol-4-yl)-3,4-dihydro-2H-quinolin-1-yl]-2-methyl-1,4-dihydroisoquinolin-3-one (50 mg, 0109 mmol), 1-methyl-4-(4,4,5,5-tetramethyl-1,3,2-dioxaboran-2-yl)-3,6-dihydro-2H-pyridine (24.42 mg), K₃PO₄ (69.69 mg, 0.328 mmol) and XPhos Pd G3 (18.53 mg, 0.0219 mmol) in THF/H₂O (V:V = 5:1) (1 mL) was placed in a microwave tube and stirred at 60 °C under microwave for 0.5 h. The reaction mixture was concentrated under reduced pressure to give a residue. The residue was purified by preparative TLC (SiO2, PE:EA = 1:1) to give 8-[7-(difluoromethyl)-6-(1-methylpyrazol-4-yl)-3,4-dihydro-2H-quinolin-1-yl]-2-methyl-6-(1-ethyl-3,6-dihydro-2-pyridin-4-yl)-1,4-dihydroisoquinolin-3-one (35 mg, 0.0676 mmol, 61.79% yield) as a yellow gum.

A mixture of 8-[7-(difluoromethyl)-6-(1-methylpyrazol-4-yl)-3,4-dihydro-2H-quinolin-1-yl]-2-methyl-6-(1-methyl-3,6-dihydro-1H-pyridin-4-yl)-1,4-dihydroisoquinolin-3-one (35 mg, 0.0676 mmol), Pd/C (30 mg, 10% purity) in MeOH (1 mL) was degassed and purged with H₂ three times, and then stirred at 25 °C under H₂ atmosphere (15 Psi) for 0.5 h. The reaction mixture was filtered and concentrated under reduced pressure to give a residue. The product was then further purified by preparative HPLC (Welch Xtimate C18 150 × 25 mm × 5 mm, mobile phase A: water (FA), mobile phase B: acetonitrile, flow rate: 25 mL/min, gradient conditions: 5% B to 50%). The pure fractions were collected and the volatiles were removed under vacuum. The residue was partitioned between acetonitrile (2 mL) and water (10 mL). The solution was lyophilized to give 8-[7-(difluoromethyl)-6-(1-methylpyrazol-4-yl)-3,4-dihydro-2H-quinolin-1-yl]-2-methyl-6-(1-methyl-4-piperidyl)-1,4-dihydroisoquinolin-3-one (1.07 mg, 0.00192 mmol, 2.83% yield, 93% purity) as a yellow gum.

### M001028:

¹H NMR (400 MHz, METHANOL-d4) δ 8.49 (br s, 1 H) 7.62 (s, 1 H) 7.49 (s, 1 H) 7.10 - 7.16 (m, 3 H) 6.50 (t, *J =* 55.41 Hz, 1 H) 6.30 (s, 1 H) 4.54 - 4.63 (m, 1 H) 4.28 - 4.51 (m, 2 H) 3.93 (s, 3 H) 3.60 - 3.72 (m, 3 H) 3.46 - 3.55 (m, 3 H) 3.02 - 3.05 (m, 1 H) 3.00 (s, 3 H) 2.94 - 2.99 (m, 2 H) 2.86 - 2.93 (m, 1 H) 2.83 (s, 3 H) 2.07 - 2.20 (m, 4 H) 1.87 - 2.00 (m, 2 H).

### Example 11

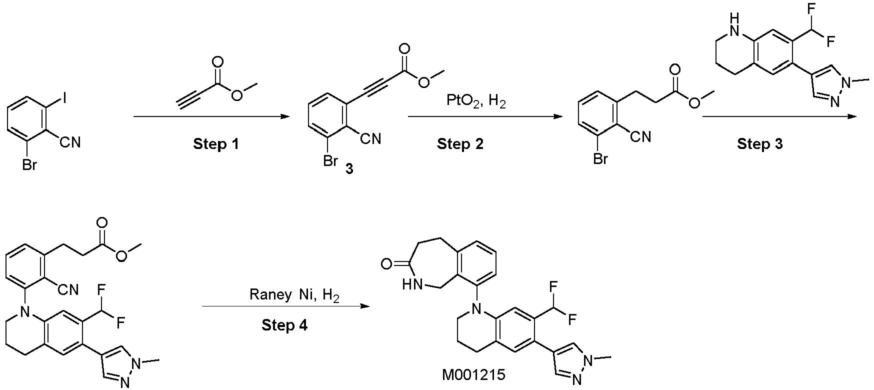

A mixture of 2-bromo-6-iodobenzonitrile (3.9 g, 0.012 mol), methyl propiolate (1.1 g, 0.012 mmol), Pd(PPh₃)₂Cl₂ (0.90 g, 0.0012 mol) and CuI (0.49 g, 0.0025 mol) in DMF (20 mL) and TEA (20 mL) was stirred at 25 °C under N₂. The mixture was heated to 50 °C and stirred at that temperature for 2 h. The reaction mixture was cooled to 25 °C, diluted with water (100 mL) and extracted with EtOAc (100 mL × 2). The combined organic phases were dried over Na₂SO₄ and filtered. The filtrate was concentrated under reduced pressure. The residue was purified by silica gel chromatography eluting with EtOAc/petroleum ether (with EtOAc from 0% to 100% in 15 min) to give methyl 3-(3-bromo-2-cyanophenyl)propanoate (1.5 g, 50% yield) as a yellow solid.

To a solution of methyl 3-(3-bromo-2-cyanophenyl)propiolate (2 g, 7.57 mmol) in EtOH (20 mL) and THF (20 mL) was added PtO₂ (171 mg, 0.75 mmol). The reaction mixture was stirred at 25 °C under hydrogen atmosphere (balloon) for 2 h. The reaction mixture was filtered through a pad of celite, and then the filtrate was concentrated under reduced pressure to give methyl 3-(3-bromo-2-cyanophenyl)propanoate (1.5 g, 66% yield) as a yellow solid.

A mixture of methyl 3-(3-bromo-2-cyanophenyl)propanoate (400 mg, 1.49 mmol), 7-(difluoromethyl)-6-(1-methylpyrazol-4-yl)-1,2,3,4-tetrahydroquinoline (314 mg, 1.19 mmol), Cs₂CO₃ (1458 mg, 4.4757 mmol), Pd2(dba)3 (136 mg, 0.14 mmol) and Xantphos (172 mg, 0.29 mmol) in dioxane (10 mL) was stirred at 25 °C under N₂. The mixture was heated to 100 °C and stirred at that temperature for 10 h. The reaction mixture was cooled to 25 °C, diluted with water (10 mL) and extracted with EtOAc (30 mL × 2). The combined organic phases were dried over Na₂SO₄ and filtered. The filtrate was concentrated under reduced pressure. The residue was purified by silica gel chromatography eluting with EtOAc/petroleum ether (with EtOAc from 0% to 100% in 15 min) to give methyl 3-{2-cyano-3-[7-(difluoromethyl)-6-(1-methylpyrazol-4-yl)-3,4-dihydro-2H-quinolin-1-yl]phenyl}propanoate (300 mg, 40% yield) as a yellow solid.

To a solution of methyl 3-{2-cyano-3-[7-(difluoromethyl)-6-(1-methylpyrazol-4-yl)-3,4-dihydro-2H-quinolin-1-yl]phenyl}propanoate (150 mg, 0.33 mmol) in MeOH (4 mL) at 25 °C was added Raney Ni (19 mg, 0.33 mmol). After the addition, the mixture was stirred at 25 °C under H₂ for 2 h. The reaction mixture was filtered through a pad of celite, and the filtrate was then concentrated under reduced pressure. The residue was purified by preparative HPLC (with CH₃CN from 10% to 70% in 10 min) to give 9-[7-(difluoromethyl)-6-(1-methylpyrazol-4-yl)-3,4-dihydro-2H-quinolin-1-yl]-1,2,4,5-tetrahydro-2-benzazepin-3-one (10 mg, 7% yield) as a white solid.

### M001215:

¹H NMR (400 MHz, DMSO-*d₆*) δ 7.87-7.84 (m, 1H), 7.72 (s, 1H), 7.47 (s, 1H), 7.35-7.30 (m, 1H), 7.25-7.20 (m, 1H), 7.15-7.05 (m, 2H), 6.80-6.55 (m, 1H), 6.16 (s, 1H), 4.30-4.13 (m, 2H), 3.88 (s, 3H), 3.50-3.45 (m, 1H), 3.25-3.15 (m, 1H), 3.02-2.80 (m, 3H), 2.72-2.60 (m, 1H), 2.54-2.50 (m, 2H), 2.13-2.03 (m, 2H).

### Example 12

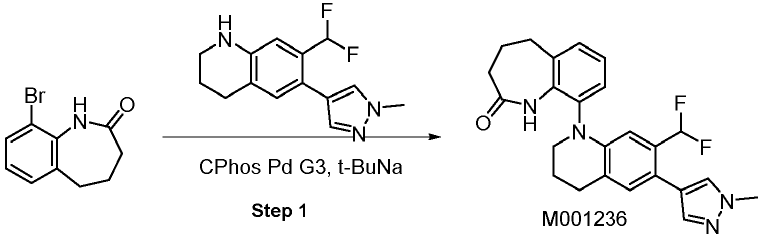

To a solution of 7-(difluoromethyl)-6-(1-methylpyrazol-4-yl)-1,2,3,4-tetrahydroquinoline (100 mg, 0.380 mmol) and 9-bromo-1,3,4,5-tetrahydro-1-benzazepin-2-one (109 mg, 0.456 mmol) in 1,4-dioxane (15 mL) were added t-BuONa (73.0 mg, 0.760 mmol) and Cphos-Pd G3 (30.6 mg, 0.0379 mmol). The reaction mixture was stirred at 90 °C for 16 h. The reaction mixture was added to water (20 mL). The aqueous phase was extracted with ethyl acetate (30 mL × 2). The combined organic phases were dried over sodium sulfate and filtered. The filtrate was concentrated under reduced pressure. The residue was purified by preparative HPLC (with CH₃CN from 45% to 75%) in 9 min to give 9-[7-(difluoromethyl)-6-(1-methylpyrazol-4-yl)-3,4-dihydro-2H-quinolin-1-yl]-1,3,4,5-tetrahydro-1-benzazepin-2-one (2.40 mg, 1.5% yield) as a white solid.

### M001236:

¹H NMR (400 MHz, DMSO-*d₆*) δ 8.90 (s, 1H), 7.71 (s, 1H), 7.46 (s, 1H), 7.24 (s, 3H), 7.08 (s, 1H), 6.70 (t, *J =* 55.2 Hz, 1H), 6.19 (s, 1H), 3.85 (s, 3H), 3.60-3.46 (m, 2H), 2.90-2.83 (m, 2H), 2.77-2.71 (m, 2H), 2.25-1.97 (m, 6H).

### Example 13

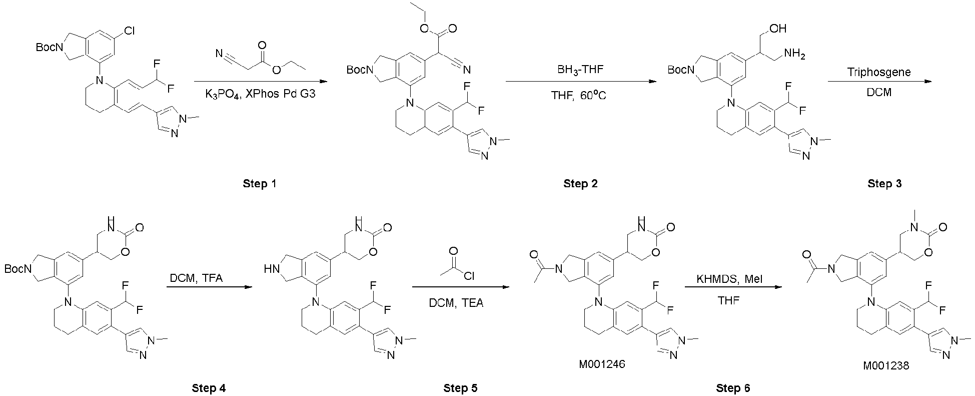

To a solution of tert-butyl 6-chloro-4-[7-(difluoromethyl)-6-(1-methylpyrazol-4-yl)-3,4-dihydro-2H-quinolin-1-yl]-1,3-dihydroisoindole-2-carboxylate (400 mg, 0.777 mmol) in dioxane (20 mL) at 25 °C were added ethyl 2-cyanoacetate (351 mg, 3.11 mmol), K₃PO₄ (495 mg, 2.33 mmol) and Xhos-Pd G3 (65.7 mg, 0.0776 mmol). The reaction mixture was stirred at 25 °C under N₂ for 2 h. The reaction mixture was added to water (20 mL). The aqueous phase was extracted with ethyl acetate (50 × 2 mL). The combined organic phases were washed with aqueous NaCl (10 mL × 2), dried over Na₂SO₄, filtered, and concentrated under reduced pressure. The residue was purified by silica gel chromatography eluting with EtOAc in petroleum from 0% to 50% to give tert-butyl 6-(1-cyano-2-ethoxy-2-oxoethyl)-4-[7-(difluoromethyl)-6-(1-methylpyrazol-4-yl)-3,4-dihydro-2H-quinolin-1-yl]-1,3-dihydroisoindole-2-carboxylate (440 mg, 95% purity, 91% yield) as a yellow solid.

To a solution of tert-butyl 6-(1-cyano-2-ethoxy-2-oxoethyl)-4-[7-(difluoromethyl)-6-(1-methylpyrazol-4-yl)-3,4-dihydro-2H-quinolin-1-yl)-1,3-dihydroisoindole-2-carboxylate (230 mg, 0.389 mol) in THF (35 mL) at 25 °C was added a borane-tetrahydrofuran complex (1 M, 23 mL). The reaction mixture was heated to 60 °C and stirred at 60 °C under nitrogen for 5 h. The reaction mixture was cooled to ambient temperature and quenched with MeOH until bubbling ceased. The mixture was concentrated under reduced pressure. The filtrate was quenched with water (50 mL) and then extracted with DCM (30 mL × 2). The combined organic phases were dried over sodium sulfate and filtered to give tert-butyl 6-(1-amino-3-hydroxypropan-2-yl)-4-[7-(difluoromethyl)-6-(1-methylpyrazol-4-yl)-3,4-dihydro-2H-quinolin-1-yl]-1,3-dihydroisoindole-2-carboxylate (180 mg, 75% yield) as a white solid.

To a solution of tert-butyl 6-(1-amino-3-hydroxypropan-2-yl)-4-[7-(difluoromethyl)-6-(1-methylpyrazol-4-yl)-3,4-dihydro-2H-quinolin-1-yl]-1,3-dihydroisoindole-2-carboxylate (180 mg, 0.0361 mmol) in DCM (60 mL) was added triphosgene (145 mg, 0.488 mmol). The reaction mixture was stirred at 25 °C under N₂ for 5 h. The mixture was quenched with water (30 mL) and then extracted with DCM (30 mL × 2). The combined organic phases were dried over sodium sulfate and filtered to give tert-butyl 4-[7-(difluoromethyl)-6-(1-methylpyrazol-4-yl)-3,4-dihydro-2H-quinolin-1-yl]-6-(2-oxo-1,3-oxazin-5-yl)-1,3-dihydroisoindole-2-carboxylate (105 mg, 50% yield) as a yellow solid. To a solution of tert-butyl 4-[7-(difluoromethyl)-6-(1-methylpyrazol-4-yl)-3,4-dihydro-2H-quinolin-1-yl]-6-(2-oxo-1,3-oxazin-5-yl)-1,3-dihydroisoindole-2-carboxylate (105 mg, 0.181 mmol) in DCM (6.0 mL) at 25 °C was added TFA (2.0 mL). The reaction mixture was stirred at 25 °C for 0.5 h. The filtrate was concentrated under reduced pressure to give 5-{7-[7-(difluoromethyl)-6-(1-methylpyrazol-4-yl)-3,4-dihydro-2H-quinolin-1-yl]-2,3-dihydro-1H-isoindol-5-yl}-1,3-oxazin-2-one (100 mg, crude) as a yellow oil. The product was directly used in the next step without further purification.

To a solution of 5-{7-[7-(difluoromethyl)-6-(1-methylpyrazol-4-yl)-3,4-dihydro-2H-quinolin-1-yl]-2,3-dihydro-1H-isoindol-5-yl}-1,3-oxazin-2-one (107 mg, 0.223 mmol) in DCM (10 mL) was added TEA (67.7 mg, 0.670 mmol). The mixture was stirred at 25 °C for 10 min. The mixture was concentrated under reduced pressure. The residue was purified by Prep-HPLC (with CH₃CN from 28% to 58% in 9 min) to give 5-{2-acetyl-7-[7-(difluoromethyl)-6-(1-methylpyrazol-4-yl)-3,4-dihydro-2H-quinolin-1-yl]-1,3-dihydroisoindol-5-yl}-1,3-oxazin-2-one (8.70 mg, 7.5% yield) as a white solid.

### M001246:

¹H NMR (400 MHz, DMSO-*d₆*) δ 7.75 (s, 1H), 7.50 (s, 1H), 7.42-7.35 (m, 1H), 7.29-7.22 (m, 2H), 7.14 (s, 1H), 6.75 (t, *J* = 55.2 Hz, 1H), 6.44-6.35 (m, 1H), 4.90-4.84 (m, 1H), 4.76-4.45 (m, 2H), 4.40-4.23 (m, 3H), 3.86 (s, 3H), 3.60-3.51 (m, 2H), 3.44-3.37 (m, 3H), 2.91-2.83 (s, 2H), 2.05-1.96 (m, 5H).

To a solution of 5-{2-acetyl-7-[7-(difluoromethyl)-6-(1-methylpyrazol-4-yl)-3,4-dihydro-2H-quinolin-1-yl]-1,3-dihydroisoindol-5-yl}-1,3-oxazin-2-one (20.0 mg, 0.0383 mmol) in THF (6.0 mL) at -60 °C was added dropwise KHMDS (1 M in THF, 0.1 mL, 0.0766 mmol) under N₂ atmosphere. After 10 min of stirring, a solution of iodomethane (8.15 mg, 0.0574 mmol) in THF (0.5 mL) was added dropwise at -30 °C under N₂ atmosphere. After the mixture was stirred at 10 °C for another 20 min, it was quenched with a NH4Cl solution (20 mL) and then extracted with ethyl acetate (20 mL × 2). The combined organic phases were dried over sodium sulfate and filtered. The filtrate was concentrated under reduced pressure. The mixture was concentrated under reduced pressure, and the residue was purified by Prep-HPLC (with CH₃CN from 35% to 45% in 8 min) to give 5-{2-acetyl-7-[7-(difluoromethyl)-6-(1-methylpyrazol-4-yl)-3,4-dihydro-2H-quinolin-1-yl]-1,3-dihydroisoindol-5-yl}-3-methyl-1,3-oxazin-2-one (5.00 mg, 23% yield) as a yellow solid.

### M001238:

¹H NMR (400 MHz, DMSO-*d₆*) δ 7.75 (s, 1H), 7.50 (s, 1H), 7.30-7.22 (m, 2H), 7.14 (s, 1H), 6.75 (t, *J =* 55.0 Hz, 1H), 6.45-6.33 (m, 1H), 4.89-4.85 (m, 1H), 4.69-4.54 (m, 2H), 4.38-4.25 (m, 3H), 3.86 (s, 3H), 3.60-3.47 (m, 5H), 2.91-2.84 (m, 5H), 2.04 (s, 3H), 2.01-1.93 (m, 2H).

### Example 14

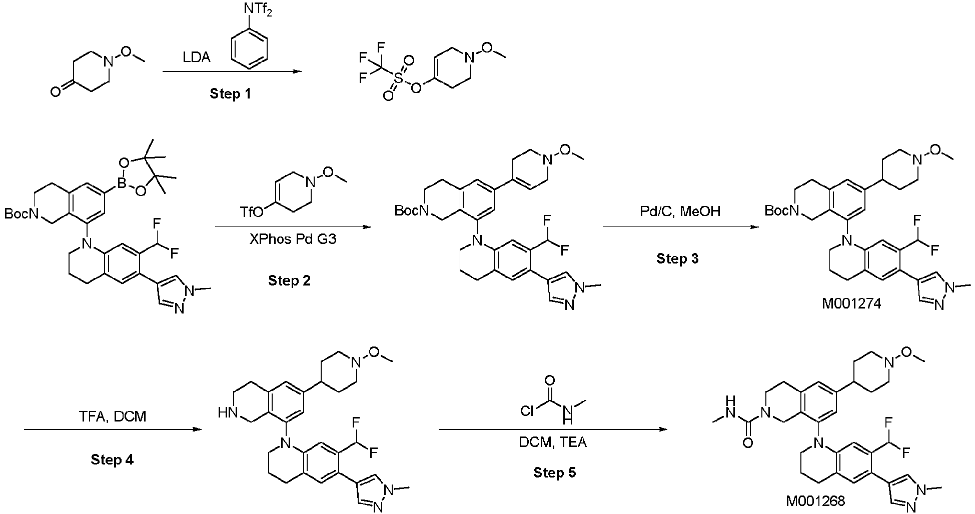

A solution of 1-methoxypiperidin-4-one (500 mg, 3.87 mmol) in THF (15 mL) was cooled to -78 °C, and LDA (2 M, 2.5 mL, 5.03 mmol) was added under N₂. The resulting mixture was stirred at the same temperature for 30 min and treated by adding a solution of 1,1,1-trifluoro-N-phenyl-N-(trifluoromethane)sulfonylmethanesulfonamide (1.80 g, 5.03 mmol) in THF (15 mL). The reaction mixture was stirred for another 30 min and warmed to 25 °C. The mixture was quenched with water (50 mL) and then extracted with ethyl acetate (60 mL × 2). The combined organic phases were dried over sodium sulfate and filtered. The filtrate was concentrated under reduced pressure. The residue was purified by silica gel chromatography eluting with 0% to 5% tetrahydrofuran in petroleum to give 1-methoxy-3,6-dihydro-2H-pyridin-4-yl trifluoromethanesulfonate (687 mg, 90% purity, 61% yield) as a colorless oil.

To a solution of tert-butyl 8-[7-(difluoromethyl)-6-(1-methylpyrazol-4-yl)-3,4-dihydro-2H-quinolin-1-yl]-6-(4,4,5,5-tetramethyl-1,3,2-dioxaborolan-2-yl)-3,4-dihydro-1H-isoquinoline-2-carboxylate (234 mg, 0.377 mmol) and 1-methoxy-3,6-dihydro-2H-pyridin-4-yl trifluoromethanesulfonate (197 mg, 0.754 mmol) in dioxane (8.0 mL) and H₂O (0.8 mL) at 25 °C were added K₂CO₃ (156 mg, 1.13 mmol) and XPhos Pd G3 (32.0 mg, 0.0377 mmol). The reaction mixture was heated to 100 °C and stirred at 100 °C under nitrogen for 2 h. The mixture was concentrated under reduced pressure. The residue was purified by silica gel chromatography eluting with methanol in DCM from 0% to 5% to give tert-butyl 8-[7-(difluoromethyl)-6-(1-methylpyrazol-4-yl)-3,4-dihydro-2H-quinolin-1-yl]-6-(1-methoxy-3,6-dihydro-2H-pyridin-4-yl)-3,4-dihydro-1H-isoquinoline-2-carboxylate (210 mg, 90% purity, 83% yield) as a brown solid.

To a solution of tert-butyl 8-[7-(difluoromethyl)-6-(1-methylpyrazol-4-yl)-3,4-dihydro-2H-quinolin-1-yl]-6-(1-methoxy-3,6-dihydro-2H-pyridin-4-yl)-3,4-dihydro-1H-isoquinoline-2-carboxylate (120 mg, 0.198 mmol) in MeOH (10 mL) was added Pd/C (21.1 mg, 0.198 mmol). The mixture was stirred at 25 °C under H₂ balloon pressure for 16 h. The mixture was filtered to remove solids and concentrated. The residue was purified by Prep-HPLC (with CH₃CN from 70% to 95% in 9 min) to give tert-butyl 8-[7-(difluoromethyl)-6-(1-methylpyrazol-4-yl)-3,4-dihydro-2H-quinolin-1-yl]-6-(1-methoxypiperidin-4-yl)-3,4-dihydro-1H-isoquinoline-2-carboxylate (70 mg, 57% yield) as a white solid.

### M001274:

¹H NMR (400 MHz, DMSO-*d₆*) δ 7.71 (s, 1H), 7.46 (s, 1H), 7.11-7.00 (m, 3H), 6.69 (t, *J* = 55.2 Hz, 1H), 6.16 (s, 1H), 4.44-4.32 (m, 1H), 4.28-4.06 (m, 1H), 3.86 (s, 3H), 3.59-3.48 (m, 3H), 3.44-3.40 (3, 3H), 3.39-3.35 (m, 2H), 2.95-2.79 (m, 4H), 2.45-2.30 (m, 2H), 2.07-1.99 (m, 2H), 1.90-1.76 (m, 2H), 1.75-1.59 (m, 2H), 1.49-1.17 (m, 11H). To a solution of tert-butyl 8-[7-(difluoromethyl)-6-(1-methylpyrazol-4-yl)-3,4-dihydro-2H-quinolin-1-yl]-6-(1-methoxypiperidin-4-yl)-3,4-dihydro-1H-isoquinoline-2-carboxylate (76.0 mg, 0.125 mmol) in DCM (6.0 mL) at 25 °C was added TFA (2.0 mL). The reaction mixture was stirred at 25 °C for 0.5 h. The mixture was concentrated under reduced pressure to give 7-(difluoromethyl)-1-[6-(1-methoxypiperidin-4-yl)-1,2,3,4-tetrahydroisoquinolin-8-yl]-6-(1-methylpyrazol-4-yl)-3,4-dihydro-2H-quinoline (65 mg, 97% yield) as a yellow oil. The product was directly used in the next step without further purification.

To a solution of 7-(difluoromethyl)-1-[6-(1-methoxypiperidin-4-yl)-1,2,3,4-tetrahydroisoquinolin-8-yl]-6-(1-methylpyrazol-4-yl)-3,4-dihydro-2H-quinoline (64.0 mg, 0.126 mmol) in DCM (6.0 mL) at 25 °C were added TEA (38.3 mg, 0.378 mmol) and N-methylcarbamoyl chloride (17.7 mg, 0.189 mmol). The mixture was stirred at 25 °C for 10 min. The mixture was quenched with water (10 mL) and then extracted with ethyl acetate (20 mL × 2). The combined organic phases were dried over sodium sulfate and filtered. The residue was purified by Prep-HPLC (with CH₃CN from 50% to 60% in 9 min) to give 8-[7-(difluoromethyl)-6-(1H-pyrazol-4-yl)-3,4-dihydro-2H-quinolin-1-yl]-6-(1-methoxypiperidin-4-yl)-N-methyl-3,4-dihydro-1H-isoquinoline-2-carboxamide (26.9 mg, 38% yield) as a white solid.

### M001268:

¹H NMR (400 MHz, DMSO-*d₆*) δ 7.72 (s, 1H), 7.47 (s, 1H), 7.08 (s, 1H), 7.04 (s, 1H), 6.99 (s, 1H), 6.69 (t, *J* = 55.2 Hz, 1H), 6.51-6.42 (m, 1H), 6.18 (s, 1H), 4.46-4.36 (m, 1H), 4.17-4.05 (m, 1H), 3.86 (s, 3H), 3.57-3.35 (m, 9H), 3.03-2.70 (m, 5H), 2.54 (d, *J=* 4.0 Hz, 3H), 2.42-2.28 (m, 2H), 2.10-1.99 (m, 2H), 1.90-1.76 (m, 2H), 1.74-1.56 (m, 2H).

### Example 15

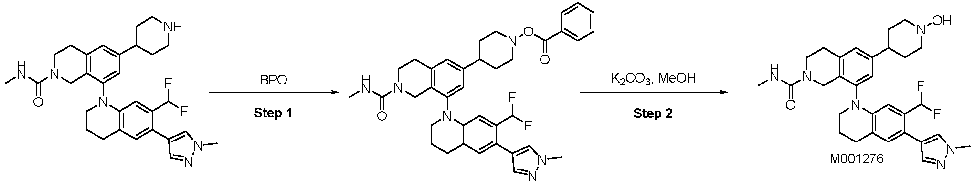

To a solution of 8-[7-(difluoromethyl)-6-(1-methylpyrazol-4-yl)-3,4-dihydro-2H-quinolin-1-yl]-N-methyl-6-(piperidin-4-yl)-3,4-dihydro-1H-isoquinoline-2-carboxamide (50.0 mg, 0.0935 mmol) in THF (2.0 mL) at 25 °C was added benzoyl peroxide (34.0 mg, 0.140 mmol). The reaction mixture was stirred at 25 °C for 2 h. The reaction mixture was purified by preparative TLC (PE/THF = 3:7) to give 4-{8-[7-(difluoromethyl)-6-(1-methylpyrazol-4-yl)-3,4-dihydro-2H-quinolin-1-yl]-2-(methylcarbamoyl)-3,4-dihydro-1H-isoquinolin-6-yl} piperidin-1-yl benzoate (20.0 mg, 29% yield) as a white solid.

To a solution of 4-{8-[7-(difluoromethyl)-6-(1-methylpyrazol-4-yl)-3,4-dihydro-2H-quinolin-1-yl]-2-(methylcarbamoyl)-3,4-dihydro-1H-isoquinolin-6-yl}piperidin-1-yl benzoate (20.0 mg, 0.0305 mmol) in MeOH (2 mL) at 25 °C was added K₂CO₃ (12.7 mg, 0.0915 mmol). The reaction mixture was stirred at 25 °C for 1 h. The mixture was quenched with water (10 mL) and then extracted with ethyl acetate (20 mL × 2). The combined organic phases were dried over sodium sulfate and filtered. The filtrate was concentrated under reduced pressure. The residue was purified by Prep-HPLC (with CH₃CN from 20% to 50%) in 10 min to give 8-[7-(difluoromethyl)-6-(1-methylpyrazol-4-yl)-3,4-dihydro-2H-quinolin-1-yl]-6-(1-hydroxypiperidin-4-yl)-N-methyl-3,4-dihydro-1H-isoquinoline-2-carboxamide (4.00 mg, 24% yield) as a white solid.

### M001276:

¹H NMR (400 MHz, DMSO-*d₆*) δ 7.93 (s, 1H), 7.71 (s, 1H), 7.47 (s, 1H), 7.08 (s, 1H), 7.04 (s, 1H), 6.99 (s, 1H), 6.83-6.45 (m, 2H), 6.18 (s, 1H), 4.46-4.38 (m, 1H), 4.15-4.06 (m, 1H), 3.86 (s, 3H), 3.55-3.41 (m, 4H), 3.25-3.05 (m, 2H), 3.00-2.77 (m, 5H), 2.54 (d, *J* = 4.2 Hz, 3H), 2.45-2.33 (m, 2H), 2.10-2.00 (m, 2H), 1.85-1.40 (m, 4H).

### Example 16

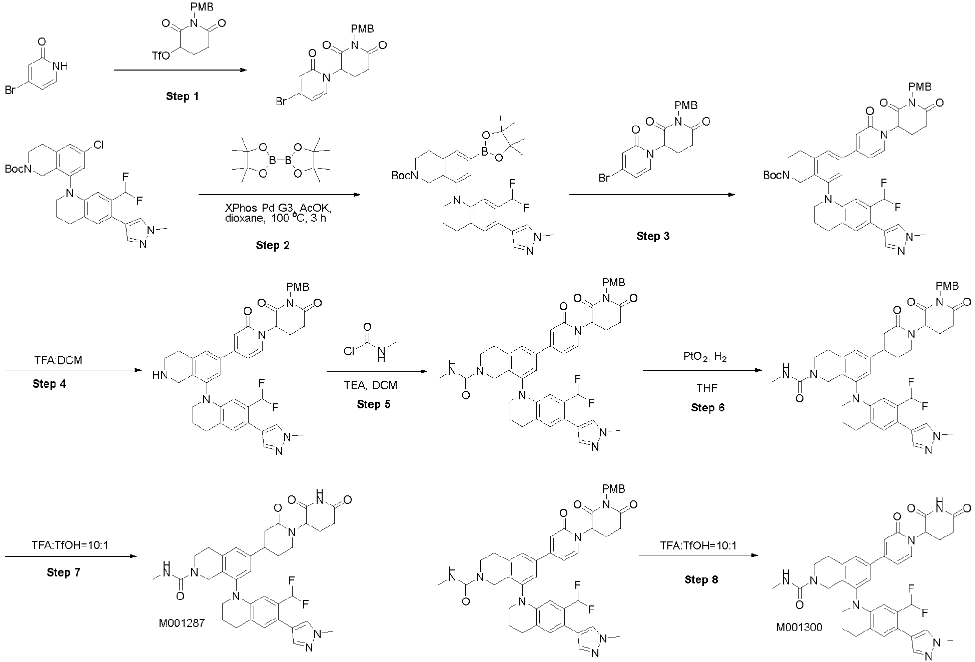

To a solution of 4-bromo-1H-pyridin-2-one (200 mg, 1.15 mmol) in THF (8.0 mL) at 0 °C was added t-BuOK (1 M in THF, 1.8 mL, 1.8 mmol). The mixture was stirred at 0 °C for 0.5 h. A solution of 1-(2-methoxy-5-methylphenyl)-2,6-dioxopiperidin-3-yl trifluoromethanesulfonate (526.0 mg, 1.4 mmol) in THF (2.0 mL) was then added to the reaction mixture at 0 °C. The mixture was stirred at 0 °C for 0.5 h. The reaction was concentrated under reduced pressure and purified by silica gel chromatography eluting with EtOAc in PE from 0% to 50% to give 3-(4-bromo-2-oxopyridin-1(2H)-yl)-1-(4-methoxybenzyl)piperidine-2,6-dione (300 mg, 64% yield) as a yellow solid.

To a solution of tert-butyl 6-chloro-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (300 mg, 0.57 mmol) in dioxane (4.0 mL) were added 4,4,5,5-tetramethyl-2-(4,4,5,5-tetramethyl-1,3,2-dioxaborolan-2-yl)-1,3,2-dioxaborolane (288.0 mg, 1.13 mmol), AcOK (167.0 mg, 1.70 mmol) and XPhos Pd G3 (95.9 mg, 0.11 mmol). The mixture was then stirred at 100 °C for 2 h. LCMS showed the starting materials were consumed and the desired product was detected. After filtration, the filtrate was concentrated under reduced pressure to give tert-butyl 8-(7-(difluoromethyl)-6-(1-methyl-1H-pyrazol-4-yl)-3,4-dihydroquinolin-1(2H)-yl)-6-(4,4,5,5-tetramethyl-1,3,2-dioxaborolan-2-yl)-3,4-dihydroisoquinoline-2(1H)-carboxylate (300 mg, 85% yield) as a yellow oil.

To a solution of tert-butyl 8-(7-(difluoromethyl)-6-(1-methyl-1H-pyrazol-4-yl)-3,4-dihydroquinolin-1(2H)-yl)-6-(4,4,5,5-tetramethyl-1,3,2-dioxaborolan-2-yl)-3,4-dihydroisoquinoline-2(1H)-carboxylate (298.6 mg, 0.48 mmol) in dioxane (5.0 mL) and H₂O (0.5 mL) were added 3-(4-bromo-2-oxopyridin-1(2H)-yl)-1-(4-methoxybenzyl) piperidine-2,6-dione (130.0 mg, 0.32 mmol), K₃PO₄ (204.3 mg, 0.96 mmol) and XPhos Pd G3 (54.3 mg, 0.06 mmol). The mixture was then stirred at 100 °C for 2 h. LCMS showed the starting materials were consumed and the desired product was detected. The residue was purified by flash column chromatography eluting with EtOAc in PE from 0% to 50% and then by flash column chromatography eluting with methanol in DCM from 0% to 5% to give tert-butyl 8-(7-(difluoromethyl)-6-(1-methyl-1H-pyrazol-4-yl)-3,4-dihydroquinolin-1(2H)-yl)-6-(1-(4-methoxybenzyl)-2,6-dioxopiperidin-3-yl)-2-oxo-1,2-dihydropyridin-4-yl)-3,4-dihydroisoquinoline-2(1H)-carboxylate (160 mg, 91% yield) as a yellow solid.

To a solution of tert-butyl 8-(7-(difluoromethyl)-6-(1-methyl-1H-pyrazol-4-yl)-3,4-dihydroquinolin-1(2H)-yl)-6-(1-(1-(4-methoxybenzyl)-2,6-dioxopiperidin-3-yl)-2-oxo-1,2-dihydropyridin-4-yl)-3,4-dihydroisoquinoline-2(1H)-carboxylate (300 mg, 0.37 mmol) in DCM (6.0 mL) was added TFA (2.0 mL). The mixture was then stirred at 25 °C for 0.5 h. LCMS showed the starting materials were consumed and the desired product was detected. The resulting mixture was concentrated under reduced pressure to give 3-(4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-2-oxopyridin-1(2H)-yl)-1-(4-methoxybenzyl)piperidine-2,6-dione (250 mg, 95% yield) as a yellow solid.

To a solution of 3-(4-(8-(7-(difluoromethyl)-6-(1-methyl-1H-pyrazol-4-yl)-3,4-dihydroquinolin-1(2H)-yl)-1,2,3,4-tetrahydroisoquinolin-6-yl)-2-oxopyridin-1(2H)-yl)-1-(4-methoxybenzyl)piperidine-2,6-dione (200 mg, 0.42 mmol) in DCM (2.0 mL) at 25 °C were added TEA (126.7 mg, 1.3 mmol) and N-methylcarbamoyl chloride (78.1 mg, 0.83 mmol). The mixture was then stirred at 25 °C for 0.5 h. The reaction was quenched by adding water (50 mL), extracted with DCM (50 mL × 3), and dried over anhydrous Na₂SO₄. After filtration, the filtrate was concentrated under reduced pressure to give 8-(7-(difluoromethyl)-6-(1-methyl-1H-pyrazol-4-yl)-3,4-dihydroquinolin-1(2H)-yl)-6-(1-(4-methoxybenzyl)-2,6-dioxopiperidin-3-yl)-2-oxo-1,2-dihydropyridin-4-yl)-N-methyl-3,4-dihydroisoquinoline-2(1H)-carboxamide (130 mg, 60% yield) as a yellow solid.

To a solution of 8-(7-(difluoromethyl)-6-(1-methyl-1H-pyrazol-4-yl)-3,4-dihydroquinolin-1(2H)-yl)-6-(1-(4-methoxybenzyl)-2,6-dioxopiperidin-3-yl)-2-oxo-1,2-dihydropyridin-4-yl)-N-methyl-3,4-dihydroisoquinoline-2(1H)-carboxamide (50 mg, 0.06 mmol) in THF (1.0 mL) at 25 °C was added PtO₂ (14.6 mg, 0.06 mmol) under H₂ atmosphere (15 PSI). The mixture was then stirred at 25 °C for 12 h. LCMS showed the starting materials were consumed and the desired product was detected. After filtration, the filtrate was concentrated under reduced pressure to give 8-(7-(difluoromethyl)-6-(1-methyl-1H-pyrazol-4-yl)-3,4-dihydroquinolin-1(2H)-yl)-6-(1'-(4-methoxybenzyl)-2,2',6'-trioxy-[1,3'-bipiperidin]-4-yl)-N-methyl-3,4-dihydroisoquinoline-2(1H)-carboxamide (40 mg, 80% yield) as a yellow solid.

To a solution of 8-(7-(difluoromethyl)-6-(1-methyl-1H-pyrazol-4-yl)-3,4-dihydroquinolin-1(2H)-yl)-6-(1'-(4-methoxybenzyl)-2,2',6'-trioxy-[1,3'-bipiperidin]-4-yl)-N-methyl-3,4-dihydroisoquinoline-2(1H)-carboxamide (50 mg, 0.06 mmol) in TFA (2.0 mL) was added TfOH (0.2 mL). The mixture was then stirred at 60 °C for 6 h. The reaction was purified by preparative HPLC eluting with CH3CN in 0.1% FA/water from 27% to 57% in 7 min to give 8-(7-(difluoromethyl)-6-(1-methyl-1H-pyrazol-4-yl)-3,4-dihydroquinolin-1(2H)-yl)-N-methyl-6-(2,2',6'-trioxy-[1,3'-bipyridin]-4-yl)-3,4-dihydroisoquinoline-2(1H)-carboxamide (0.9 mg, 3% yield) as a white solid.

### M001287:

¹H NMR (400 MHz, DMSO-*d₆*) δ 10.80 (s, 1H), 7.71 (s, 1H), 7.47 (s, 1H), 7.10 (d, *J* = 11.2 Hz, 3H), 6.70 (t, *J* = 55.2 Hz, 1H), 6.48-6.45 (m, 1H), 6.18 (s, 1H), 4.44 (d, *J =* 16.8 Hz, 2H), 4.40-4.29 (m, 1H), 4.11 (d, *J =* 16.8 Hz, 2H), 3.86 (s, 3H), 3.57-3.51 (m, 2H), 3.50-3.42 (m, 2H), 3.40-3.35 (m, 2H), 3.30-3.16 (m, 2H), 3.16-3.05 (m, 2H), 2.97-2.77 (m, 5H), 2.55 (*d, J =* 4.4 Hz, 3H), 2.14-1.87 (m, 4H).

To a solution of 8-(7-(difluoromethyl)-6-(1-methyl-1H-pyrazol-4-yl)-3,4-dihydroquinolin-1(2H)-yl)-6-(1-(4-methoxybenzyl)-2,6-dioxopiperidin-3-yl)-2-oxo-1,2-dihydropyridin-4-yl)-N-methyl-3,4-dihydroisoquinoline-2(1H)-carboxamide (50 mg, 0.06 mmol) in TFA (2.0 mL) was added TfOH (0.2 mL). The mixture was then stirred at 60 °C for 6 h. The reaction was purified by preparative HPLC eluting with CH₃CN in 0.1% FA/water from 26% to 56% in 7 min to give 8-(7-(difluoromethyl)-6-(1-methyl-1H-pyrazol-4-yl)-3,4-dihydroquinolin-1(2H)-yl)-6-(1-(2,6-dioxopiperidin-3-yl)-2-oxo-1,2-dihydropyridin-4-yl)-N-methyl-3,4-dihydroisoquinoline-2(1H)-carboxamide (5.3 mg, 13% yield) as a white solid.

### M001300:

¹H NMR (400 MHz, DMSO-*d₆*) δ 11.03 (s, 1H), 7.73-7.70 (m, 2H), 7.59 (s, 1H), 7.55 (s, 1H), 7.48 (s, 1H), 7.12 (s, 1H), 6.76 (t, *J* = 55.1 Hz, 1H), 6.73 (s, 1H), 6.69-6.63 (m, 1H), 6.57-6.51 (m, 1H), 6.21 (s, 1H), 4.52 (d, *J* = 17.2 Hz, 2H), 4.17 (d, *J* = 17.2 Hz, 2H), 3.86 (s, 3H), 3.69-3.54 (m, 2H), 3.54-3.41 (m, 2H), 3.04-2.82 (m, 4H), 2.61 (s, 1H), 2.56 *(d, J =* 4.4 Hz, 3H), 2.10-2.00 (m, 4H).

### Example 17

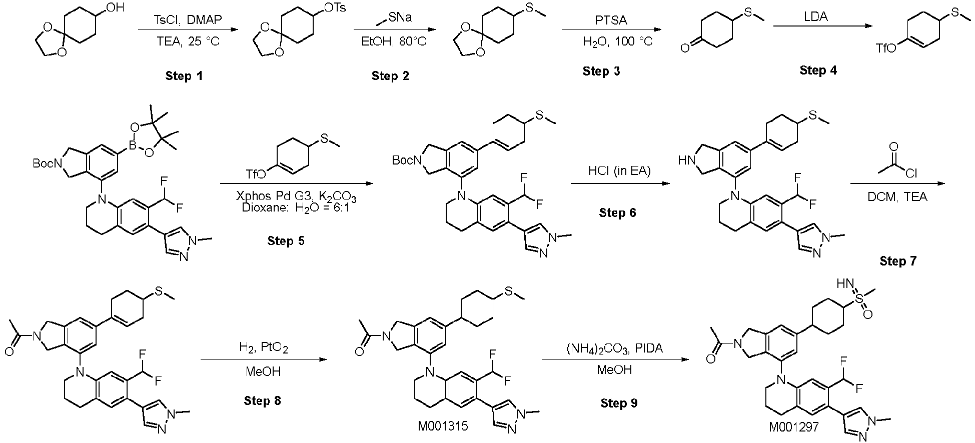

To a solution of 1,4-dioxaspiro[4.5]decan-8-ol (10 g, 0.063 mol), 4-DMAP (0.77 g, 0.0063 mol) and TEA (19 g, 0.19 mol) in DCM (200 mL) was added TsCl (18 g, 0.095 mol). The reaction mixture was stirred at 25 °C for 16 h. LCMS showed the desired product was formed. The solvent was removed under reduced pressure. The residue was purified by flash chromatography eluting with EtOAc in PE from 0% to 30% to give 1,4-dioxaspiro[4.5]dec-8-yl 4-methylbenzenesulfonate (10 g, 48%) as a white solid.

To a solution of 1,4-dioxaspiro[4.5]dec-8-yl 4-methylbenzenesulfonate (5.0 g, 0.02 mol) in EtOH (50 mL) was added methyl sodium sulfate (2.2 g, 0.032 mol). The reaction mixture was stirred at 80 °C for 30 min. The solvent was removed under reduced pressure. The solid was dissolved in water and extracted with EtOAc (200 mL × 2). The combined organic layers were dried over Na₂SO₄, filtered and concentrated under reduced pressure. The residue was purified by flash chromatography eluting with EA in PE from 0% to 20% to give 8-(methylthio)-1,4-dioxaspiro[4.5]decane (2.2 g, 55%) as a yellow oil.

To a solution of 8-(methylsulfonyl)-1,4-dioxaspiro[4.5]decane (2.2 g, 5.9 mmol) in H₂O (5.0 mL) was added PTSA (2.2 g, 11.6 mmol). The reaction mixture was stirred at 100 °C for 16 h. The reaction was extracted with EtOAc (200 mL). The organic layer was dried over Na₂SO₄, filtered, and concentrated under reduced pressure. The residue was purified by flash chromatography eluting with EA in PE from 0% to 20% to give 4-(methylthio)cyclohexan-1-one (1.2 g, 78%) as a yellow oil.

To a solution of 4-(methylthio)cyclohexan-1-one (1.2 g, 8.4 mmol) in THF (15 mL) at -78 °C was added LDA (4.5 mL, 2 M THF, 9.0 mmol). The reaction mixture was stirred at the same temperature for 1.0 h, and then 1,1,1-trifluoro-N-phenyl-N-((trifluoromethyl)sulfonyl)methanesulfonamide (3.2 g, 9.0 mmol) was added. The reaction mixture was stirred at 25 °C for 16 h. The reaction mixture was quenched with NH₄Cl (aq) and extracted with EA (200 mL × 2). The combined organic layers were dried over Na₂SO₄, filtered, and concentrated under reduced pressure. The residue was purified by flash chromatography eluting with EA in PE from 0% to 3% to give 4-(methylthio)cyclohex-1-en-1-yl trifluoromethanesulfonate (0.8 g, 35%) as a pale yellow oil.

To a solution of tert-butyl 4-(7-(difluoromethyl)-6-(1-methyl-1H-pyrazol-4-yl)-3,4-dihydroquinolin-1(2H)-yl)-6-(4,4,5,5-tetramethyl-1,3,2-dioxaborolan-2-yl)isoindoline-2-carboxylate (1.0 g, 1.6 mmol) and tert-butyl 4-(methylthio)cyclohex-1-en-1-yl trifluoromethanesulfonate (0.46 g, 1.6 mmol) in dioxane (20.0 mL) and H₂O (3 mL) were added Xphos 1 Pd G3 (0.13 g, 0.16 mmol) and K₂CO₃ (0.34 g, 2.5 mmol). The reaction mixture was stirred at 100 °C for 2.0 h. The solvent was removed under reduced pressure. The residue was purified by flash chromatography eluting with EtOAc in PE from 0% to 60% to give tert-butyl 4-(7-(difluoromethyl)-6-(1-methyl-1H-pyrazol-4-yl)-3,4-dihydroquinolin-1(2H)-yl)-6-(4-(methylthio)cyclohex-1-en-1-yl)isoindoline-2-carboxylate (0.50 g, 50%) as a pale yellow solid.

A solution of tert-butyl 4-(7-(difluoromethyl)-6-(1-methyl-1H-pyrazol-4-yl)-3,4-dihydroquinolin-1(2H)-yl)-6-(4-(methylthio)cyclohex-1-en-1-yl)isoindoline-2-carboxylate (0.50 g, 0.80 mmol) in HCl (3.0 mL, 4 M EA) was stirred at 25 °C for 30 min. LCM showed the target product was formed. The solid was removed under reduced pressure, and 7-(difluoromethyl)-6-(1-methyl-1H-pyrazol-4-yl)-1-(6-(4-(methylthio)cyclohex-1-en-1-yl)isoindol-4-yl)-1,2,3,4-tetrahydroquinoline (0.46 g, crude) was obtained as a white solid.

To a solution of 7-(difluoromethyl)-6-(1-methyl-1H-pyrazol-4-yl)-1-(6-(4-(methylthio)cyclohex-1-en-1-yl)isoindol-4-yl)-1,2,3,4-tetrahydroquinoline (0.46 g, 0.92 mmol) and TEA (0.28 g, 2.7 mmol) in DCM (5.0 mL) was added acetyl chloride (0.14 g, 12.2 mmol). The reaction mixture was stirred at 25 °C for 30 min. The reaction was quenched with water and extracted with DCM (100 mL). The organic layer was dried over Na₂SO₄, filtered, and concentrated under reduced pressure to give 1-(4-(7-(difluoromethyl)-6-(1-methyl-1H-pyrazol-4-yl)-3,4-dihydroquinolin-1(2H)-yl)-6-(4-(methylthio)cyclohex-1-en-1-yl)isoindolin-2-yl)ethan-1-one (0.42 g, 86%) as a white solid.

To a solution of 1-(4-(7-(difluoromethyl)-6-(1-methyl-1H-pyrazol-4-yl)-3,4-dihydroquinolin-1(2H)-yl)-6-(4-(methylthio)cyclohex-1-en-1-yl)isoindol-2-yl)ethan-1-one (0.42 g, 0.77 mmol) in MeOH (5.0 mL) was added PtO₂ (0.42 g). The reaction mixture was stirred at 25 °C under H₂ atmosphere for 48 h. PtO₂ was filtered out, and the solvent was removed under reduced pressure. The residue was purified by Pre-HPLC (with CH₃CN from 33% to 43% in 2 min) to give 1-(4-(7-(difluoromethyl)-6-(1-methyl-1H-pyrazol-4-yl)-3,4-dihydroquinolin-1(2H)-yl)-6-(4-(methylthio)cyclohexyl)isoindol-2-yl)ethan-1-one (0.1 g, 24%) as a white solid.

### M001315:

¹H NMR (400 MHz, DMSO-*d₆*) δ 7.74 (s, 1H), 7.49 (s, 1H), 7.15-7.07 (m, 3H), 6.87 (s, 0.25 H), 6.74 (s, 0.50 H), 6.60 (s, 0.25 H), 6.45-6.33 (m, 1H), 4.90-4.50 (m, 3H), 4.28 (s, 1H), 3.86 (s, 3H), 3.54 (d, *J =* 4.8 Hz, 2H), 2.87 (s, 2H), 2.64-2.53 (m, 2H), 2.10-1.96 (m, 10H), 1.87 (d, *J* = 11.6 Hz, 2H), 1.60-1.47 (m, 2H), 1.38 (d, *J =* 9.6 Hz, 2H). To a solution of 1-(4-(7-(difluoromethyl)-6-(1-methyl-1H-pyrazol-4-yl)-3,4-dihydroquinolin-1(2H)-yl)-6-(4-(methylthio)cyclohexyl)isoindol-2-yl)ethan-1-one (90 mg, 0.17 mmol) and ammonium carbamate (19 mg, 0.25 mmol) in MeOH (3.0 mL) was added PIDA (113 mg, 0.35 mmol). The reaction mixture was stirred at 0 °C for 30 min. LCMS showed the target product was formed. The reaction was quenched with water and extracted with EA (10 mL × 3). The combined organic layers were dried overNa₂SO₄, filtered, and concentrated under reduced pressure. The residue was purified by Pre-HPLC (with CH₃CN from 20% to 35% in 3 min) to give (4-(2-acetyl-7-(7-(difluoromethyl)-6-(1-methyl-1H-pyrazol-4-yl)-3,4-dihydroquinolin-1(2H)-yl)isoindol-5-yl)cyclohexyl)(imino)(methyl)-16-sulfanone (16 mg, 17%) as a pale yellow solid.

### M001297:

¹H NMR (400 MHz, DMSO) δ 7.74 (s, 1H), 7.49 (s, 1H), 7.13 (d, *J* = 6.0 Hz, 3H), 6.88 (s, 0.25 H), 6.74 (s, 0.50 H), 6.61 (s, 0.25 H), 6.46-6.34 (m, 1H), 4.95-4.55 (m, 3H), 4.29 (s, 1H), 3.86 (s, 3H), 3.54 (s, 3H), 2.98 (s, 1H), 2.88 (s, 2H), 2.83 (d, *J=* 4.0 Hz, 3H), 2.58 (s, 1H), 2.21 (s, 2H), 2.05-1.90 (m, 7H), 1.65-1.45 (m, 4H).

### Example 18

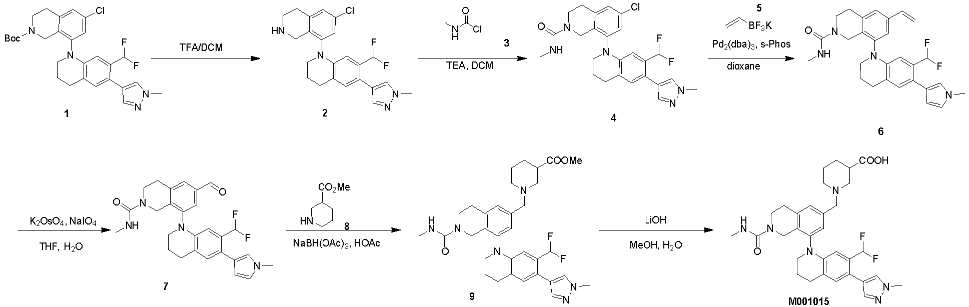

To a solution of tert-butyl 6-chloro-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (120 mg, 226.83 µmol) in DCM (4 mL) was added TFA (1.54 g, 13.51 mmol). The mixture was stirred at 25 °C for 0.5 h. The mixture was concentrated to give compound 2 (120 mg, 221.02 µmol, 97.4% yield, TFA) as a yellow solid.

To a solution of methyl 1-((8-(7-(difluoromethyl)-6-(1-methyl-1H-pyrazol-4-yl)-3,4-dihydroquinolin-1(2H)-yl)-1,2,3,4-tetrahydroisoquinolin-6-yl)methyl)piperidine-3-carboxylate (50 mg, 75.34 µmol, TFA) in DCM (3 mL) were added TEA (22.87 mg, 226.01 µmol) and methylcarbamoyl chloride (17.61 mg, 188.34 µmol). The mixture was stirred at 15 °C for 16 h. The mixture was extracted with DCM (10 mL) and washed with brine (5 mL). The organic layer was dried over Na₂SO₄, filtered and concentrated to give compound 4 (40 mg, 65.93 µmol, 87.5% yield) as a yellow oil.

A solution of tert-butyl 6-chloro-8-(7-(difluoromethyl)-6-(1-methyl-1H-pyrazol-4-yl)-3,4-dihydroquinolin-1(2H)-yl)-3,4-dihydroisoquinoline-2(1H)-carboxylate (0.7 g, 1.32 mmol), potassium vinyltrifluoroborate (265.86 mg, 1.98 mmol), Pd2(dba)3 (121.17 mg, 132.32 µmol), s-Phos (108.64 mg, 264.64 µmol) and t-BuONa (381.49 mg, 3.97 mmol) in dioxane (10 mL) was degassed and purged with N₂ three times, and the mixture was then stirred at 100 °C under N₂ atmosphere for 16 h. The mixture was extracted with EtOAc (20 mL) and washed with brine (10 mL). The organic layer was dried over Na₂SO₄, filtered and concentrated to give a crude product. The crude product was purified by silica gel column chromatography (petroleum ether/ethyl acetate from 100% to 70%) to give compound 6 (593 mg, 1.14 mmol, 86.3% yield) as a yellow oil.

To a solution of tert-butyl 8-(7-(difluoromethyl)-6-(1-methyl-1H-pyrrol-3-yl)-3,4-dihydroquinolin-1(2H)-yl)-6-ethenyl-3,4-dihydroisoquinoline-2(1H)-carboxylate (593 mg, 1.14 mmol) in H₂O (2 mL) and THF (8 mL) was added K₂OsO4 (21.71 mg, 114.12 µmol). The mixture was cooled to 0 °C, and then NaIO4 (488.19 mg, 2.28 mmol, 126.47 µL, 2 eq) was added. The mixture was then heated to 15 °C and stirred for 16 h. The mixture was extracted with EtOAc (20 mL) and washed with brine (10 mL). The organic layer was dried over Na₂SO₄, filtered and concentrated to give a crude product. The crude product was purified by silica gel column chromatography (petroleum ether/ethyl acetate from 100% to 30%) to give compound 7 (451 mg, 864.65 µmol, 75.8% yield) as a yellow oil.

To a solution of methyl 1-((8-(7-(difluoromethyl)-6-(1-methyl-1H-pyrazol-4-yl)-3,4-dihydroquinolin-1(2H)-yl)-1,2,3,4-tetrahydroisoquinolin-6-yl)methyl)piperidine-3-carboxylate (50 mg, 75.34 µmol, TFA) in DCM (3 mL) were added TEA (22.87 mg, 226.01 µmol) and methylcarbamoyl chloride (17.61 mg, 188.34 µmol). The mixture was stirred at 15 °C for 16 h. The mixture was extracted with DCM (10 mL) and washed with brine (5 mL). The organic layer was dried over Na₂SO₄, filtered and concentrated to give compound 6 (40 mg, 65.93 µmol, 87.5% yield, crude) as a yellow oil.

To a solution of methyl 1-((8-(7-(difluoromethyl)-6-(1-methyl-1H-pyrazol-4-yl)-3,4-dihydroquinolin-1(2H)-yl)-2-(methylcarbamoyl)-1,2,3,4-tetrahydroisoquinolin-6-yl)methyl)piperidine-3-carboxylate (40 mg, 65.93 µmol) in MeOH (2 mL) and H₂O (1 mL) was added LiOH (4.74 mg, 197.79 µmol). The mixture was stirred at 15 °C for 0.5 h. The mixture was concentrated to give a crude product, and the crude product was purified by prep-HPLC (FA) to give M001015 (3.25 mg, 5.48 µmol, 8.3% yield) as a gray solid.

### M001015:

¹H NMR (400MHz, CD₃Cl) δ 8.23 (br s, 1H), 7.72 (s, 1H), 7.47 (s, 1H), 7.34 -6.96 (m, 3H), 6.88 - 6.09 (m, 1H), 6.18 (s, 1H), 4.56 - 4.07 (m, 3H), 3.85 (s, 3H),3.01 - 2.63 (m, 8H), 2.59 - 2.53 (m, 6H), 2.42 - 2.28 (m, 2H), 2.18 - 1.89 (m, 4H),1.83 - 1.51 (m, 2H), 1.45 - 1.30 (m, 1H).

### Example 19

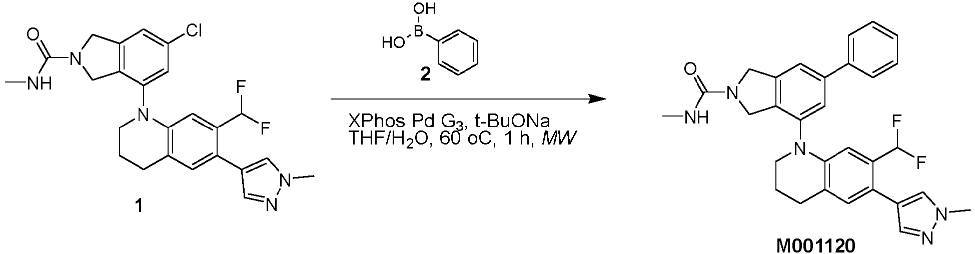

Phenylboronic acid (19.38 mg, 158.92 µmol), 6-chloro-4-(7-(difluoromethyl)-6-(1-methyl-1H-pyrazol-4-yl)-3,4-dihydroquinolin-1(2H)-yl)-N-methylisoindoline-2-carboxamide (50 mg, 105.95 µmol), XPhos Pd G3 (8.97 mg, 10.59 µmol) and K₃PO₄ (67.47 mg, 317.84 µmol) were dissolved in THF (2 mL) and H₂O (0.5 mL) and then placed in a microwave tube. The microwave tube was heated at 60 °C under microwave for 1 h. The mixture was extracted with EtOAc (10 mL) and washed with brine (5 mL × 2). The organic layer was dried over Na₂SO₄, filtered and concentrated to give a crude product. The crude product was purified by silica gel column chromatography (petroleum ether/ethyl acetate from 100% to 0%) to give M001120 (3.66 mg, 7.13 µmol, 6.7% yield) as a yellow solid.

### M001120:

¹H NMR (400MHz, DMSO-*d₆*) δ 7.75 (s, 1H), 7.69 (br d, *J* = 7.70 Hz, 2H), 7.56 (s,1H), 7.48-7.51 (m, 2H), 7.44-7.47 (m, 1H), 7.33-7.41 (m, 1H), 7.14 (s, 1H), 6.60-6.91 (m, 1H), 6.29-6.46 (m, 2H), 4.68 (br s, 2H), 4.26-4.47 (m, 2H), 3.85 (s, 3H), 3.63 (br s, 1H), 2.86-2.93 (m, 2H), 2.55-2.63 (m, 4H), 1.98-2.09 (m, 2H)

### Example 20

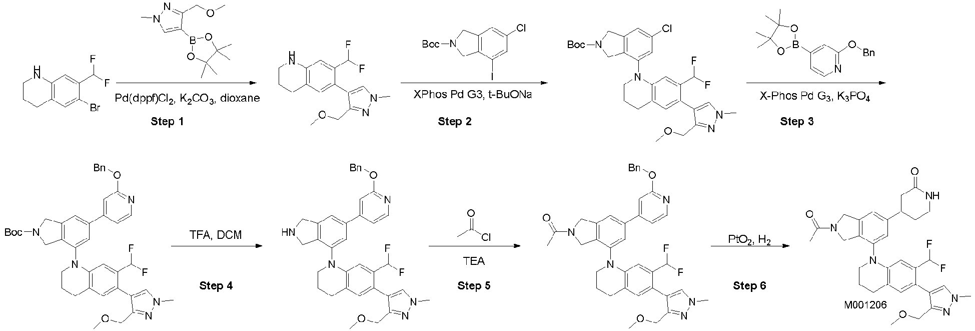

To a solution of 6-bromo-7-(difluoromethyl)-1,2,3,4-tetrahydroquinoline (600 mg, 2.30 mmol) and 3-(methoxymethyl)-1-methyl-4-(4,4,5,5-tetramethyl-1,3,2-dioxaborolan-2-yl)pyrazole (635 mg, 2.52 mmol) in dioxane (20 mL) and H₂O (2.0 mL) were added K₂CO₃ (949 mg, 6.87 mmol) and Pd(dppf)Cl2 (168 mg, 0.230 mmol). The reaction mixture was stirred at 100 °C under N₂ for 2 h. The mixture was quenched with water (50 mL) and then extracted with ethyl acetate (80 mL × 2). The combined organic phases were washed with aqueous NaCl (10 mL × 2), dried over Na₂SO₄, filtered, and concentrated under reduced pressure. The filtrate was concentrated under reduced pressure. The residue was purified by silica gel chromatography eluting with EtOAc in petroleum from 0% to 30% to give 7-(difluoromethyl)-6-[3-(methoxymethyl)-1-methylpyrazol-4-yl]-1,2,3,4-tetrahydroquinoline (700 mg, 85% yield) as a yellow oil.

To a solution of 7-(difluoromethyl)-6-[3-(methoxymethyl)-1-methylpyrazol-4-yl]-1,2,3,4-tetrahydroquinoline (797 mg, 2.59 mmol) and tert-butyl (6-chloro-4-iodo-1,3-dihydroisoindol-2-yl)carboxylate (1974 mg, 5.19 mmol) in dioxane (60 mL) at 25 °C were added t-BuONa (748 mg, 7.78 mmol) and XPhos Pd G3 (219 mg, 0.259 mmol). The reaction mixture was stirred at 90 °C for 16 h. The mixture was quenched with water (50 mL) and then extracted with ethyl acetate (80 mL × 2). The combined organic phases were dried over sodium sulfate and filtered. The filtrate was concentrated under reduced pressure. The residue was purified by silica gel chromatography eluting with THF in PE from 0% to 25% to give tert-butyl {6-chloro-4-[7-(difluoromethyl)-6-[3-(methoxymethyl)-1-methylpyrazol-4-yl]-3,4-dihydro-2H-quinolin-1-yl]-1,3-dihydroisoindol-2-yl}carboxylate (1.01 g, 66% yield) as a brown solid.

To a solution of tert-butyl {6-chloro-4-[7-(difluoromethyl)-6-[3-(methoxymethyl)-1-methylpyrazol-4-yl]-3,4-dihydro-2H-quinolin-1-yl]-1,3-dihydroisoindol-2-yl}carboxylate (200 mg, 0.357 mmol) and 2-(1-methylphenoxy)-4-(4,4,5,5-tetramethyl-1,3,2-dioxaborolan-2-yl)pyridine (167 mg, 0.536 mmol) in THF/H₂O = 10:1 (11 mL) at 25 °C were added K₃PO₄ (227 mg, 1.07 mmol) and XPhos Pd G3 (60.4 mg, 0.0714 mmol). The reaction mixture was stirred at 60 °C under N₂ for 2 h. The mixture was quenched with water (50 mL) and then extracted with ethyl acetate (80 mL × 2). The combined organic phases were dried over sodium sulfate and filtered. The filtrate was concentrated under reduced pressure. The residue was purified by silica gel chromatography eluting with THF in petroleum from 0% to 60% to give tert-butyl {4-[7-(difluoromethyl)-6-[3-(methoxymethyl)-1-methylpyrazol-4-yl]-3,4-dihydro-2H-quinolin-1-yl]-6-[2-(1-methylphenoxy)pyridin-4-yl]-1,3-dihydroisoindol-2-yl}carboxylate (113 mg, **44%** yield) as a brown solid.

To a solution of tert-butyl {4-[7-(difluoromethyl)-6-[3-(methoxymethyl)-1-methylpyrazol-4-yl]-3,4-dihydro-2H-quinolin-1-yl]-6-[2-(1-methylphenoxy) pyridin-4-yl]-1,3-dihydroisoindol-2-yl}carboxylate (113 mg, 0.159 mmol) in DCM (6.0 mL) at 25 °C was added TFA (2.0 mL). The reaction mixture was stirred at 25 °C for 0.5 h. The mixture was concentrated under reduced pressure to give 7-(difluoromethyl)-6-[3-(methoxymethyl)-1-methylpyrazol-4-yl]-1-{6-[2-(1-methylphenoxy)pyridin-4-yl]-2,3-dihydro-1H-isoindol-4-yl}-3,4-dihydro-2H-quinoline (120 mg, 92% yield) as a brown oil. The product was directly used in the next step without further purification.

To a solution of 7-(difluoromethyl)-6-[3-(methoxymethyl)-1-methylpyrazol-4-yl]-1-{6-[2-(1-methylphenoxy)pyridin-4-yl]-2,3-dihydro-1H-isoindol-4-yl}-3,4-dihydro-2H-quinoline (97.0 mg, 0.159 mmol) in DCM (5.0 mL) were added TEA (48.4 mg, 0.478 mmol) and acetyl chloride (18.8 mg, 0.239 mmol). The reaction mixture was stirred at 25 °C for 10 min. The mixture was quenched with water (20 mL) and then extracted with ethyl acetate (50 mL × 2). The combined organic phases were dried over sodium sulfate and filtered. The filtrate was concentrated under reduced pressure. The residue was purified by silica gel chromatography eluting with methanol in DCM from 0% to 5% to give 1-{4-[7-(difluoromethyl)-6-[3-(methoxymethyl)-1-methylpyrazol-4-yl]-3,4-dihydro-2H-quinolin-1-yl]-6-[2-(1-methylphenoxy)pyridin-4-yl]-1,3-dihydroisoindol-2-yl}ethanone (71.0 mg, 62% yield) as a yellow solid.

To a solution of 1-{4-[7-(difluoromethyl)-6-[3-(methoxymethyl)-1-methylpyrazol-4-yl]-3,4-dihydro-2H-quinolin-1-yl]-6-[2-(1-methylphenoxy)pyridin-4-yl]-1,3-dihydroisoindol-2-yl}ethene (99.0 mg, 0.155 mmol) in MEOH (8.0 mL) was added PtO₂ (34.5 mg, 0.152 mmol). The mixture was stirred at 25 °C under H₂ balloon pressure for 16 h. The mixture was filtered to remove solids and concentrated. The residue was purified by Prep-HPLC (with CH₃CN from 40% to 70% in 10 min) to give 4-{2-acetyl-7-[7-(difluoromethyl)-6-[3-(methoxymethyl)-1-methylpyrazol-4-yl]-3,4-dihydro-2H-quinolin-1-yl]-1,3-dihydroisoindol-5-yl}piperidin-2-one (15.1 mg, 18% yield) as a white solid.

### M001206:

¹H NMR (400 MHz, DMSO-d6) δ 7.63-7.52 (m, 2H), 7.25- 7.14 (m, 2H), 7.09- 7.03 (m, 1H), 6.65 (d, J = 55.4 Hz, 1H), 6.45- 6.35 (m, 1H), 4.90- 4.84 (m, 1H), 4.74- 4.50 (m, 2H), 4.37- 4.27 (m, 1H), 4.18 (s, 2H), 3.84 (s, 3H), 3.62-3.54 (m, 2H), 3.22- 3.17 (m, 4H), 3.14- 3.02 (m, 2H), 2.90- 2.84 (m, 2H), 2.37-2.30 (m, 2H), 2.05-1.87 (m, 7H).

### Example 21

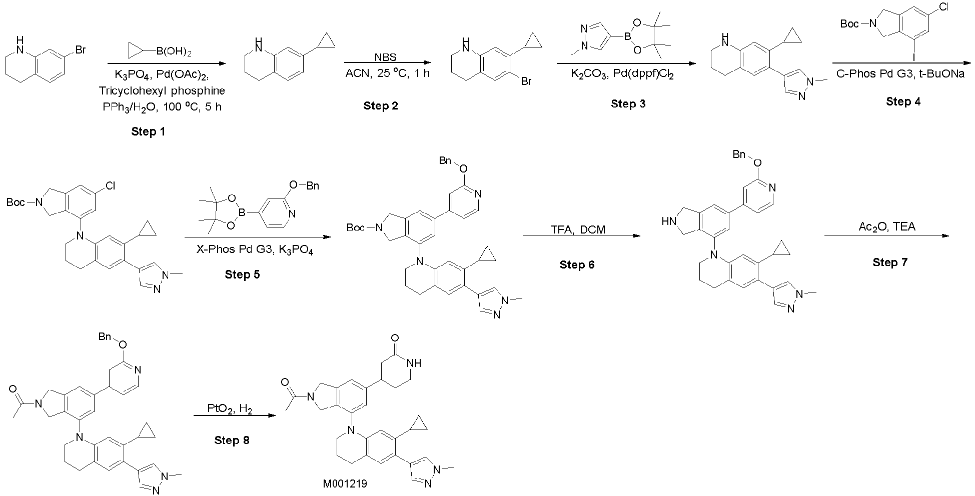

To a solution of 7-bromo-1,2,3,4-tetrahydroquinoline (2.0 g, 0.0094 mol) and cyclopropylboranediol (1.6 g, 0.019 mol) in toluene/water (11 mL) were added K₃PO₄ (6.0 g, 0.028 mol), tricyclohexyl phosphine (0.3 g, 0.0009 mol) and Pd(OAc)₂ (0.2 g, 0.0009 mol). The mixture was stirred at 100 °C for 5 h. The mixture was concentrated under reduced pressure. The residue was purified by silica gel chromatography eluting with EtOAc/petroleum ether (with EtOAc from 0% to 20% in 15 min) to give 7-cyclopropyl-1,2,3,4-tetrahydroquinoline (1.2 g, 66% yield) as a yellow gum.

To a solution of 7-cyclopropyl-1,2,3,4-tetrahydroquinoline (1.1 g, 0.0062 mol) in ACN (20 mL) at 0 °C was added N-bromosuccinimide (1.2 g, 0.0065 mol). The mixture was stirred at 25 °C for 1 h. The mixture was concentrated under reduced pressure. The residue was purified by silica gel chromatography eluting with EtOAc/petroleum ether (with EtOAc from 0% to 30% in 20 min) to give 6-bromo-7-cyclopropyl-1,2,3,4-tetrahydroquinoline (1.3 g, 74% yield) as a yellow liquid.

To a solution of 6-bromo-7-cyclopropyl-1,2,3,4-tetrahydroquinoline (0.9 g, 0.0054 mol) and 1-methyl-4-(4,4,5,5-tetramethyl-1,3,2-dioxaborolan-2-yl)pyrazole (1.1 g, 0.0054 mmol) in dioxane/water (22 mL) were added K₂CO₃ (1.5 g, 0.011 mol) and Pd(dppf)Cl2 (0.3 g, 0.0003 mmol). The mixture was stirred at 90 °C for 5 h. The mixture was concentrated under reduced pressure. The residue was purified by silica gel chromatography eluting with EtOAc/petroleum ether (with EtOAc from 0% to 50% in 20 min) to give 7-cyclopropyl-6-(1-methylpyrazol-4-yl)-1,2,3,4-tetrahydroquinoline (0.7 g, 64% yield) as a yellow gum.

To a solution of 7-cyclopropyl-6-(1-methylpyrazol-4-yl)-1,2,3,4-tetrahydroquinoline (300 mg, 3.2 mmol) and tert-butyl (6-chloro-4-iodo-1,3-dihydroisoindol-2-yl)carboxylate (1322 mg, 3.5 mmol) in dioxane (30 mL) were added Cphos-Pd G3 (509 mg, 0.63 mmol) and t-BuONa (607 mg, 6.3 mmol). The mixture was stirred at 90 °C for 2 h. The mixture was concentrated under reduced pressure. The residue was purified by silica gel chromatography eluting with EtOAc/petroleum ether (with EtOAc from 0% to 50% in 15 min) to give tert-butyl {6-chloro-4-[7-cyclopropyl-6-(1-methylpyrazol-4-yl)-3,4-dihydro-2H-quinolin-1-yl]-1,3-dihydroisoindol-2-yl}carboxylate (400 mg, 23% yield) as a yellow gum.

To a solution of tert-butyl {6-chloro-4-[7-cyclopropyl-6-(1-methylpyrazol-4-yl)-3,4-dihydro-2H-quinolin-1-yl]-1,3-dihydroisoindol-2-yl}carboxylate (200 mg, 0.40 mmol) and 2-(1-methylphenoxy)-4-(4,4,5,5-tetramethyl-1,3,2-dioxaborolan-2-yl)pyridine (247 mg, 0.79 mmol) in dioxane/water (11 mL) were added K₂CO₃ (252 mg, 1.2 mmol) and X-Phos Pd G3 (67 mg, 0.079 mmol). The mixture was stirred at 90 °C for 2 h. The mixture was concentrated under reduced pressure. The residue was purified by silica gel chromatography eluting with EtOAc/petroleum ether (with EtOAc from 0% to 50% in 15 min) to give tert-butyl {4-[7-cyclopropyl-6-(1-methylpyrazol-4-yl)-3,4-dihydro-2H-quinolin-1-yl]-6-[2-(1-methylphenoxy)pyridin-4-yl]-1,3-dihydroisoindol-2-yl}carboxylate (100 mg, 35% yield) as a yellow gum.

A mixture of tert-butyl {4-[7-cyclopropyl-6-(1-methylpyrazol-4-yl)-3,4-dihydro-2H-quinolin-1-yl]-6-[2-(1-methylphenoxy)pyridin-4-yl]-1,3-dihydroisoindol-2-yl}carboxylate (100 mg, 0.15 mmol) in DCM/TFA (6 mL) was stirred at 25 °C for 0.1 h. The mixture was concentrated under reduced pressure to give 7-cyclopropyl-1-{6-[2-(1-methylphenoxy)pyridin-4-yl]-2,3-dihydro-1H-isoindol-4-yl}-6-(1-methylpyrazol-4-yl)-3,4-dihydro-2H-quinoline (80 mg, 85% yield) as a yellow gum.

To a solution of 7-cyclopropyl-1-{6-[2-(1-methylphenoxy)pyridin-4-yl]-2,3-dihydro-1H-isoindol-4-yl}-6-(1-methylpyrazol-4-yl)-3,4-dihydro-2H-quinoline (80 mg, 0.14 mmol) and TEA (44 mg, 0.43 mmol) in DCM (10 mL) was added acetyl chloride (23 mg, 0.29 mmol). The mixture was stirred at 25 °C for 0.1 h and concentrated under reduced pressure. The residue was purified by silica gel chromatography eluting with EtOAc/petroleum ether (with EtOAc from 0% to 50% in 10 min) to give 1-{4-[7-cyclopropyl-6-(1-methylpyrazol-4-yl)-3,4-dihydro-2H-quinolin-1-yl]-6-[2-(1-methylphenoxy)pyridin-4-yl]-1,3-dihydroisoindol-2-yl}ethenone (70 mg, 73% yield) as a yellow gum. To a solution of 1-{4-[7-cyclopropyl-6-(1-methylpyrazol-4-yl)-3,4-dihydro-2H-quinolin-1-yl]-6-[2-(1-methylphenoxy)pyridin-4-yl]-1,3-dihydroisoindol-2-yl}ethene (70 mg, 0.12 mmol) in MeOH (5 mL) was added PtO₂ (27 mg, 0.12 mmol). The mixture was stirred at 25 °C for 16 h. The mixture was filtered and concentrated under reduced pressure. The residue was purified by Pre-HPLC eluting with CH₃CN in water containing 40% to 50% CH₃CN in 10 min to give 4-{2-acetyl-7-[7-cyclopropyl-6-(1-methylpyrazol-4-yl)-3,4-dihydro-2H-quinolin-1-yl]-1,3-dihydroisoindol-5-yl}piperidin-2-one (30 mg, 49% yield) as a white solid.

### M001219:

¹H NMR (400 MHz, DMSO-d6) δ 7.84 (s, 1H), 7.60 (s, 1H), 7.56 (s, 1H), 7.14-7.01 (m, 2H), 7.02 (s, 1H), 5.88-5.82 (m,, 1H), 4.85 (s, 1H), 4.64 (s, 1H), 4.59 (s, 1H), 4.26 (s, 1H), 3.85 (s, 3H), 3.49 (d, J = 5.2 Hz, 2H), 3.20 (s, 2H), 3.07 (s, 1H), 2.79-2.78 (m, 2H), 2.36-2.29 (m, 2H), 2.04 (d, J = 5.2 Hz, 1H), 2.00-1.92 (m, 4H), 1.87-1.84 (m, 3H), 0.77-0.69 (m, 2H), 0.21-0.13 (m, 2H).

### Example 22

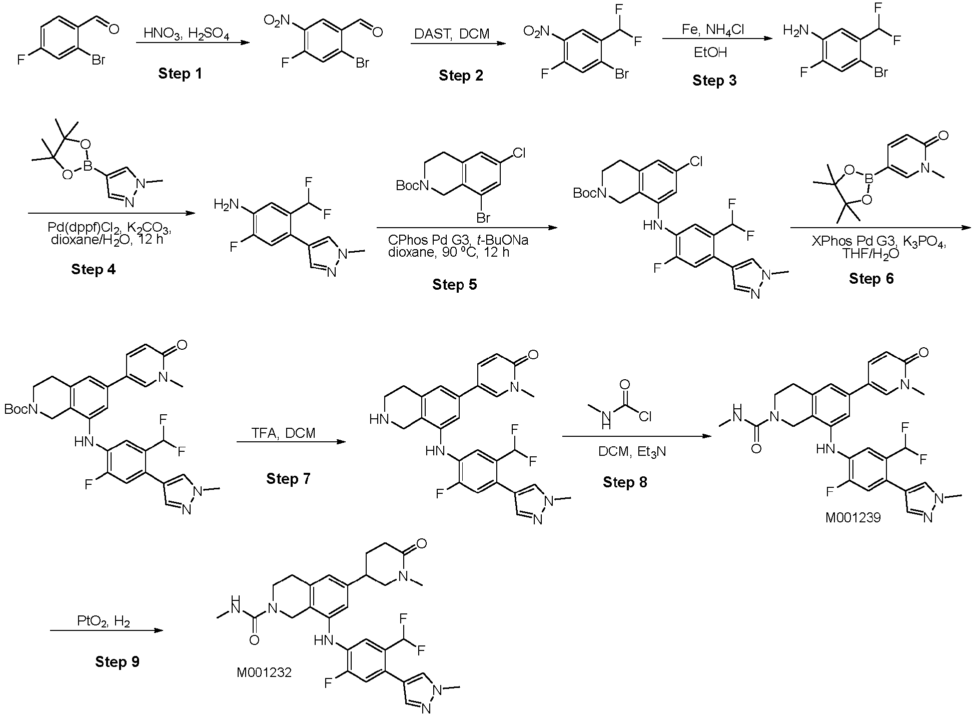

To a stirred solution of 2-bromo-4-fluorobenzaldehyde (5.0 g, 0.024 mol) in H₂SO₄ (30.0 mL) at 0 °C was added nitric acid (1.71 g, 0.027 mol). The reaction mixture was heated to 25 °C and stirred at that temperature for 2 h. The reaction mixture was quenched with water (200 mL) and then extracted with EtOAc (50 mL × 2). The combined organic phases were dried over Na₂SO₄ and then filtered. The filtrate was concentrated under reduced pressure to give 2-bromo-4-fluoro-5-nitrobenzaldehyde (5.6 g, 78% yield) as a yellow solid.

To a stirred solution of 2-bromo-4-fluoro-5-nitrobenzaldehyde (1.5 g, 0.006 mol) in DCM (20.0 mL) at 0 °C was added DAST (1.93 g, 0.012 mol). The reaction mixture was heated to 25 °C and stirred at that temperature for 12 h. The reaction mixture was quenched with water (200 mL) and then extracted with EtOAc (50 mL × 2). The combined organic phases were dried over Na₂SO₄, filtered and concentrated. The residue was purified by silica gel chromatography eluting with EtOAc/PE (with EtOAc from 0% to 5%) in 10 min to give 1-bromo-2-(difluoromethyl)-5-fluoro-4-nitrobenzene (1.4 g, 78% yield) as a yellow solid.

To a solution of 1-bromo-2-(difluoromethyl)-5-fluoro-4-nitrobenzoate (1.4 g, 0.005 mol) in EtOH (20.0 mL) and H₂O (5.0 mL) were added Fe (1.45 g, 0.026 mol) and NH4Cl (2.78 g, 0.052 mol). The reaction solution was heated to 90 °C and stirred at that temperature for 2 h. The mixture was cooled to 25 °C, filtered to remove solids, and concentrated. The residue was purified by silica gel chromatography eluting with EtOAc/PE (with EtOAc from 0% to 5%) in 10 min to give 4-bromo-5-(difluoromethyl)-2-fluoroaniline (0.85 g, 62% yield) as a white solid.

To a solution of 4-bromo-5-(difluoromethyl)-2-fluoroaniline (850 mg, 3.54 mmol) in 1,4-dioxane (15.0 mL) and H₂O (2.0 mL) were added 1-methyl-4-(4,4,5,5-tetramethyl-1,3,2-dioxaborolan-2-yl)pyrazole (884 mg, 4.24 mmol), K₂CO₃ (979 mg, 7.08 mmol) and Pd(dppf)Cl2 (259 mg, 0.354 mmol). The reaction solution was heated to 100 °C and stirred at that temperature under N₂ for 10 h. The mixture was cooled to 25 °C and concentrated under reduced pressure. The residue was purified by silica gel chromatography eluting with EtOAc/PE (with EtOAc from 0% to 20%) in 10 min to give 5-(difluoromethyl)-2-fluoro-4-(1-methyl-1H-pyrazol-4-yl)aniline (790 mg, 88% yield) as a white solid.

To a solution of 5-(difluoromethyl)-2-fluoro-4-(1-methyl-1H-pyrazol-4-yl)aniline (500 mg, 2.07 mmol) in 1,4-dioxane (10.0 mL) were added tert-butyl 8-bromo-6-chloro-3,4-dihydro-1H-isoquinoline-2-carboxylate (719 mg, 2.07 mmol), t-BuONa (398 mg, 4.14 mmol) and Cphos-Pd G3 (167 mg, 0.207 mmol). The reaction solution was heated to 90 °C and stirred at that temperature under N₂ for 12 h. The mixture was cooled to 25 °C and concentrated under reduced pressure. The residue was purified by silica gel chromatography eluting with MeOH/DCM (with MeOH from 0 to 10%) in 10 min to give tert-butyl 6-chloro-8-((5-(difluoromethyl)-2-fluoro-4-(1-methyl-1H-pyrazol-4-yl)phenyl)amino)-3,4-dihydroisoquinoline-2(1H)-carboxylate (780 mg, 45% yield) as a yellow solid.

To a solution of tert-butyl 6-chloro-8-(5-(difluoromethyl)-2-fluoro-4-(1-methyl-1H-pyrazol-4-yl)phenyl)amino)-3,4-dihydroisoquinoline-2(1H)-carboxylate (150 mg, 0.295 mmol) in THF (6.0 mL) and H₂O (1.0 mL) were added 1-methyl-5-(4,4,5,5-tetramethyl-1,3,2-dioxaborolan-2-yl)pyridin-2-one (139 mg, 0.591 mmol), K₃PO₄ (188 mg, 0.887 mmol) and XPhos Pd G3 (25.0 mg, 0.029 mmol). The reaction solution was heated to 80 °C and stirred at that temperature under N₂ for 12 h. The mixture was cooled to 25 °C and concentrated under reduced pressure. The residue was purified by silica gel chromatography eluting with MeOH/DCM (with MeOH from 0 to 10%) in 10 min to give tert-butyl 8-((5-(difluoromethyl)-2-fluoro-4-(1-methyl-1H-pyrazol-4-yl)phenyl)amino)-6-(1-methyl-6-oxo-1,6-dihydropyridin-3-yl)-3,4-dihydroisoquinoline-2(1H)-carboxylate (150 mg, 83% yield) as a yellow solid.

To a solution of tert-butyl 8-((5-(difluoromethyl)-2-fluoro-4-(1-methyl-1H-pyrazol-4-yl)phenyl)amino)-6-(1-methyl-6-oxo-1,6-dihydropyridin-3-yl)-3,4-dihydroisoquinoline-2(1H)-carboxylate (150 mg, 0.258 mmol) in DCM (5.0 mL) was added TFA (1.0 mL). The solution was stirred at 25 °C for 1 h. The mixture was concentrated under reduced pressure to give 5-(8-((5-(difluoromethyl)-2-fluoro-4-(1-methyl-1H-pyrazol-4-yl)phenyl)amino)-1,2,3,4-tetrahydroisoquinolin-6-yl)-1-methylpyridin-2(1H)-one (110 mg, 84% yield) as a yellow oil.

To a solution of 5-(8-((5-(difluoromethyl)-2-fluoro-4-(1-methyl-1H-pyrazol-4-yl)phenyl)amino)-1,2,3,4-tetrahydroisoquinolin-6-yl)-1-methylpyridin-2(1H)-one (110 mg, 0.229 mmol) in DCM (5.0 mL) were added Et3N (70.0 mg, 0.688 mmol) and N-methylcarbamoyl chloride (64.0 mg, 0.688 mmol). The reaction solution was stirred at 25 °C for 1 h. The mixture was concentrated under reduced pressure. The residue was purified by Prep-HPLC (with CH₃CN from 25% to 55% in 8 min) to give a white solid c (80.0 mg, 65% yield).

### M001239:

¹H NMR (400 MHz, DMSO-d6) δ 8.03 (d, J = 2.0 Hz, 1H), 7.87 (s, 1H), 7.71-7.68 (m, 1H), 7.61 (d, J = 8.0 Hz, 2H), 7.34 (d, J = 12.0 Hz, 1H), 7.14 (d, J = 8.0 Hz, 2H), 7.06-6.75 (m, 2H), 6.48-6.43 (m, 2H), 4.43 (s, 2H), 3.88 (s, 3H), 3.55-3.52 (m, 2H), 3.47 (s, 3H), 2.84 (s, 2H), 2.59 (d, J = 4.0 Hz, 3H).

To a solution of 8-((5-(difluoromethyl)-2-fluoro-4-(1-methyl-1H-pyrazol-4-yl)phenyl)amino)-N-methyl-6-(1-methyl-6-oxo-1,6-dihydropyridin-3-yl)-3,4-dihydroisoquinoline-2(1H)-carboxamide (70 mg, 0.130 mmol) in MeOH (6.0 mL) was added PtO₂ (30.0 mg, 0.130 mmol). The solution was stirred at 25 °C under H₂ for 12 h. The mixture was filtered to remove solids and concentrated. The residue was purified by Prep-HPLC (with CH₃CN from 25% to 55% in 9 min) to give 8-((5-(difluoromethyl)-2-fluoro-4-(1-methyl-1H-pyrazol-4-yl)phenyl)amino)-N-methyl-6-(1-methyl-6-oxopiperidin-3-yl)-3,4-dihydroisoquinoline-2(1H)-carboxamide (42.5 mg, 50% yield) as a white solid.

### M001232:

¹H NMR (400 MHz, DMSO-d6) δ 7.87 (s, 1H), 7.62 (s, 1H), 7.50 (s, 1H), 7.34 (d, J = 12.0 Hz, 1H), 7.15 (d, J = 8.0 Hz, 1H), 7.05-6.76 (m, 3H), 6.49-6.41 (m, 1H), 4.39 (s, 2H), 3.89 (s, 3H), 3.52-3.49 (m, 2H), 3.32-3.24 (m, 2H), 3.05-2.99 (m, 1H), 2.79-2.76 (m, 5H), 2.59 (d, J = 4.0 Hz, 3H), 2.36-2.22 (m, 2H), 1.97-1.82 (m, 2H).

| **Compound ID** | **Structure Formula** | **¹H-NMR; m/z** |
|---|---|---|
| M001001 | 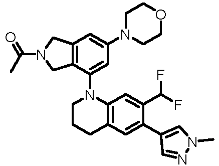 | ¹H NMR (400MHz, DMSO-d6) d 7.74 (s, 1H), 7.49 (s, 1H), 7.11 (s, 1H), 6.91 - 6.80 (m, 2H), 6.79 - 6.59 (m, 1H), 6.49 - 6.36 (m, 1H), 4.81 (br s, 1H), 4.60 (br s, 1H), 4.55 - 4.12 (m, 2H), 3.86 (s, 3H), 3.76 - 3.69 (m, 4H), 3.61 - 3.48 (m, 3H), 3.15 3.09 (m, 3H), 2.86 (br s, 2H), 2.05 - 1.92 (m, 5H) |
| M001002 | 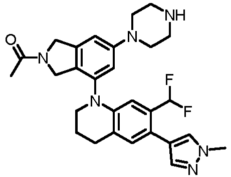 | ¹H NMR (400MHz, DMSO-d6) d 9.46 (br s, 2H), 7.77 (br s, 1H), 7.49 (br s, 1H), 7.11 (br s, 1H), 6.96 - 6.80 (m, 2H), 6.73 (br s, 1H), 6.46 - 6.33 (m, 1H), 4.80 (br s, 1H), 4.68 - 4.32 (m, 3H), 3.82 (br s, 3H), 3.59 - 3.48 (m, 2H), 3.45 - 3.32 (m, 4H), 3.25 3.08 (m, 4H), 2.91 - 2.78 (m, 2H), 2.08 - 1.88 (m, 5H) |
| M001003 | 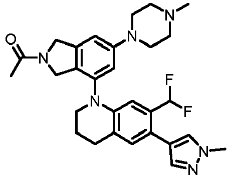 | ¹H NMR (400MHz, DMSO-d6) d 7.74 (s, 1H), 7.49 (s, 1H), 7.10 (s, 1H), 6.90 - 6.79 (m, 2H), 6.78 - 6.59 (m, 1H), 6.47 - 6.37 (m, 1H), 4.79 (br s, 1H), 4.59 (s, 1H), 4.54 - 4.12 (m, 2H), 3.86 (s, 3H), 3.58 - 3.52 (m, 2H), 3.21 - 3.06 (m, 4H), 2.86 (br s, 2H), 2.45 (br d, J = 3.9 Hz, 4H), 2.22 (d, J = 4.2 Hz, 3H), 2.04 - 1.92 (m, 5H) |
| M001004 | 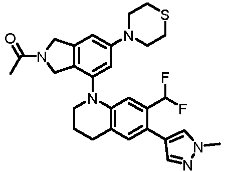 | ¹H NMR (400MHz, DMSO-d6) d 7.74 (s, 1H), 7.49 (s, 1H), 7.11 (s, 1H), 6.92 - 6.56 (m, 3H), 6.51 - 6.38 (m, 1H), 4.80 (br s, 1H), 4.60 (s, 1H), 4.52 (br s, 1H), 4.20 (br s, 1H), 3.86 (s, 3H), 3.63 - 3.51 (m, 6H), 2.91 - 2.83 (m, 2H), 2.70 - 2.61 (m, 4H), 2.05 - 1.95 (m, 5H) |
| M001006 | 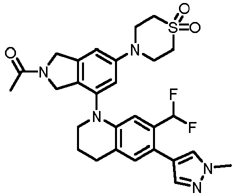 | ¹H NMR (400MHz, DMSO-d6) d = 7.74 (d, J=2.4 Hz, 1H), 7.49 (s, 1H), 7.11 (s, 1H), 6.98 - 6.92 (m, 1H), 6.91 - 6.58 (m, 1H), 6.48 - 6.40 (m, 1H), 4.81 (br s, 1H), 4.61 (s, 1H), 3.86 (s, 3H), 3.79 (br s, 4H), 3.56 (br s, 2H), 3.12 (br s, 4H), 2.86 (br s, 2H), 2.04 - 2.00 (m, 3H), 2.00 - 1.95 (m, 2H) |
| M001014 | 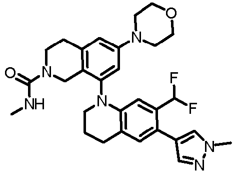 | ¹H NMR (400MHz, CD3Cl) d 7.53 (s, 1H), 7.40 (s, 1H), 7.05 (s, 1H), 6.67 (d, J = 3.9 Hz, 2H), 6.62 - 6.24 (m, 2H), 4.46 (d, J = 15.9 Hz, 1H), 4.36 - 4.27 (m, 1H), 4.16 4.06 (m, 1H), 3.95 (s, 3H), 3.85 (t, J = 4.8 Hz, 4H), 3.78 - 3.68 (m, 1H), 3.63 - 3.43 (m, 3H), 3.20 - 3.09 (m, 4H), 3.01 - 2.83 (m, 4H), 2.79 (d, J = 4.6 Hz, 3H), 2.24 2.03 (m, 2H) |
| M001015 | 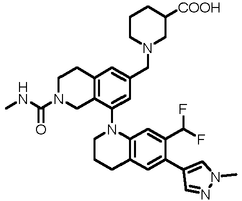 | ¹H NMR (400MHz, DMSO-d6) d 8.23 (br s, 1H), 7.72 (s, 1H), 7.47 (s, 1H), 7.34 6.96 (m, 3H), 6.88 - 6.09 (m, 1H), 6.18 (s, 1H), 4.56 - 4.07 (m, 3H), 3.85 (s, 3H), 3.01 - 2.63 (m, 8H), 2.59 - 2.53 (m, 6H), 2.42 - 2.28 (m, 2H), 2.18 - 1.89 (m, 4H), 1.83 - 1.51 (m, 2H), 1.45 - 1.30 (m, 1H) |
| M001016 | 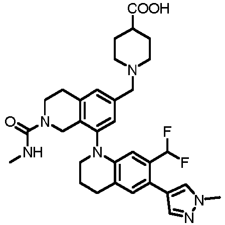 | ¹H NMR (400MHz, DMSO-d6) d 7.72 (s, 1H), 7.46 (s, 1H), 7.09 (br s, 1H), 7.02 (br s, 1H), 6.82 - 6.55 (m, 1H), 6.49 (br d, J = 4.2 Hz, 1H), 6.19 (s, 1H), 4.59 - 4.34 (m, 2H), 4.31 - 4.11 (m, 2H), 3.92 - 3.79 (m, 4H), 3.01 - 2.69 (m, 7H), 2.60 - 2.53 (m, 5H), 2.20 - 1.87 (m, 5H), 1.83 - 1.67 (m, 1H), 1.52 (br s, 2H) |
| M001017 | 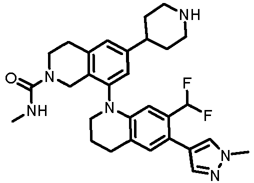 | ¹H NMR: (400 MHz, d6-DMSO) δ 8.360 (br s, 1H), 7.719 (s, 1H), 7.469 (s, 1H), 7.091 (s, 1H), 7.016 (s, 1H), 6.971 (s, 1H), 6.698 (t, J = 55.1 Hz, 1H), 6.494 (s, 1H), 6.211 - 6.145 (m, 1H), 4.417 (d, J = 16.4 Hz, 1H), 4.160 - 4.070 (m, 1H), 3.857 (s, 3H), 3.562 - 3.461 (m, 4H), 3.173 (d, J = 10.8 Hz, 2H), 2.984 - 2.656 (m, 7H), 2.540 (d, J = 4.4 Hz, 3H), 2.411 - 2.312 (m, 1H), 2.110 - 1.978 (m, 2H), 1.832 - 1.561 (m, 3H). |
| M001018 | 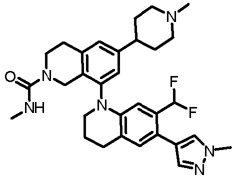 | ¹H NMR: (400 MHz, MeOD) δ 7.600 (s, 1H), 7.469 (s, 1H), 7.100 (s, 1H), 7.072 (s, 1H), 7.042 (s, 1H), 6.473 (t, J = 55.6 Hz, 1H), 6.243 (s, 1H), 4.622 - 4.545 (m, 1H), 4.208 (d, J = 16.4 Hz, 1H), 3.915 (s, 3H), 3.628 - 3.489 (m, 4H), 3.371 - 3.323 (m, 3H), 2.989 - 2.910 (m, 4H), 2.778 - 2.664 (m, 6H), 2.662 - 2.553 (m, 3H), 2.241 - 2.060 (m, 2H), 2.042 - 1.789 (m, 3H). |
| M001019 | 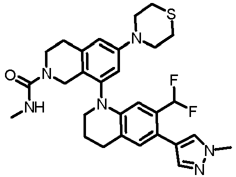 | ¹H NMR (400MHz, CD3Cl) d 7.53 (s, 1H), 7.40 (s, 1H), 7.05 (s, 1H), 6.69 - 6.62 (m, 2H), 6.61 - 6.28 (m, 2H), 4.45 ( d, J = 15.9 Hz, 1H), 4.36 - 4.30 (m, 1H), 4.11 (br d, J = 16.0 Hz, 1H), 3.96 (s, 3H), 3.81 - 3.68 (m, 1H), 3.62 - 3.42 (m, 6H), 3.03 - 2.83 (m, 4H), 2.82 - 2.76 (m, 3H), 2.76 - 2.66 (m, 4H), 2.21 - 2.01 (m, 2H) |
| M001021 | 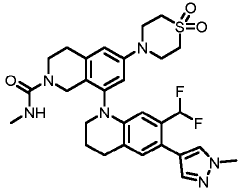 | ¹H NMR (400MHz, CD3Cl) δ 7.78 (d, J = 10.4 Hz, 1H), 7.53 (s, 1H), 7.40 (s, 1H), 7.15 - 7.01 (m, 1H), 6.74 - 6.40 (m, 2H), 6.35 (s, 1H), 4.68 - 4.12 (m, 5H), 3.96 (s, 3H), 3.86 - 3.67 (m, 2H), 3.64 - 3.45 (m, 4H), 3.16 - 2.85 (m, 7H), 2.81 (s, 3H), 2.23 - 2.04 (m, 2H). |
| M001024 | 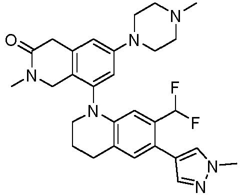 | ¹H NMR (400 MHz, DMSO-d6) d 10.35 (br s, 0.9 H) 7.72 (s, 1 H) 7.48 (s, 1 H) 7.11 (s, 1 H) 6.86 (s, 2 H) 6.56 6.74 (m, 1 H) 6.25 (s, 1 H) 4.27 - 4.35 (m, 1 H) 4.07 - 4.14 (m, 1 H) 3.86 (s, 3 H) 3.79 - 3.85 (m, 2 H) 3.56 - 3.63 (m, 2 H) 3.53 - 3.55 (m, 2 H) 2.96 - 3.18 (m, 5 H) 2.89 - 2.91 (m, 1 H) 2.87 (s, 3 H) 2.80 - 2.82 (m, 2 H) 2.512.51 (m, 3 H) 2.02 - 2.10 (m, 2 H). |
| M001027 | 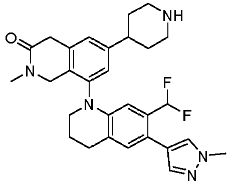 | ¹H NMR (400 MHz, METHANOL-d4): d 7.62 (s, 1H), 7.48 (s, 1H), 7.15 - 7.10 (m, 2H), 6.50 (t, J = 55.3 Hz, 1H), 6.30 (s, 1H), 4.55 - 4.26 (m, 2H), 3.92 (s, 3H), 3.74 - 3.59 (m, 3H), 3.58 - 3.42 (m, 4H), 3.20 - 3.08 (m, 2H), 3.04 - 2.86 (m, 6H), 2.23 - 2.06 (m, 4H), 2.02 - 1.77 (m, 2H). |
| M001028 | 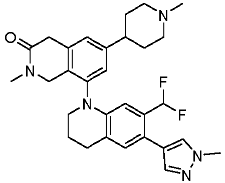 | ¹H NMR (400 MHz, METHANOL-d4) d 8.49 (br s, 0.9 H) 7.62 (s, 1 H) 7.49 (s, 1 H) 7.10 - 7.16 (m, 3 H) 6.50 (t, J = 55.41 Hz, 1 H) 6.30 (s, 1 H) 4.54 - 4.63 (m, 1 H) 4.28 - 4.51 (m, 2 H) 3.93 (s, 3 H) 3.60 - 3.72 (m, 3 H) 3.46 - 3.55 (m, 3 H) 3.02 - 3.05 (m, 1 H) 3.00 (s, 3 H) 2.94 - 2.99 (m, 2 H) 2.86 - 2.93 (m, 1 H) 2.83 (s, 3 H) 2.07 - 2.20 (m, 4 H) 1.87 - 2.00 (m, 2 H). |
| M001030 | 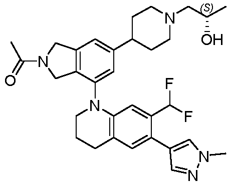 | ¹H NMR (400MHz, METHANOL-d4) 8.51 (br s, 1H), 7.63 (d, J=3.7 Hz, 1H), 7.50 (br s, 1H), 7.46 - 7.11 (m, 3H), 6.68 - 6.36 (m, 2H), 4.95-4.93 (m, 2H), 4.80 (br d, J=7.0 Hz, 1H), 4.69 (br s, 1H), 4.55 - 4.30 (m, 2H), 4.16-4.14 (m, 1H), 3.93 (s, 3H), 3.70 - 3.50 (m, 4H), 3.12 - 2.70 (m, 6H), 2.29 - 1.91 (m, 7H), 1.40 - 1.05 (m, 4H). |
| M001032 | 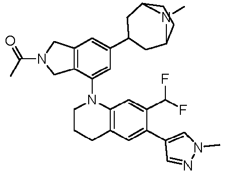 | ¹H NMR (400MHz, DMSO-d6) d 8.29 (br s, 1H), 7.74 (s, 1H), 7.49 (s, 1H), 7.28 (br d, J=14.2 Hz, 1H), 7.24 - 7.09 (m, 2H), 6.89 - 6.56 (m, 1H), 6.45 - 6.31 (m, 1H), 4.85 (br s, 1H), 4.65 (br s, 1H), 4.28 (br s, 1H), 3.86 (s, 3H), 3.54 (br d, J=5.1 Hz, 2H), 3.42-3.21 (m, 5H), 3.04 (br s, 2H), 2.87 (br s, 2H), 2.41 - 2.31 (m, 2H), 2.25 (br s, 3H), 2.05 - 1.95 (m, 6H), 1.78-1.75 (m 2H), 1.45-1.42 (m, 2H). |
| M001033 | 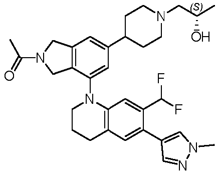 | ¹H NMR (400MHz, MeOD) d 7.62 (s, 1H), 7.49 (s, 1H), 7.17 - 6.99 (m, 3H), 6.68 6.32 (m, 1H), 6.27 (s, 1H), 4.61 (br d, J = 16.8 Hz, 2H), 4.40 - 4.01 (m, 1H), 3.94 (s, 3H), 3.68 - 3.43 (m, 4H), 3.20 - 2.81 (m, 8H), 2.77 - 2.61 (m, 5H), 2.32 - 2.06 (m, 4H), 1.99 - 1.80 (m, 3H), 1.32 - 1.16 (m, 2H) |
| M001035 | 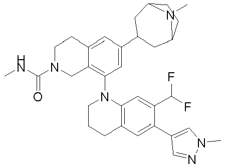 | ¹H NMR (400 MHz, MeOD) d 7.62 (br s, 1H), 7.47 (br s, 1H), 7.38 (br s, 1H ), 7.30 (br s, 1H), 7.09 (br s, 1H), 6.30-6.68 (m, 1H), 6.23 (br s, 1H), 4.61 (br d, J = 14.55 Hz, 1H), 4.24-4.39 (m, 1H), 3.77-4.00 (m, 5H), 3.53-3.67 (m, 4H), 2.97 (br s, 3H), 2.58-2.83 (m, 10H), 2.37-2.55 (m, 1H), 2.01-2.29 (m, 5H), 1.81 (br s, 2H) |
| M001040 | 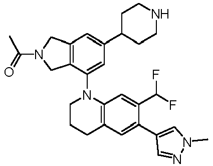 | ¹H NMR (400MHz, DMSO-d6) d 8.30 (br s, 1H), 7.71 (br s, 1H), 7.47 (s, 1H), 7.35 6.98 (m, 3H), 6.90 - 6.53 (m, 1H), 6.43 - 6.28 (m, 1H), 4.85 (br s, 1H), 4.72 - 4.38 (m, 1H), 4.26 (br s, 1H), 3.83 (s, 3H), 3.70 - 3.16 (m, 6H), 3.06 - 2.61 (m, 5H), 2.12 1.59 (m, 8H) |
| M001041 | 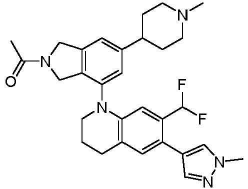 | ¹H NMR (400MHz, METHANOL-d4) d 7.65 (d, J=3.8 Hz, 1H), 7.52 (d, J=3.2 Hz, 1H), 7.44 - 7.18 (m, 2H), 7.14 - 7.14 (m, 1H), 6.77 - 6.72 (m, 2H), 4.96 (br s, 1H), 4.81 (br s, 1H), 4.69 (br s, 1H), 4.49 (br s, 1H), 3.94 (s, 3H), 3.82 - 3.42 (m, 4H), 3.09-3.07 (m 2H), 2.97-2.95 (m, 3H), 2.89-2.85 (m, 3H), 2.40 - 1.72 (m, 9H) |
| M001042 | 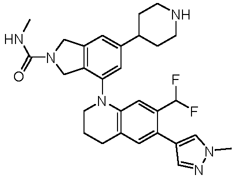 | ¹H NMR (400MHz, CHLOROFORM-d) d = 7.74 (br s, 1H), 7.62 (br s, 1H), 7.56 7.36 (m, 1H), 6.86 - 6.46 (m, 2H), 4.93 (br s, 2H), 4.66 (br s, 3H), 4.16 (br s, 3H), 3.78 (br s, 2H), 3.68 (br s, 3H), 3.37 (br s, 1H), 3.22 (br d, J=9.9 Hz, 1H), 3.12 (br s, 3H), 3.06 (br s, 3H), 2.94 (br s, 1H), 2.80 - 2.59 (m, 2H), 2.55 (br s, 1H), 2.29 (br s, 3H), 2.18 (br s, 2H), 2.07 (br s, 2H), 1.47 (br s, 2H) |
| M001043 | 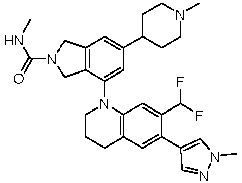 | ¹H NMR (400MHz, CHLOROFORM-d) d = 7.53 (s, 1H), 7.40 (s, 1H), 7.07 7.01 (m, 3H), 6.60 - 6.44 (m, 2H), 4.71 (br s, 2H), 4.45 (br s, 2H), 4.24 (br d, J=4.8 Hz, 1H), 3.95 (s, 3H), 3.56 (br s, 2H), 3.44 (br d, J=11.2 Hz, 2H), 2.91 (br s, 2H), 2.86 (d, J=4.5 Hz, 3H), 2.72 - 2.55 (m, 5H), 2.24 - 2.14 (m, 4H), 2.13 2.04 (m, 4H), 1.98 (br d, J=13.8 Hz, 3H) |
| M001044 | 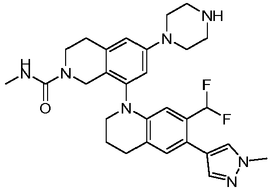 | ¹H NMR (400MHz, METHANOL-d4) d 8.49 (br s, 1H), 7.61 (s, 1H), 7.48 (s, 1H), 7.07 (s, 1H), 6.87 (s, 1H), 6.82 (d, J=2.1 Hz, 1H), 6.67 - 6.34 (m, 1H), 6.28 (s, 1H), 4.55 (d, J=16.4 Hz, 1H), 4.14 (d, J=16.3 Hz, 1H), 3.92 (s, 3H), 3.67 - 3.57 (m, 2H), 3.56 - 3.49 (m, 2H), 3.44-3.37 (m, 4H), 3.35 - 3.31 (m, 4H), 3.04 - 2.85 (m, 4H), 2.70 (s, 3H), 2.29 - 2.16 (m, 1H), 2.15 - 2.06 (m, 1H). |
| M001045 | 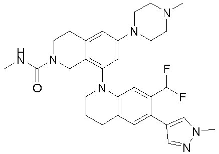 | ¹H NMR (400MHz, METHANOL-d4) d 8.47 (br s, 1H), 7.61 (s, 1H), 7.54 - 7.40 (m, 1H), 7.19 - 6.97 (m, 1H), 6.85 (d, J=2.1 Hz, 1H), 6.79 (d, J=2.3 Hz, 1H), 6.66 - 6.32 (m, 1H), 6.29 (s, 1H), 4.54 (d, J=16.4 Hz, 1H), 4.14 (d, J=16.3 Hz, 1H), 3.92 (s, 3H), 3.72 - 3.41 (m, 4H), 3.38 - 3.32 (m, 5H), 3.07-3.02 (m, 3H), 3.01 - 2.87 (m, 4H), 2.86 - 2.56 (m, 6H), 2.23-2.20 (m, 1H), 2.15 - 2.03 (m, 1H). |
| M001046 | 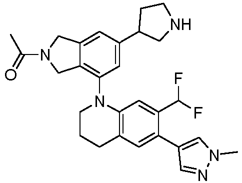 | ¹H NMR (400MHz, METHANOL-d4) d 8.53 (s, 1H), 7.63 (d, J=4.8 Hz, 1H), 7.50 (d, J=4.2 Hz, 1H), 7.36 - 7.19 (m, 2H), 7.12 (d, J=6.4 Hz, 1H), 6.71 - 6.29 (m, 2H), 4.95 (br s, 1H), 4.79 (s, 1H), 4.66 (br s, 1H), 4.47 (br s, 1H), 3.92 (d, J=2.1 Hz, 3H), 3.80 - 3.49 (m, 5H), 3.38 (br s, 1H), 3.28 - 3.14 (m, 1H), 3.01 - 2.88 (m, 2H), 2.52-2.46 (m, 1H), 2.25 - 1.95 (m, 6H) |
| M001047 | 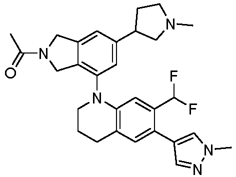 | ¹H NMR (400MHz, METHANOL-d4) d 8.48 (br s, 1H), 7.64 (d, J=4.0 Hz, 1H), 7.50 (d, J=3.8 Hz, 1H), 7.28 - 7.26 (m, 2H), 7.13 (d, J=6.0 Hz, 1H), 6.81 - 6.30 (m, 2H), 4.95 (s, 1H), 4.86 - 4.75 (m, 1H), 4.74 - 4.56 (m, 1H), 4.47 (br s, 1H), 3.93 (s, 3H), 3.83 3.70 (m, 2H), 3.67-3.62 (m, 2H), 3.59 - 3.47 (m, 2H), 3.39-3.34 (m, 1H), 3.04 - 2.86 (m, 5H), 2.70 - 2.47 (m, 1H), 2.36 - 2.19 (m, 1H), 2.17 - 2.01 (m, 5H) |
| M001048 | 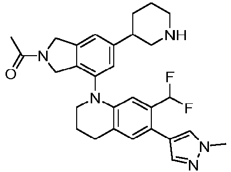 | ¹H NMR (400MHz, METHANOL-d4) d 7.64 (d, J=3.7 Hz, 1H), 7.50 (d, J=3.3 Hz, 1H), 7.40 - 7.18 (m, 2H), 7.13 (br d, J=5.4 Hz, 1H), 6.70 - 6.35 (m, 2H), 4.95 (br s, 1H), 4.83 - 4.77 (m, 1H), 4.67 (br s, 1H), 4.47 (br s, 1H), 3.93 (s, 3H), 3.77 - 3.72 (m, 1H), 3.64 (br s, 2H), 3.54 - 3.36 (m, 2H), 3.26 - 2.79 (m, 5H), 2.18 - 2.06 (m, 6H), 1.91 - 1.85 (m, 2H). |
| M001049 | 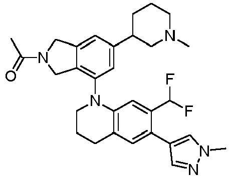 | ¹H NMR (400MHz, METHANOL-d4) d 7.64 (d, J=4.3 Hz, 1H), 7.50 (d, J=3.8 Hz, 1H), 7.39 - 7.17 (m, 2H), 7.13 (d, J=5.9 Hz, 1H), 6.73 - 6.33 (m, 2H), 4.97 (s, 1H), 4.79 (s, 1H), 4.67 (br s, 1H), 4.47 (br s, 1H), 3.93 (s, 3H), 3.76 - 3.57 (m, 2H), 3.52 - 3.34 (m, 2H), 3.16 - 3.04 (m, 1H), 3.04 - 2.89 (m, 3H), 2.89 - 2.65 (m, 4H), 2.36 - 1.56 (m, 9H). |
| M001050 | 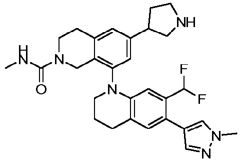 | ¹H NMR (400MHz, DMSO-d6) δ 7.68 (s, 1H), 7.44 (s, 1H), 7.15 - 7.04 (m, 3H), 6.80 - 6.47 (m, 1H), 6.12 (s, 1H), 4.43 (br d, J = 16.8 Hz, 1H), 4.09 - 3.96 (m, 3H), 3.88 - 3.77 (m, 4H), 3.62 - 3.28 (m, 7H), 3.25 - 3.10 (m, 1H), 3.08 - 2.95 (m, 1H), 2.91 - 2.76 (m, 4H), 2.39 - 2.24 (m, 1H), 2.13 - 1.79 (m, 3H). |
| M001051 | 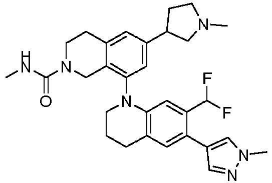 | ¹H NMR (400MHz, DMSO-d6) δ 8.31 (br s, 1H), 7.71 (s, 1H), 7.46 (s, 1H), 7.19 6.99 (m, 3H), 6.89 - 6.50 (m, 1H), 6.15 (s, 1H), 4.43 (br d, J = 16.8 Hz, 1H), 4.07 (br dd, J = 5.8, 16.8 Hz, 1H), 3.84 (s, 3H), 3.54 - 3.24 (m, 6H), 3.09 (br s, 2H), 2.97 2.73 (m, 5H), 2.61 (br d, J = 4.2 Hz, 3H), 2.53 (br s, 3H), 2.33 (br s, 1H), 2.12 - 1.81 (m, 3H). |
| M001052 | 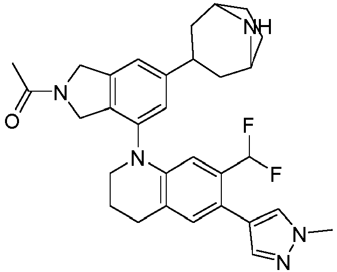 | ¹H NMR (400MHz, DMSO-d6) d 8.36 (br s, 1H), 7.74 (s, 1H), 7.49 (s, 1H), 7.37 - 7.10 (m, 3H), 6.90 - 6.56 (m, 1H), 6.45 - 6.23 (m, 1H), 4.87 (br s, 1H), 4.66 (br s, 1H), 4.28 (br s, 1H), 3.97 - 3.72 (m, 4H), 3.55-3.52 (m, 1H), 3.50 - 3.09 (m, 8H), 2.89-2.87 (m, 2H), 2.33-2.31 (m, 2H), 2.04-2.01 (m, 6H), 1.77-1.74 (m, 1H), 1.65 - 1.58 (m, 1H). |
| M001053 | 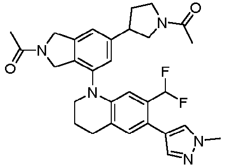 | ¹H NMR (400MHz, METHANOL-d4) d 7.63 (br d, J=3.4 Hz, 1H), 7.50 (br d, J=3.3 Hz, 1H), 7.30 - 7.08 (m, 3H), 6.70 - 6.37 (m, 2H), 4.94 (br s, 1H), 4.79 (br s, 1H), 4.66 (br s, 1H), 4.47 (br s, 1H), 4.06 - 3.95 (m, 1H), 3.92 (s, 3H), 3.75-3.72 (m, 1H), 3.68 - 3.38 (m, 5H), 2.94-2.92 (m, 2H), 2.48 - 2.26 (m, 1H), 2.18 - 2.02 (m, 9H). |
| M001054 | 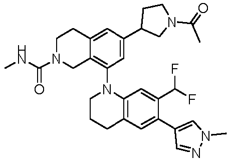 | ¹H NMR (400MHz, DMSO-d6) d 7.72 (s, 1H), 7.47 (s, 1H), 7.17 - 7.00 (m, 3H), 6.90 6.54 (m, 1H), 6.51 - 6.41 (m, 1H), 6.18 (s, 1H), 4.44 (br d, J = 16.5 Hz, 1H), 4.16 - 3.98 (m, 1H), 3.91 - 3.78 (m, 4H), 3.63 - 3.40 (m, 7H), 2.98 - 2.80 (m, 4H), 2.67 (br s, 2H), 2.33 (br s, 2H), 2.05 (br s, 4H), 1.96 - 1.88 (m, 3H) |
| M001055 | 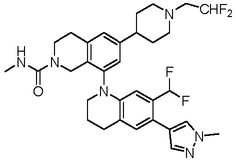 | ¹H NMR (400MHz, DMSO-d6) δ 8.40 (s, 1H), 7.71 (s, 1H), 7.46 (s, 1H), 7.06 (br d, J = 13.4 Hz, 2H), 6.99 (s, 1H), 6.86 - 6.48 (m, 1H), 6.31 - 5.94 (m, 2H), 4.41 (br d, J 16.9 Hz, 1H), 4.14 - 4.07 (m, 1H), 3.85 (s, 3H), 3.41 - 3.31 (m, 2H), 3.00 - 2.80 (m, 6H), 2.79 - 2.65 (m, 4H), 2.53 (br s, 3H), 2.27 - 1.96 (m, 4H), 1.74 - 1.47 (m, 6H). |
| M001056 | 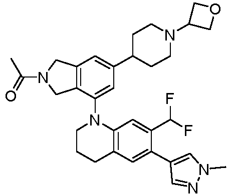 | ¹H NMR (400MHz, DMSO-d6) d 7.75 (s, 1H), 7.50 (s, 1H), 7.26 - 7.02 (m, 3H), 6.94 - 6.56 (m, 1H), 6.48 - 6.31 (m, 1H), 4.86 (br s, 1H), 4.70 - 4.63 (m, 1H), 4.57 - 4.52 (m, 1H), 4.43-4.41 (m, 1H), 4.30-4.28 (m, 1H), 3.86 (s, 3H), 3.57 - 3.45 (m, 7H), 2.99 - 2.75 (m, 5H), 2.08 - 1.96 (m, 5H), 1.96 - 1.66 (m, 5H) |
| M001057 | 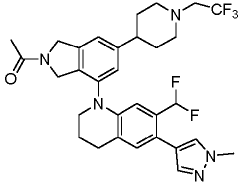 | ¹H NMR (400MHz, METHANOL-d4) 8.49 (br s, 1H), 7.70 - 7.60 (m, 1H), 7.57 - 7.41 (m, 1H), 7.34 - 7.00 (m, 3H), 6.78 - 6.25 (m, 2H), 4.82 - 4.75 (m, 1H), 4.63-4.61 (m, 3H), 4.46 (br s, 1H), 3.92 (s, 3H), 3.75 - 3.48 (m, 2H), 3.15 - 3.05 (m, 4H), |
| | | 2.95-2.93 (m, 2H), 2.75 - 2.39 (m, 3H), 2.19 - 2.04 (m, 5H), 1.87 - 1.78 (m, 3H). |
| M001058 | 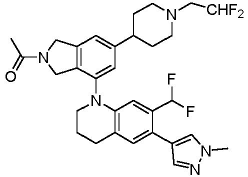 | ¹H NMR (400MHz, METHANOL-d4) d 7.63 (d, J=3.7 Hz, 1H), 7.50 (d, J=3.9 Hz, 1H), 7.30 - 6.96 (m, 3H), 6.70 - 6.33 (m, 2H), 6.22 - 5.87 (m, 1H), 5.12 (br s, 1H), 4.78 (br s, 1H), 4.65 (br s, 1H), 4.46 (br s, 1H), 3.92 (s, 3H), 3.62 (br s, 2H), 3.24 - 3.06 (m, 2H), 2.98 - 2.82 (m, 4H), 2.66-2.63 (m, 1H), 2.52 - 2.36 (m, 2H), 2.19 - 2.04 (m, 5H), 1.92 - 1.77 (m, 4H) |
| M001059 | 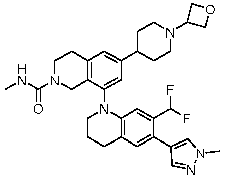 | ¹H NMR (400MHz, DMSO-d6) δ 7.69 (s, 1H), 7.45 (s, 1H), 7.04-7.07(d, J = 12 Hz, 2H), |
| | | 6.97 (s, 1H), 6.53 - 6.80 (m, 1H), 6.15 (s, 1H), 4.49-4.52 (m, 2H), 4.39 - 4.42 (m, 3H), 3.83 (s, 3H), 3.39 - 3.51 (m, 4H), 2.72 - 2.82 (m, 8H), 2.50 (br s, 3H), 2.01 (s, 2H), 1.82 - 1.85 (m, 2H), 1.62 - 1.79 (m, 4H). |
| M001060 | 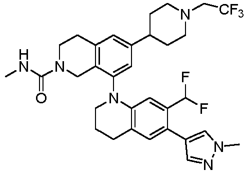 | ¹H NMR (400MHz, DMSO-d6) δ 8.38 (br s, 1H), 7.70 (s, 1H), 7.45 (s, 1H), 7.06 (br d, J = 9.9 Hz, 2H), 6.98 (s, 1H), 6.87 - 6.48 (m, 1H), 6.16 (s, 1H), 4.41 (br d, J = 16.8 Hz, 1H), 4.09 (br d, J = 16.8 Hz, 1H), 3.84 (s, 3H), 3.55 - 3.37 (m, 4H), 3.18 - 3.08 |
| | | (m, 2H), 3.00 - 2.78 (m, 6H), 2.53 (br s, 3H), 2.45 - 2.35 (m, 3H), 2.09 - 1.96 (m, 2H), |
| | | 1.73 - 1.55 (m, 4H). |
| M001061 | 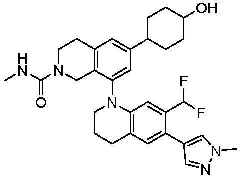 | m/z+=550.3 |
| M001062 | 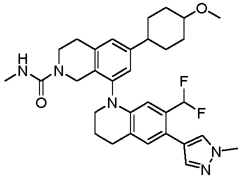 | m/z+=564.3 |
| M001063 | 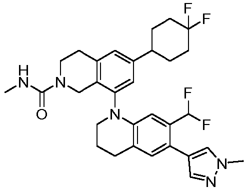 | ¹H NMR (400 MHz, DMSO-d6) d 7.72 (s, 1H), 7.47 (s, 1H), 7.07 (br d, J = 12.72 Hz, 2H), 7.00 (s, 1H), 6.54-6.85 (m, 1H), 6.50 (br d, J = 4.16 Hz, 1 H), 6.17 (s, 1H), 4.41 (br d, J = 16.75 Hz, 1H), 4.12 (br d, J = 16.87 Hz, 1H), 3.85 (s, 3H), 3.45-3.51 (m, 1H), 2.76-2.97 (m, 4H), 2.65-2.71 (m, 1H), 2.54 (br d, J = 3.91 Hz, 3H), 2.08 (s, 9H), 1.82-1.87 (m, 2H), 1.58-1.70 (m, 2H) |
| M001064 | 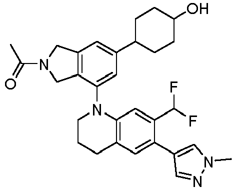 | ¹H NMR (400 MHz, DMSO-d6) d 7.74 (s, 1H), 7.49 (s, 1H), 7.07-7.17 (m, 3H), 6.55-6.92 (m, 1H), 6.25-6.48 (m, 1H), 4.84 (br s, 1H), 4.63 (br s, 1H), 4.54-4.60 (m, 1H), 4.27 (br s, 1H), 3.86 (s, 3H), 3.53 (br s, 3H), 2.87 (br s, 2H), 1.95-2.07 (m, 6H), 1.87-1.93 (m, 2H), 1.71-1.82 (m, 2H), 1.44-1.55 (m, 2H), 1.23-1.31 (m, 2H) |
| M001065 | 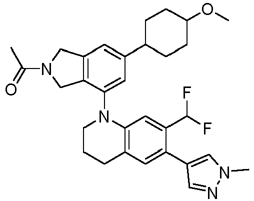 | ¹H NMR (400 MHz, DMSO-*d₆*) δ 7.68 (s, 1H), 7.45 (s, 1H), 7.10 - 7.02 (m, 3H), 6.81 - 6.68 (m, 1H), 4.84 (s, 1H), 4.59 - 4.56 (m, 2H), 4.32 (s, 1H), 3.86 (s, 3H), 3.53 - 3.51 (m, 4H), 3.44 - 3.42 (m, 1H), 3.25 - 3.21 (m, 3H), 2.89 - 2.87 (m, 2H), 2.68 - 2.65 (m, 1H), 2.15 - 1.92 (m, 5H), 1.75 - 1.53 (m, 6H), 1.30 - 1.25 (m, 1H), 0.90 - 0.85 (m, 1H). |
| M001066 | 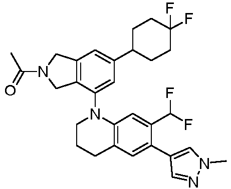 | ¹H NMR (400 MHz, METHANOL-d4) d 7.63 (d, J = 4.00 Hz, 1 H) 7.50 (d, J = 4.13 Hz, 1 H) 7.17 - 7.20 (m, 1 H) 7.12 (m, J = 5.90, 5.90 Hz, 2 H) 6.36 - 6.70 (m, 2 H) 4.78 (s, 1 H) 4.65 (s, 1 H) 4.61 (s, 1 H) 4.46 (s, 1 H) 3.93 (d, J = 1.25 Hz, 3 H) 3.57 - 3.67 (m, 2 H) 2.89 - 2.99 (m, 2 H) 2.69 - 2.82 (m, 1 H) 2.16 (s, 3 H) 2.05 - 2.13 (m, 4 H) 1.76 - 1.99 (m, 6 H). |
| M001067 | 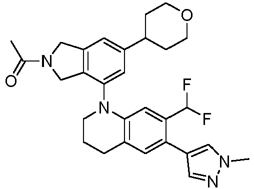 | ¹H NMR (400MHz, DMSO-d6) d 7.75 (s, 1H), 7.49 (s, 1H), 7.33 - 6.98 (m, 3H), 6.97 - 6.56 (m, 1H), 6.55 - 6.23 (m, 1H), 4.86 (br s, 1H), 4.65 (br s, 1H), 4.28 (br s, 1H), 3.95-3.92 (m, 2H), 3.86 (s, 3H), 3.56-3.54 (m, 2H), 3.47 - 3.38 (m, 3H), 2.91 - 2.77 (m, 3H), 2.10 - 1.91 (m, 5H), 1.83 - 1.52 (m, 4H). |
| M001068 | 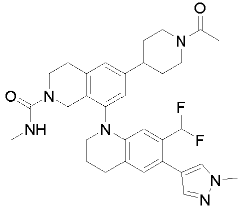 | ¹H NMR (400MHz, CDCl3) d 7.53 (s, 1H), 7.40 (s, 1H), 7.06 (br s, 1H), 7.00 - 6.91 (m, 1H), 6.64 - 6.27 (m, 2H), 4.77 (br d, J = 11.1 Hz, 1H), 4.54 (br d, J = 16.1 Hz, 1H), 4.41 4.29 (m, 1H), 4.17 (br d, J = 14.9 Hz, 1H), 4.01 - 3.89 (m, 1H), 3.81 - 3.69 (m, 1H), 3.60 3.44 (m, 3H), 3.15 (br t, J = 13.8 Hz, 1H), 3.01 - 2.86 (m, 4H), 2.80 (br d, J = 3.9 Hz, 3H), 2.74 - 2.66 (m, 1H), 2.65 - 2.54 (m, 1H), 2.30 - 1.98 (m, 6H), 1.87 (br d, J = 14.5 Hz, 2H) |
| M001069 | 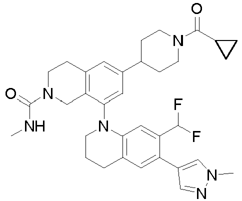 | ¹H NMR (400MHz, CDCl3) d 7.53 (s, 1H), 7.40 (s, 1H), 7.06 (s, 1H), 6.97 (d, J = 4.8 Hz, 2H), 6.64 - 6.28 (m, 2H), 4.76 (br d, J = 12.8 Hz, 1H), 4.54 (br d, J = 16.1 Hz, 1H), 4.43 4.25 (m, 2H), 4.17 (br d, J = 16.3 Hz, 1H), 3.80 - 3.69 (m, 1H), 3.61 - 3.41 (m, 3H), 3.18 (br t, J = 12.0 Hz, 1H), 3.06 - 2.83 (m, 4H), 2.82 - 2.58 (m, 4H), 2.23 - 2.06 (m, 2H), 1.97 - 1.45 (m, 8H), 1.00 (br s, 2H), 0.82 - 0.67 (m, 2H) |
| M001070 | 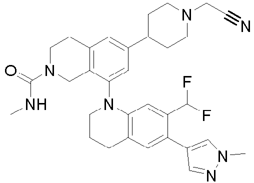 | ¹H NMR (400MHz, CDCl3) d 7.53 (s, 1H), 7.40 (s, 1H), 7.06 (s, 1H), 6.98 (br d, J = 3.7 Hz, 2H), 6.63 - 6.27 (m, 2H), 4.53 (br d, J = 16.3 Hz, 1H), 4.35 (br d, J = 4.4 Hz, 1H), 4.16 (br d, J = 16.4 Hz, 1H), 3.96 (s, 3H), 3.81 - 3.70 (m, 1H), 3.62 - 3.45 (m, 5H), 2.96 - 2.86 (m, 5H), 2.80 (d, J = 4.5 Hz, 3H), 2.56 - 2.42 (m, 3H), 2.20 - 2.07 (m, 2H), 1.95 - 1.86 (m, 2H), 1.83 - 1.73 (m, 2H) |
| M001071 | 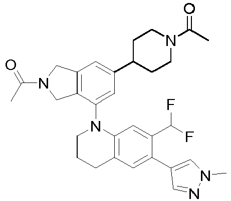 | ¹H NMR (400 MHz, METHANOL-d4) d 7.63 (d, J = 4.00 Hz, 1 H) 7.50 (d, J = 4.13 Hz, 1 H) 7.18 (d, J =4.50 Hz, 1 H) 7.08 - 7.15 (m, 2 H) 6.35 - 6.69 (m, 2 H) 4.93 (s, 1 H) 4.78 (s, 1 H) 4.63 - 4.71 (m, 2 H) 4.43 - 4.49 (m, 1 H) 4.00 - 4.08 (m, 1 H) 3.90 - 3.95 (m, 3 H) 3.58 - 3.67 (m, 2 H) 3.18 - 3.28 (m, 1 H) 2.85 - 2.97 (m, 3 H) 2.67 - 2.77 (m, 1 H) 2.15 (s, 2 H) 2.12 - 2.14 (m, 3 H) 2.08 - 2.12 (m, 2 H) 2.07 (s, 1 H) 1.84 - 2.00 (m, 2 H) 1.54 - 1.79 (m, 2 H). |
| M001072 | 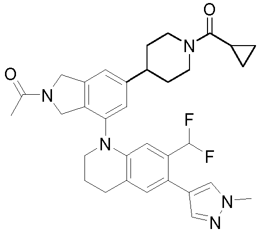 | ¹H NMR (400 MHz, METHANOL-d4) d 7.63 (d, J = 3.88 Hz, 1 H) 7.50 (d, J = 4.13 Hz, 1 H) 7.19 (d, J = 4.13 Hz, 1 H) 7.08 - 7.15 (m, 2 H) 6.36 - 6.69 (m, 2 H) 4.93 (s, 1 H) 4.78 (s, 1 H) 4.62 - 4.70 (m, 2 H) 4.42 - 4.53 (m, 2 H) 3.93 (d, J = 1.38 Hz, 3 H) 3.59 - 3.66 (m, 2 H) 3.21 - 3.29 (m, 1 H) 2.90 - 2.97 (m, 3 H) 2.69 - 2.84 (m, 1 H) 2.16 (s, 2 H) 2.08 - 2.14 (m, 2 H) 2.07 (s, 1 H) 1.96 - 2.04 (m, 2 H) 1.86 - 1.95 (m, 1 H) 1.57 - 1.78 (m, 2 H) 0.86 - 0.91 (m, 2 H) 0.78 - 0.85 (m, 2 H). |
| M001073 | 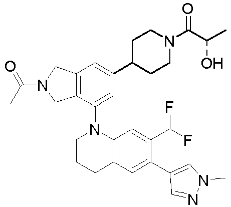 | ¹H NMR (400MHz, DMSO-d6) d 7.75 (s, 1H), 7.49 (s, 1H), 7.28 - 7.02 (m, 3H), 6.90 - 6.58 (m, 1H), 6.51 - 6.24 (m, 1H), 4.85 (br s, 2H), 4.64 (br s, 1H), 4.59 - 4.43 (m, 2H), 4.28-4.25 (m, 1H), 4.10-4.07 (m, 1H), 3.86 (s, 3H), 3.63 - 3.56 (m, 1H), 3.08 (br s, 1H), 2.89-2.85 (m, 3H), 2.75 - 2.52 (m, 2H), 2.12 - 1.96 (m, 5H), 1.83-1.79 (m, 2H), 1.69 - 1.42 (m, 2H), 1.38 - 1.15 (m, 3H). |
| M001074 | 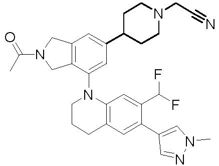 | ¹H NMR (400 MHz, METHANOL-d4) d 7.63 (d, J = 4.00 Hz, 1 H) 7.50 (d, J = 4.00 Hz, 1 H) 7.18 (d, J = 3.13 Hz, 1 H) 7.09 - 7.15 (m, 2 H) 6.36 - 6.68 (m, 2 H) 4.93 (s, 1 H) 4.87 (s, 1 H) 4.62 - 4.68 (m, 1 H) 4.43 - 4.48 (m, 1 H) 3.91 - 3.94 (m, 3 H) 3.68 - 3.70 (m, 2 H) 3.59 - 3.65 (m, 2 H) 2.96 - 3.02 (m, 2 H) 2.90 - 2.95 (m, 2 H) 2.57 - 2.70 (m, 1 H) 2.40 - 2.52 (m, 2 H) 2.16 (s, 2 H) 2.09 - 2.14 (m, 2 H) 2.07 (s, 1 H) 1.89 - 1.98 (m, 2 H) 1.78 - 1.89 (m, 2 H) |
| M001075 | 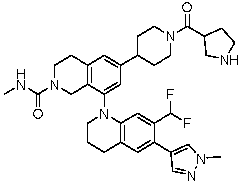 | ¹H NMR (400MHz, CDCl3) δ 7.52 (s, 1H), 7.39 (s, 1H), 6.94 (s, 1H), 6.31-6.59 (m, 2H), 4.71-4.74 (m, 1H), 4.51-4.55 (d, *J =* 16, 1H), 4.41 (s, 1H), 4.11-4.18 (m, 1H), 3.95-3.98 (m, 5H), 3.73 (s, 1H), 3.33-3.72 (m, 8H), 2.92-3.16 (m, 5H), 2.74-2.92 (m, 4H), 2.31 (s, 1H), 2.12 (s, 3H), 1.90-1.97 (m, 2H), 1.60 (s, 1H). |
| M001076 | 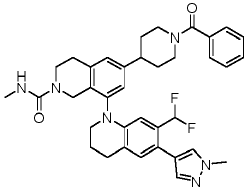 | ¹H NMR (400MHz, CDCl3) d 7.53 (s, 1H), 7.44 - 7.38 (m, 6H), 7.06 (s, 1H), 6.98 (d, J = 4.9 Hz, 2H), 6.64 - 6.24 (m, 2H), 4.95 - 4.78 (m, 1H), 4.54 (br d, J = 16.4 Hz, 1H), 4.36 (br d, J = 4.9 Hz, 1H), 4.17 (br d, J = 16.5 Hz, 1H), 3.96 (s, 3H), 3.93 3.83 (m, 1H), 3.79 - 3.69 (m, 1H), 3.61 - 3.44 (m, 3H), 3.09 (br s, 1H), 3.02 - 2.85 (m, 5H), 2.85 - 2.68 (m, 4H), 2.21 - 2.08 (m, 2H), 2.06 - 1.76 (m, 3H) |
| M001079 | 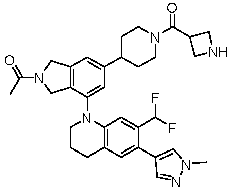 | ¹H NMR (400MHz, DMSO-d6) d 8.36 (s, 1H), 7.75 (s, 1H), 7.49 (s, 1H), 7.31 - 6.97 (m, 3H), 6.92 - 6.57 (m, 1H), 6.54 - 6.28 (m, 1H), 4.85 (br s, 1H), 4.64 (br s, 1H), 4.54 - 4.26 (m, 3H), 4.09 - 4.02 (m, 1H), 3.93 - 3.89 (m, 2H), 3.64-3.45 (m, 6H), 3.08-3.02 (m, 2H), 2.91 - 2.79 (m, 4H), 2.67-2.64 (m, 2H), 2.09 - 1.93 (m, 5H), 1.80-1.77 (m, 2H), 1.59 - 1.30 (m, 2H). |
| M001080 | 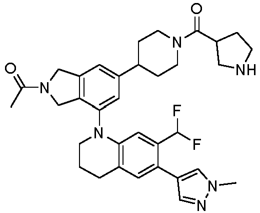 | ¹H NMR (400MHz, CHLOROFORM-d) d = 7.51 (br d, J=6.6 Hz, 1H), 7.39 (br d, J=7.0 Hz, 1H), 7.10 - 6.94 (m, 3H), 6.78 - 6.27 (m, 2H), 4.81 (br d, J=9.8 Hz, 4H), 4.73 (br d, J=11.9 Hz, 2H), 4.55 (br s, 3H), 4.15 - 3.97 (m, 3H), 3.94 (s, 3H), 3.65 - 3.37 (m, 6H), 3.18 (br d, J=9.0 Hz, 2H), 2.98 - 2.87 (m, 2H), 2.83 - 2.63 (m, 2H), 2.37 - 2.11 (m, 4H), 2.11 - 2.02 (m, 5H), 1.96 (br d, J=15.5 Hz, 2H), 1.74 - 1.49 (m, 3H), 1.35 - 1.20 (m, 4H), 0.97 - 0.76 (m, 4H) |
| M001081 | 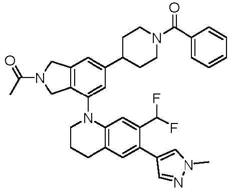 | ¹H NMR (400MHz, CHLOROFORM-d) d = 7.53 (br d, J=7.0 Hz, 1H), 7.56 - 7.48 (m, 1H), 7.42 (br s, 5H), 7.11 - 6.99 (m, 3H), 6.66 - 6.28 (m, 2H), 4.83 (br d, J=5.7 Hz, 3H), 4.57 (br s, 2H), 3.95 (s, 4H), 3.57 (br d, J=5.0 Hz, 2H), 3.11 (br s, 1H), 2.99 - 2.66 (m, 4H), 2.19 - 2.04 (m, 5H), 1.57 (br s, 9H) |
| M001082 | 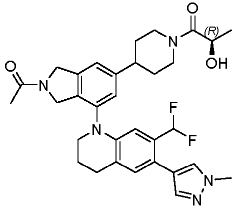 | ¹H NMR (400MHz, DMSO-d6) ä = 7.74 (s, 1H), 7.49 (s, 1H), 7.19 - 7.09 (m, 3H), 6.89 - 6.36 (m, 1H), 4.93 - 4.79 (m, 2H), 4.71 - 4.44 (m, 3H), 4.36 - 4.02 (m, 2H), 3.85 (s, 3H), 3.68 (s, 1H), 3.54 (br s, 1H), 3.23 - 2.97 (m, 2H), 2.86 (br s, 3H), 2.65 (br s, 1H), 2.06 - 2.00 (m, 3H), 1.97 (s, 2H), 1.81 (br s, 2H), 1.76 - 1.35 (m, 3H), 1.35 - 1.24 (m, 1H), 1.23 - 1.09 (m, 3H) |
| M001083 | 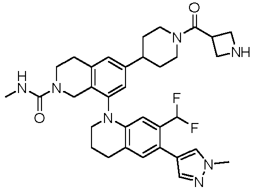 | ¹H NMR (400 MHz, DMSO-d6) d 8.33 (s, 1H), 7.71 (s, 1H), 7.46 (s, 1H), 6.99-7.11 (m, 3H), 6.52-6.86 (m, 1H), 6.48 (br d, J = 4.52 Hz, 1H), 6.17 (s, 1H), 4.36-4.55 (m, 2H), 4.10 (br dd, J = 8.07, 16.63 Hz, 1H), 3.87-3.92 (m, 1H), 3.85 (s, 3H), 3.83 (br s, 1H), 3.71-3.81 (m, 4H), 2.97-3.06 (m, 2H), 2.85-2.92 (m, 3H), 2.82 (br d, J = 6.60 Hz, 2H), 2.69-2.77 (m, 2H), 2.66 (br d, J = 7.58 Hz, 1H), 2.54 (d, J = 4.16 Hz, 3H), 1.93-2.11 (m, 3H), 1.70-1.80 (m, 2H), 1.39-1.54 (m, 2H) |
| M001084 | 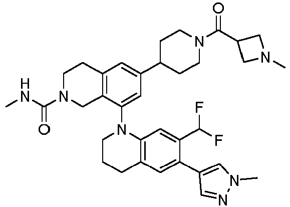 | ¹H NMR (400 MHz, DMSO-d6) d 8.21 (br s, 1H), 7.72 (br s, 1H), 7.46 (br s, 1H), 6.95-7.15 (m, 3H), 6.43-6.88 (m, 2H), 6.16 (br s, 1H), 4.33-4.54 (m, 2H), 4.10 (br d, J = 11.37 Hz, 1H), 3.85 (br s, 3H), 2.66-3.10 (m, 10H), 2.57-2.65 (m, 2H), 2.25 (br s, 2H), 2.07 (br s, 9H), 1.73 (br s, 2H), 1.32-1.57 (m, 2H) |
| M001085 | 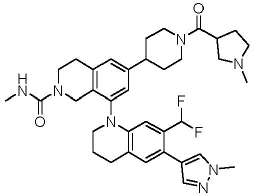 | ¹H NMR (400 MHz, DMSO-d6) d 8.24 (br s, 1H), 7.72 (br s, 1H), 7.46 (br s, 1H), 6.96-7.13 (m, 3H), 6.54-6.88 (m, 1H), 6.49 (br s, 1H), 6.17 (br s, 1H), 4.34-4.60 (m, 2H), 4.10 (br d, J = 13.57 Hz, 2H), 3.94-4.04 (m, 2H), 3.76-3.91 (m, 5H), 2.99-3.11 (m, 3H), 2.69-2.95 (m, 9H), 2.28 (br s, 2H), 1.88-2.16 (m, 6H), 1.76 (br s, 2H), 1.28-1.59 (m, 3H) |
| M001086 | 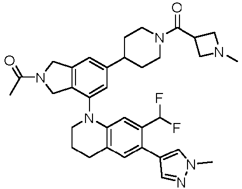 | ¹H NMR (400MHz, DMSO-d6) d 8.23 (s, 1H), 7.75 (s, 1H), 7.49 (s, 1H), 7.21 - 7.08 (m, 3H), 6.91 - 6.56 (m, 1H), 6.51 - 6.25 (m, 1H), 4.84 (br s, 1H), 4.64 (br s, 1H), 4.51-4.48 (m, 1H), 4.27-4.24 (m, 1H), 3.86 (s, 3H), 3.39 - 3.16 (m, 6H), 3.12 - 2.99 (m, 2H), 2.97 - 2.73 (m, 4H), 2.57-2.34 (m, 2H), 2.25 (s, 3H), 2.06 - 1.95 (m, 5H), 1.81 - 1.71 (m, 2H), 1.58 - 1.42 (m, 2H). |
| M001087 | 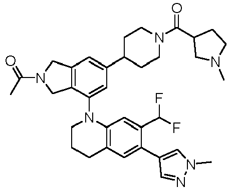 | ¹H NMR (400MHz, DMSO-d6) d = 8.18 (br s, 1H), 7.73 (s, 1H), 7.48 (s, 1H), 7.19 - 7.09 (m, 3H), 6.89 - 6.53 (m, 1H), 6.48 - 6.28 (m, 1H), 4.84 (br s, 1H), 4.68 - 4.42 (m, 3H), 4.27 (br s, 1H), 4.02 (br s, 1H), 3.85 (s, 3H), 3.53 (br s, 1H), 3.07 (br s, 1H), 2.86 (br s, 3H), 2.80 (br s, 2H), 2.32 - 2.24 (m, 3H), 2.02 (s, 2H), 1.99 (br s, 2H), 1.97 (br s, 3H), 1.79 (br d, J=11.9 Hz, 3H) |
| M001088 | 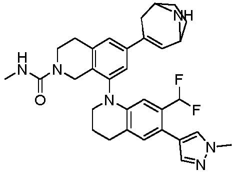 | ¹H NMR (400 MHz, DMSO-d6) d 8.35 (s, 1H), 7.72 (s, 1H), 7.47 (s, 1H), 7.22 (br t, J = 9.72 Hz, 2H), 7.10 (s, 1H), 6.55-6.86 (m, 1H), 6.46-6.55 (m, 2H), 6.14 (s, 1H), 4.46 (br d, J = 17.24 Hz, 1H), 4.08 (br d, J = 17.24 Hz, 1H), 3.93-3.99 (m, 2H), 3.86 (s, 3H), 2.82-2.91 (m, 4H), 2.54 (br d, J = 4.16 Hz, 3H), 2.29-2.38 (m, 1H), 2.08 (s, 9H), 1.83-1.97 (m, 1H), 1.66 (br s, 1H) |
| M001089 | 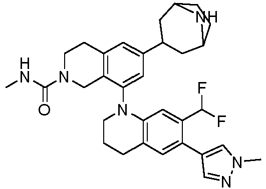 | ¹H NMR (400 MHz, DMSO-d6+D2O) d 8.40 (br s, 1H), 7.71 (br s, 1H), 7.46 (br s, 1H), 7.14-7.33 (m, 2H), 7.09 (br s, 1H), 6.45-6.88 (m, 1H), 6.11 (br s, 1H), 4.41 (br d, J = 15.77 Hz, 1H), 4.17 (br d, J = 16.26 Hz, 1H), 3.85 (br s, 4H), 3.10 (br s, 1H), 2.74-2.99 (m, 5H), 2.54 (br s, 4H), 2.24 (br s, 4H), 2.06 (br s, 5H), 1.76 (br s, 2H), 1.56 (br s, 1H), 1.22 (br s, 1H) |
| M001090 | 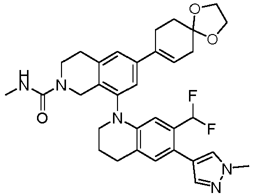 | ¹H NMR (400MHz, DMSO-d6) d 7.73 (s, 1H), 7.48 (s, 1H), 7.31 - 7.05 (m, 3H), 6.89 - 6.45 (m, 2H), 6.17 (s, 1H), 6.02 (br s, 1H), 4.45 (br d, J=16.9 Hz, 1H), 4.10 (br d, J = 16.8 Hz, 1H), 3.91 (s, 4H), 3.86 (s, 3H), 3.63 - 3.49 (m, 4H), 2.99 - 2.75 (m, 4H), 2.55 (br d, J=4.0 Hz, 5H), 2.34 (br s, 2H), 2.05 (br s, 2H), 1.79 (br t, J = |
| | | 6.3 Hz, 2H) |
| M001092 | 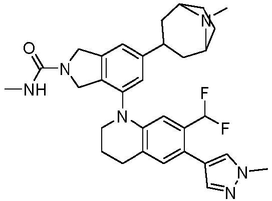 | ¹H NMR (400MHz, DMSO-d6) d = 7.63 (s, 1H), 7.42 - 7.30 (m, 2H), 7.26 (br s, 1H), 7.22 6.95 (m, 2H), 6.78 - 6.47 (m, 1H), 6.19 (br s, 2H), 4.53 (br s, 2H), 4.25 (br s, 2H), 3.87 (br s, 1H), 3.75 (br s, 3H), 3.62 - 3.45 (m, 3H), 3.09 (br s, 1H), 2.79 (br s, 3H), 2.54 (br d, J=4.0 Hz, 3H), 2.48 (br s, 3H), 2.36 - 2.16 (m, 4H), 1.93 (br s, 4H), 1.73 (br s, 1H), 1.61 1.48 (m, 2H) |
| M001093 | 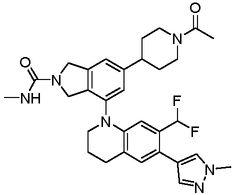 | ¹H NMR (400MHz, CHLOROFORM-d) d = 7.48 (s, 1H), 7.35 (s, 1H), 7.00 (s, 1H), 6.95 (s, 2H), 6.57 - 6.26 (m, 2H), 4.80 - 4.65 (m, 3H), 4.40 (br s, 2H), 4.19 (br d, J=4.4 Hz, 1H), 3.90 (s, 3H), 3.86 (br s, 1H), 3.51 (br s, 2H), 3.11 (br t, J=12.6 Hz, 1H), 2.86 (br s, 2H), 2.80 (d, J=4.4 Hz, 3H), 2.72 (br d, J=11.7 Hz, 1H), 2.61 - 2.49 (m, 1H), 2.07 (s, 3H), 2.06 2.01 (m, 2H), 1.86 (br t, J=14.5 Hz, 3H) |
| M001094 | 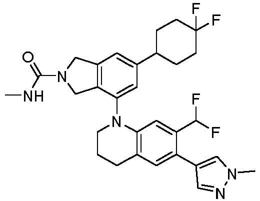 | ¹H NMR (400 MHz, DMSO-d6) d 7.81 (s, 1H), 7.56 (s, 1H), 7.22 (s, 1H), 7.17 (br d, J = 6.85 Hz, 2H), 6.64-6.98 (m, 1H), 6.42 (s, 1H), 6.36 (br d, J = 3.79 Hz, 1H), 4.66 (br s, 2H), 4.26-4.49 (m, 2H), 3.93 (s, 3H), 3.61 (br s, 1H), 2.94 (br s, 2H), 2.79-2.85 (m, 1H), 2.62-2.66 (m, 3H), 2.12-2.21 (m, 2H), 2.03-2.11 (m, 3H), 1.96 (br d, J = 11.00 Hz, 2H), 1.67-1.82 (m, 2H), 1.22-1.40 (m, 2H) |
| M001095 | 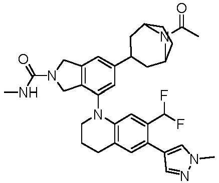 | ¹H NMR (400MHz, DMSO-d6) δ = 7.74 (s, 1H), 7.49 (s, 1H), 7.20 (br d, J=18.6 Hz, 2H), 6.90 - 6.60 (m, 1H), 6.36 - 6.24 (m, 2H), 4.60 (br s, 2H), 4.52 - 4.22 (m, 3H), 3.87 (s, 3H), 3.56 (br s, 1H), 2.89 (br s, 2H), 2.59 (d, J=4.3 Hz, 3H), 2.44 - 2.25 (m, 3H), 1.82 (br d, J=11.1 Hz, 2H), 1.73 - 1.59 (m, 3H), 1.24 (s, 1H) |
| M001096 | 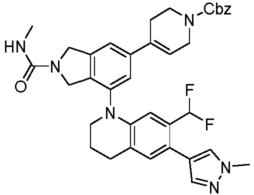 | ¹H NMR (400MHz, CHLOROFORM-d) d = 7.52 (s, 1H), 7.39 (s, 1H), 7.37 - 7.35 (m, 3H), 7.14 (s, 2H), 7.04 (s, 1H), 6.60 - 6.31 (m, 2H), 5.16 (s, 2H), 4.72 (br s, 2H), 4.44 (br s, 2H), 4.22 (br d, J=4.5 Hz, 1H), 4.14 (br s, 2H), 3.94 (s, 3H), 3.71 (br s, 2H), 3.56 (br s, 2H), 2.90 (br s, 2H), 2.84 (d, J=4.5 Hz, 3H), 2.52 (br s, 2H), 2.13 - 2.05 (m, 2H) |
| M001097 | 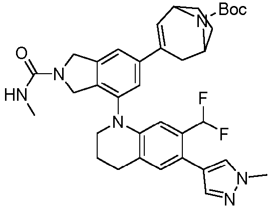 | ¹H NMR (400MHz, CHLOROFORM-d) d 7.55 (br s, 1H), 7.42 (br s, 1H), 7.16 (br s, 2H), 7.06 (br s, 1H), 6.67 - 6.26 (m, 3H), 4.74 (br s, 1H), 4.58 - 4.36 (m, 4H), 4.25 (br s, 1H), 3.96 (s, 3H), 3.58 (br s, 2H), 2.99 - 2.86 (m, 4H), 2.29 - 2.17 (m, 2H), 2.112-2.09 (, 2H), 2.03-1.96 (m, 2H), 1.78 - 1.55 (m, 3H), 1.48 (s, 9H). |
| M001098 | 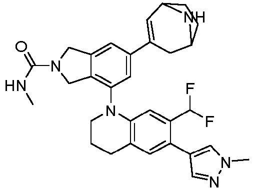 | ¹H NMR (400MHz, DMSO-d6) d 8.32 (br s, 1H), 7.74 (s, 1H), 7.49 (s, 1H), 7.41 - 7.23 (m, 2H), 7.12 (s, 1H), 7.18 - 7.06 (m, 1H), 6.90 - 6.58 (m, 1H), 6.33-6.30 (m, 2H), 4.61 (br s, 2H), 4.29 (br s, 1H), 4.05 (br s, 1H), 3.86 (s, 3H), 3.19 - 3.02 (m, 4H), 2.90-2.86 (m, 3H), 2.58 (br s, 3H), 2.47 - 2.27 (m, 2H), 2.25 - 1.52 (m, 6H). |
| M001099 | 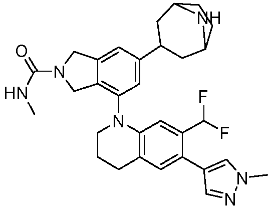 | ¹ H NMR (400MHz, DMSO-d6) d= 8.46 (br s, 1H), 7.73 (s, 1H), 7.48 (s, 1H), 7.36 (s, 1H), 7.28 (s, 1H), 7.15 - 7.08 (m, 1H), 6.98 - 6.49 (m, 1H), 6.30 (s, 2H), 4.62 (br s, 2H), 4.35 (br s, 2H), 3.98 (br s, 2H), 3.86 (s, 3H), 3.54 (br s, 2H), 3.16 (br s, 1H), 2.88 (br s, 2H), 2.76 (br s, 2H), 2.67 (br s, 3H), 2.58 (br d, J=4.2 Hz, 4H), 2.33 (br s, 3H), 2.29 - 2.19 (m, 2H), 2.04 (br d, J=4.9 Hz, 2H), 1.81 (br s, 2H), 1.74 - 1.60 (m, 2H) |
| M001101 | 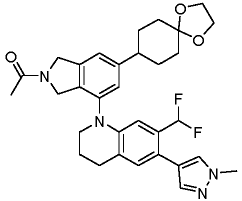 | ¹H NMR (400MHz, CHLOROFORM-d) d 7.53 (br d, J=6.8 Hz, 1H), 7.39 (br s, 1H), 7.10 - 7.01 (m, 3H), 6.63 - 6.35 (m, 2H), 4.82 (br s, 2H), 4.55 (br s, 2H), 3.98 (br s, 4H), 3.95 (br s, 3H), 3.59-3.55 (m, 2H), 2.92-2.87 (m, 2H), 2.60-5.56 (m, 1H), 2.17-2.13 (m, 2H), 2.09-2.05 (m, 4H), 1.90-1.86 (m, 4H), 1.82-1.74 (m, 2H), 1.72-1.65 (m, 3H), 1.24 (br s, 1H). |
| M001102 | 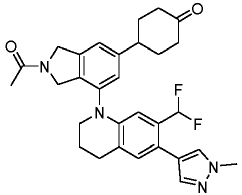 | ¹H NMR (400MHz, CHLOROFORM-d) d 7.52 (br s, 1H), 7.39 (br s, 1H), 7.11 - 7.02 (m, 3H), 6.65 - 6.29 (m, 2H), 4.84 (br s, 2H), 4.56 (br s, 2H), 3.94 (br s, 3H), 3.57 (br s, 2H), 3.11 - 2.88 (m, 3H), 2.52-2.48 (m, 4H), 2.23-2.20 (m, 2H), 2.16-2.14 (m, 2H), 2.09-2.06 (m, 4H), 1.96-1.92 (m, 3H). |
| M001103 | 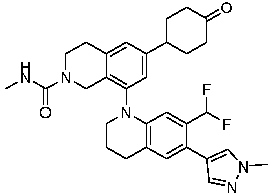 | ¹H NMR (400 MHz, DMSO-d6) d 7.72 (br s, 1H), 7.47 (br s, 1H), 7.09 (br d, J = 5.99 Hz, 2H), 6.55-6.89 (m, 1H), 6.48 (br s, 1H), 6.18 (br s, 1H), 4.42 (br d, J = 16.02 Hz, 1H), 4.05-4.21 (m, 1H), 3.75-3.98 (m, 4H), 2.76-3.11 (m, 6H), 2.62-2.71 (m, 1H), 2.53-2.62 (m, 6H), 2.24 (br d, J = 12.84 Hz, 2H), 1.75-2.12 (m, 6H) |
| M001116 | 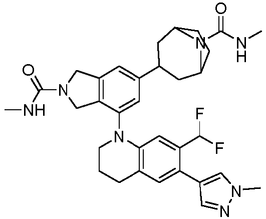 | ¹H NMR (400MHz, CHLOROFORM-d) δ = 7.47 (s, 1H), 7.35 (s, 1H), 7.04 - 6.97 (m, 2H), 6.57 - 6.36 (m, 2H), 4.66 (br s, 2H), 4.45 - 4.29 (m, 3H), 4.18 (br d, J=4.3 Hz, 2H), 3.90 (s, 4H), 3.50 (br s, 1H), 2.92 - 2.85 (m, 2H), 2.80 (d, J=4.6 Hz, 4H), 2.75 (br d, J=4.6 Hz, 1H), 2.45 (td, J=7.2, 14.2 Hz, 2H), 2.07 - 2.01 (m, 2H), 2.01 - 1.95 (m, 2H), 1.20 (s, 6H), 0.87 - 0.72 (m, 3H) |
| M001117 | 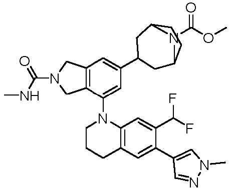 | ¹H NMR (400MHz, CHLOROFORM-d) δ = 7.54 (s, 1H), 7.40 (s, 1H), 7.05 (br s, 3H), 6.54 (s, 1H), 6.45 (s, 1H), 4.72 (br s, 2H), 4.06 - 3.88 (m, 4H), 3.79 - 3.66 (m, 4H), 3.56 (br s, 2H), 2.95 - 2.83 (m, 6H), 2.75 - 2.66 (m, 1H), 2.50 (br s, 2H), 2.17 - 1.91 (m, 6H), 1.38 - 1.22 (m, 5H). |
| M001118 | 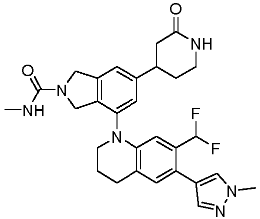 | ¹H NMR (400 MHz, DMSO-d6) δ 7.74 (s, 1H), 7.54 (s, 1H), 7.49 (s, 1H), 7.18 (s, 1H), 7.15 (s, 1H), 7.12 (s, 1H), 6.74 (m, 1H), 6.36 (s, 1H), 6.28 (d, J = 4.0 Hz, 1H), 4.60 (s, 2H), 4.30 (s, 2H), 3.86 (s, 3H), 3.55 (s, 2H), 3.25 - 3.18 (m, 2H), 3.10 (d, J = 4.0 Hz, 1H), 2.88 (s, 2H), 2.58 (d, J = 4.0 Hz, 3H), 2.36 (m, 2H), 2.05 - 1.99 (m, 2H), 1.93 (d, J = 12.0 Hz, 1H), 1.85 (m, 1H). |
| M001119 | 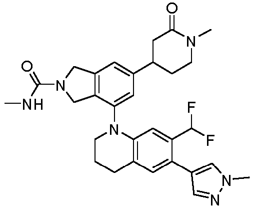 | ¹H NMR (400 MHz, DMSO-d6) δ 7.74 (s, 1H), 7.49 (s, 1H), 7.18 (s, 1H), 7.12 (d, J = 6.4 Hz, 2H), 6.74 (t, J = 55.2 Hz, 1H), 6.35 (s, 1H), 6.28 (q, J = 4.0 Hz, 1H), 4.60 (s, 2H), 4.30 (s, 2H), 3.86 (s, 3H), 3.55 (s, 2H), 3.41 - 3.35 (m, 1H), 3.31 - 3.25 (m, 1H), 3.16 - 3.09 (m, 1H), 2.87 (d, J = 5.6 Hz, 2H), 2.83 (s, 3H), 2.58 (d, J = 4.4 Hz, 3H), 2.48 - 2.33 (m, 2H), 2.04 - 1.93(m, 4H). |
| M001120 | 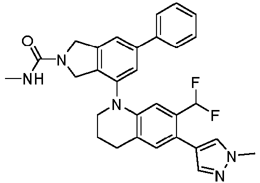 | ¹H NMR (400 MHz, DMSO-d6) d 7.75 (s, 1H), 7.69 (br d, J = 7.70 Hz, 2H), 7.56 (s, 1H), 7.48-7.51 (m, 2H), 7.44-7.47 (m, 1H), 7.33-7.41 (m, 1H), 7.14 (s, 1H), 6.60-6.91 (m, 1H), 6.29-6.46 (m, 2H), 4.68 (br s, 2H), 4.26-4.47 (m, 2H), 3.85 (s, 3H), 3.63 (br s, 1H), 2.86-2.93 (m, 2H), 2.55-2.63 (m, 4H), 1.98-2.09 (m, 2H) |
| M001121 | 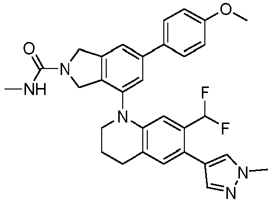 | ¹H NMR (400 MHz, DMSO-d6) d 7.60-7.75 (m, 2H), 7.50-7.53 (m, J = 8.60 Hz, 2H), 7.42-7.48 (m, 1H), 7.08-7.19 (m, 1H), 6.99-7.07 (m, 3H), 6.39-6.79 (m, 1H), 6.28-6.37 (m, 1H), 4.53-4.75 (m, 4H), 4.24-4.42 (m, 1H), 3.86 (s, 2H), 3.81 (s, 2H), 3.80 (s, 3H), 2.82-2.94 (m, 1H), 2.53-2.69 (m, 5H), 1.93-2.11 (m, 1H) |
| M001122 | 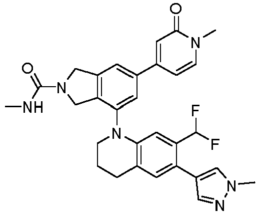 | ¹H NMR (400 MHz, DMSO-d6) δ 7.71 (d, J = 5.6 Hz, 2H), 7.57 (s, 1H), 7.52 (s, 1H), 7.47 (s, 1H), 7.14 (s, 1H), δ 6.71 (t, J = 55.2 Hz, 1H), 6.68 (d, J = 2.0 Hz, 1H) 6.54 (dd, J = 7.2, 2.0 Hz, 1H), 6.44 (s, 1H), 6.11 (dd, J = 8.4, 4.0 Hz, 1H), 4.82 - 4.70 (m, 2H), 4.50 - 4.41 (m, 2H), 3.87 (s, 3H), 3.68 - 3.59 (m, 2H), 3.46 (s, 3H), 2.91 (t, J = 6.0 Hz, 2H), 2.63 (d, J = 4.4 Hz, 3H), 2.11 - 2.04 (m, 2H). |
| M001123 | 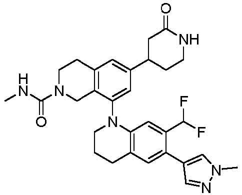 | ¹H NMR (400 MHz, DMSO-d6) δ 7.72 (s, 1H), 7.53 (s, 1H), 7.47 (s, 1H), 7.11 - 7.04 (m, 3H), 6.70 (m, 1H), 6.48 (d, J = 4.0 Hz, 1H), 6.18 (s, 1H), 4.48 - 4.40 (m, 1H), 4.11 (m, 1H), 3.86 (s, 3H), 3.55 - 3.41 (m, 4H), 3.19 (d, J = 8.0 Hz, 2H), 3.01 (s, 2H), 2.88 (s, 1H), 2.86 - 2.81 (m, 2H), 2.54 (d, J = 4.0 Hz, 3H), 2.31 (m, 2H), 2.04 (s, 2H), 1.87 (s, 1H), 1.79 (s, 1H). |
| M001124 | 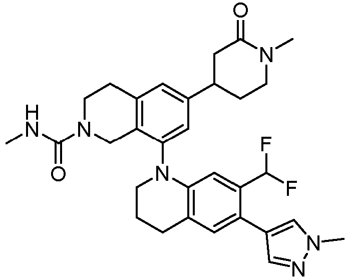 | ¹H NMR (400 MHz, DMSO-d6) δ 7.72 (s, 1H), 7.47 (s, 1H), 7.06 (m, 3H), 6.70 (m, 1H), 6.48 (d, J = 4.0 Hz, 1H), 6.17 (s, 1H), 4.43 (d, J = 16.0 Hz, 1H), 4.16 - 4.08 (m, 1H), 3.86 (s, 3H), 3.59 - 3.41 (m, 4H), 3.28 - 3.22 (m, 1H), 3.05 (s, 1H), 2.81 (m, 7H), 2.54 (d, J = 4.0 Hz, 3H), 2.43 - 2.30 (m, 2H), 2.18 - 1.81 (m, 5H). |
| M001125 | 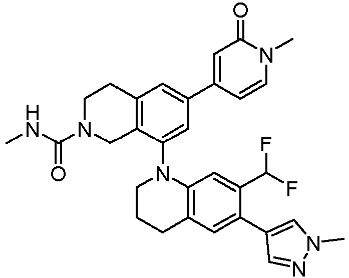 | ¹H NMR (400 MHz, DMSO-d6) δ 7.78 - 7.72 (m, 2H), 7.56 (s, 1H), 7.52 (d, J = 1.6 Hz, 1H), 7.48 (s, 1H), 7.12 (s, 1H), 6.74 (m, 2H), 6.58 (m, 1H), 6.54 (d, J = 4.0 Hz, 1H), 6.20 (s, 1H), 4.51 (d, J = 16.0 Hz, 1H), 4.16 (d, J = 16.0 Hz, 1H), 3.86 (s, 3H), 3.60 (m, 2H), 3.53 - 3.40 (m, 5H), 2.90 (m, 4H), 2.55 (d, J = 4.0 Hz, 3H), 2.08 (d, J = 4.0 Hz, 2H). |
| M001126 | 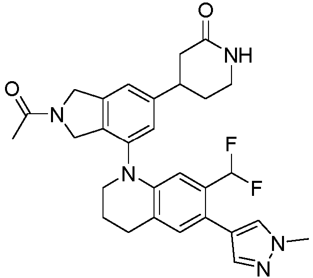 | ¹H NMR (400 MHz, DMSO-*d₆*) δ 7.75 (s, 1H), 7.10 (s, 1H), 7.49 (s, 1H), 7.50 - 7.13 (m, 3H), 6.75 - 6.25 (m, 1H), 6.44 - 6.37 (m, 1H), 4.86 - 4.66 (m, 2H), 4.65 - 4.28 (m, 2H), 3.86 (s, 3H), 3.60 - 3.50 (m, 2H), 3.25 - 3.15 (m, 2H), 3.14 - 3.05 (m, 1H), 2.87 (s, 2H), 2.45 - 2.25 (m, 2H), 2.20 - 1.75 (m, 7H). |
| M001127 | 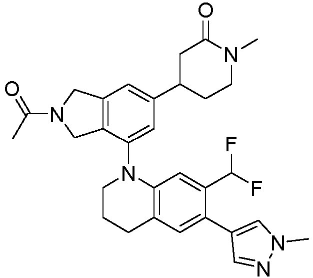 | ¹H NMR (400 MHz, DMSO-d6) δ 7.75 (s, 1H), 7.49 (s, 1H), 7.19 (d, J = 14.4 Hz, 2H), 7.13 (d, J = 5.6 Hz, 1H), 6.75 (t, J = 55.2 Hz, 1H), 6.40 (d, J = 26 Hz, 1H), 4.86 (s, 1H), 4.65 (s, 1H), 4.28 (s, 1H), 3.86 (s, 3H), 3.66 - 3.47 (m, 2H), 3.45 - 3.35 (m, 1H), 3.31 - 3.23 (m, 1H), 3.19 - 3.03 (m, 1H), 2.87 (s, 2H), 2.84 (s, 3H), 2.82 (s, 1H), 2.49 - 2.42 (m, 1H), 2.43 - 2.35 (m, 1H), 2.03 (s, 3H), 2.02 - 1.99 (m, 2H), 1.98 - 1.86 (m, 2H). |
| M001128 | 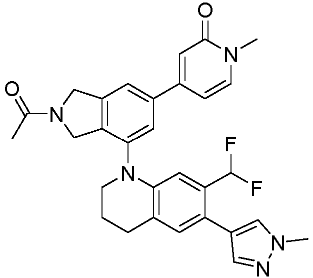 | ¹H NMR (400 MHz, DMSO) δ 7.79 (d, J = 7.2 Hz, 1H), 7.76 - 7.75 (m, 1H), 7.64 (d, J = 13.2 Hz, 1H), 7.59 (d, J = 9.6 Hz, 1H), 7.50 (s, 1H), 7.16 (s, 1H), 6.83 (d, J = 55.2 Hz, 1H), 6.71 (dd, J = 16.8, 1.6 Hz, 1H), 6.61 - 6.55 (m, 1H), 6.44 (d, J = 26.8 Hz, 1H), 4.93 (s, 1H), 4.72 (s, 1H), 4.34 (s, 1H), 3.86 (s, 3H), 3.62 (s, 2H), 3.45 (d, J = 4.4 Hz, 3H), 2.89 (s, 2H), 2.06 (s, 3H), 2.07 - 2.00 (m, 1H), 2.04 - 1.95 (m, 2H). |
| M001129 | 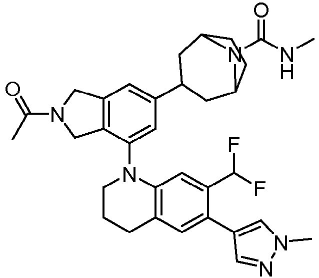 | ¹H NMR (400 MHz, DMSO-d6) δ 7.74 (s, 1H), 7.49 (s, 1H), 7.08 (m, 3H), 6.73 (m, 1H), 6.35 (m, 2H), 4.84 (s, 1H), 4.64 (s, 1H), 4.23 (s, 3H), 3.86 (s, 3H), 3.54 (d, J = 4.0 Hz, 2H), 3.18 (s, 2H), 2.87 (s, 2H), 2.58 (m, 3H), 2.04 - 1.97 (m, 5H), 1.89 - 1.76 (m, 4H), 1.67 (m, 4H). |
| M001132 | 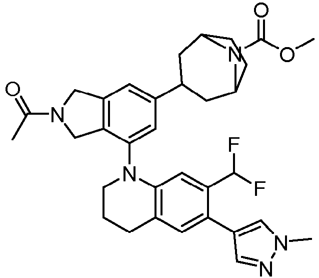 | ¹H NMR (400 MHz, DMSO-d6) δ 7.74 (s, 1H), 7.49 (s, 1H), 7.19 - 7.07 (m, 3H), 6.73 (m, 1H), 6.38 (d, J = 24.0 Hz, 1H), 4.84 (s, 1H), 4.64 (s, 1H), 4.23 (s, 3H), 3.86 (s, 3H), 3.61 (t, J = 4.0 Hz, 3H), 3.58 - 3.52 (m, 2H), 3.30 - 3.18 (m, 2H), 2.87 (s, 2H), 2.04 - 1.97 (m, 5H), 1.87 (d, J = 36.0 Hz, 4H), 1.72 (s, 4H). |
| M001137 | 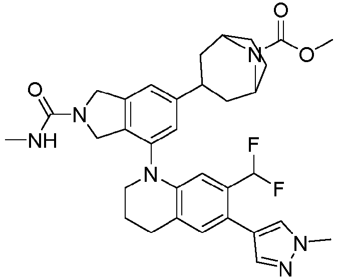 | ¹H NMR (400 MHz, DMSO-*d₆*) δ 7.73 (s, 1H), 7.48 (s, 1H), 7.20 (d, *J* = 22.4 Hz, 1H), 7.13 - 7 |
| | | .08 (m, 2H), 6.73 (t, *J* = 55.2 Hz, 1H), 6.33 (s, 1H), 6.28 (d, *J* = 4.4 Hz, 1H), 4.59 (s, 2H), 4.29 (s, 2H), 4.22 - 4.19 (m, 2H), 3.86 (s, 3H), 3.61 (d, *J* = 4.4 Hz, 3H), 3.54 (s, 2H), 3.24 - 3.21 (m, 1H), 2.92 - 2.87 (m, 2H), 2.66 - 2.57 (m, 1H), 2.58 - 2.56 (m, 3H), 2.34 - 2.19 (m, 1H), 2.03 - 2.00 (m, 2H), 1.91 - 1.82 (m, 2H), 1.73 - 1.68 (m, 2H), 1.67 - 1.61 (m, 2H). |
| M001142 | 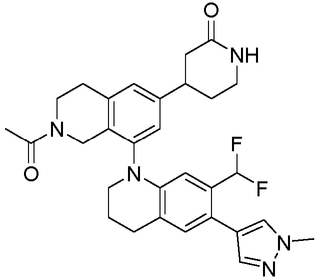 | ¹H NMR (400 MHz, DMSO-d6) δ 7.72 (s, 1H), 7.54 (s, 1H), 7.47 (s, 1H), 7.15 - 7.07 (m, 3H), 6.84-6.57 (m, 1H), 6.18 (d, J = 3.6 Hz, 1H), 4.51-4.47 (m, 1H), 4.30-4.23 (m, 1H), 3.86 (s, 3H), 3.71 - 3.46 (m, 4H), 3.24 - 3.16 (m, 2H), 3.10 - 2.76 (m, 6H), 2.39 - 2.24 (m, 2H), 2.05 (s, 3H), 1.93 - 1.74 (m, 3H). |
| M001143 | 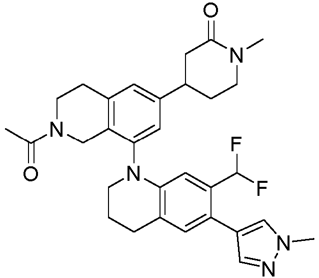 | ¹H NMR (400 MHz, DMSO-*d₆*) δ 7.72 (s, 1H), 7.47 (s, 1H), 7.10 (s, 2H), 7.06 (s, 1H), 6.69 (t, *J* = 55.2 Hz, 1H), 6.18 (s, 1H), 4.52 - 4.47(m, 1H), 4.31 - 4.24 (m, 1H), 3.85 (s, 3H), 3.70 - 3.58 (m, 2H), 3.58 - 3.47 (m, 1H), 3.46 - 3.33 (m, 3H), 3.31 - 3.21 (m, 3H), 3.13 - 3.02 (m, 1H), 3.02 - 2.85 (m, 4H), 2.82 (d, *J* = 8.8 Hz, 4H), 2.44 - 2.30 (m, 2H), 2.05 (s, 3H), 2.02 - 1.92 (m, 3H), 1.87 (s, 1H). |
| M001144 | 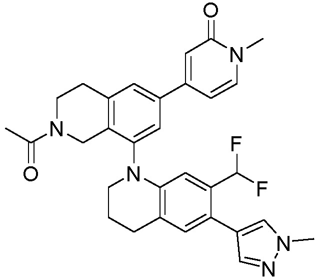 | ¹H NMR (400 MHz, DMSO-*d₆*) δ 7.76 (s, 1H), 7.73 (s, 1H), 7.60 (s, 1H), 7.56 (s, 1H), 7.48 (s, 1H), 7.13 (s, 1H), 6.72 (s, 1H) 6.70 (t, *J* = 57.2 Hz, 1H). 6.60 - 6.56 (m, 1H), 6.21 (s, 1H), 4.60 - 4.54(m, 1H), 4.37 - 4.29(m, 1H), 3.86 (s, 3H), 3.68 - 3.63 (m, 3H), 3.44 (s, 4H), 3.01 - 2.49 (m, 4H), 2.07 (s, 1H), 1.89 (s, 1H). |
| M001153 | 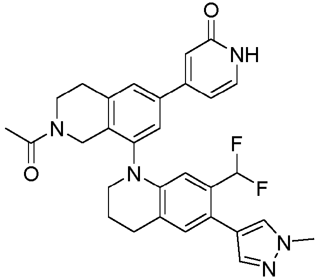 | ¹H NMR (400 MHz, DMSO-d6) δ 11.59 (s, 1H), 7.73 (s, 1H), 7.57 (d, J = 2.4 Hz, 1H), 7.53 (d, J = 2.8 Hz, 1H), 7.48 (s, 1H), 7.43 (d, J = 3.6 Hz, 1H), 7.13 (d, J = 2.4 Hz, 1H), 6.85-6.58 (m, 1H), 6.62 (s, 1H),6.52 (d, J = 3.2 Hz, 1H), 6.21 (d, J = 5.6 Hz, 1H), 4.58 (d, J = 8.6 Hz, 1H), 4.37-4.30 (m, 1H), 3.86 (s, 3H), 3.67-3.62 (m, 3H), 3.50 - 3.41 (m, 1H), 3.08 - 2.84 (m, 4H), 1.98-1.89 (m, 5H) |
| M001154 | 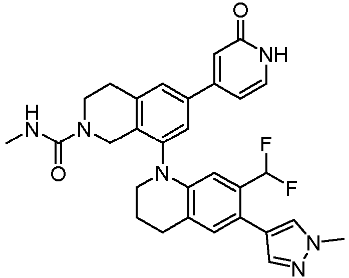 | m/z+=545.2 |
| M001155 | 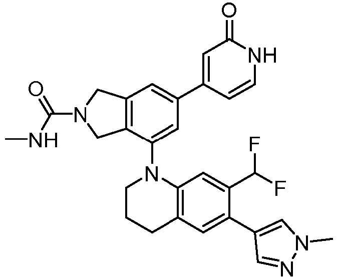 | ¹H NMR (400 MHz, DMSO-*d₆*) δ 7.73 (s, 1H), 7.60 - 7.42 (m, 4H), 7.13 (s, 1H), 6.80 - 6.50 (m, 1H), 6.38 - 6.33 (m, 2H), 4.65 - 4.60 (m, 2H), 4.35 - 4.34 (m, 2H), 3.84 (s, 3H), 3.45 - 3.43 (m, 2H), 2.88 - 2.80 (m, 2H), 2.55 - 2.52 (m, 3H), 2.03 - 1.98 (m, 2H). |
| M001156 | 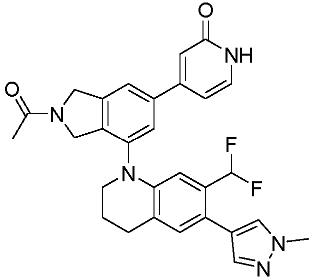 | ¹H NMR (400 MHz, DMSO-*d₆*) δ 11.31 (s, 1H), 7.70 (s, 1H), 7.57 - 7.39 (m, 4H), 7.13 (s, 1H), 6.85 - 6.53 (m, 1H), 6.50 - 6.44 (m, 3H), 4.93 (s, 1H), 4.73 - 4.66 (m, 2H), 4.38 (s, 1H), 3.80 (s, 3H), 3.63 - 3.61 (m, 2H), 2.90 - 2.88 (m, 2H), 2.10 - 1.98 (m, 5H). |
| M001161 | 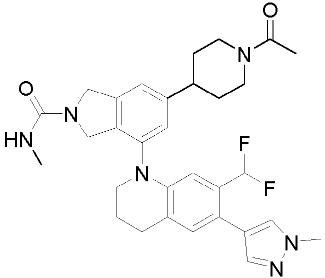 | ¹H NMR (400 MHz, DMSO-*d₆*) δ 7.74 (s, 1H), 7.49 (s, 1H), 7.15 (s, 1H), 7.11 (s, 2H), 6.87-6.60 (s, 1H), 6.35 (s, 1H), 6.28 (d, *J =* 2.4 Hz, 1H), 4.59 (s, 2H), 4.51 (d, *J* = 6.4 Hz, 1H), 4.30 (s, 1H), 3.92 (s, 1H), 3.86 (s, 3H), 3.54 (s, 2H), 3.13-3.17 (m, 1 H), 2.8 7 --2. 8 1 (m, 4H), 2.58 (d, *J* = 2.4 Hz, 4H), 2.01 (d, *J =* 3.2 Hz, 5H), 1.79-1.76 (m, 2H), 1.61-1.59 (m, 1H), 1.45-1.42 (m, 1H). |
| M001162 | 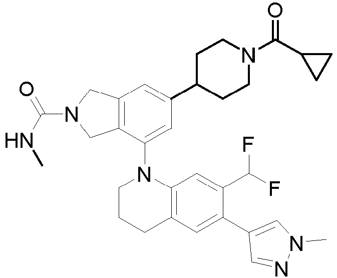 | ¹H NMR (400 MHz, DMSO-*d₆*) δ 7.74 (s, 1H), 7.49 (s, 1H), 7.16 (s, 1H), 7.11 (s, 2H), 6.87 -6.59 (m, 1H), 6.36 (s, 1H), 6.28 (d, *J* = 2.2 Hz, 1H), 4.59 (s, 2H), 4.52 (d, *J =* 5.6 Hz, 1H), 4.40 - 4.25 (m, 3H), 3.86 (s, 3H), 3.55 (s, 2H), 3.17 (d, *J* = 6.0 Hz, 1H), 2.90 - 2.82 (m, 3H), 2.59-2.49 (m, 4H), 2.01-1.98 (m, 3H), 1.89 - 1.73 (m, 2H), 1.60 (d, *J* = 5.6 Hz, 1H), 1.48 (s, 1H), 0.69 (d, *J* = 4.4 Hz, 4H). |
| M001163 | 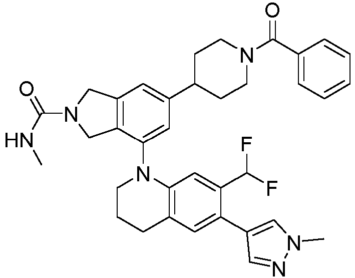 | ¹H NMR (400 MHz, DMSO-*d₆*) δ 7.74 (s, 1H), 7.49 (s, 1H), 7.44 (d, *J* = 0.8 Hz, 4H), 7.19 (s, 1H), 7.16 (s, 1H), 7.12 (s, 1H), 6.87 -6.60(m, 1H), 6.35 (s, 1H), 6.28 (d, *J =* 2.0 Hz, 1H), 4.60 (s, 3H), 4.30 (s, 2H), 3.86 (s, 3H), 3.65 (s, 1H), 3.56 (s, 2H), 3.12 (s, 1H), 2.88 (s, 4H), 2.58 (d, *J* = 2.2 Hz, 3H), 2.03-2.00 (m, 2H), 1.87 (s, 1H), 1.65-1.61 (m, 3H). |
| M001164 | 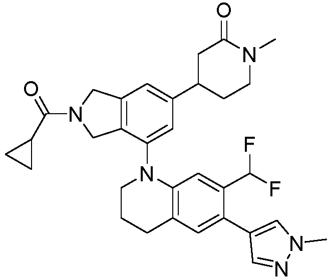 | ¹H NMR (400 MHz, DMSO-*d₆*) δ 7.74 (s, 1H), 7.49 (s, 1H), 7.21 - 7.15 (m, 2H), 7.12 (s, 1H), 6.75 (t, *J* = 55.2Hz, 1H), 6.40 (*d, J =* 18.4 Hz, 1H), 5.05 (s, 1H), 4.78 (s, 1H), 4.68 (s, 1H), 4.32 (s, 1H), 3.86 (s, 3H), 3.60 - 3.53 (m, 2H), 3.47 - 3.36 (m, 1H), 3.20 - 3.03 (m, 1H), 2.92 - 2.88 (m, 2H), 2.84 (d, *J* = 5.2 Hz, 3H), 2.44 - 2.36 (m, 2H), 2.15 - 1.94 (m, 4H), 1.91 - 1.62 (m, 2H), 0.92 - 0.51 (m, 4H). |
| M001166 | 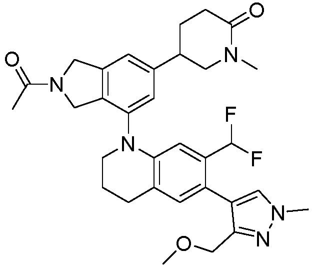 | ¹H NMR (400 MHz, DMSO-*d₆*) δ 7.68 - 7.52 (m, 1H), 7.29 - 7.17 (m, 2H), 7.10 - 7.02 (m, 1H), 6.74 - 6.45 (m, 1H), 6.45 - 6.32 (m, 1H), 4.93 - 4.84 (m, 1H), 4.77 - 4.46 (m, 2H), 4.37 - 4.26 (m,1H), 4.18 (s, 2H), 3.89 - 3.78 (m, 3H), 3.62 - 3.54 (m, 2H), 3.45 - 3.32 (m, 4H), 3.20 - 3.18 (m, 2H), 2.90 - 2.76 (m, 5H), 2.39 - 2.28 (m, 2H), 2.08 - 1.87 (m, 7H). |
| M001167 | 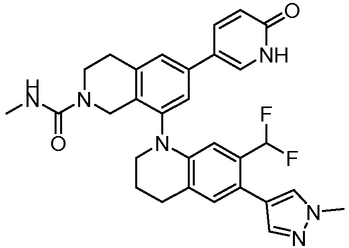 | m/z+=545.4 |
| M001168 | 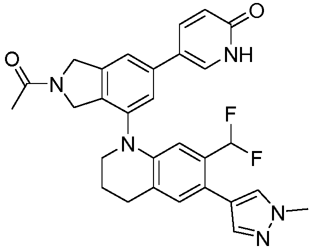 | m/z+=516.2 |
| M001169 | 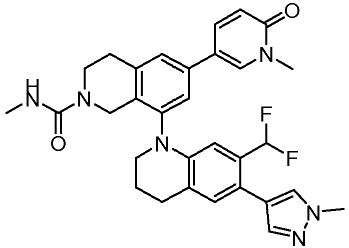 | m/z+=559.4 |
| M001170 | 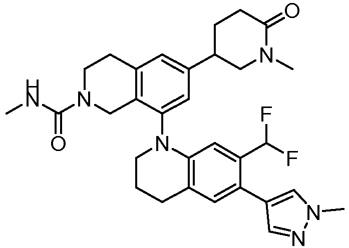 | ¹H NMR (400 MHz, DMSO-*d₆*) δ 7.72 (s, 1H), 7.47 (s, 1H), 7.12 (s, 1H), 7.09 (s, 2H), 6.70 (m, 1H), 6.48 (q, *J* = 4.0 Hz, 1H), 6.17 (d, *J* = 2.8 Hz, 1H), 4.44 (d, *J*= 16.8 Hz, 1H), 4.10 (d, *J* = 16.8 Hz, 1H), 3.85 (s, 3H), 3.58 - 3.36 (m, 6H), 3.12 - 3.04 (m, 1H), 2.93-2.83 (m, 4H), 2.80 (s, 3H), 2.54 (d, *J* = 4.4 Hz, 3H), 2.37 - 2.30 (m, 2H), 2.09 - 1.85 (m, 4H). |
| M001171 | 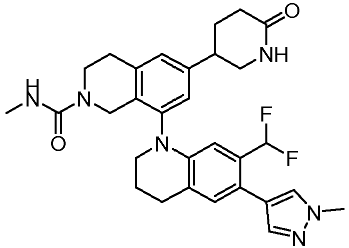 | m/z+=549.3 |
| M001172 | 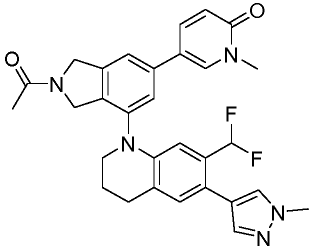 | m/z+=530.3 |
| M001173 | 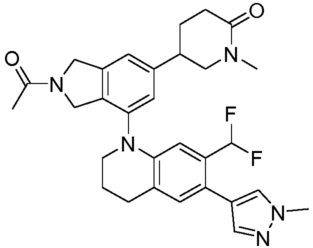 | ¹H NMR (400 MHz, DMSO-*d₆*) δ 7.75 (s, 1H), 7.50 (s, 1H), 7.22 (t, *J* = 9.2 Hz, 2H), 7.13 (s, 1H), 6.75 (t, *J* = 55.2 Hz, 1H), 6.45 - 6.35 (m, 1H), 4.87 (s, 1H), 4.66 (s, 1H), 4.62 (s, 1H), 4.28 (s, 1H), 3.86 (s, 3H), 3.61 - 3.53 (m, 2H), 3.37 - 3.43 (m, 2H),, 3.21 - 3.14 (m, 1H), 2.88 (s, 2H), 2.82 (d, *J* = 6.4 Hz, 3H), 2.38 - 2.31 (m, 2H),, 2.09 - 1.98 (m, 7H). |
| M001174 | 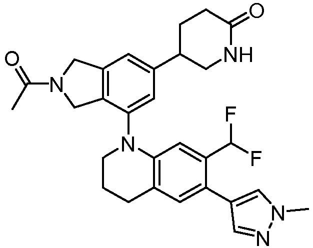 | ¹H NMR (400 MHz, DMSO-*d₆*) δ 7.75 (s, 1H), 7.58 (s, 1H), 7.49 (s, 1H), 7.24 - 7.19 (m, 2H), 7.13 (s, 1H), 6.88 - 6.61 (m, 1H), 6.39 (d, *J* = 24.0 Hz, 1H), 4.86 - 4.65 (m, 3H), 4.29 (s, 1H), 3.86 (s, 3H), 3.55 (s, 2H), 3.28 - 3.17 (m, 2H), 3.05 (s, 1H), 2.88 (s, 2H), 2.33 - 2.25 (m, 2H), 2.03 (s, 3H), 2.01 - 1.90 (m, 4H). |
| M0011175 | 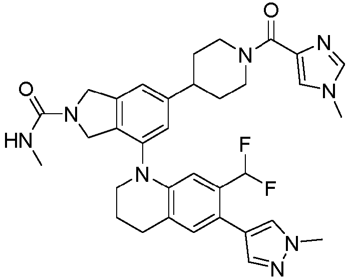 | ¹H NMR (400 MHz, DMSO-*d₆*) δ 7.74 (s, 1H), 7.63 (s, 1H), 7.60 (d, *J* = 1.2 Hz, 1H), 7.49 (s, 1H), 7.15 (s, 1H), 7.10 (s, 2H), 6.87 -6.60(m, 1H), 6.35 (s, 1H), 6.28 (d, *J =* 2.4 Hz, 1H), 4.59 (s, 2H), 4.29 (s, 2H), 3.85 (d, *J* = 4.8 Hz, 3H), 3.68 (s, 3H), 3.54 (s, 2H), 3.05 (s, 1H), 2.87 (s, 4H), 2.58 (d, *J* = 2.4 Hz, 3H), 2.52 (s, 1H), 2.01-1.97 (m, 2H), 1.84 (d, *J* = 5.6 Hz, 2H), 1.57 (d, *J* = 4.8 Hz, 2H). |
| M001176 | 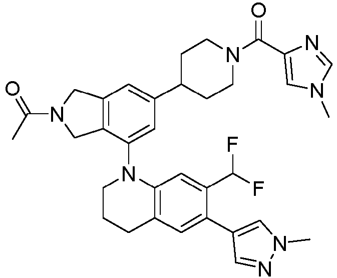 | ¹H NMR (400 MHz, DMSO-*d₆*) δ 7.74 (s, 1H), 7.67 - 7.57 (m, 2H), 7.49 (s, 1H), 7.18 - 7.11 (m, 3H), 6.74 (t, *J* = 55.2 Hz, 1H), 6.40 (d, *J* = 27.6 Hz, 1H), 4.84 (s, 1H), 4.64 (s, 1H), 4.60 (s, 1H), 4.28 (s, 1H), 3.86 (s, 3H), 3.67 (s, 3H), 3.55 - 3.53 (m, 2H), 2.90 - 2.81 (m, 4H), 2.58 - 2.50 (m, 1H), 2.06 - 1.93 (m, 5H), 1.86 - 1.79 (m, 2H), 1.62 - 1.54 (m, 2H). |
| M001178 | 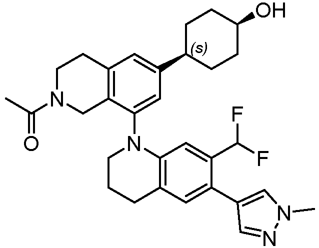 | ¹H NMR (400 MHz, DMSO-*d₆*) δ 7.72 (s, 1H), 7.47 (s, 1H), 7.09 (s, 1H), 7.05 (s, 1H), 7.01 (s, 1H), 6.69 (t, *J* = 55.2, 1H), 6.17 (s, 1H).4.54 (s, 1H), 4.47 (*d, J =* 17.2 Hz, 1H), 4.26 (d, *J* = 17.2 Hz, 1H), 3.86 (s, 3H), 3.65 - 3.60 (m, 2H), 3.56 - 3.45 (m, 2H), 3.47 - 3.37 (m, 1H), 3.05 - 2.76 (m, 4H), 2.45 - 2.38(m, 1H), 2.04 (s, 3H), 2.03 - 1.98 (m, 1H), 1.90 - 1.85(m, 3H), 1.79 -1.70 (m, 2H), 1.57 - 1.36 (m, 2H), 1.35 - 1.16 (m, 2H). |
| M001179 | 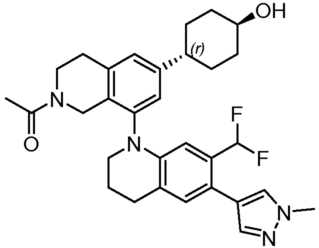 | ¹H NMR (400 MHz, DMSO-*d₆*) δ 7.72 (s, 1H), 7.47 (s, 1H), 7.09 (s, 1H), 7.05 (s, 1H), 7.00 (s, 1H), 6.69 (t, *J =* 55.2, 1H), 6.20 (s, 1H), 4.47 (d, *J* = 17.2 Hz, 1H), 4.33 (d, *J =* 3.2 Hz, 1H), 4.29 (d, *J =* 17.2 Hz, 1H),3.85 (s, 4H), 3.68 - 3.60 (m, 2H), 3.55 - 3.45 (m, 1H), 3.44 - 3.37 (m, 1H), 3.10 - 2.74 (m, 4H), 2.48 - 2.43 (m, 1H), 2.05 (s, 3H), 2.04 - 1.98 (m, 1H), 1.88 (s, 1H), 1.83 - 1.77 (m, 2H), 1.74 - 1.70(m, 2H), 1.55 - 1.45(m, 4H). |
| M001180 | 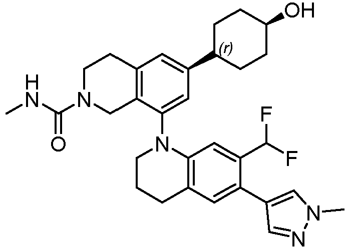 | ¹H NMR (400 MHz, DMSO-*d₆*) δ 7.71 (s, 1H), 7.46 (s, 1H), 7.08 (s, 1H), 7.01 (s, 1H), 6.97 (s, 1H), 6.69 (t, *J* = 55.2 Hz, 1H), 6.47 - 6.44 (m, 1H), 6.17 (s, 1H), 4.57 - 4.49 (m,, 1H), 4.40 (d, *J* = 16.8 Hz, 1H), 4.10 (d, *J* = 16.8 Hz, 1H), 3.85 (s, 3H), 3.54 - 3.39 (m, 5H), 2.93 - 2.78 (m, 4H), 2.54 (d, *J* = 4.4 Hz, 3H), 2.44 - 2.38 (m, 1H), 2.09 - 1.99 (m, 2H), 1.90 - 1.86 (m, 2H), 1.76 - 1.72 (m, 2H), 1.47 - 1.41 (m, 2H), 1.30 - 1.20 (m, 2H). |
| M001181 | 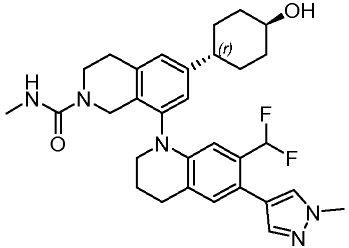 | ¹H NMR (400 MHz, DMSO-*d₆*) δ 7.71 (s, 1H), 7.46 (s, 1H), 7.08 (s, 1H), 7.02 (s, 1H), 6.96 (s, 1H), 6.69 (t, *J* = 55.2 Hz, 1H), 6.48 - 6.45 (m, 1H), 6.19 (s, 1H), 4.41 (d, *J* = 16.8 Hz, 1H), 4.34 - 4.30(m, 1H), 4.13 (*d, J =* 16.8 Hz, 1H), 3.85 (s, 4H), 3.54 - 3.41 (m, 4H), 2.93 - 2.80 (m, 4H), 2.54 (d, *J* = 4.4 Hz, 3H), 2.46 - 2.43 (m, 1H), 2.10 - 2.02 (m, 2H), 1.84 - 1.69 (m, 4H), 1.53 - 1.45 (m, 4H). |
| M001184 | 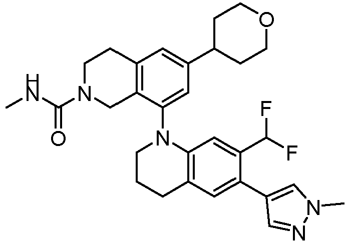 | ¹H NMR (400 MHz, DMSO-*d₆*) δ 7.72 (s, 1H), 7.47 (s, 1H), 7.08 (s, 1H), 7.05 (s, 1H), 7.01 (s, 1H), 6.69 (t, *J* = 55.2 Hz, 1H), 6.47 (d, *J* = 4.4 Hz, 1H), 6.19 (s, 1H), 4.46 - 4.36 (m, 1H), 4.15 - 4.06 (m, 1H), 3.95 - 3.89 (m, 2H), 3.86 (s, 3H), 3.53 - 3.39 (m, 6H), 2.93 - 2.79 (m, 4H), 2.77 - 2.70 (m, 1H), 2.54 (d, *J* = 4.4 Hz, 3H), 2.10 - 1.98 (m, 2H), 1.72 - 1.59 (m, 4H). |
| M001186 | 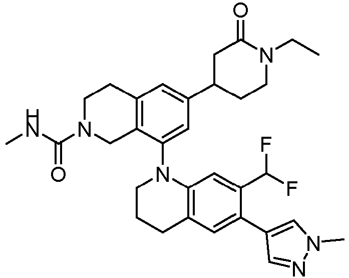 | ¹H NMR (400 MHz, DMSO-*d₆*) δ 7.72 (s, 1H), 7.47 (s, 1H), 7.08 (d, *J* = 8.0 Hz, 2H), 7.04 (d, *J* = 8.0 Hz, 1H), 6.84 - 6.56 (m, 1H), 6.48 (d, *J* = 4.4 Hz, 1H), 6.17 (s, 1H), 4.48 - 4.40 (m, 1H), 4.16 - 4.08 (m, 1H), 3.86 (s, 3H), 3.58 - 3.41 (m, 4H), 3.31 (d, *J=* 8.0 Hz, 2H), 3.28 - 3.24 (m, 2H), 3.06-3.02 (m, 1H), 2.95 - 2.78 (m, 4H), 2.54 (d, *J* = 4.0 Hz, 3H), 2.44 - 2.29 (m, 2H), 2.11 - 1.95 (m, 3H), 1.92 - 1.82 (m, 1H), 1.04 - 0.95 (m, 3H). |
| M001187 | 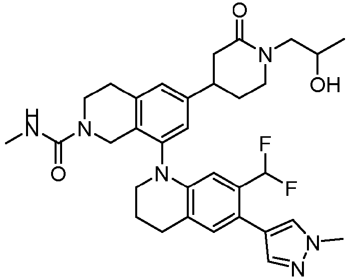 | m/z+=607.5 |
| M001188 | 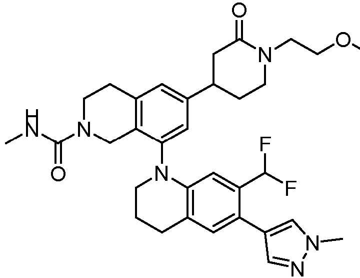 | ¹H NMR (400 MHz, DMSO-*d₆*) δ 7.72 (s, 1H), 7.47 (s, 1H), 7.09 - 7.05 (m, 3H), 6.84 - 6.56 (m, 1H), 6.48 (d, *J* = 4.0 Hz, 1H), 6.17 (s, 1H), 4.43 (d, *J* = 16.0 Hz, 1H), 4.13 - 4.07 (m, 1H), 3.86 (s, 3H), 3.53 - 3.38 (m, 10H), 3.23 (d, *J =* 8.0 Hz, 3H), 3.05 (s, 1H), 2.94 - 2.79 (m, 4H), 2.54 (d, *J* = 4.0 Hz, 3H), 2.45 - 2.34 (m, 2H), 2.04 - 1.88 (m, 4H). |
| M001189 | 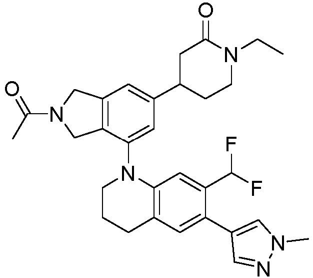 | ¹H NMR **(400** MHz, DMSO-*d₆*) δ 7.75 (s, 1H), 7.49 (s, 1H), 7.22 - 7.16 (m, 2H), 7.14 - 7.13 (m, 1H), 6.74 (t, *J* = 55.2 Hz, 1H), 6.40 (d, *J* = 26.0 Hz, 1H), 4.86 (s, 1H), 4.65 (s, 1H), 4.60 (s, 1H), 4.28 (s, 1H), 3.86 (s, 3H), 3.59 - 3.52 (m, 2H), 3.39 - 3.35 (m, 2H), 3.30 - 3.26 (m, 2H), 3.17 - 3.05 (m, 1H), 2.87 (s, 2H), 2.43 - 2.25 (m, 2H), 2.11 - 1.96 (m, 6H), 1.97 - 1.79 (m, 1H), 1.05 - 0.99 (m, 3H). |
| M001190 | 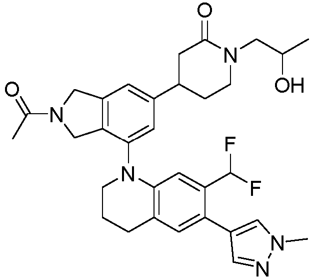 | ¹H NMR (400 MHz, DMSO-*d₆*) δ 7.75 (s, 1H), 7.49 (s, 1H), 7.18 (t, *J* = 10.2 Hz, 2H), 7.13 (s, 1H), 6.75 (t, *J* = 55.2 Hz, 1H), 6.46 - 6.35 (m, 1H), 4.86 (s, 1H), 4.72 - 4.55 (m, 2H), 4.28 (s, 1H), 3.93 - 3.86 (m, 1H), 3.86 (s, 3H), 3.66 - 3.40 (m, 5H), 3.18 - 3.06 (m, 2H), 2.88 (s, 2H), 2.49 - 2.34 (m, 2H), 2.03 (s, 3H), 2.01 - 1.91 (m, 4H), 1.05 - 0.99 (m, 3H). |
| M001191 | 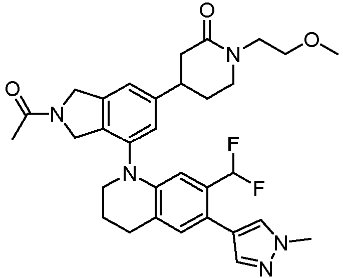 | ¹H NMR (400 MHz, DMSO-*d₆*) δ 7.75 (s, 1H), 7.49 (s, 1H), 7.21 - 7.15 (m, 2H), 7.13 (s, 1H), 6.75 (t, *J* = 55.2 Hz, 1H), 6.48 - 6.35 (m, 1H), 4.86 (s, 1H), 4.77 - 4.40 (m, 2H), 4.28 (s, 1H), 3.86 (s, 3H), 3.56 (d, *J* = 4.8 Hz, 2H), 3.47 - 3.38 (m, 6H), 3.24 (d, *J=* 3.6 Hz, 3H), 3.13 (d,*J* = 4.4 Hz, 1H), 2.87 (s, 1H), 2.48 - 2.34 (m, 1H), 2.03 (s, 3H), 2.01 - 1.90 (m, 4H). |
| M001192 | 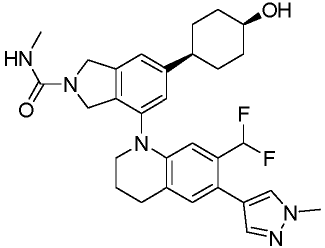 | ¹H NMR (400 MHz, DMSO-*d₆*) δ 7.73 (s, 1H), 7.48 (s, 1H), 7.11 (d, *J* = 4.4 Hz, 1H), 7.06 (s, 1H), 6.88 - 6.58 (m, 1H), 6.35 (s, 1H), 6.29 - 6.25 (m, 1H), 4.60 - 4.54 (m, 3H), 4.28 (s, 2H), 3.86 (s, 3H), 3.53 (s, 2H), 3.49 - 3.39 (m, 1H), 2.88 - 2.86 (m, 2H), 2.58 (d, *J* = 4.0 Hz, 3H), 2.49 - 2.44 (m, 1H), 2.05 - 1.96 (m, 2H), 1.94 - 1.86 (m, 2H), 1.83 - 1.74 (m, 2H), 1.55 - 1.42 (m, 2H), 1.33 - 1.22 (m, 2H). |
| M001193 | 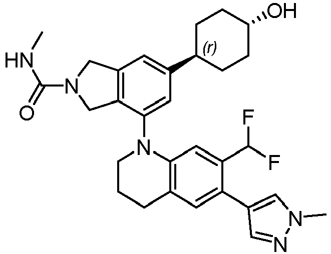 | ¹H NMR (400 MHz, DMSO-*d₆*) δ 7.74 (s, 1H), 7.49 (s, 1H), 7.11 (d, *J* = 2.4 Hz, 1H), 7.05 (s, 1H), 6.88 -6.58 (m, 1H), 6.37 (s, 1H), 6.29 - 6.25 (m, 1H), 4.60 (s, 2H), 4.40 - 4.25 (m, 3H), 3.89 - 3.84 (m, 4H), 3.54 (s, 2H), 2.90 - 2.84 (m, 2H), 2.58 (d, *J* = 4.0 Hz, 3H), 2.56 - 2.52 (m, 1H), 2.05 - 1.98 (m, 2H), 1.89 - 1.77 (m, 2H), 1.76 - 1.68 (m, 2H), 1.58 - 1.47 (m, 4H). |
| M001194 | 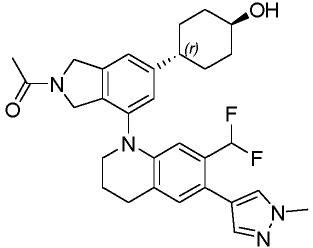 | ¹H NMR (400 MHz, DMSO-*d₆*) δ 8.45 (brs, 0.3H), 7.74 (s, 1H), 7.49 (s, 1H), 7.15 - 7.06 (m, 3H), 6.88 - 6.58 (m, 1H), 6.44 - 6.35 (m, 1H), 4.84 (s, 1H), 4.64 - 4.54 (m, 3H), 4.27 (s, 1H), 3.86 (s, 3H), 3.56 -3.51 (m, 2H), 3.49 - 3.39 (m, 1H), 2.88 - 2.86 (m, 2H), 2.49 - 2.44 (m, 1H), 2.04 - 1.96 (m, 5H), 1.94 - 1.86 (m, 2H), 1.82 - 1.73 (m, 2H), 1.55 - 1.42 (m, 2H), 1.33 - 1.22 (m, 2H). |
| M001195 | 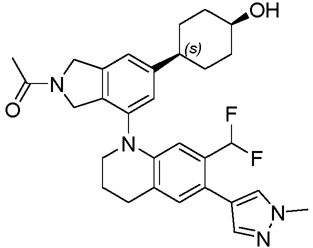 | ¹H NMR (400 MHz, DMSO-*d₆*) δ 7.74 (s, 1H), 7.49 (s, 1H), 7.15-7.06 (m, 3H), 6.88 - 6.58 (m, 1H), 6.46 - 6.36 (m, 1H), 4.85 (s, 1H), 4.65 (s, 2H), 4.38 - 4.25 (m, 2H), 3.90 - 3.82 (m, 4H), 3.57 - 3.52 (m, 2H), 2.90 - 2.84 (m, 2H), 2.59 - 2.52 (m, 1H), 2.04 - 1.96 (m, 5H), 1.86 - 1.78 (m, 2H), 1.77 - 1.68 (m, 2H), 1.57 - 1.48 (m,4 H). |
| M001196 | 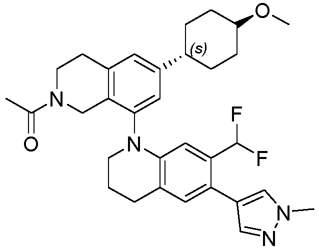 | ¹H NMR (400 MHz, DMSO-*d₆*) δ 7.72 (s, 1H), 7.47 (s, 1H), 7.09 (s, 1H), 7.06 (s, 1H), 7.02 (s, 1H), 6.69 (t, *J* = 55.6, 1H), 6.18 (s, 1H), 4.47 (*d*, *J =* 17.6 Hz, 1H), 4.27 (d, *J =* 17.2 Hz, 1H), 3.85 (s, 3H), 3.65-3.60 (m, 2H), 3.53 - 3.39 (m, 3H), 3.24 (s, 3H), 3.18 - 3.13 (m, 1H), 2.98 - 2.84 (m, 4H), 2.48 - 2.43 (m, 1H), 2.08 (s, 2H), 2.04 (s, 3H), 1.87 (s, 1H), 1.84 - 1.76(m, 2H), 1.50-1.41 (m, 2H), 1.25 - 1.17 (m, 2H). |
| M001197 | 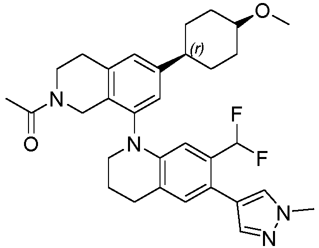 | ¹H NMR (400 MHz, DMSO-*d₆*) δ 7.72 (s, 1H), 7.47 (s, 1H), 7.09 (s, 1H), 7.04 (s, 1H), 6.97 (s, 1H), 6.69 (t, *J* = 55.6, 1H), 6.18 (s, 1H), 4.47 (d, *J* = 17.6 Hz, 1H), 4.28 (d, *J* = 17.2 Hz, 1H), 3.85 (s, 3H) 3.68 - 3.60(m, 2H), 3.54 - 3.48(m, 1H), 3.44(s, 1H), 3.41 - 3.38(m, 1H), 3.22(s, 3H), 2.98 - 2.80(m, 4H), 2.58 - 2.56(m, 1H), 2.04(s, 3H), 2.03 - 1.99(m, 1H), 1.95 - 1.91(m, 2H), 1.88 (s, 1H).1.71 - 1.59(m,2H), 1.53 - 1.44(m, 4H). |
| M001198 | 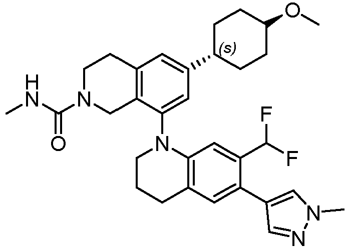 | ¹H NMR (400 MHz, DMSO-*d₆*) δ 7.71 (s, 1H), 7.46 (s, 1H), 7.08 (s, 1H), 7.02 (s, 1H), 6.97 (s, 1H), 6.69 (t, *J* = 55.2 Hz, 1H), 6.48 - 6.44 (m, 1H), 6.18 (s, 1H), 4.41 (d, *J* = 16.8 Hz, 1H), 4.11 (d,*J* = 16.8 Hz, 1H), 3.85 (s, 3H), 3.52 - 3.42 (m, 4H), 3.24 (s, 3H), 3.17 - 3.11 (m, 1H), 2.93 - 2.78 (m, 4H), 2.54 (d, *J* = 4.4 Hz, 3H), 2.47 - 2.42 (m, 1H), 2.10 - 2.0 (m, 4H), 1.84 - 1.76 (m, 2H), 1.51 - 1.39 (m, 2H), 1.25 - 1.15 (m, 2H). |
| M001199 | 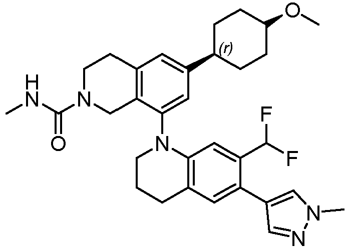 | ¹H NMR (400 MHz, DMSO-*d₆*) δ 7.71 (s, 1H), 7.46 (s, 1H), 7.08 (s, 1H), 7.00 (s, 1H), 6.93 (s, 1H), 6.69 (t, *J* = 55.2 Hz, 1H), 6.48 - 6.45 (m, 1H), 6.18 (s, 1H), 4.40 (d, *J* = 16.8 Hz, 1H), 4.12 (d,*J* = 16.8 Hz, 1H), 3.85 (s, 3H), 3.54 - 3.43 (m, 5H), 3.21 (s, 3H), 2.98 - 2.75 (m, 5H), 2.54 (d, *J* = 4.4 Hz, 3H), 2.09 - 2.00 (m, 2H), 1.96 - 1.89 (m, 2H), 1.69 - 1.58 (m, 2H), 1.52 - 1.43 (m, 4H). |
| M001200 | 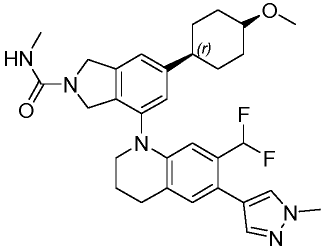 | ¹H NMR (400 MHz, DMSO-*d₆*) δ 7.74 (s, 1H), 7.49 (s, 1H), 7.10 - 7.03 (m, 2H), 7.01 (s, 1H), 6.87 - 6.60 (m, 1H), 6.36 (s, 1H), 6.28 - 6.26 (m, 1H), 4.60 (s, 2H), 4.30 (s, 2H), 3.86 (s, 3H), 3.60 (s, 2H), 3.46 (s, 1H), 3.23 (s, 3H), 2.88 - 2.86 (m, 2H), 2.60 - 2.55 (m, 3H), 2.53 - 2.51 (m, 1H), 2.10 - 1.90 (m, 4H), 1.75 - 1.45 (m, 6H). |
| M001201 | 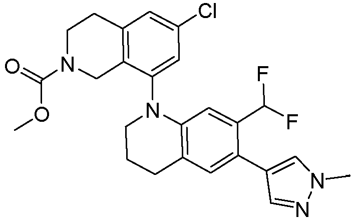 | ¹H NMR (400 MHz, DMSO-*d₆*) δ 7.74 (s, 1H), 7.49 (s, 1H), 7.31 (d, *J =* 2.0 Hz, 1H), 7.28 (d, *J =* 2.0 Hz, 1H), 7.13 (s, 1H), 6.73 (t, *J* = 55.2 Hz, 1H), 6.17 (s, 1H), 4.46 - 4.41 (m, 1H), 4.25 (s, 1H), 3.86 (s, 3H), 3.63 - 3.50 (m, 6H), 3.43 - 3.34 (m, 1H), 3.03 - 2.76 (m, 4H), 2.04 - 2.00 (m, 2H). |
| M001202 | 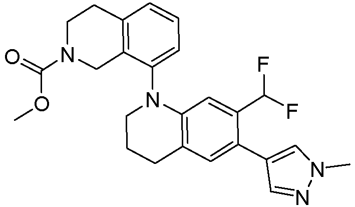 | ¹H NMR (400 MHz, DMSO-*d₆*) δ 7.73 (s, 1H), 7.48 (s, 1H), 7.34 (t, *J* = 7.6 Hz, 1H), 7.20 (d, *J* = 7.6 Hz, 1H), 7.15 (d,*J* = 7.6 Hz, 1H), 7.11 (s, 1H), 6.70 (t, *J* = 55.2 Hz, 1H), 6.16 (s, 1H), 4.49 - 4.45 (m, 1H), 4.29 (s, 1H), 3.86 (s, 3H), 3.76 - 3.60 (m, 1H), 3.62 - 3.49 (m, 5H), 3.44 - 3.34 (m, 1H), 3.03 - 2.83 (m, 4H), 2.22 - 1.82 (m, 2H). |
| M001203 | 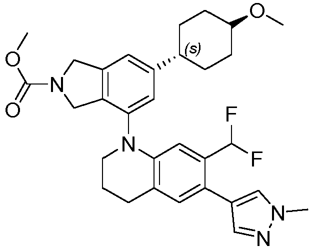 | ¹H NMR (400 MHz, DMSO-*d₆*) δ 7.75 (s, 1H), 7.50 (s, 1H),7.20- 7.03 (m, 3H), 6.80 - 6.60 (m, 1H), 6.39 - 6.37 (m, 1H), 4.67 - 4.65 (m, 2H), 4.50 - 4.25 (m, 2H), 3.86 (s, 3H), 3.70 - 3.60 (m, 3H), 3.50 (s, 2H), 3.25 (s, 3H), 3.24 - 3.12 (m, 1H), 2.87 - 2.85 (m, 2H), 2.54 - 2.50 (m, 1H), 2.15 - 1.85 (m, 4H), 1.70 - 1.50 (m, 2H), 1.55 - 1.45 (m, 2H), 1.27 - 1.18 (m, 2H). |
| M001204 | 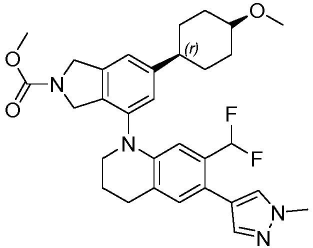 | ¹H NMR (400 MHz, DMSO-*d₆*) δ 7.75 (s, 1H), 7.50 (s, 1H),7.20- 7.03 (m, 3H), 6.88 - 6.60 (m, 1H), 6.41 - 6.39 (m, 1H), 4.67 - 4.65 (m, 2H), 4.50 - 4.25 (m, 2H), 3.86 (s, 3H), 3.60 - 3.55 (m, 3H), 3.50 (s, 2H), 3.40 - 3.38 (m, 1H), 3.23 (s, 3H), 2.87 - 2.85 (m, 2H), 2.54 - 2.50 (m, 1H), 2.05 - 1.95 (m, 4H), 1.75 - 1.30 (m, 6H). |
| M001206 | 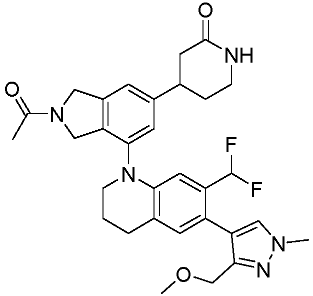 | ¹H NMR (400 MHz, DMSO-*d₆*) δ 7.63 - 7.52 (m, 2H), 7.25 - 7.14 (m, 2H), 7.09 - 7.03 (m, 1H), 6.65 (d, *J* = 55.4 Hz, 1H), 6.45 - 6.35 (m, 1H), 4.90 - 4.84 (m, 1H), 4.74 - 4.50 (m, 2H), 4.37 - 4.27 (m, 1H), 4.18 (s, 2H), 3.84 (s, 3H), 3.62 - 3.54 (m, 2H), 3.22 - 3.17 (m, 4H), 3.14 - 3.02 (m, 2H), 2.90 - 2.84 (m, 2H), 2.37 - 2.30 (m, 2H), 2.05 - 1.87 (m, 7H). |
| M001207 | 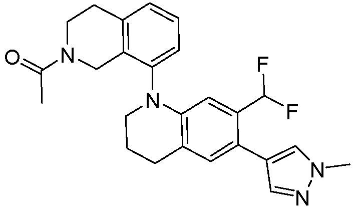 | ¹H NMR (400 MHz, DMSO-*d₆*) δ 7.72 (s, 1H), 7.47 (s, 1H), 7.39 - 7.29 (m, 1H), 7.23 - 7.07 (m, 1H), 6.86 - 6.54 (m, 1H), 6.20 - 6.10 (m, 1H), 4.61 - 4.47 (m, 1H), 4.40 - 4.24 (m, 1H), 3.85 (s, 3H), 3.69 - 3.41 (m, 4H), 3.01 - 2.81 (m, 4H), 2.10 - 1.99 (m, 4H), 1.91 - 1.85 (m, 1H). |
| M001208 | 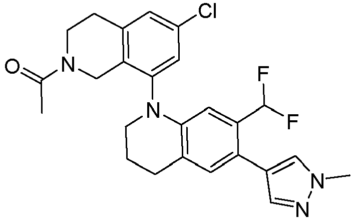 | ¹H NMR (400 MHz, DMSO-*d₆*) δ 7.74 (s, 1H), 7.49 (s, 1H), 7.34 - 7.25 (m, 2H), 7.16 - 7.11 (m, 1H), 6.73 (td, *J* = 55.0, 5.6 Hz, 0H), 6.21 - 6.14 (m, 1H), 4.59 - 4.43 (m, 1H), 4.34 - 4.20(m, 1H), 3.86 (s, 3H), 3.70 - 3.41 (m, 4H), 3.00 - 2.81 (m, 4H), 2.09 - 1.98 (m, 4H), 1.91 - 1.85 (m, 1H). |
| M001209 | 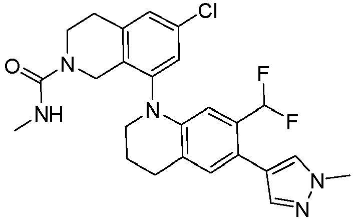 | ¹H NMR (400 MHz, DMSO-*d₆*) δ 7.73 (s, 1H), 7.48 (s, 1H), 7.27 (s, 1H), 7.23 (d, *J* = 1.6 Hz, 1H), 7.12 (s, 1H), 6.73 (t, *J* = 55.2 Hz, 1H), 6.54 - 6.47 (m, 1H), 6.17 (s, 1H), 4.51 - 4.37 (m, 1H), 4.16 - 4.05 (m, 1H), 3.86 (s, 3H), 3.59 - 3.49 (m, 2H), 3.47 - 3.39 (m, 2H), 2.93 - 2.84 (m, 4H), 2.58 - 2.53 (m, 3H), 2.08 - 2.00 (m, 2H). |
| M001210 | 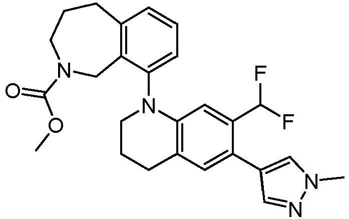 | ¹H NMR (400 MHz, DMSO-*d₆*) δ 7.72 (s, 1H), 7.47 (s, 1H), 7.36 - 7.24 (m, 1H), 7.22 - 7.15 (m, 1H), 7.13 - 7.05 (m, 2H), 6.88 - 6.52 (m, 1H), 6.21 - 5.98 (m, 1H), 4.95 - 4.78 (m, 0.4H), 4.69 - 4.53 (m, 0.6H), 4.43 - 4.30 (m, 0.6H), 4.05 - 3.95 (m, 1H), 3.85 (s, 3H), 3.76 - 3.47 (m, 4H), 3.32 - 3.27 (m, 3H), 3.05 - 2.85 (m, 4H), 2.20 - 2.00 (m, 2H), 1.87 - 1.95 (m, 2H). |
| M001211 | 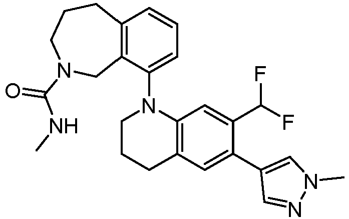 | ¹H NMR (400 MHz, DMSO-*d₆*) δ 7.73 (s, 1H), 7.48 (s, 1H), 7.30 - 7.22 (m, 1H), 7.20 - 7.14 (m, 1H), 7.13 - 7.05 (m, 2H), 6.71 (t, *J* = 55.2 Hz, 1H), 6.20 (s, 1H), 5.87 (s, 1H), 4.75 - 4.63 (m, 1H), 4.19 - 4.05 (m, 1H), 3.86 (s, 3H), 3.62 - 3.46 (m, 4H), 3.00 - 2.83 (m, 4H), 2.40 (d, *J* = 2.8 Hz, 3H), 2.24 - 2.10 (m, 1H), 2.08 - 1.96 (m, 1H), 1.82 - 1.60 (m, 1H). |
| M001212 | 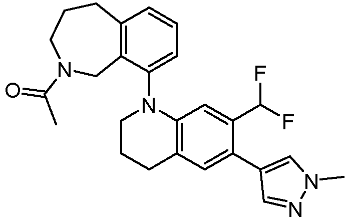 | ¹H NMR (400 MHz, DMSO-*d₆*) δ 7.78 - 7.66 (m, 1H), 7. 51 - 7.44 (m, 1H), 7.32 - 7.24 (m, 1H), 7.19 - 7.05 (m, 3H), 6.87 - 6.54 (m, 1H), 6.33 - 6.28 (m, 0.4H), 6.05 - 5.97 (m, 0.6H), 5.20 - 4.96 (m, 1H), 4.65 - 4.40 (m, 1H), 3.87 - 3.84 (m, 3H), 3.81 - 3.52 (m, 4H), 3.01 - 2.85 (m, 4H), 2.13 - 1.99 (m, 2H), 1.96 - 1.87 (s, 3H), 1.84 - 1.77 (m, 2H). |
| M001213 | 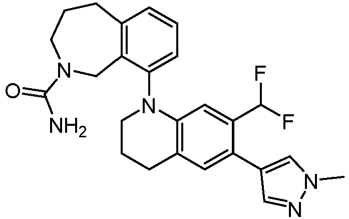 | ¹H NMR (400 MHz, DMSO-*d₆*) δ 7.72 (s, 1H), 7.47 (s, 1H), 7.33 - 7.23 (m, 1H), 7.22 - 7.14 (m, 1H), 7.12 - 7.06 (m, 2H), 6.70 (t, *J* = 55.2 Hz, 1H), 6.13 (s, 1H), 5.71 (s, 2H), 4.85 - 4.70 (m, 1H), 4.00 - 3.90 (m, 1H), 3.86 (s, 3H), 3.77 - 3.68 (m, 1H), 3.62 - 3.51 (m, 2H), 3.33 - 3.27 (m, 1H), 3.01 - 2.82 (m, 4H), 2.21 - 2.07 (m, 1H), 2.04 - 1.92 (m, 1H), 1.85 - 1.73 (m, 1H), 1.71 - 1.58 (m, 1H). |
| M001214 | 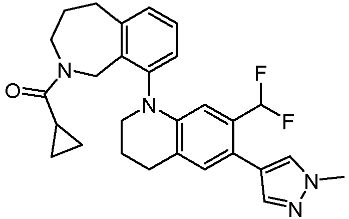 | ¹H NMR (400 MHz, DMSO-*d₆*) δ 7.78 - 7.68 (m, 1H), 7.54 - 7.42 (m, 1H), 7.32 - 7.24 (m, 1H), 7.21 - 7.04 (m, 3H), 6.88 - 6.54 (m, 1H), 6.36 - 6.02 (m, 1H), 5.06 - 4.97 (m, 0.4H), 4.86 - 4.74 (m, 0.6H), 4.74 - 4.64 (m, 0.6H), 4.19 - 4.09 (m, 1H), 4.04 - 3.89 (m, 1H), 3.86 (s, 3H), 3.67 - 3.44 (m, 3H), 3.02 - 2.81 (m, 5H), 2.06 - 1.84 (m, 4H), 1.79 - 1.76 (m, 1H), 0.66 - 0.36 (m, 4H). |
| M001215 | 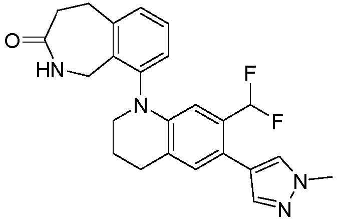 | ¹H NMR (400 MHz, DMSO-*d₆*) δ 7.87 - 7.84 (m, 1H), 7.72 (s, 1H), 7.47 (s, 1H), 7.35 - 7.30 (m, 1H), 7.25 - 7.20 (m, 1H), 7.15 - 7.05 (m, 2H), 6.80 - 6.55 (m, 1H), 6.16 (s, 1H), 4.30 - 4.13 (m, 2H), 3.88 (s, 3H), 3.50 - 3.45 (m, 1H), 3.25 - 3.15 (m, 1H), 3.02 - 2.80 (m, 3H), 2.72 - 2.60 (m, 1H), 2.54 - 2.50 (m, 2H), 2.13 - 2.03 (m, 2H). |
| M001217 | 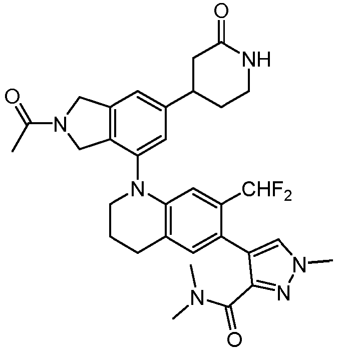 | ¹H NMR (400 MHz, DMSO-*d₆*) δ 8.52 (s, 1H), 7.72 - 7.70 (m, 1H), 7.57 (s, 1H), 7.25 - 7.18 (m, 2H), 6.90 (s, 1H), 6.80 - 6.53 (m, 1H), 6.41 - 6.33 (m, 1H), 4.87 (s, 1H), 4.66 - 4.50 (m, 2H), 4.29 (s, 1H), 3.88 (s, 3H), 3.53 - 3.50 (m, 2H), 3.25 - 3.20 (m, 2H), 3.10 - 3.18 (m, 1H), 2.80 - 2.75 (m, 5H), 2.30 - 2.25 (m, 2H), 2.13 - 1.70 (m, 7H). |
| M001218 | 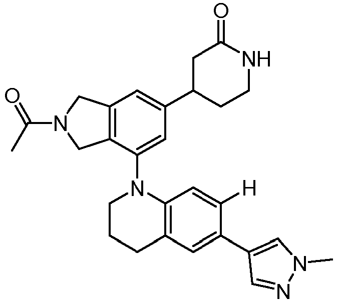 | ¹H NMR (400 MHz, DMSO-*d₆*) δ 7.90 (s, 1H), 7.66 (s, 1H), 7.56 (s, 1H), 7.22 (s, 1H), 7.17 - 7.09 (m, 2H), 7.07 - 7.03 (m, 1H), 6.16 - 6.09 (m, 1H), 4.87 - 4.52 (m, 3H), 4.24 (s, 1H), 3.81 (s, 3H), 3.53 (d, *J* = 4.0 Hz, 2H), 3.21 (d, *J* = 4.0 Hz, 2H), 3.08 (d, *J* = 4.0 Hz, 2H), 2.85 - 2.82 (m, 2H), 2.35 - 2.33 (m, 2H), 2.02 - 1.83 (m, 7H). |
| M001219 | 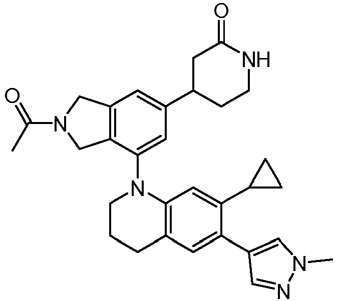 | ¹H NMR (400 MHz, DMSO-*d₆*) δ 7.84 (s, 1H), 7.60 (s, 1H), 7.56 (s, 1H), 7.14-7.01 (m, 2H), 7.02 (s, 1H), 5.88-5.82 (m_{"} 1H), 4.85 (s, 1H), 4.64 (s, 1H), 4.59 (s, 1H), 4.26 (s, 1H), 3.85 (s, 3H), 3.49 (d, *J* = 5.2 Hz, 2H), 3.20 (s, 2H), 3.07 (s, 1H), 2.79-2.78 (m, 2H), 2.36 - 2.29 (m, 2H), 2.04 (d, *J =* 5.2 Hz, 1H), 2.00 - 1.92 (m, 4H), 1.87-1.84 (m, 3H), 0.77 - 0.69 (m, 2H), 0.21 - 0.13 (m, 2H). |
| M001221 | 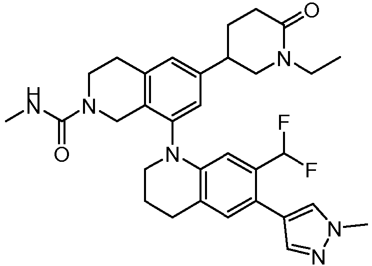 | ¹H NMR (400 MHz, DMSO-*d₆*) δ 7.72 (s, 1H), 7.47 (s, 1H), 7.17 - 7.07 (m, 3H), 6.70 (t, *J* = 55.2 Hz, 1H), 6.50 - 6.48 (m, 1H), 6.18 (d, *J* = 1.6 Hz, 1H), 4.44 (d,*J* = 16.8 Hz, 1H), 4.10 (d, *J* = 16.8 Hz, 1H), 3.85 (s, 3H), 3.58 - 3.50 (m, 2H), 3.49 - 3.41 (m, 2H), 3.38 - 3.35 (m, 1H), 3.27 - 3.16 (m, 2H), 3.09 - 3.03 (m, 1H), 2.98 - 2.79 (m, 4H), 2.54 (d, *J* = 4.4 Hz, 3H), 2.39 - 2.24 (m, 3H), 2.11 - 2.02 (m, 2H), 2.02 - 1.93 (m, 1H), 1.92 - 1.87 (m, 1H), 1.03 - 0.99 (m, 3H). |
| M001222 | 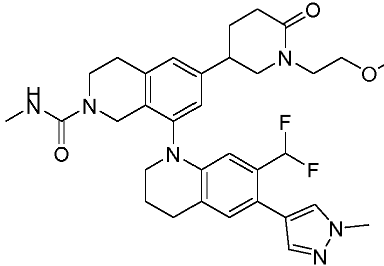 | ¹H NMR (400 MHz, DMSO-*d₆*) δ 7.72 (s, 1H), 7.47 (s, 1H), 7.11 (d, *J* = 16.0 Hz, 3H), 6.84 - 6.56 (m, 1H), 6.48 (d, *J* = 4.0 Hz, 1H), 6.17 (d, *J* = 4.0 Hz, 1H), 4.44 (d, *J* = 16.0 Hz, 1H), 4.10 (d, *J* = 16.0 Hz, 1H), 3.86 (s, 3H), 3.57 - 3.38 (m, 10H), 3.21 (d, *J* = 8.0 Hz, 3H), 3.08 - 3.06 (m, 1H), 2.89 - 2.82 (m, 4H), 2.54 (d, *J* = 4.0 Hz, 3H), 2.36 - 2.20 (m, 2H), 2.09 - 2.03 (m, 2H), 1.98 - 1.92 (m, 2H). |
| M001223 | 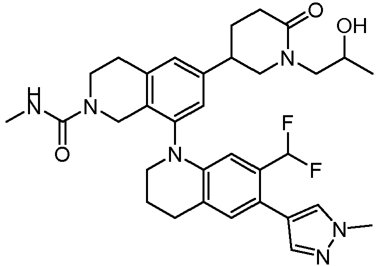 | ¹H NMR (400 MHz, DMSO-*d₆*) δ 7.72 (s, 1H), 7.47 (s, 1H), 7.12 (s, 1H), 7.09 (s, 2H), 6.70 (t, *J* = 55.2 Hz, 1H), 6.52 - 6.46 (m, 1H), 6.19 - 6.14 (m, 1H), 4.68 - 4.62 (m, 1H), 4.48 - 4.40 (m, 1H), 4.14 - 4.05 (m, 1H), 3.86 (s, 3H), 3.54 - 3.42 (m, 5H), 3.10 - 3.02 (m, 2H), 2.95 - 2.79 (m, 5H), 2.54 (d, *J =* 4.2 Hz, 3H), 2.35 - 2.32 (m, 2H), 2.10 - 1.86 (m, 6H), 1.00 (d, *J* = 6.2 Hz, 3H). |
| M001224 | 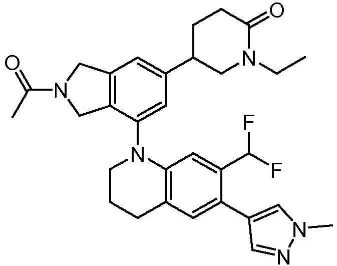 | ¹H NMR (400 MHz, DMSO-*d₆*) δ 7.75 (s, 1H), 7.49 (s, 1H), 7.24 (t, *J* = 7.6 Hz, 2H), 7.13 (s, 1H), 6.75 (*t, J =* 55.2 Hz, 1H), 6.41 (d, *J* = 28.0 Hz, 1H), 4.86 (s, 1H), 4.66 (s, 1H), 4.62 (s, 1H), 4.29 (s, 1H), 3.86 (s, 3H), 3.69 - 3.51 (m, 2H), 3.33 - 3.22 (m, 2H), 3.16 - 3.11 (m, 1H), 2.88 (s, 2H), 2.43 - 2.22 (m, 3H), 2.13 - 1.86 (m, 8H), 1.05 - 0.99 (m, 3H). |
| M001225 | 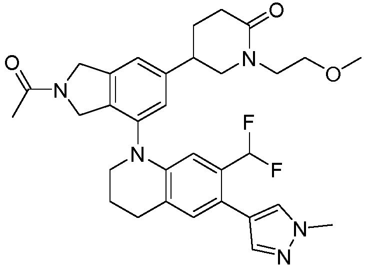 | ¹H NMR (400 MHz, DMSO-*d₆*) δ 7.75 (d, *J =* 2.0 Hz, 1H), 7.49 (s, 1H), 7.25 - 7.20 (m, 2H), 7.13 (s, 1H), 6.89 - 6.61 (m, 1H), 6.40 (d, *J =* 28.0 Hz, 1H), 4.87 - 4.66 (m, 3H), 4.28 (s, 1H), 3.86 (s, 3H), 3.56 - 3.35 (m, 8H), 3.22 (d, *J* = 4.0 Hz, 3H), 3.15 - 3.13 (m, 1H), 2.88 (s, 2H), 2.38 - 2.33 (m, 2H), 2.04 - 1.98 (m, 7H). |
| M001226 | 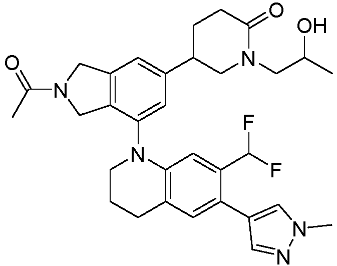 | ¹H NMR (400 MHz, DMSO-*d₆*) δ 7.75 (s, 1H), 7.50 (s, 1H), 7.27 - 7.18 (m, 2H), 7.13 (s, 1H), 6.75 (t, *J* = 55.2 Hz, 1H), 6.45 - 6.34 (m, 1H), 4.90 - 4.84 (m, 1H), 4.70 - 4.58 (m, 2H), 4.36 - 4.18 (m, 1H), 3.86 (s, 3H), 3.65 - 3.40 (m, 5H), 3.28 - 3.00 (m, 3H), 2.92 - 2.83 (m, 2H), 2.40 - 2.31 (m, 2H), 2.06 - 1.90 (m, 7H), 1.01 (t, *J* = 5.6 Hz, 3H). |
| M001227 | 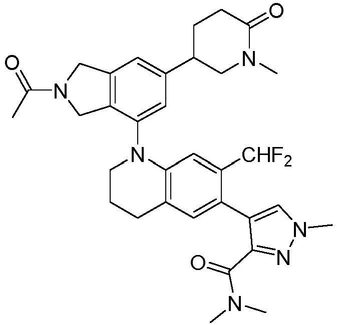 | ¹H NMR (400 MHz, DMSO-*d₆*) δ 7.72 - 7.70 (m, 1H), 7.25 - 7.23 (m, 2H), 6.90 (s, 1H), 6.80 - 6.53 (m, 1H), 6.40 - 6.33 (m, 1H), 4.87 (s, 1H), 4.61 - 4.60 (m, 2H), 4.30 (s, 1H), 3.88 (s, 3H), 3.60 - 3.50 (m, 2H), 3.49 - 3.40 (m, 2H), 3.17 - 3.16 (m, 1H), 2.90 - 2.70 (m, 11H), 2.36 - 2.29 (m, 2H), 2.10 - 1.80 (m, 7H). |
| M001228 | 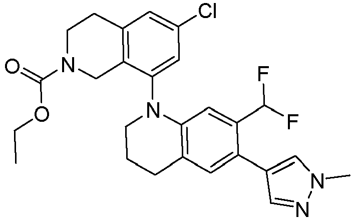 | ¹H NMR (400 MHz, DMSO-*d₆*) δ 7.74 (s, 1H), 7.49 (s, 1H), 7.30 (d, *J* = 8.8 Hz, 2H), 7.14 (s, 1H), 6.73 (t, *J* = 55.2 Hz, 1H), 6.17 (s, 1H), 4.50 - 4.38 (m, 1H), 4.34 - 4.13 (m, 1H), 4.07 - 3.94 (m, 2H), 3.86 (s, 3H), 3.65 - 3.49 (m, 3H), 3.45 - 3.37 (m, 1H), 2.96 - 2.79 (m, 4H), 2.09 - 1.96 (m, 2H), 1.122 - 1.00 (m, 3H). |
| M001229 | 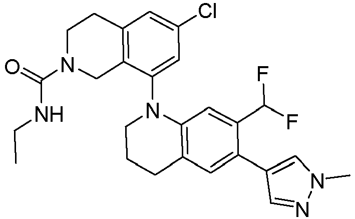 | ¹H NMR (400 MHz, DMSO-*d₆*) δ 7.74 (s, 1H), 7.49 (s, 1H), 7.28 (s, 1H), 7.24 (s, 1H), 7.12 (s, 1H), 6.89 - 6.57 (m, 2H), 6.17 (s, 1H), 4.52 - 4.39 (m, 1H), 4.16 - 4.01 (m, 1H), 3.86 (s, 3H), 3.59 - 3.49 (m, 2H), 3.48 - 3.39 (m, 2H), 3.05 - 2.99 (m, 2H), 2.93 - 2.80 (m, 4H), 2.07 - 2.01 (m, 2H), 0.98 (t, *J* = 7.2 Hz, 2H). |
| M001230 | 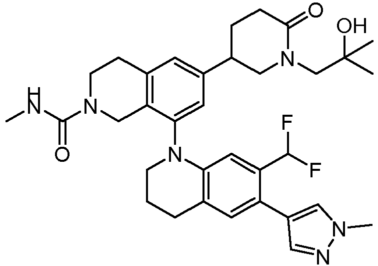 | ¹H NMR (400 MHz, DMSO-*d₆*) δ 7.75 (s, 1H), 7.49 (s, 1H), 7.20 - 7.05 (m, 2H), 6.80 - 6.53 (m, 1H), 6.53 - 6.50 (m, 1H), 6.25 - 6.20 (m, 1H), 4.55 (s, 1H), 4.46 - 4.42 (m, 1H), 4.13 - 4.06 (m, 1H), 3.86 (s, 3H), 3.63 - 3.50 (m, 6H), 3.30 - 3.25 (m, 4H), 3.10 - 3.02 (m, 1H), 2.95 - 2.75 (m, 4H), 2.55 - 2.50 (m, 3H), 2.35 - 1.30 (m, 2H), 2.13 - 1.85 (m, 4H), 1.10 - 1.05 (m, 6H),. |
| M001231 | 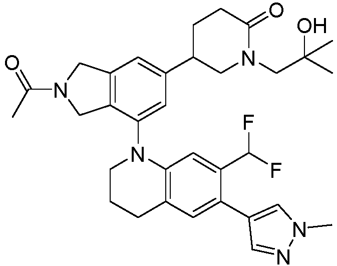 | ¹H NMR (400 MHz, DMSO-*d₆*) δ 7.75 (s, 1H), 7.49 (s, 1H), 7.26 - 7.20 (m, 2H), 7.13 (s, 1H), 6.75 (t, *J* = 55.2 Hz, 1H), 6.39 (d, *J* = 25.6 Hz, 1H), 4.87 (s, 1H), 4.66 (s, 1H), 4.61 (s, 1H), 4.54 (s, 1H), 4.27 (s, 1H), 3.86 (s, 3H), 3.64 - 3.47 (m, 4H), 3.30 - 3.22 (m, 2H), 3.18 - 3.12 (m, 1H), 2.88 (s, 2H), 2.45 - 2.37 (m, 3H), 2.03 - 1.97 (m, 6H), 1.07 - 1.05 (m, 6H). |
| M001232 | 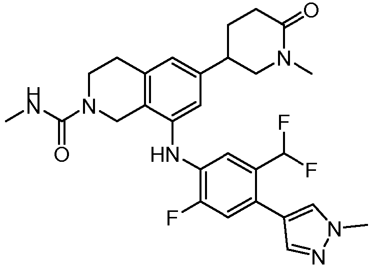 | ¹H NMR (400 MHz, DMSO-*d₆*) δ 7.87 (s, 1H), 7.62 (s, 1H), 7.50 (s, 1H), 7.34 (d, *J* = 12.0 Hz, 1H), 7.15 (d, *J* = 8.0 Hz, 1H), 7.05 - 6.76 (m, 3H), 6.49 - 6.41 (m, 1H), 4.39 (s, 2H), 3.89 (s, 3H), 3.52 - 3.49 (m, 2H), 3.32 - 3.24 (m, 2H), 3.05 - 2.99 (m, 1H), 2.79 - 2.76 (m, 5H), 2.59 (d, *J* = 4.0 Hz, 3H), 2.36 - 2.22 (m, 2H), 1.97 - 1.82 (m, 2H). |
| M001233 | 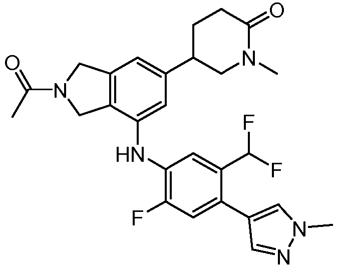 | ¹H NMR (400 MHz, DMSO-*d₆*) δ 7.99 - 7.88 (m, 2H), 7.65 (d, *J* = 4.0 Hz, 1H), 7.41 - 7.28 (m, 2H), 7.10 - 6.78 (m, 3H), 4.79 (d, *J* = 32.0 Hz, 2H), 4.56 (d, *J* = 44.0 Hz, 2H), 3.89 (s, 3H), 3.29 (s, 2H), 3.07 (d, *J* = 4.0 Hz, 1H), 2.79 (d, *J* = 4.0 Hz, 3H), 2.33 - 2.28 (m, 2H), 2.06 (d, *J*= 4.0 Hz, 3H), 1.98 - 1.85 (m, 2H). |
| M001234 | 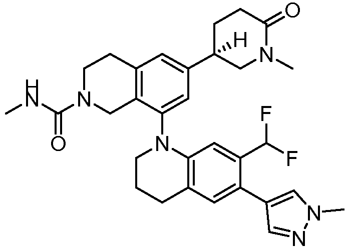 | H NMR (400 MHz, DMSO-*d₆*) δ 7.72 (s, 1H), 7.47 (s, 1H), 7.12 (s, 1H), 7.09 (s, 2H), 6.70 (m, 1H), 6.50-6.47 (m, 1H), 6.17 (d, *J* = 2.8 Hz, 1H), 4.44 (d, *J* = 16.8 Hz, 1H), 4.09 (d, *J* = 16.8 Hz, 1H), 3.86 (s, 3H), 3.58 - 3.36 (m, 6H), 3.11 - 3.05 (m, 1H), 2.93-2.83 (m, 4H), 2.80 (s, 3H), 2.54 (d, *J* = 4.4 Hz, 3H), 2.35 - 2.27 (m, 2H), 2.09 - 1.91 (m, 4H). |
| M001235 | 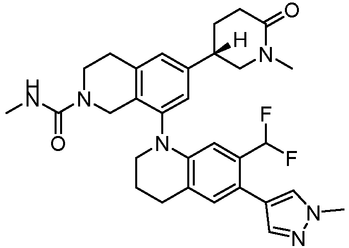 | ¹H NMR (400 MHz, DMSO-*d₆*) δ 7.72 (s, 1H), 7.47 (s, 1H), 7.12 (s, 1H), 7.09 (s, 2H), 6.70 (m, 1H), 6.50-6.47 (m, 1H), 6.17 (d, *J* = 2.8 Hz, 1H), 4.44 (d, *J* = 16.8 Hz, 1H), 4.09 (d, *J* = 16.8 Hz, 1H), 3.85 (s, 3H), 3.60 - 3.42 (m, 6H), 3.12 - 3.05 (m, 1H), 2.88-2.83 (m, 4H), 2.80 (s, 3H), 2.54 (d, *J* = 4.0 Hz, 3H), 2.35 - 2.27 (m, 2H), 2.09 - 1.91 (m, 4H). |
| M001236 | 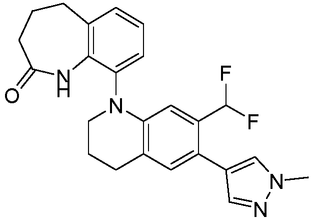 | ¹H NMR (400 MHz, DMSO-*d₆*) δ 8.90 (s, 1H), 7.71 (s, 1H), 7.46 (s, 1H), 7.24 (s, 3H), 7.08 (s, 1H), 6.70 (t, *J* = 55.2 Hz, 1H), 6.19 (s, 1H), 3.85 (s, 3H), 3.60 - 3.46 (m, 2H), 2.90 - 2.83 (m, 2H), 2.77 - 2.71 (m, 2H), 2.25 - 1.97 (m, 6H). |
| M001237 | 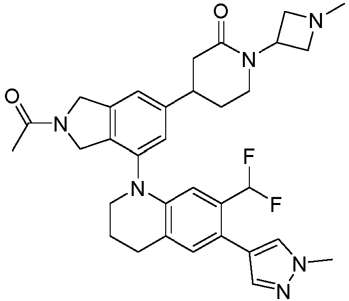 | m/z+=589.4 |
| M001238 | 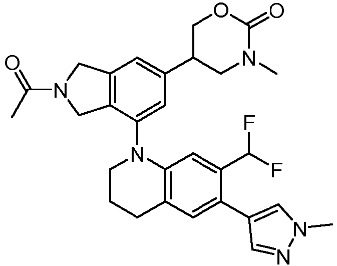 | ¹H NMR (400 MHz, DMSO-d6) δ 7.75 (s, 1H), 7.50 (s, 1H), 7.30 - 7.22 (m, 2H), 7.14 (s, 1H), 6.75 (t, J = 55.0 Hz, 1H), 6.45 - 6.33 (m, 1H), 4.89 - 4.85 (m, 1H), 4.69 - 4.54 (m, 2H), 4.38 - 4.25 (m, 3H), 3.86 (s, 3H), 3.60 - 3.47 (m, 5H), 2.91 - 2.84 (m, 5H), 2.04 (s, 3H), 2.01 - 1.93 (m, 2H). |
| M001239 | 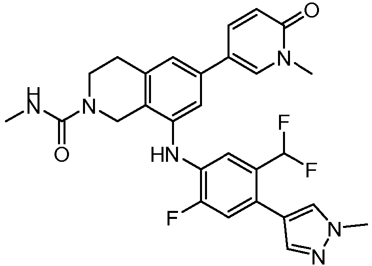 | ¹H NMR (400 MHz, DMSO-*d₆*) δ 8.03 (d, *J=* 2.0 Hz, 1H), 7.87 (s, 1H), 7.71 - 7.68 (m, 1H), 7.61 (d, *J* = 8.0 Hz, 2H), 7.34 *(d, J=* 12.0 Hz, 1H), 7.14 *(d, J=* 8.0 Hz, 2H), 7.06 - 6.75 (m, 2H), 6.48 - 6.43 (m, 2H), 4.43 (s, 2H), 3.88 (s, 3H), 3.55 - 3.52 (m, 2H), 3.47 (s, 3H), 2.84 (s, 2H), 2.59 (d, *J* = 4.0 Hz, 3H). |
| M001240 | 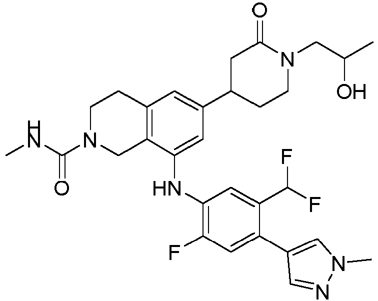 | ¹H NMR (400 MHz, DMSO-*d₆*) δ 7.87 (s, 1H), 7.62 (s, 1H), 7.48 (s, 1H), 7.34 (d, *J* = 12.0 Hz, 1H), 7.15 (d, *J* = 8.0 Hz, 1H), 7.04 - 6.77 (m, 3H), 6.45 (d, *J* = 4.0 Hz, 1H), 4.69 - 4.65 (m, 1H), 4.39 (s, 2H), 3.88 (s, 3H), 3.87 - 3.80 (m, 1H), 3.52 - 3.49 (m, 2H), 3.40 (s, 1H), 3.30 (d, *J* = 4.0 Hz, 1H), 3.08 - 2.98 (m, 2H), 2.77 (s, 2H), 2.59 (d, *J* = 4.0 Hz, 3H), 2.45 - 2.40 (m, 2H), 2.32 - 2.29 (m, 1H), 1.90 - 1.82 (m, 2H), 1.01 - 0.99 (m, 3H). |
| M001241 | 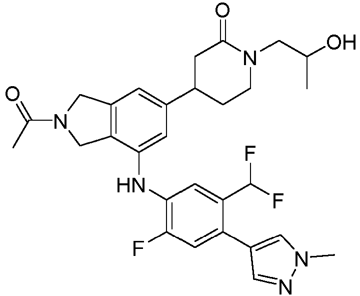 | ¹H NMR (400 MHz, DMSO-*d₆*) δ 7.95 - 7.89 (m, 2H), 7.64 (d, *J* = 4.0 Hz, 1H), 7.41 - 7.28 (m, 2H), 7.09 - 6.79 (m, 2H), 6.74 (s, 1H), 4.82 (s, 1H), 4.75 (s, 1H), 4.70 - 4.64 (m, 1H), 4.61 (s, 1H), 4.50 (s, 1H), 3.89 - 3.87 (m, 5H), 3.56 - 3.39 (m, 2H), 3.12 - 3.04 (m, 2H), 2.42 (s, 1H), 2.35 - 2.29 (m, 1H), 2.05 (d, *J* = 4.0 Hz, 3H), 1.91 - 1.83 (m, 2H), 1.02 - 0.99 (m, 3H). |
| M001242 | 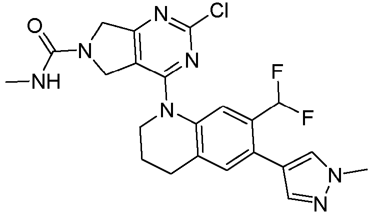 | m/z+=474.2 |
| M001243 | 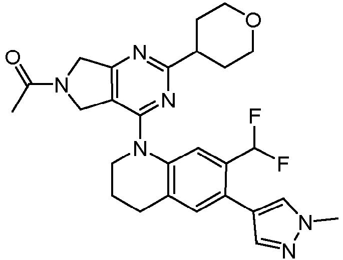 | m/z+=509.4 |
| M001244 | 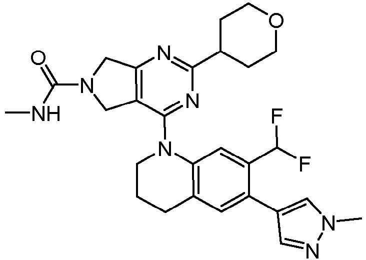 | m/z+=524.5 |
| M001245 | 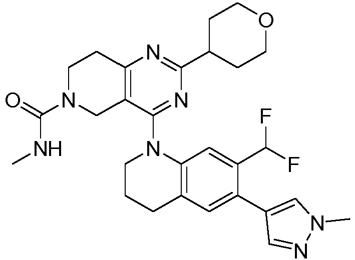 | H NMR: EB2743-48-P1N1, (400 MHz, MeOD). δ 7.74 (s, 1H), 7.60 (s, 1H), 7.30 (s, 1H), 6.84 - 6.82 (m, 1H), 6.82 - 6.53 (m, 1H), 4.62 (s, 1H), 4.07 (s, 3H), 4.04 (d, *J* = 2.0 Hz, 1H), 3.97 (s, 3H), 3.92 (t, *J* = 6.0 Hz, 2H), 3.68 (t, *J=* 6.4 Hz, 2H), 3.58 (dt, *J* = 2.4, 11.8 Hz, 2H), 3.06 - 2.90 (m, 5H), 2.68 (s, 3H), 2.18 - 2.10 (m, 2H), 2.07 - 1.96 (m, 2H), 1.94 - 1.87 (m, 2H) |
| M001246 | 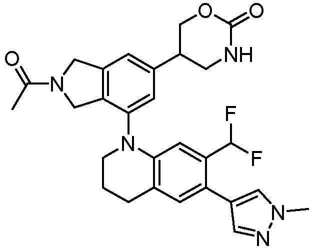 | ¹H NMR (400 MHz, DMSO-*d₆*) δ 7.75 (s, 1H), 7.50 (s, 1H), 7.42 - 7.35 (m, 1H), 7.29 - 7.22 (m, 2H), 7.14 (s, 1H), 6.75 (t, *J=* 55.2 Hz, 1H), 6.44 - 6.35 (m, 1H), 4.90 - 4.84 (m, 1H), 4.76 - 4.45 (m, 2H), 4.40 - 4.23 (m, 3H), 3.86 (s, 3H), 3.60 - 3.51 (m, 2H), 3.44 - 3.37 (m, 3H), 2.91 - 2.83 (s, 2H), 2.05 - 1.96 (m, 5H). |
| M001247 | 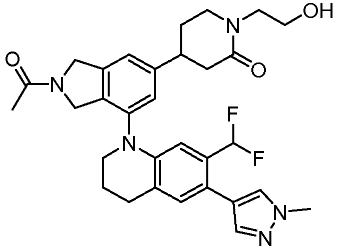 | ¹H NMR (400 MHz, DMSO-*d₆*) δ 7.75 (s, 1H), 7.50 (s, 1H), 7.20 - 7.12 (m, 3H), 6.75 (t, *J* = 55.2 Hz, 1H), 6.46 - 6.36 (m, 1H), 4.90 - 4.84 (m, 1H), 4.80 - 4.49 (m, 3H), 4.33 - 4.22 (m, 1H), 3.86 (s, 3H), 3.69 - 3.43 (m, 6H), 3.40 - 3.36 (m, 2H), 3.18 - 3.08 (m, 1H), 2.91 - 2.83 (m, 2H), 2.45 - 2.31 (m, 2H), 2.07 - 1.91 (m, 7H). |
| M001248 | 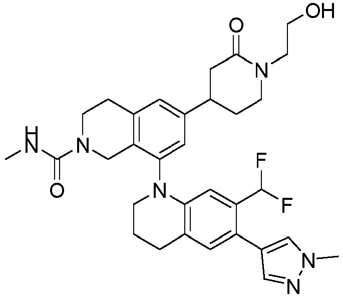 | ¹H NMR (400 MHz, DMSO-*d₆*) δ 7.72 (s, 1H), 7.47 (s, 1H), 7.15 - 6.97 (m, 3H), 6.70 (t, *J* = 55.2 Hz, 1H), 6.53 - 6.42 (m, 1H), 6.18 (s, 1H), 4.80 - 4.33 (m, 2H), 4.15 - 4.06 (m, 1H), 3.86 (s, 3H), 3.45 - 3.29 (m, 10H), 3.09 - 3.01 (m, 1H), 2.94 - 2.79 (m, 4H), 2.54 (d, *J* = 4.0 Hz, 3H), 2.46 - 2.37 (m, 2H), 2.12 - 1.85 (m, 4H). |
| M001249 | 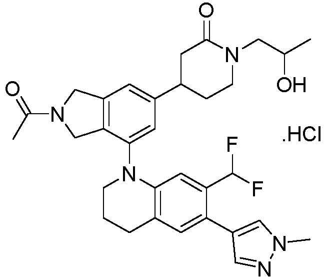 | ¹H NMR (400 MHz, DMSO-*d₆*) δ 7.76 (s, 1H), 7.50 (s, 1H), 7.25 - 7.10 (m, 3H), 6.75 (t, *J* = 55.2 Hz, 1H), 6.46 - 6.35 (m, 1H), 4.89 - 4.83 (m, 1H), 4.72 - 4.57 (m, 2H), 4.30 - 4.26 (m, 1H), 3.94 - 3.88 (m, 1H), 3.86 (s, 3H), 3.59 - 3.30 (m, 5H), 3.19 - 3.05 (m, 2H), 2.88 (s, 2H), 2.46 - 2.36 (m, 2H), 2.06 - 1.91 (m, 7H), 1.02 (s, 3H). |
| M001250 | 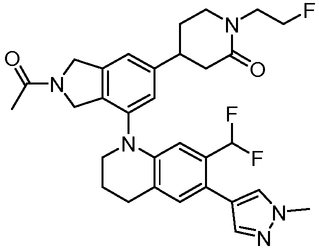 | ¹H NMR (400 MHz, DMSO-*d₆*) δ 7.75 (s, 1H), 7.49 (s, 1H), 7.22 - 7.16 (m, 2H), 7.13 (s, 1H), 6.75 (t, *J* = 55.2 Hz, 1H), 6.45 - 6.35 (m, 1H), 4.90 - 4.83 (m, 1H), 4.74 - 4.44 (m, 4H), 4.38 - 4.20 (s, 3H), 3.86 (s, 3H), 3.67 - 3.53 (m, 4H), 3.51 - 3.38 (m, 2H), 3.21 - 3.10 (m, 1H), 2.91 - 2.84 (m, 2H), 2.57 - 2.52 (m, 1H), 2.47 - 2.38 (m, 1H), 2.07 - 1.96 (m, 7H). |
| M001251 | 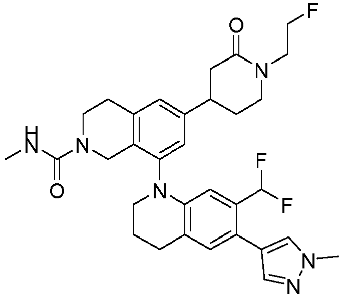 | ¹H NMR (400 MHz, DMSO-*d₆*) δ 7.72 (s, 1H), 7.47 (s, 1H), 7.14 - 7.04 (m, 3H), 6.70 (t, *J* = 55.2 Hz, 1H), 6.53 - 6.43 (m, 1H), 6.18 (s, 1H), 4.66 - 4.55 (m, 1H), 4.52 - 4.38 (m, 2H), 4.17 - 4.06 (m, 1H), 3.86 (s, 3H), 3.68 - 3.55 (m, 2H), 3.55 - 3.35(m, 6H), 3.13 - 3.03 (m, 1H), 2.97 - 2.79 (m, 4H), 2.54 (d, *J* = 4.2 Hz, 3H), 2.48 - 2.35 (m, 2H), 2.11 - 1.87 (m, 4H). |
| M001252 | 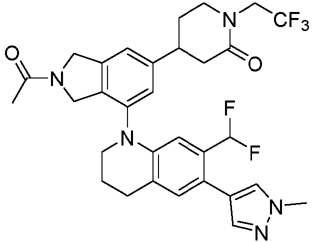 | ¹H NMR (400 MHz, DMSO-*d₆*) δ 7.75 (s, 1H), 7.49 (s, 1H), 7.23 - 7.19 (m, 2H), 7.13 (s, 1H), 6.74 (t, *J* = 55.2 Hz, 1H), 6.40 (d, *J* = 25.6 Hz, 1H), 4.86 (s, 1H), 4.65 (s, 1H), 4.60 (s, 1H), 4.29 (s, 1H), 4.24 - 4.10 (m, 2H), 3.86 (s, 3H), 3.56 - 3.53 (s, 2H), 3.51 - 3.44 (m, 2H), 3.24 - 3.10 (m, 2H), 2.87 (s, 2H), 2.62 - 2.57 (m, 1H), 2.10 - 1.93 (m, 7H). |
| M001253 | 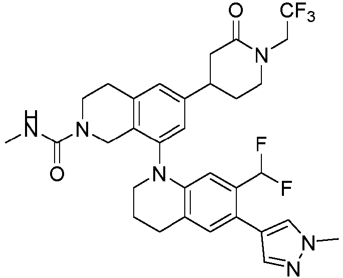 | ¹H NMR (400 MHz, DMSO-*d₆*) δ 7.72 (s, 1H), 7.47 (s, 1H), 7.09 (s, 3H), 6.63 (d, *J* = 55.2 Hz, 1H), 6.48 (d, *J* = 4.4 Hz, 1H), 6.17 (s, 1H), 4.46 - 4.41 (m, 1H), 4.22 - 4.17 (m, 2H), 4.15 - 4.03 (m, 1H), 3.85 (s, 3H), 3.65 - 3.50 (m, 2H), 3.53 - 3.38 (m, 4H), 3.21 - 3.04 (m, 1H), 3.04 - 2.86 (m, 2H), 2.84 - 2.80 (m, 2H), 2.57 - 2.54 (m, 3H), 2.45 - 2.43 (m, 2H), 2.16 - 1.87 (m, 4H). |
| M001254 | 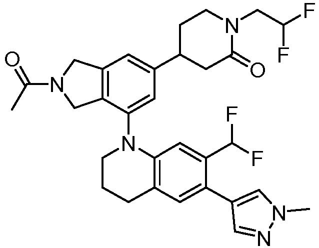 | ¹H NMR (400 MHz, DMSO) δ 7.75 (s, 1H), 7.49 (s, 1H), 7.19 (t, *J =* 8.0 Hz, 2H), 7.13 (s, 1H), 6.74 (t, *J* = 55.2 Hz, 1H), 6.40 (d, *J* = 26.4 Hz, 1H), 6.31 - 5.95 (m, 1H), 4.86 (s, 1H), 4.65 (s, 1H), 4.61 (s, 1H), 4.28 (s, 1H), 3.86 (s, 3H), 3.73 - 3.68 (s, 2H), 3.62 - 3.53 (m, 2H), 3.52 - 3.40 (m, 2H), 3.19 - 3.16 (m, 1H), 2.87 (s, 2H), 2.57- 2.54 (m, 1H), 2.49 - 2.40 (m, 1H), 2.06 - 1.91 (m, 7H). |
| M001255 | 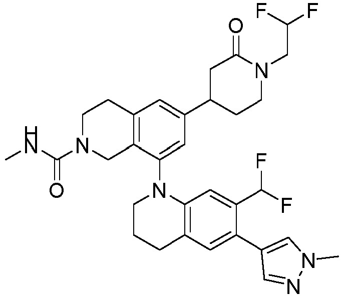 | ¹H NMR (400 MHz, DMSO-*d₆*) δ 7.72 (s, 1H), 7.47 (s, 1H), 7.09 - 7.07 (m, 3H), 6.70 (t, *J* = 55.2 Hz, 1H), 6.48 (d, *J* = 4.4 Hz, 1H), 6.18 (s, 1H), 6.30 - 5.94 (m, 1H), 4.46 - 4.41 (m, 1H), 4.13 - 4.08 (m, 1H), 3.86 (s, 3H), 3.77 - 3.68 (m, 2H), 3.63 - 3.39 (m, 6H), 3.17 - 3.01 (m, 1H), 3.00 - 2.74 (m, 4H), 2.57 - 2.54 (m, 3H), 2.47 - 2.24 (m, 2H), 2.10 - 2.01 (m, 3H), 1.95 - 1.86 (m, 1H). |
| M001256 | 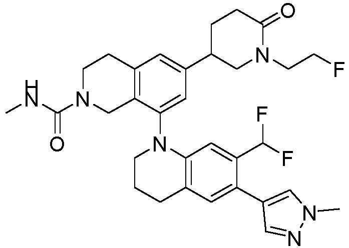 | ¹H NMR (400 MHz, DMSO-*d₆*) δ 7.72 (s, 1H), 7.47 (s, 1H), 7.14 - 7.04 (m, 3H), 6.70 (t, *J* = 55.2 Hz, 1H), 6.56 - 6.47 (m, 1H), 6.17 (d, *J* = 4.0 Hz, 1H), 4.66 - 4.55 (m, 1H), 4.52 - 4.38 (m, 2H), 4.17 - 4.06 (m, 1H), 3.85 (s, 3H), 3.58 - 3.41 (m, 8H), 3.13 - 3.03 (m, 1H), 2.97 - 2.79 (m, 4H), 2.54 (d, *J* = 4.4 Hz, 3H), 2.42 - 2.32 (m, 2H), 2.09 - 1.91 (m, 4H). |
| M001257 | 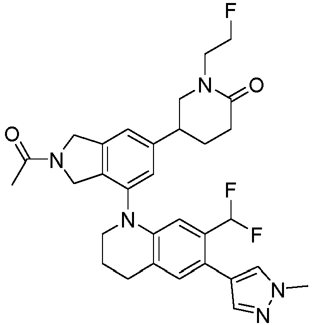 | ¹H NMR (400 MHz, DMSO-*d₆*) δ 7.75 (s, 1H), 7.49 (s, 1H), 7.26 - 7.21 (m, 2H), 7.13 (s, 1H), 6.75 (t, *J* = 55.2 Hz, 1H), 6.45 - 6.35 (m, 1H), 4.90 - 4.83 (m, 1H), 4.74 - 4.44 (m, 4H), 4.32 - 4.23 (m, 1H), 3.86 (s, 3H), 3.64 - 3.44 (m, 6H), 3.21 - 3.13 (m, 1H), 2.91 - 2.85 (m, 2H), 2.43 - 2.34 (m, 2H), 2.06 - 1.96 (m, 7H). |
| M001258 | 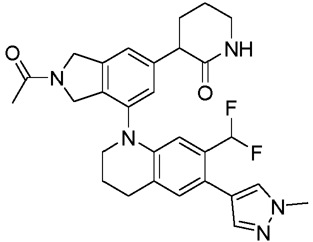 | ¹H NMR (400 MHz, DMSO-*d₆*) δ 7.75 (s, 1H), 7.64 (d, *J* = 7.6 Hz, 1H), 7.49 (s, 1H), 7.12 (s, 1H), 7.10 - 7.03 (m, 2H), 6.74 (t, *J* = 55.2 Hz, 1H), 6.43 *(d, J=* 29.2 Hz, 1H), 4.86 (s, 1H), 4.65 (s, 1H), 4.62 (s, 1H), 4.29 (s, 1H), 3.86 (s, 3H), 3.69 - 3.43 (m, 3H), 3.29 - 3.09 (m, 2H), 2.87 (s, 2H), 2.54 (s, 3H), 2.14 - 1.93 (m, 4H), 1.86 - 1.56 (m, 2H). |
| M001260 | 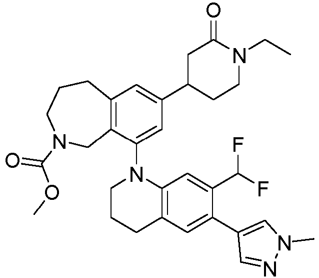 | ¹H NMR (400 MHz, DMSO-*d₆*) δ 7.71 (s, 1H), 7.47 (s, 1H), 7.09 (d, *J* = 8.0 Hz, 2H), 7.02 (d, *J* = 8.0 Hz, 1H), 6.86 - 6.53 (m, 1H), 6.09 (d, *J* = 48.0 Hz, 1H), 4.85 - 3.91 (m, 2H), 3.85 (s, 3H), 3.69 (s, 1H), 3.54 (d, *J* = 28.0 Hz, 3H), 3.39 - 3.34 (m, 2H), 3.31 - 3.19 (m, 5H), 3.02 - 2.88 (m, 5H), 2.49 - 2.30 (m, 2H), 2.16 - 1.98 (m, 3H), 1.91 - 1.58 (m, 3H), 1.03 - 0.96 (m, 3H). |
| M001261 | 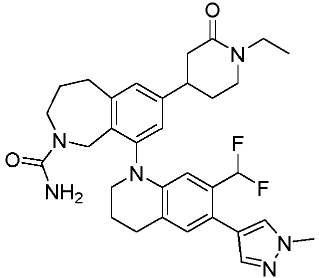 | ¹H NMR (400 MHz, DMSO-*d₆*) δ 7.72 (s, 1H), 7.47 (s, 1H), 7.08 (s, 2H), 7.00 (d, *J* = 8.0 Hz, 1H), 6.83 - 6.56 (m, 1H), 6.14 (s, 1H), 5.67 (s, 2H), 4.72 (d, *J* = 12.0 Hz, 1H), 3.91 (d, *J* = 16.0 Hz, 1H), 3.86 (s, 3H), 3.73 (d, *J* = 16.0 Hz, 1H), 3.58 (s, 2H), 3.38 - 3.34 (m, 1H), 3.30 - 3.21 (m, 3H), 3.05 - 2.84 (m, 5H), 2.44 - 2.30 (m, 3H), 2.18 - 2.07 (m, 1H), 2.00 (d, *J* = 8.0 Hz, 2H), 1.89 - 1.85 (m, 1H), 1.75 (s, 1H), 1.65 (s, 1H), 1.04 - 0.96 (m, 3H). |
| M001262 | 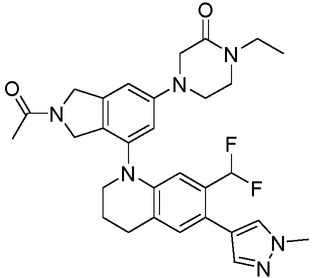 | ¹H NMR (400 MHz, DMSO-*d₆*) δ 7.75 (s, 1H), 7.50 (s, 1H), 7.12 (s, 1H), 6.88 - 6.83 (m, 2H), 6.80 - 6.61 (m, 1H), 6.51 - 6.40 (m, 1H), 4.87 (s, 1H), 4.60 - 4.50 (m, 2H), 4.19 (s, 1H), 3.88 (s, 3H), 3.80 - 3.75 (m, 2H), 3.60 - 3.52 (m, 6H), 3.50 - 3.30 (m, 2H), 2.89 - 2.86 (m, 2H), 2.12 - 1.95 (m, 5H), 1.10 - 1.02 (m, 3H). |
| M001263 | 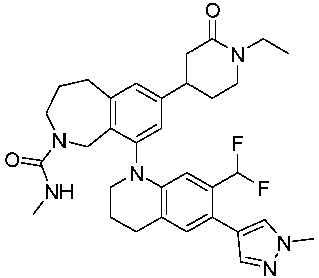 | ¹H NMR (400 MHz, DMSO-*d₆*) δ 7.73 (s, 1H), 7.48 (s, 1H), 7.09 (d, *J* = 8.0 Hz, 2H), 6.99 (d, *J* = 8.0 Hz, 1H), 6.85 - 6.58 (m, 1H), 6.20 (s, 1H), 5.85 (s, 1H), 4.65 (d, *J=* 16.0 Hz, 1H), 4.06 (d, *J=* 12.0 Hz, 1H), 3.86 (s, 3H), 3.54 (d, *J* = 20.0 Hz, 4H), 3.30 - 3.21 (m, 3H), 3.01 - 2.88 (m, 5H), 2.47 (s, 1H), 2.43 - 2.32 (m, 5H), 2.20 - 2.09 (m, 1H), 1.98 (d, *J* = 12.0 Hz, 2H), 1.91 - 1.80 (m, 1H), 1.71 (s, 2H), 1.03 - 0.96 (m, 3H). |
| M001264 | 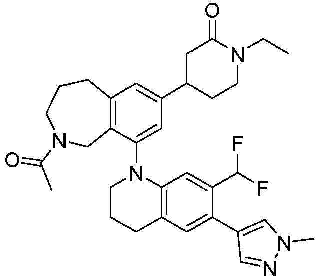 | ¹H NMR (400 MHz, DMSO-*d₆*) δ 7.72 (d, *J=* 16.0 Hz, 1H), 7.47 (d, *J* = 16.0 Hz, 1H), 7.10 - 7.00 (m, 3H), 6.82 - 6.54 (m, 1H), 6.17 (d, *J* = 116.0 Hz, 1H), 5.04 (d, *J= 16.0* Hz, 1H), 4.51 (s, 1H), 3.86 (d, *J* = 4.0 Hz, 3H), 3.78 (d, *J* = 20.0 Hz, 1H), 3.66 (s, 1H), 3.60 - 3.52 (m, 1H), 3.44 - 3.36 (m, 2H), 3.29 - 3.22 (m, 2H), 3.01 - 2.88 (m, 5H), 2.45 - 2.29 (m, 2H), 2.22 - 1.66 (m, 10H), 1.05 - 0.96 (m, 3H). |
| M001268 | 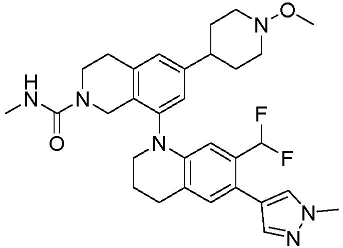 | ¹H NMR (400 MHz, DMSO-*d₆*) δ 7.72 (s, 1H), 7.47 (s, 1H), 7.08 (s, 1H), 7.04 (s, 1H), 6.99 (s, 1H), 6.69 (t, *J* = 55.2 Hz, 1H), 6.51 - 6.42 (m, 1H), 6.18 (s, 1H), 4.46 - 4.36 (m, 1H), 4.17 - 4.05 (m, 1H), 3.86 (s, 3H), 3.57 - 3.35 (m, 9H), 3.03 - 2.70 (m, 5H), 2.54 (d, *J* = 4.0 Hz, 3H), 2.42 - 2.28 (m, 2H), 2.10 - 1.99 (m, 2H), 1.90 - 1.76 (m, 2H), 1.74 - 1.56 (m, 2H). |
| M001269 | 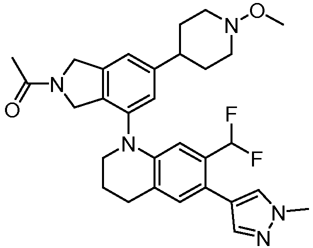 | ¹H NMR (400 MHz, DMSO-*d₆*) δ 7.75 (s, 1H), 7.49 (s, 1H), 7.20 - 7.06 (m, 3H), 6.74 (t, *J* = 55.2 Hz, 1H), 6.50 - 6.34 (m, 1H), 4.86 - 4.83 (m, 1H), 4.66 - 4.58 (m, 2H), 4.31 - 4.24 (m, 1H), 3.86 (s, 3H), 3.59 - 3.51 (m, 2H), 3.50 - 3.43 (m, 3H), 3.42 - 3.34 (m, 2H), 2.91 - 2.84 (m, 2H), 2.61 - 2.56 (m, 2H), 2.44 - 2.41 (m, 1H), 2.05 - 1.95 (m, 5H), 1.91 - 1.81 (m, 2H), 1.78 - 1.64 (m, 2H). |
| M001270 | 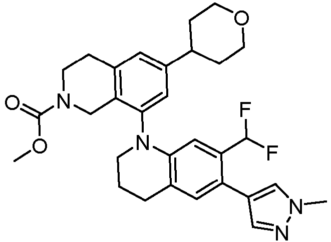 | ¹H NMR (400 MHz, DMSO-*d₆*) δ 7.73 (s, 1H), 7.48 (s, 1H), 7.09 (d, *J=* 7.2 Hz, 2H), 7.05 (s, 1H), 6.70 (t, *J* = 55.2 Hz, 1H), 6.19 (s, 1H), 4.50 - 4.36 (m, 1H), 4.36 - 4.15 (m, 1H), 3.99 - 3.89 (m, 2H), 3.86 (s, 3H), 3.67 - 3.48 (m, 6H), 3.44 - 3.36 (m, 3H), 2.99 - 2.81 (m, 4H), 2.79 - 2.71 (m, 1H), 2.11 - 1.95 (m, 2H), 1.72 - 1.57 (m, 4H). |
| M001271 | 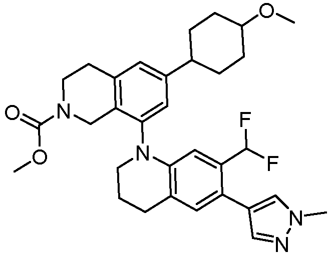 | ¹H NMR (400 MHz, DMSO-*d₆*) δ 7.73 (s, 1H), 7.47 (s, 1H), 7.10 (s, 1H), 7.03 (s, 1H), 6.97 (s, 1H), 6.70 (t, *J* = 55.2 Hz, 1H), 6.18 (s, 1H), 4.47 - 4.36 (m, 1H), 4.35 - 4.16 (m, 1H), 3.86 (s, 3H), 3.67 - 3.47 (m, 6H), 3.45 - 3.42 (m, 1H), 3.41 - 3.35 (m, 1H), 3.21 (s, 3H), 2.98 - 2.78 (m, 4H), 2.59 - 2.51 (m, 1H), 2.06 - 1.89 (m, 4H), 1.70 - 1.58 (m, 2H), 1.54 - 1.42 (m, 4H). |
| M001272 | 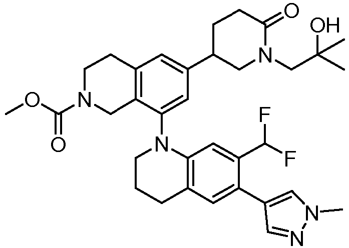 | ¹H NMR (400 MHz, DMSO-*d₆*) δ 7.73 (s, 1H), 7.48 (s, 1H), 7.19 - 7.09 (m, 3H), 6.70 (t, *J* = 55.2 Hz, 1H), 6.21 - 6.12 (m, 1H), 4.557 - 4.50 (m, 1H), 4.48 - 4.38 (m, 1H), 4.36 - 4.14 (m, 1H), 3.86 (s, 3H), 3.65 - 3.48 (m, 8H), 3.42 - 3.37 (m, 1H), 3.30 - 3.22 (m, 2H), 3.13 - 3.03 (m, 1H), 2.96 - 2.80 (m, 4H), 2.44 - 2.34 (m, 2H), 2.08 - 1.88 (m, 4H), 1.05 (d, *J* = 4.0 Hz, 6H). |
| M001274 | 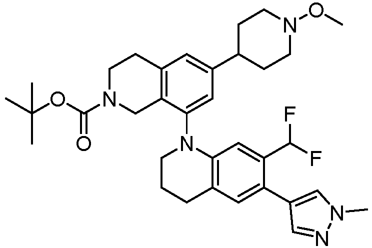 | ¹H NMR (400 MHz, DMSO-*d₆*) δ 7.71 (s, 1H), 7.46 (s, 1H), 7.11 - 7.00 (m, 3H), 6.69 (t, *J =* 55.2 Hz, 1H), 6.16 (s, 1H), 4.44 - 4.32 (m, 1H), 4.28 - 4.06 (m, 1H), 3.86 (s, 3H), 3.59 - 3.48 (m, 3H), 3.44 - 3.40 (3, 3H), 3.39 - 3.35 (m, 2H), 2.95 - 2.79 (m, 4H), 2.45 - 2.30 (m, 2H), 2.07 - 1.99 (m, 2H), 1.90 - 1.76 (m, 2H), 1.75 - 1.59 (m, 2H), 1.49 - 1.17 (m, 11H). |
| M001275 | 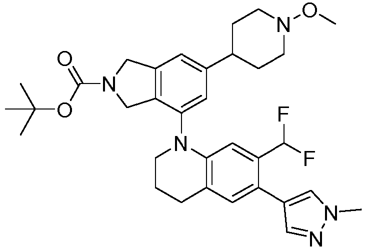 | ¹H NMR (400 MHz, DMSO-*d₆*) δ 7.75 (s, 1H), 7.49 (s, 1H), 7.20 - 7.00 (m, 3H), 6.75 (t, *J =* 55.2 Hz, 1H), 6.45 - 6.31 (m, 1H), 4.68 - 4.56 (m, 2H), 4.40 - 4.17 (m, 2H), 3.86 (s, 3H), 3.61 - 4.49 (m, 3H), 3.48 - 3.41 (m, 5H), 2.92 - 2.83 (m, 2H), 2.46 - 2.32 (m, 2H), 2.04 - 1.95 (m, 2H), 1.92 - 1.78 (m, 2H), 1.78 - 1.62 (m, 2H), 1.47 - 1.34 (m, 9H). |
| M001276 | 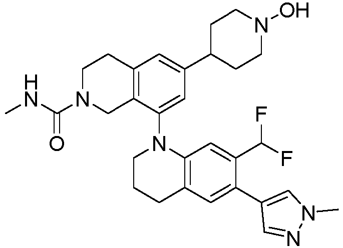 | ¹H NMR (400 MHz, DMSO-*d₆*) δ 7.93 (s, 1H), 7.71 (s, 1H), 7.47 (s, 1H), 7.08 (s, 1H), 7.04 (s, 1H), 6.99 (s, 1H), 6.83 - 6.45 (m, 2H), 6.18 (s, 1H), 4.46 - 4.38 (m, 1H), 4.15 - 4.06 (m, 1H), 3.86 (s, 3H), 3.55 - 3.41 (m, 4H), 3.25 - 3.05 (m, 2H), 3.00 - 2.77 (m, 5H), 2.54 (d, *J* = 4.2 Hz, 3H), 2.45 - 2.33 (m, 2H), 2.10 - 2.00 (m, 2H), 1.85 - 1.40 (m, 4H). |
| M001277 | 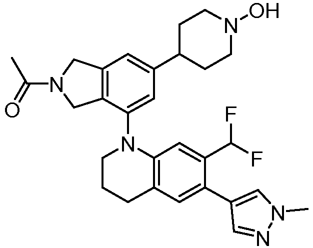 | ¹H NMR (400 MHz, DMSO-*d₆*) δ 7.97 (s, 1H), 7.74 (s, 1H), 7.49 (s, 1H), 7.20 - 7.02 (m, 3H), 6.74 (t, *J* = 55.2 Hz, 1H), 6.49 - 6.31 (m, 1H), 4.89 - 4.81 (m, 1H), 4.75 - 4.45 (m, 2H), 4.34 - 4.22 (m, 1H), 3.86 (s, 3H), 3.58 - 3.52 (m, 2H), 3.20 - 3.13 (m, 2H), 2.91 - 2.84 (m, 2H), 2.59 - 2.54 (m, 1H), 2.45 - 2.37 (m, 2H), 2.07 - 1.96 (m, 5H), 1.90 - 1.62 (m, 4H). |
| M001280 | 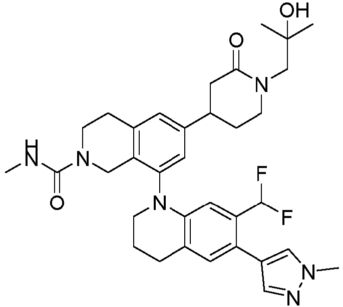 | ¹H NMR (400 MHz, DMSO-*d₆*) δ 7.72 (s, 1H), 7.47 (s, 1H), 7.08 (d, *J* = 6.4 Hz, 3H), 6.83-6.56 (m,1H), 6.48 (s, 1H), 6.18 (s, 1H), 4.45-4.40 (m, 1H), 4.12-4.07 (m, 1H), 3.86 (s, 3H), 3.49 (d, *J* = 12.2 Hz, 6H), 3.34 (s, 2H), 3.19-3.16 (m, 1H), 3.08 (s, 1H), 2.87-2.82 (m, 4H), 2.54 (d, *J* = 3.8 Hz, 3H), 2.45 - 2.36 (m, 2H), 2.03-1.93 (m, 4H), 1.06 (s, 6H). |
| M001281 | 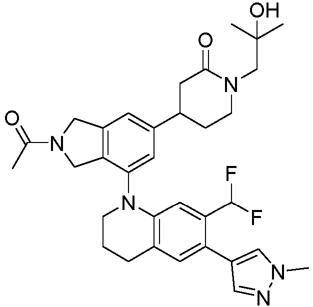 | ¹H NMR (400 MHz, DMSO-*d₆*) δ 7.75 (s, 1H), 7.49 (s, 1H), 7.21-7.16 (m, 2H), 7.13 (s, 1H), 6.88-6.60 (m, 1H), 6.414-6.37(m, 1H), 4.86 (s, 1H), 4.68-4.59 (m, 2H), 4.28 (s, 1H), 3.86 (s, 3H), 3.53 -3.51(m, 4H), 3.26 - 3.13 (m, 2H), 2.88 (s, 2H), 2.42-2.32 (m, 3H), 2.06 - 1.89 (m, 8H), 1.08-1.06 (m, 6H). |
| M001287 | 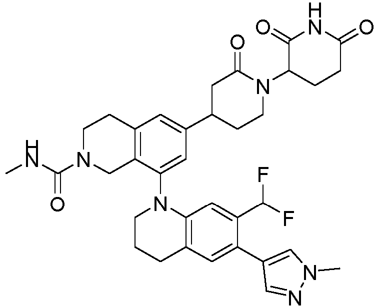 | ¹H NMR (400 MHz, DMSO-*d₆*) δ 10.80 (s, 1H), 7.71 (s, 1H), 7.47 (s, 1H), 7.10 (d, *J=* 11.2 Hz, 3H), 6.70 (t, *J* = 55.2 Hz, 1H), 6.48 - 6.45 (m, 1H), 6.18 (s, 1H), 4.44 (d, *J=* 16.8 Hz, 2H), 4.40 - 4.29 (m, 1H), 4.11 *(d, J=* 16.8 Hz, 2H), 3.86 (s, 3H), 3.57 - 3.51 (m, 2H), 3.50 - 3.42 (m, 2H), 3.40 - 3.35 (m, 2H), 3.30 - 3.16 (m, 2H), 3.16 - 3.05 (m, 2H), 2.97 - 2.77 (m, 5H), 2.55 (d, *J* = 4.4 Hz, 3H), 2.14 - 1.87 (m, 4H). |
| M001288 | 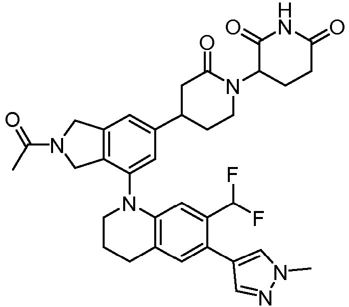 | ¹H NMR (400 MHz, DMSO-*d₆*) δ 10.82 (s, 1H), 7.75 (s, 1H), 7.49 (s, 1H), 7.28 - 7.17 (m, 2H), 7.13 (s, 1H), 6.89 - 6.61 (m, 1H), 6.41 (d, *J=* 26.0 Hz, 1H), 5.07 - 4.53 (m, 4H), 4.29 (s, 1H), 3.86 (s, 3H), 3.56 (s, 2H), 3.39 (d, *J* = 9.2 Hz, 2H), 3.22 (s, 2H), 2.87 (s, 2H), 2.81 - 2.58 (m, 2H), 2.33 (s, 1H), 2.12 - 1.74 (m, 9H). |
| M001289 | 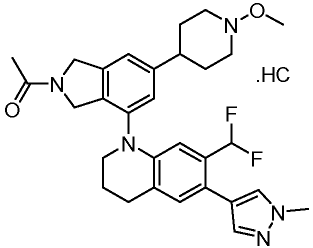 | ¹H NMR (400 MHz, DMSO-*d₆*) δ 7.75 (s, 1H), 7.50 (s, 1H), 7.23 - 7.03 (m, 3H), 6.75 (t, *J* = 55.2 Hz, 1H), 6.46 - 6.32 (m, 1H), 4.90 - 4.84 (m, 1H), 4.70 - 4.58 (m, 2H), 3.99 - 3.93 (m, 1H), 3.86 (s, 5H), 3.77 - 3.48 (m, 7H), 3.05 - 2.70 (m, 5H), 2.08 - 1.82 (m, 9H). |
| M001296 | 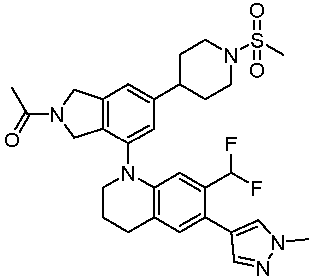 | ¹H NMR (400 MHz, DMSO) δ 7.74 (s, 1H), 7.49 (s, 1H), 7.25 - 7.10 (m, 3H), 6.88 (s, 0.25H), 6.74 (s, 0.52H), 6.60 (s, 0.25H), 6.48 - 6.32 (m, 1H), 4.86 (s, 1H), 4.72 - 4.51 (m, 2H), 4.28 (s, 1H), 3.86 (s, 3H), 3.73 - 3.62 (m, 2H), 3.61 - 3.50 (m, 2H), 2.93 - 2.62 (m, 8H), 2.06 - 1.96 (m, 5H), 1.94 - 1.86 (m, 2H), 1.80 - 1.61 (m, 3H). |
| M001297 | 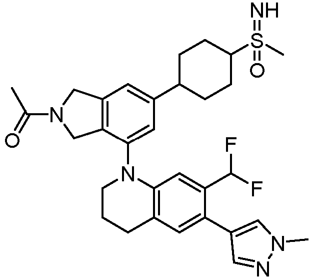 | ¹H NMR (400 MHz, DMSO) δ 7.74 (s, 1H), 7.49 (s, 1H), 7.13 (d, *J* = 6.0 Hz, 3H), 6.88 (s, 0.25 H), 6.74 (s, 0.50 H), 6.61 (s, 0.25 H), 6.46 - 6.34 (m, 1H), 4.95 - 4.55 (m, 3H), 4.29 (s, 1H), 3.86 (s, 3H), 3.54 (s, 3H), 2.98 (s, 1H), 2.88 (s, 2H), 2.83 (d, *J* = 4.0 Hz, 3H), 2.58 (s, 1H), 2.21 (s, 2H), 2.05 - 1.90 (m, 7H), 1.65 - 1.45 (m, 4H). |
| M001298 | 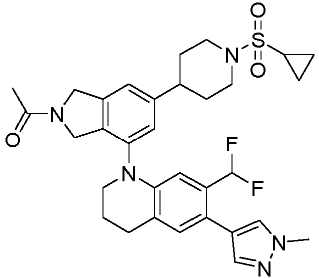 | ¹H NMR (400 MHz, DMSO) δ 8.39 (s, 0.14H), 7.75 (s, 1H), 7.49 (s, 1H), 7.25 - 7.09 (m, 3H), 6.89 (s, 0.26H), 6.75 (s, 0.21H), 6.61 (s, 0.27H), 6.48 - 6.33 (m, 1H), 4.86 (s, 1H), 4.75 - 4.52 (m, 2H), 4.29 (s, 1H), 3.86 (s, 3H), 3.78 - 3.66 (m, 2H), 3.63 - 3.50 (m, 2H), 3.00 - 2.83 (m, 4H), 2.80 - 2.70 (m, 1H), 2.65 - 2.57 (m, 1H), 2.08 - 1.96 (m, 5H), 1.89 (t, J = 10.8 Hz, 2H), 1.79 - 1.60 (m, 1H), 1.09 - 0.88 (m, 41H). |
| M001299 | 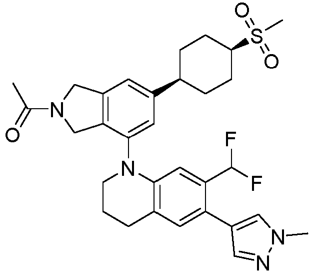 | H NMR (400 MHz, DMSO-*d₆*) δ 7.75 (s, 1H), 7.49 (s, 1H), 7.28 - 6.97 (m, 3H), 6.87 (s, 0.25H), 6.73 (s, 0.5H), 6.60 (s, 0.25H), 6.40 (d, *J* = 24.0 Hz, 1H), 4.91 - 4.16 (m, 4H), 3.86 (s, 3H), 3.55 (d, *J* = 4.8 Hz, 2H), 3.02 - 2.74 (m, 6H), 2.21 - 2.13 (m, 2H), 2.08 - 1.19 (m, 12H). |
| M001300 | 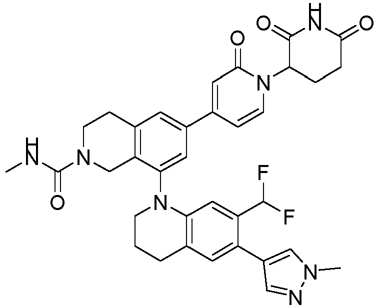 | ¹H NMR (400 MHz, DMSO-*d₆*) δ 11.03 (s, 1H), 7.73 - 7.70 (m, 2H), 7.59 (s, 1H), 7.55 (s, 1H), 7.48 (s, 1H), 7.12 (s, 1H), 6.76 (t, *J* = 55.1 Hz, 1H), 6.73 (s, 1H), 6.69 - 6.63 (m, 1H), 6.57 - 6.51 (m, 1H), 6.21 (s, 1H), 4.52 (d, *J* = 17.2 Hz, 2H), 4.17 *(d, J=* 17.2 Hz, 2H), 3.86 (s, 3H), 3.69 - 3.54 (m, 2H), 3.54 - 3.41 (m, 2H), 3.04 - 2.82 (m, 4H), 2.61 (s, 1H), 2.56 (d, *J* = 4.4 Hz, 3H), 2.10 - 2.00 (m, 4H). |
| M001301 | 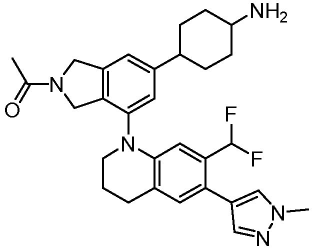 | ¹H NMR (400 MHz, DMSO-*d₆*) δ 8.40 (s, 1H), 7.74 (s, 1H), 7.49 (s, 1H), 7.16 - 7.10 (m, 2H), 6.74 (t, *J* = 55.2 Hz, 1H), 6.40 (d, *J* = 28.0 Hz, 1H), 4.85 (s, 1H), 4.64 (s, 1H), 4.60 (s, 1H), 4.28 (s, 1H), 3.86 (s, 3H), 3.57 - 3.48 (m, 4H), 2.98 - 2.94 (m, 1H), 2.87 - 2.80 (m, 2H), 2.64 - 2.53 (m, 1H), 2.47 - 2.42 (m, 1H), 2.19 - 1.96 (m, 6H), 1.87 - 1.81 (s, 2H), 1.62 - 1.47 (m, 2H), 1.43 - 1.36 (m, 2H). |
| M001302 | 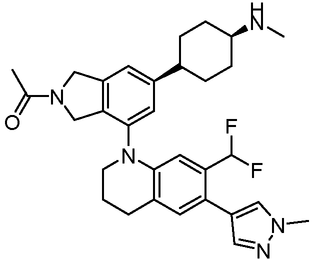 | ¹H NMR (400 MHz, DMSO-*d₆*) δ 8.09 (s, 1H), 7.74 (s, 1H), 7.49 (s, 1H), 7.21 - 7.08 (m, 2H), 6.74 (t, *J* = 55.2 Hz, 1H), 6.40 (d, *J* = 28.0 Hz, 1H), 4.86 (s, 1H), 4.66 (s, 1H), 4.38 - 4.19 (m, 2H), 3.86 (s, 3H), 3.56 - 3.51 (m, 2H), 3.09 - 3.06 (m, 1H), 2.90 - 2.86 (m, 2H), 2.42 (s, 3H), 2.08 - 1.93 (m, 5H), 1.43 - 1.29 (m, 6H), 0.97 - 0.79 (m, 4H). |
| M001303 | 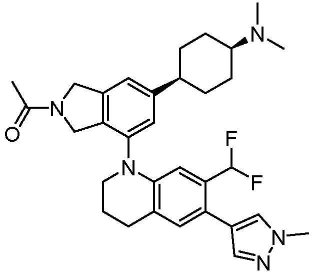 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001304 | 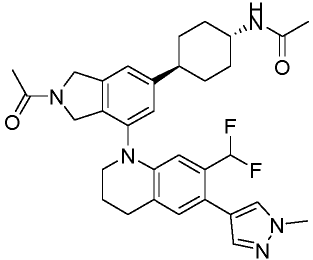 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001305 | 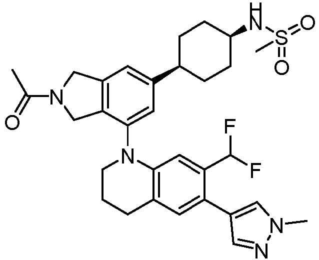 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001306 | 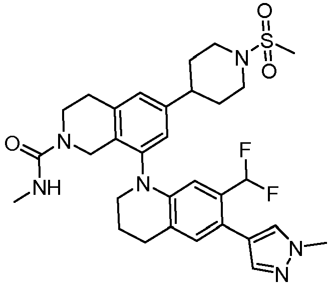 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001307 | 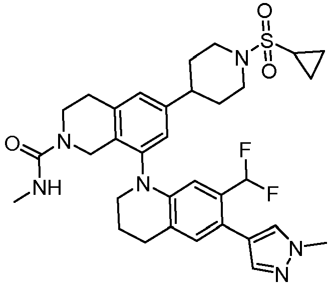 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001308 | 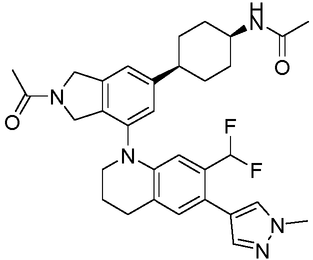 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001309 | 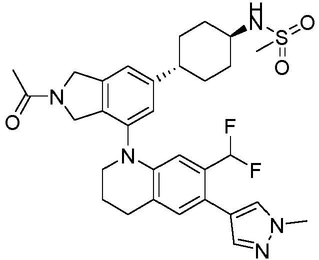 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001310 | 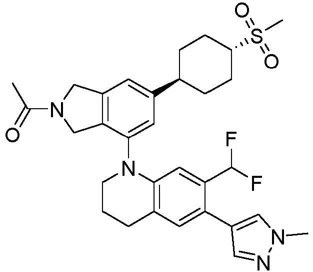 | ¹H NMR (400 MHz, DMSO-*d₆*) δ 7.75 (s, 1H), 7.49 (s, 1H), 7.17-7.06 (m, 3H), 6.87 (s, 0.25H), 6.74 (s, 0.5H), 6.60 (s, 0.25H), 6.44-6.37 (m, 1H), 4.87-4.29 (m, 4H), 3.86 (s, 3H), 3.55 (d, *J=* 4.8 Hz, 2H), 3.31-3.26 (m, 1H), 2.97 (d, *J* = 4.8 Hz, 3H), 2.88 (s, 2H), 2.82-2.72 (m, 1H), 2.15 *(d, J=* 12.8 Hz,2H), 2.09-1.97 (m, 7H), 1.91-1.83 (m, 2H), 1.73-1.67 (m, 2H). |
| M001311 | 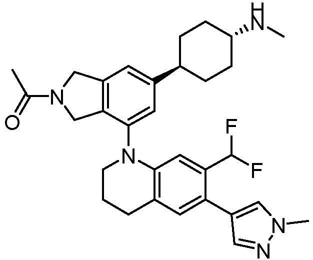 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001312 | 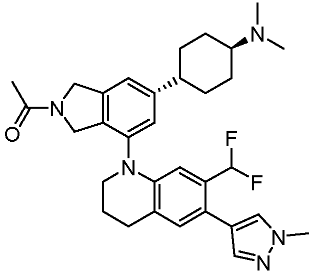 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001313 | 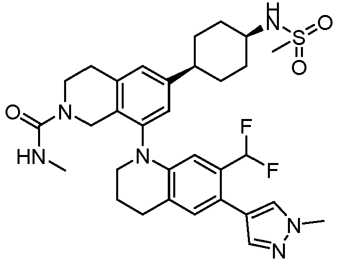 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001314 | 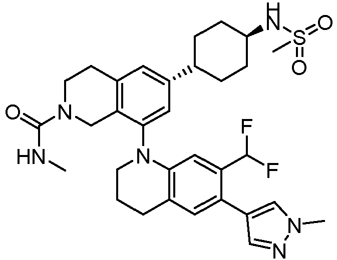 | ¹H NMR (400 MHz, DMSO-*d₆*) δ 8.09 (s, 1H), 7.74 (s, 1H), 7.49 (s, 1H), 7.16 - 7.09 (m, 2H), 6.74 (t, *J* = 55.2 Hz, 1H), 6.40 (d, *J* = 28.0 Hz, 1H), 4.85 (s, 1H), 4.65 (s, 1H), 4.35 - 4.14 (m, 2H), 3.86 (s, 3H), 3.62 - 3.46 (m, 2H), 3.30 (s, 3H), 3.09 - 3.06 (m, 1H), 2.89 - 2.84 (m, 2H), 2.12 - 1.97 (m, 5H), 1.92 - 1.86 (m, 2H), 1.55 - 1.46 (m, 2H), 1.40 - 1.17 (m, 4H), 0.97 - 0.72 (m, 2H). |
| M001315 | 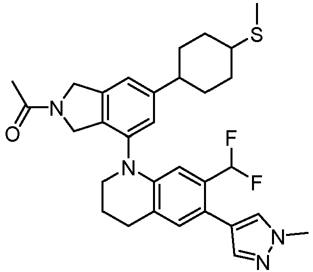 | ¹H NMR (400 MHz, DMSO-*d₆*) δ 7.74 (s, 1H), 7.49 (s, 1H), 7.15 - 7.07 (m, 3H), 6.87 (s, 0.25 H), 6.74 (s, 0.50 H), 6.60 (s, 0.25 H), 6.45 - 6.33 (m, 1H), 4.90 - 4.50 (m, 3H), 4.28 (s, 1H), 3.86 (s, 3H), 3.54 (d, *J* = 4.8 Hz, 2H), 2.87 (s, 2H), 2.64 - 2.53 (m, 2H), 2.10 - 1.96 (m, 10H), 1.87 (d, *J* = 11.6 Hz, 2H), 1.60 - 1.47 (m, 2H), 1.38 (d, *J* = 9.6 Hz, 2H). |
| M001316 | 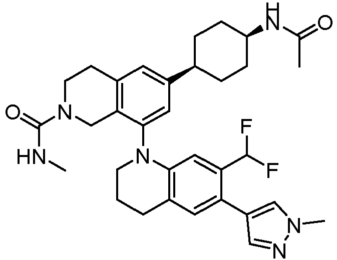 | ¹H NMR (400 MHz, DMSO-*d₆*) δ 7.81 *(d, J=* 7.6 Hz, 1H), 7.71 (s, 1H), 7.47 (s, 1H), 7.13 - 7.02 (m, 3H), 6.69 (t, *J* = 55.2 Hz, 1H), 6.49 - 6.46 (m, 1H), 6.18 (s, 1H), 4.42 (d, *J* = 16.4 Hz, 1H), 4.11 (d, *J* = 16.4 Hz, 1H), 3.96 (s, 1H), 3.86 (s, 3H), 3.58 - 3.40 (m, 4H), 2.89 - 2.81 (m, 4H), 2.55 (d, *J* = 4.0 Hz, 3H), 2.49 - 2.42 (m, 1H), 2.12 - 2.02 (m, 2H), 1.84 (s, 3H), 1.77 - 1.61 (m, 4H), 1.62 - 1.41 (m, 4H). |
| M001317 | 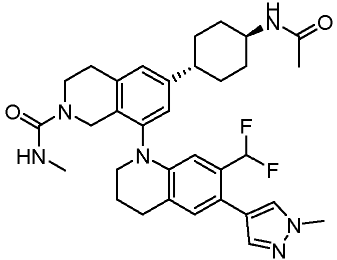 | ¹H NMR (400 MHz, DMSO-*d₆*) δ 7.74 (s, 1H), 7.71 (s, 1H), 7.46 (s, 1H), 7.15 - 6.93 (m, 3H), 6.69 (t, *J* = 55.2 Hz, 1H), 6.49 - 6.46 (m, 1H), 6.18 (s, 1H), 4.41 (d, *J* = 16.4 Hz, 1H), 4.10 *(d, J=* 16.8 Hz, 1H), 3.85 (s, 3H), 3.65 - 3.37 (m, 5H), 3.03 - 2.77 (m, 4H), 2.54 (d, *J* = 4.4 Hz, 3H), 2.49 - 2.42 (m, 1H), 2.15 - 2.00 (m, 2H), 1.89 - 1.70 (m, 7H), 1.53 - 1.44 (m, 2H), 1.34 - 1.10 (m, 2H). |
| M001318 | 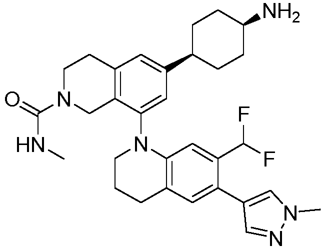 | ¹H NMR (400 MHz, DMSO-*d₆*) δ 7.71 (s, 1H), 7.46 (s, 1H), 7.09 - 7.08 (m, 3H), 6.69 (t, *J* = 55.2 Hz, 1H), 6.49 - 6.46 (m, 1H), 6.19 (s, 1H), 4.41 *(d, J=* 16.0 Hz, 1H), 4.12 (dd, *J* = 16.4, 5.2 Hz, 1H), 3.85 (s, 3H), 3.59 - 3.41 (m, 4H), 2.91 - 2.82 (m, 4H), 2.54 (d, *J =* 4.4 Hz, 3H), 2.46 - 2.44 (m, 2H), 2.15 - 1.98 (m, 2H), 1.88 - 1.61 (m, 6H), 1.66 - 1.36 (m, 4H). |
| M001319 | 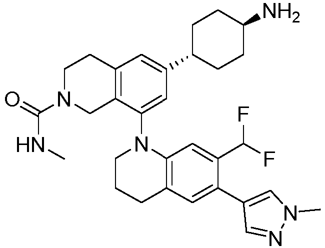 | ¹H NMR (400 MHz, DMSO-*d₆*) δ 7.71 (s, 1H), 7.46 (s, 1H), 7.15 - 6.98 (m, 3H), 6.69 (t, *J* = 55.2 Hz, 1H), 6.50 - 6.40 (m, 1H), 6.17 (s, 1H), 4.41 *(d, J=* 16.4 Hz, 1H), 4.10 (dd, *J* = 16.4, 5.2 Hz, 1H), 3.85 (s, 3H), 3.53 - 3.44 (m, 4H), 2.91 - 2.80 (m, 4H), 2.54 (d, *J =* 4.4 Hz, 3H), 2.46 - 2.42 (m, 2H), 2.07 - 2.03 (m, 2H), 1.98 - 1.70 (m, 6H), 1.512 - 1.44 (m, 2H), 1.33 - 1.26 (m, 2H). |
| M001320 | 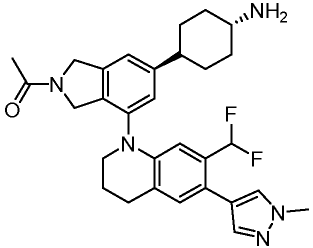 | ¹H NMR (400 MHz, DMSO-*d₆*) δ 7.98 (s, 3H), 7.75 (s, 1H), 7.49 (s, 1H), 7.19 - 7.10 (m, 3H), 6.74 (t, *J* = 55.2 Hz, 1H), 6.45 - 6.35 (m, 1H), 4.92 - 7.80 (m, 1H), 4.71 - 4.50 (m, 2H), 4.36 - 4.20 (m, 1H), 3.86 (s, 3H), 3.56 - 3.49 (m, 2H), 3.14 - 3.00 (m, 1H), 2.93 - 2.83 (m, 2H), 2.61 - 2.52 (m, 1H), 2.07 - 1.96 (m, 7H), 1.92 - 1.80 (m, 2H), 1.62 - 1.40 (m, 4H). |
| M001321 | 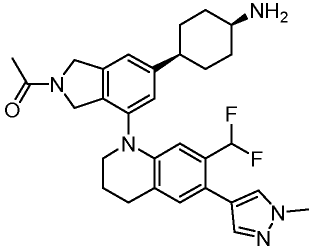 | ¹H NMR (400 MHz, DMSO-*d₆*) δ 7.99 (s, 3H), 7.75 (s, 1H), 7.49 (s, 1H), 7.28 - 7.18 (m, 2H), 7.13 (s, 1H), 6.74 (t, *J* = 55.2 Hz, 1H), 6.46 - 6.34 (m, 1H), 4.92 - 4.81 (m, 1H), 4.74 - 4.52 (m, 2H), 4.45 - 4.22 (m, 1H), 3.86 (s, 3H), 3.62 - 3.51 (m, 2H), 3.45 - 3.42 (m, 1H), 2.92 - 2.84 (m, 2H), 2.65 - 2.55 (m, 1H), 2.05 - 1.98 (m, 5H), 1.89 - 1.72 (m, 6H), 1.69 - 1.58 (m, 2H). |
| M001322 | 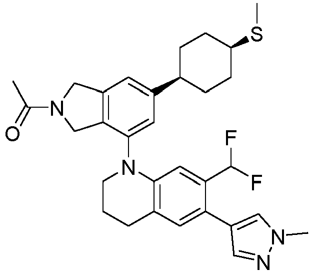 | ¹H NMR (400 MHz, CDCl3-d) δ 7.53 (d, J = 6.6 Hz, 1H), 7.40 (d, J = 7.6 Hz, 1H), 7.10 - 6.96 (m, 3H), 6.65 - 6.30 (m, 2H), 4.89 - 4.79 (m, 2H), 4.61 - 4.48 (m, 2H), 3.95 (s, 3H), 3.65 - 3.53 (m, 2H), 3.00 - 2.85 (m, 2H), 2.64 - 2.48 (m, 2H), 2.23 - 2.05 (m, 10H), 2.04 - 1.94 (m, 2H), 1.59 - 1.39 (m, 4H). |
| M001323 | 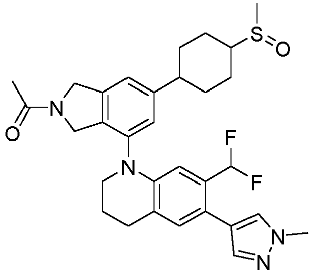 | ¹H NMR (400 MHz, DMSO-*d₆*) δ 7.75 (s, 1H), 7.49 (s, 1H), 7.12 (s, 3H), 6.88 (s, 0.25 H), 6.74 (s, 0.50 H), 6.61 (s, 0.25 H), 6.46 - 6.35 (m, 1H), 4.92 - 4.48 (m, 3H), 4.29 (s, 1H), 3.86 (s, 3H), 3.54 (s, 2H), 3.34 (s, 4H), 2.87 (s, 2H), 2.58 (d, *J* = 4.4 Hz, 1H), 2.05 - 1.92 (m, 9H), 1.64 - 1.43 (m, 4H). |
| M001324 | 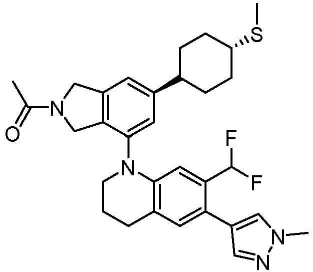 | ¹H NMR (400 MHz, CDCl3-d) δ 7.53 (d, J = 6.8 Hz, 1H), 7.40 (d, J = 7.6 Hz, 1H), 7.12 - 6.99 (m, 3H), 6.66 - 6.28 (m, 2H), 4.91 - 4.75 (m, 2H), 4.64 - 4.42 (m, 2H), 3.95 (s, 3H), 3.65 - 3.54 (m, 2H), 3.18 - 3.08 (m, 1H), 3.02 - 2.85 (m, 2H), 2.60 - 2.50 (m, 1H), 2.18 - 2.14 (m, 2H), 2.14 - 2.05 (m, 6H), 2.05 - 1.96 (m, 2H), 1.94 - 1.87 (m, 2H), 1.85 - 1.75 (m, 2H), 1.72 - 1.64 (m, 2H). |
| M001327 | 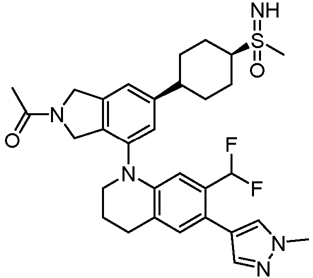 | ¹H NMR (400 MHz, DMSO) δ 7.74 (s, 1H), 7.49 (s, 1H), 7.20 - 7.19 (m, 3H), 6.87 (s, 0.25 H), 6.74 (s, 0.5 H), 6.60 (s, 0.25 H), 6.45 -6.35 (m, 1H), 4.95 - 4.56 (m, 3H), 4.28 (s, 1H), 3.86 (s, 3H), 3.54 (s, 3H), 3.11 (d, *J* = 5.2 Hz, 1H), 2.90 - 2.82 (m, 6H), 2.16 - 1.94 (m, 9H), 1.92 - 1.82 (m, 2H), 1.70 (s, 2H). |
| M001328 | 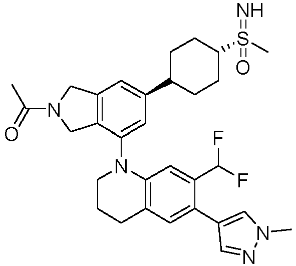 | ¹H NMR (400 MHz, DMSO) δ 7.75 (s, 1H), 7.49 (s, 1H), 7.17 - 7.09 (m, 3H), 6.88 (s, 0.25 H), 6.75 (s, 0.50 H), 6.61 (s, 0.25H), 6.45 - 6.35 (m, 1H), 4.90 - 4.55 (m, 3H), 4.28 (s, 1H), 3.86 (s, 3H), 3.55 (s, 2H), 3.02 (s, 1H), 2.87 (s, 5H), 2.56 (s, 1H), 2.22 (s, 2H), 2.05 - 1.94 (m, 7H), 1.63 - 1.51 (m, 4H). |
| M001329 | 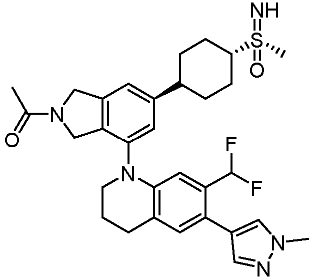 | ¹H NMR (400 MHz, DMSO) δ 7.75 (s, 1H), 7.49 (s, 1H), 7.18 - 7.08 (m, 3H), 6.88 (s, 0.25 H), 6.74 (s, 0.50 H), 6.60 (s, 0.25 H), 6.46 - 6.36 (m, 1H), 4.90 - 4.55 (m, 3H), 4.28 (s, 1H), 3.86 (s, 3H), 3.55 (s, 3H), 2.98 (s, 1H), 2.91 - 2.83 (m, 5H), 2.56 (s, 1H), 2.21 (s, 2H), 2.05 - 1.92 (m, 7H), 1.63 - 1.48 (m, 4H). |
| M001330 | 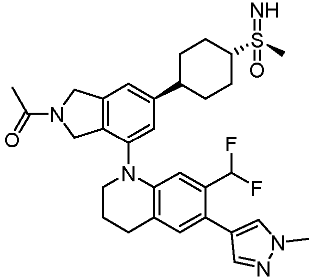 | ¹H NMR (400 MHz, DMSO) δ 7.75 (s, 1H), 7.49 (s, 1H), 7.12 (s, 3H), 6.88 (s, 0.25 H), 6.74 (s, 0.50 H), 6.60 (s, 0.25 H), 6.45 - 6.36 (m, 1H), 4.90 - 4.54 (m, 3H), 4.28 (s, 1H), 3.86 (s, 3H), 3.59 - 3.51 (m, 3H), 2.98 (s, 1H), 2.91 - 2.82 (m, 5H), 2.54 (s, 1H), 2.21 (s, 2H), 2.04 - 1.92 (m, 7H), 1.61 - 1.48 (m, 4H). |
| M001332 | 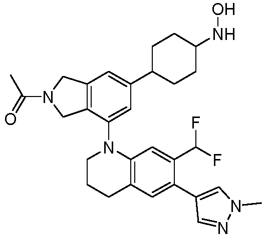 | m/z+=536.82 |
| M001340 | 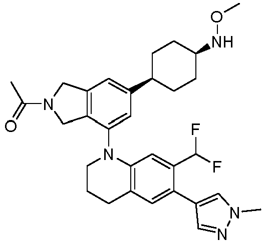 | ¹H NMR (400 MHz, DMSO-*d₆*) δ 7.74 (s, 1H), 7.49 (s, 1H), 7.18 - 7.04 (m, 3H), 6.73 (t, *J* = 55.2 Hz, 1H), 6.45 - 6.35 (m, 1H), 4.90 - 4.80 (m, 1H), 4.70 - 4.50 (m, 2H), 4.40 - 4.15 (m, 1H), 3.86 (s, 3H), 3.57 - 3.52 (m, 2H), 3.40 (s, 3H), 2.94 - 2.82 (m, 2H), 2.83 - 2.71 (m, 1H), 2.58 - 2.52 (m, 1H), 2.06 - 2.00 (m, 3H), 1.99 - 1.96 (m, 2H), 1.95 - 1.87 (m, 2H), 1.86 - 1.76 (m, 2H), 1.55 - 1.38 (m 2H), 1.24 - 1.03 (m, 2H). |
| M001341 | 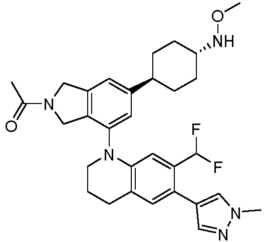 | ¹H NMR (400 MHz, DMSO-*d₆*) δ 7.75 (s, 1H), 7.49 (s, 1H), 7.19 - 7.08 (m, 3H), 6.74 (t, *J* = 55.2 Hz, 1H), 6.47 - 6.36 (m, 1H), 4.92 - 4.80 (m, 1H), 4.71 - 4.50 (m, 2H), 4.40 - 4.20 (m, 1H), 3.87 (s, 3H), 3.59 - 3.52 (m, 2H), 3.44 (s, 3H), 3.12 - 3.04 (m, 1H), 2.93 - 2.85 (m, 2H), 2.64 - 2.53 (m, 1H), 2.09 - 1.95 (m, 5H), 1.90 - 1.70 (m, 4H), 1.60 - 1.44 (m, 4H). |

### Biological Activity Example 1

22RV1 or vCap cells were cultured in RPMI-1640 medium (10% fetal bovine serum and 100 units (per mL) of penicillin/streptomycin dual antibody were added to RPMI-1640) in a 37 °C, 5% CO₂ cell incubator. Cells at the logarithmic growth phase were inoculated onto a 96-well cell culture plate at 5 × 10³ cells/100 µL/well and cultured overnight. Solutions of different concentrations of an active compound in DMSO were added, with the final concentration being 0.5% DMSO. The positive control was 1% TweeN₂0, and the negative control was 0.5% DMSO. After 72 h of culture, an equal volume of CellTiter-Glo was added to each well, and the mixtures were mixed homogeneously by shaking for 2 min. The plate was let stand at room temperature for 10 min, and fluorescence signal values were read using a microplate reader. Data were analyzed using Prism 7.0 and graphs were generated. The values are shown as mean ± SEM (N = 2). The negative control group was a 0% inhibition group, and the positive control group was a 100% inhibition group. Inhibition rates were calculated as follows: %inhibition = (Icompound - IZPE) / (IHPE - IZPE) × 100, where Icompound represents the signal value of the compound group, IHPE represents the signal value of the 100% inhibition group, and IZPE represents the signal value of the negative control group. The X-axis represents compound concentration (µM), and the Y-axis represents % inhibition.

Note: A < 1 µM, and 1 µM < B

| Compound | 22Rv1 Cell IC50 (µM) | vCap Cell IC50 (µM) |
|---|---|---|
| GNE-781 | B | B |
| M001001 | B | B |
| M001002 | B | B |
| M001003 | B | B |
| M001004 | B | B |
| M001014 | B | B |
| M001015 | B | B |
| M001016 | B | B |
| M001017 | B | B |
| M001018 | B | B |
| M001019 | B | B |
| M001021 | B | B |
| M001024 | B | B |
| M001027 | B | B |
| M001028 | B | B |
| M001030 | B | B |
| M001032 | B | B |
| M001033 | B | B |
| M001035 | B | B |
| M001040 | B | B |
| M001041 | B | B |
| M001042 | B | B |
| M001043 | B | B |
| M001044 | B | B |
| M001045 | B | B |
| M001046 | B | B |
| M001047 | B | B |
| M001048 | B | B |
| M001049 | B | B |
| M001050 | B | B |
| M001051 | B | B |
| M001052 | B | B |
| M001053 | B | A |
| M001054 | B | B |
| M001055 | B | B |
| M001056 | B | B |
| M001057 | B | B |
| M001058 | B | B |
| M001059 | B | B |
| M001060 | B | B |
| M001163 | B | B |
| M001064 | B | A |
| M001065 | A | A |
| M001066 | B | B |
| M001067 | B | A |
| M001068 | A | A |
| M001069 | A | B |
| M001070 | B | B |
| M001071 | B | B |
| M001072 | B | B |
| M001073 | B | B |
| M001074 | A | A |
| M001075 | B | B |
| M001076 | B | A |
| M001079 | B | B |
| M001080 | B | B |
| M001081 | A | A |
| M001083 | B | B |
| M001084 | B | B |
| M001085 | B | B |
| M001086 | B | B |
| M001087 | B | B |
| M001087 | B | B |
| M001088 | B | B |
| M001089 | B | B |
| M001090 | B | B |
| M001092 | B | B |
| M001093 | B | B |
| M001094 | B | B |
| M001095 | B | B |
| M001096 | B | B |
| M001097 | B | B |
| M001098 | B | B |
| M001099 | B | B |
| M001101 | A | A |
| M001102 | B | A |
| M001103 | B | B |
| M001105 | A | B |
| M001111 | B | A |
| M001116 | B | B |
| M001118 | B | B |
| M001119 | B | B |
| M001120 | B | B |
| M001121 | B | B |
| M001122 | B | B |
| M001123 | B | A |
| M001124 | A | A |
| M001125 | B | A |
| M001126 | A | A |
| M001127 | A | A |
| M001128 | B | B |
| M001129 | A | A |
| M001130 | B | A |
| M001131 | B | B |
| M001132 | B | A |
| M001137 | B | B |
| M001142 | B | B |
| M001143 | B | B |
| M001144 | B | B |
| M001149 | B | B |
| M001153 | B | B |
| M001155 | B | B |
| M001156 | B | B |
| M001161 | B | A |
| M001162 | B | B |
| M001163 | B | B |
| M001164 | B | B |
| M001170 | A | A |
| M001173 | A | A |
| M001174 | B | A |
| M001175 | A | B |
| M001176 | A | A |
| M001178 | B | B |
| M001179 | B | B |
| M001180 | B | A |
| M001181 | B | B |
| M001184 | B | A |
| M001186 | A | A |
| M001188 | A | A |
| M001189 | A | A |
| M001190 | A | A |
| M001191 | A | A |
| M001192 | B | B |
| M001193 | B | B |
| M001194 | B | A |
| M001195 | B | A |
| M001196 | B | B |
| M001197 | B | B |
| M001198 | B | B |
| M001199 | B | B |
| M001200 | B | B |
| M001201 | B | B |
| M001202 | B | B |
| M001203 | B | B |
| M001204 | B | B |
| M001207 | B | B |
| M001208 | B | B |
| M001209 | B | B |
| M001210 | B | B |
| M001211 | B | B |
| M001212 | B | B |
| M001213 | B | B |
| M001214 | B | B |
| M001215 | B | B |
| M001217 | B | B |
| M001218 | B | B |
| M001219 | A | A |
| M001221 | A | A |
| M001222 | A | A |
| M001223 | B | |
| M001224 | A | A |
| M001225 | B | A |
| M001226 | B | |
| M001227 | B | |
| M001228 | B | B |
| M001229 | B | B |
| M001230 | A | A |
| M001231 | A | A |
| M001232 | B | |
| M001233 | B | |
| M001234 | A | A |
| M001235 | A | A |
| M001236 | B | B |
| M001237 | | |
| M001238 | B | |
| M001239 | A | A |
| M001240 | B | |
| M001241 | B | |
| M001242 | B | B |
| M001243 | B | B |
| M001244 | B | B |
| M001245 | B | B |
| M001246 | B | B |
| M001247 | B | |
| M001248 | B | |
| M001249 | A | A |
| M001250 | B | |
| M001251 | B | |
| M001252 | A | A |
| M001253 | A | A |
| M001254 | A | |
| M001255 | A | |
| M001256 | B | |
| M001257 | B | |
| M001258 | B | |
| M001260 | B | B |
| M001261 | B | B |
| M001262 | B | |
| M001263 | B | B |
| M001264 | B | B |
| M001268 | B | A |
| M001269 | B | B |
| M001270 | B | |
| M001271 | B | |
| M001272 | B | B |
| M001274 | B | B |
| M001275 | B | B |
| M001276 | B | |
| M001277 | B | |
| M001280 | A | B |
| M001281 | A | A |
| M001287 | B | |
| M001288 | B | B |
| M001289 | A | B |
| M001296 | A | A |
| M001297 | A | A |
| M001298 | A | A |
| M001299 | A | A |
| M001300 | B | |
| M001301 | B | B |
| M001302 | B | |
| M001303 | B | |
| M001304 | A | A |
| M001305 | A | |
| M001306 | B | |
| M001307 | A | A |
| M001308 | A | |
| M001309 | A | |
| M001310 | A | A |
| M001311 | B | |
| M001312 | B | |
| M001313 | B | A |
| M001314 | B | |
| M001315 | B | A |
| M001316 | B | |
| M001317 | B | |
| M001318 | B | |
| M001319 | B | |
| M001320 | B | B |
| M001321 | B | B |
| M001322 | B | B |
| M001323 | B | A |
| M001324 | B | A |
| M001327 | B | B |
| M001328 | B | B |
| M001329 | B | B |
| M001330 | B | B |
| M001340 | B | B |
| M001341 | B | B |

The biochemical activity of the EP300 modulators was determined using the Ep300 AlphaLISA^{®} assay kit. The assay is based on the biotinylation of residues in the peptide substrate by enzymatic transfer of acetyl groups from acetyl-CoA to particular lysine. The compounds were incubated with EP300 and then with an antibody combination of an antibody that specifically binds to an acetylated peptide and acceptor beads that bind to an acetylated peptide, followed by streptavidin-labeled donor beads. The alpha counts were read, the data were analyzed using Prism 7.0, and the IC50 values were calculated. The values are shown as mean ± SEM (N = 2).

The biochemical activity of the CBP inhibitors was determined using the CBP AlphaLISA^{®} assay kit. The assay is based on the binding of the CBP bromodomain to the acetylated histone substrate. The compounds were incubated with CBP and the biotinylated substrate for 30 min. Then acceptor beads were added, followed by 15 min of incubation, and donor beads were added, followed by 15 min of incubation. Then the alpha counts were read, the data were analyzed using Prism 7.0, and the IC50 values were calculated. The values are shown as mean ± SEM (N = 2).

A < 10 nM, B > 10 nM

| Compound | EP300 ALPHA IC50 nM | CBP ALPHA IC50 nM |
|---|---|---|
| GNE-781 | A | A |
| M001065 | | A |
| M001076 | | A |
| M001067 | A | A |
| M001126 | | B |
| M001173 | | A |
| M001174 | | A |
| M001190 | | A |
| M001191 | | A |
| M001202 | A | A |
| M001242 | B | B |
| M001243 | B | B |
| M002244 | B | B |
| M001245 | A | A |

The embodiments of the technical solutions of the present disclosure have been described above by way of example. It should be understood that the protection scope of the present disclosure is not limited to the embodiments described above. Any modification, equivalent replacement, improvement and the like made by those skilled in the art without departing from the spirit and principle of the present disclosure shall fall within the protection scope of the claims.

## Claims

1. A compound represented by formula I and a racemate, a stereoisomer, a tautomer, an isotopic derivative, a nitrogen oxide, a solvate, a polymorph, a metabolite, an ester, a prodrug or a pharmaceutically acceptable salt thereof: 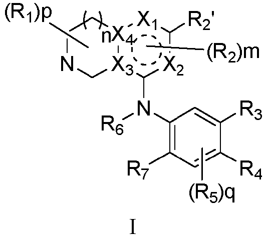 wherein:
n = 0, 1 or 2;
m = 0, 1, 2 or 3;
p = 0, 1, 2, 3, 4 or 5;
q = 0, 1 or 2;
X₁, X₂, X₃ and X₄ are each independently selected from C and N;
each R₁ is the same or different and is independently selected from oxo (=O), halogen, CN, NH₂, COOH, OH, and the following groups that are unsubstituted or optionally substituted with 1, 2 or more R₁a: C₁₋₁₂ alkyl, C₁₋₁₂ alkoxy, and -(C=O)R;
the R is selected from C₁₋₁₂ alkyl, C₁₋₁₂ alkoxy, NH₂-, C₁₋₁₂ alkyl-NH-, N,N-di C₁₋₁₂ alkylamino, and C₃₋₁₂ cycloalkyl; each R₁a is the same or different and is independently selected from oxo (=O), halogen, CN, NH₂, COOH, OH, C₁₋₁₂ alkyl, and C₁₋₁₂ alkoxy;
each R₂ is the same or different and is independently selected from oxo (=O), halogen, CN, NH₂, COOH, OH, C₁₋₁₂ alkyl, and C₁₋₁₂ alkoxy;
R₂' is selected from H, halogen, CN, NH₂, COOH, OH, and the following groups that are unsubstituted or optionally substituted with 1, 2 or more R₂a: C₆₋₁₄ aryl, 5- to 14-membered heteroaryl, 3- to 14-membered heterocyclyl, C₃₋₁₂ cycloalkyl, and C₁₋₁₂ alkyl;
each R₂a is the same or different and is independently selected from H, oxo (=O), halogen, COOH, OH, and the following groups that are unsubstituted or optionally substituted with 1, 2 or more R₂b: NH₂, C₁₋₁₂ alkyl, C₁₋₁₂ alkoxy, C₁₋₁₂ alkylthio, C₁₋₁₂ alkyl-NH-, N,N-di C₁₋₁₂ alkylamino, C₁₋₁₂ alkyl C(=O)-, C₁₋₁₂ alkoxy C(=O)-, C₁₋₁₂ alkyl-NHC(=O)-, N,N-di C₁₋₁₂ alkylaminocarbonyl, C₆₋₁₄ aryl, 5- to 14-membered heteroaryl, 3- to 14-membered heterocyclyl, C₃₋₁₂ cycloalkyl, C₆₋₁₄ aryl C(=O)-, 5- to 14-membered heteroaryl C(=O)-, 3- to 14-membered heterocyclyl C(=O)-, C₃₋₁₂ cycloalkyl C(=O)-, C₁₋₁₂ alkyl S(=O)₂-, C₁₋₁₂ alkyl S(=O)-, C₁₋₁₂ alkyl S(=O)(=NH)-, C₃₋₁₂ cycloalkyl S(=O)₂-, C₁₋₁₂ alkyl-C(=O)-NH-, C₁₋₁₂ alkyl-S(=O)₂-NH-;
each R₂b is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino;
R₃ is selected from H, halogen, CN, NH₂, COOH, OH, and the following groups that are unsubstituted or optionally substituted with 1, 2 or more R₃a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), N,N-di C₁₋₁₂ alkylaminocarbonyl, C₆₋₁₄ aryl, 5- to 14-membered heteroaryl, 3- to 14-membered heterocyclyl, and C₃₋₁₂ cycloalkyl;
each R₃a is the same or different and is independently selected from oxo (=O), halogen, CN, NH₂, COOH, OH, and the following groups that are unsubstituted or optionally substituted with 1, 2 or more R₃b: C₁₋₁₂ alkyl, C₁₋₁₂ alkoxy, C₁₋₁₂ alkyl-NH-, N,N-di C₁₋₁₂alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
each R₃b is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino;
R₄ is selected from H, halogen, CN, NH₂, COOH, OH, and the following groups that are unsubstituted or optionally substituted with 1, 2 or more R₄a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), N,N-di C₁₋₁₂ alkylaminocarbonyl, C₆₋₁₄ aryl, 5- to 14-membered heteroaryl, 3- to 14-membered heterocyclyl, and C₃₋₁₂ cycloalkyl;
each R₄a is the same or different and is independently selected from oxo (=O), halogen, CN, NH₂, COOH, OH, and the following groups unsubstituted or optionally substituted with 1, 2 or more R₄b: C₁₋₁₂ alkyl, C₁₋₁₂ alkoxy, C₁₋₁₂ alkyl-NH-, N,N-di C₁₋₁₂alkylamino, C₁₋₁₂ alkyl C(=O)-, C₁₋₁₂ alkoxy C(=O)-, C₁₋₁₂ alkyl-NHC(=O)-, and N,N-di C1-12 alkylaminocarbonyl;
each R₄b is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino;
each R₅ is the same or different and is independently selected from oxo (=O), halogen, CN, NH₂, COOH, OH, and the following groups that are unsubstituted or optionally substituted with 1, 2 or more R₅a: C₁₋₁₂ alkyl, and C₁₋₁₂ alkoxy;
each R₅a is the same or different and is independently selected from oxo (=O), halogen, CN, NH₂, COOH, OH, C₁₋₁₂ alkyl, and C₁₋₁₂ alkoxy;
R₆ is selected from H, halogen, CN, NH₂, COOH, OH, and the following groups that are unsubstituted or optionally substituted with 1, 2 or more R₆a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), and N,N-di C₁₋₁₂ alkylaminocarbonyl;
each R₆a is the same or different and is independently selected from oxo (=O), halogen, CN, NH₂, COOH, OH, C₁₋₁₂ alkyl, C₁₋₁₂ alkoxy, C₁₋₁₂ alkyl-NH-, N,N-di C₁₋₁₂ alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
R₇ is selected from H, halogen, CN, NH₂, COOH, OH, and the following groups that are unsubstituted or optionally substituted with 1, 2 or more R₇a: C₁₋₁₂ alkyl, C₁₋₁₂ alkoxy, C₁₋₁₂ alkyl-NH-, N,N-di C₁₋₁₂ alkylamino, C₁₋₁₂ alkyl-NHC(=O), and N,N-di C₁₋₁₂ alkylaminocarbonyl;
each R₇a is the same or different and is independently selected from oxo (=O), halogen, CN, NH₂, COOH, OH, C₁₋₁₂ alkyl, C₁₋₁₂ alkoxy, C₁₋₁₂ alkyl-NH-, N,N-di C₁₋₁₂ alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
or together R₆ and R₇ form the following group that is unsubstituted or optionally substituted with 1, 2 or more Rsa: 5- to 14-membered heteroaryl, or 3- to 14-membered heterocyclyl, wherein the 5- to 14-membered heteroaryl or the 3- to 14-membered heterocyclyl contains at least one N atom;
each Rsa is the same or different and is independently selected from oxo (=O), halogen, CN, NH₂, COOH, OH, and the following groups that are unsubstituted or optionally substituted with 1, 2 or more Rsb: C₁₋₁₂ alkyl, C₁₋₁₂ alkoxy, C₁₋₁₂ alkyl-NH-, N,N-di C₁₋₁₂alkylamino, C₁₋₁₂ alkyl C(=O)-, C₁₋₁₂ alkoxy C(=O)-, C₁₋₁₂ alkyl-NHC(=O)-, and N,N-di C₁₋₁₂ alkylaminocarbonyl;
each Rsb is the same or different and is independently selected from oxo (=O), halogen, CN, NH₂, COOH, OH, C₁₋₁₂ alkyl, C₁₋₁₂ alkoxy, C₁₋₁₂ alkyl-NH-, and N,N-di C₁₋₁₂ alkylamino.

2. The compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to claim 1, wherein in the formula I, the moiety the following structures: 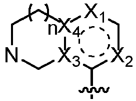 is selected from

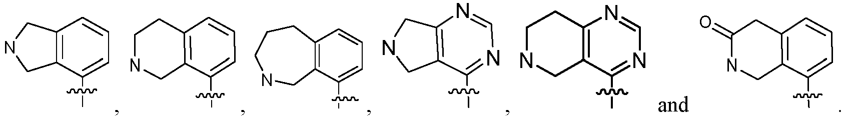

3. The compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to claim 1 or 2, wherein each R₁ is the same or different and is independently selected from oxo (=O), C₁₋₆ alkyl, C₁₋₆ alkoxy, and -(C=O)R, wherein the R is selected from C₁₋₆ alkyl, C₁₋₆ alkoxy, NH₂-, C₁₋₆ alkyl-NH-, and C₃₋₆ cycloalkyl.

4. The compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to any one of claims 1 to 3, wherein R₂' is selected from H, Cl, and C₆₋₁₀ aryl, 5-to 9-membered heteroaryl, 3- to 12-membered heterocyclyl, C₃₋₈ cycloalkyl and C₁₋₃ alkyl that are unsubstituted or optionally substituted with 1, 2 or more R₂a, wherein the heterocyclyl contains 1-4 heteroatoms selected from N, O and S;
preferably, R₂' is selected from H, Cl, and the following structures that are unsubstituted or optionally substituted with 1, 2 or more R₂a: 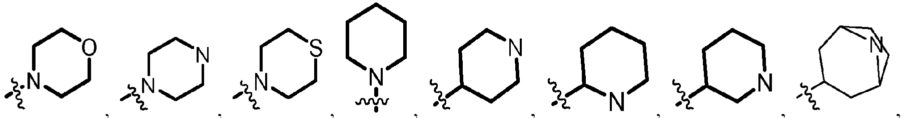 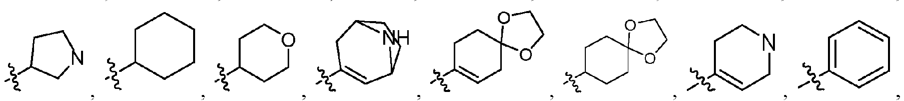 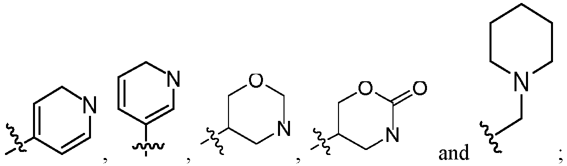
more preferably, R₂' is selected from the following structures: 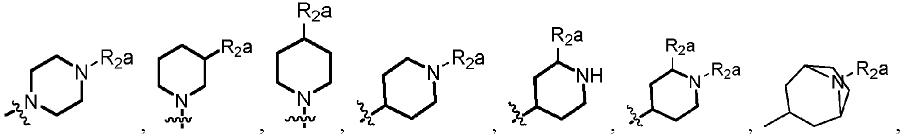 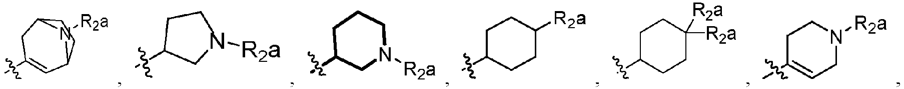 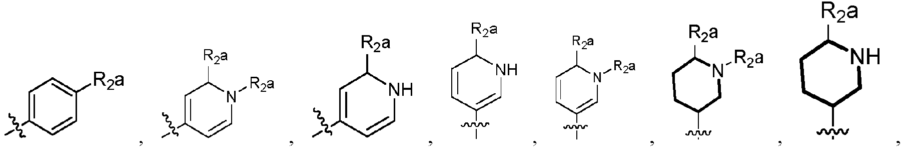 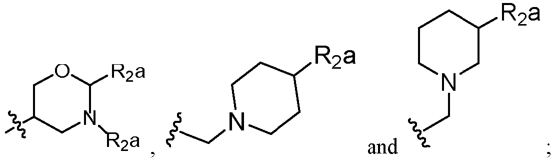 preferably, R₂a is selected from H, halogen (such as F), CN, methyl, ethyl, oxo (=O), methoxy, OH, COOH, 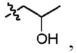 CH₃C(=O)-, -CH₂CHF₂, -CH₂CF₃, -Cbz, -Boc, amino, methylamino, dimethylamino, methylthio,

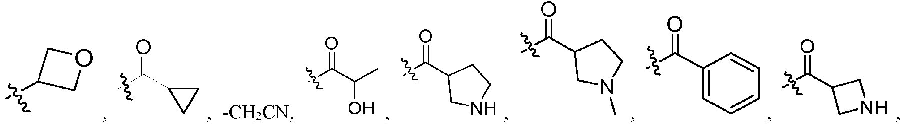

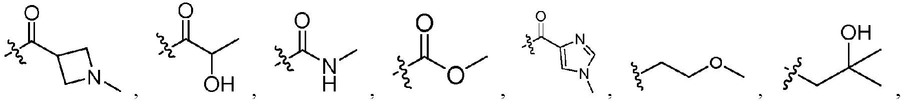

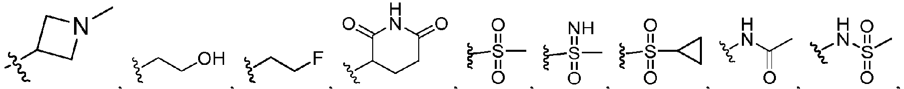

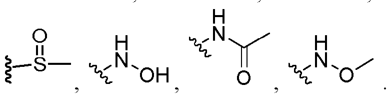

5. The compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to any one of claims 1 to 4, wherein
R₃ is selected from H, and the following groups that are unsubstituted or optionally substituted with 1, 2 or more R₃a: C₁₋₆ alkyl, C₁₋₆ alkoxy, and C₃₋₈ cycloalkyl, wherein each R₃a is the same or different and is independently selected from halogen, CN, NH₂, COOH, and OH;
R₄ is selected from 5- to 6-membered heteroaryl (e.g., pyrazolyl) that is unsubstituted or optionally substituted with 1, 2 or more R₄a, wherein each R₄a is the same or different and is independently selected from the following groups that are unsubstituted or optionally substituted with 1, 2 or more R₄b: C₁₋₆ alkyl, C₁₋₆ alkoxy, and *N*,*N*-di C₁₋₆ alkylaminocarbonyl, wherein each R₄b is the same or different and is independently selected from oxo (=O), halogen, CN, NH₂, COOH, OH, C₁₋₆ alkyl, and C₁₋₆ alkoxy;
preferably, R₄ is selected from 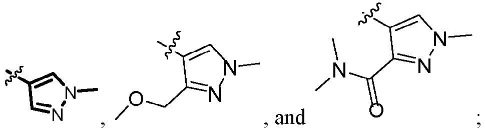
each R₅ is the same or different and is independently selected from H, halogen, C₁₋₆ alkyl, and C₁₋₆ alkoxy;
preferably, R₆ is selected from H, halogen, C₁₋₆ alkyl and C₁₋₆ alkoxy; preferably, R₆ is selected from H;
preferably, R₇ is selected from H, halogen, C₁₋₆ alkyl and C₁₋₆ alkoxy; preferably, R₇ is selected from F;
more preferably, together R₆ and R₇ form the following groups that are unsubstituted or optionally substituted with 1, 2 or more Rsa: 5- to 6-membered heteroaryl, and 5- to 6-membered heterocyclyl;
most preferably, together R₆ and R₇ form the following structure that is unsubstituted or optionally substituted with 1, 2 or more Rsa:

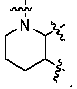

6. The compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to any one of claims 1 to 5, wherein the formula I is further selected from the following structure represented by formula I-1: 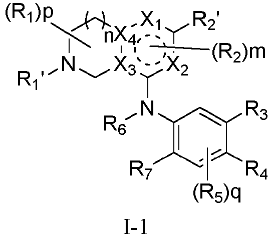
wherein X₁, X₂, X₃, X₄, R₁, R₂, R₂', R₃, R₄, R₅, R₆, R₇, R, n, m, p and q are as defined in any one of claims 1-5; R₁' has the definition of R₁;
preferably, R₁' is selected from oxo (=O), C₁₋₆ alkyl, C₁₋₆ alkoxy, and -(C=O)R, wherein the R is selected from C₁₋₆ alkyl, C₁₋₆ alkoxy, NH₂-, C₁₋₆ alkyl-NH-, and C₃₋₆ cycloalkyl;
preferably, R₁' is selected from H, CH₃, -C(=O)CH₃, -C(=O)OCH₃, -C(=O)OCH₂CH₃, -C(=O)OCH(CH₃)₃, CH₃NHC(=O)-, CH₃CH₂NHC(=O)-, NH₂C(=O)-, and cyclopropyl-C(=O)-;
preferably, R₁' is- (C=O)CH₃;
preferably, the formula I is further selected from the following structures represented by formula II, formula III and formula IV: 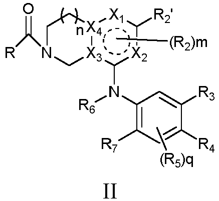 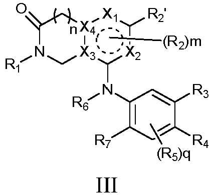 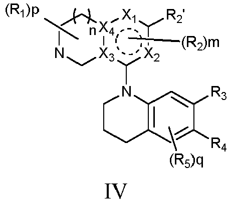
in the formula II, formula III and formula IV, X₁, X₂, X₃, X₄, R₁, R₂, R₂', R₃, R₄, R₅, R₆, R₇, R, n, m, p and q are as defined in any one of claims 1-5;
preferably, the formula I is further selected from the following structures represented by formula IIa and formula IIIa: 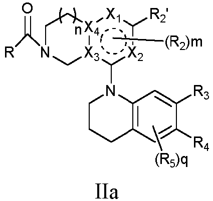 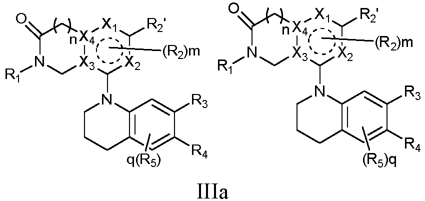
In the formula IIa and formula IIIa, X₁, X₂, X₃, X₄, R₁, R₂, R₂', R₃, R₄, R₅, R, n, m, p and q are as defined in the compound represented by formula (I).

7. The compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to any one of claims 1 to 6, wherein exemplary specific compounds of the compound represented by formula (I) are as follows:
| Compound ID | Structural formula | Compound ID | Structural formula |
|---|---|---|---|
| M001001 | 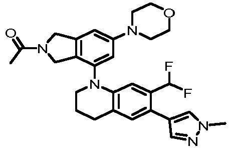 | M001191 | 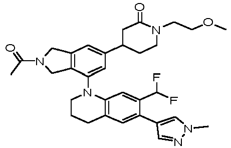 |
| M001002 | 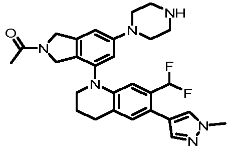 | M001192 | 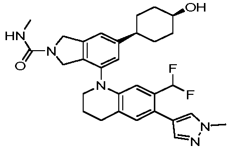 |
| M001003 | 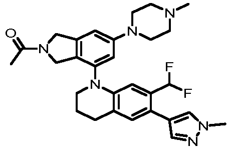 | M001193 | 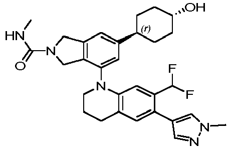 |
| M001004 | 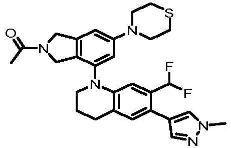 | M001194 | 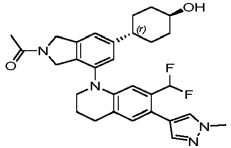 |
| M001006 | 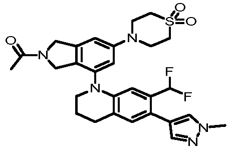 | M001195 | 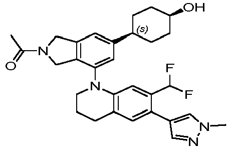 |
| M001014 | 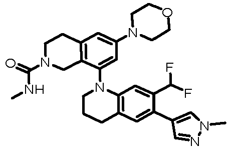 | M001196 | 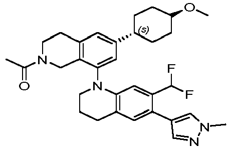 |
| M001015 | 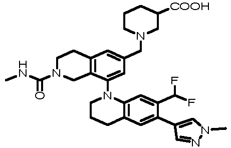 | M001197 | 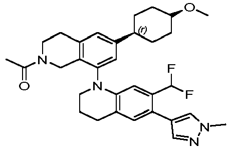 |
| M001016 | 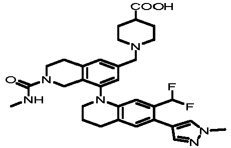 | M001198 | 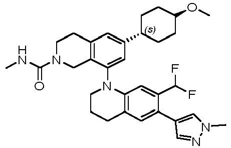 |
| M001017 | 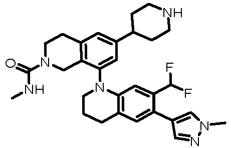 | M001199 | 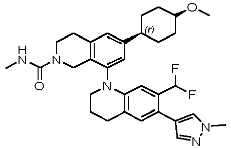 |
| M001018 | 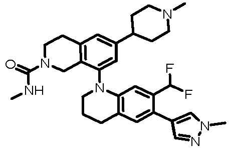 | M001200 | 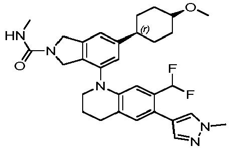 |
| M001019 | 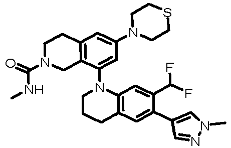 | M001201 | 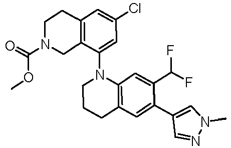 |
| M001021 | 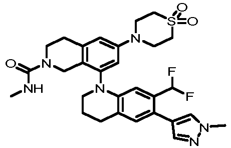 | M001202 | 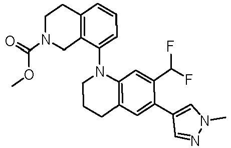 |
| M001024 | 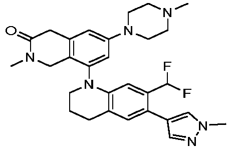 | M001203 | 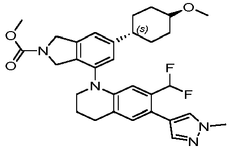 |
| M001027 | 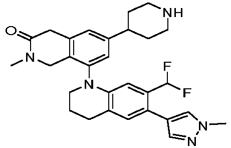 | M001204 | 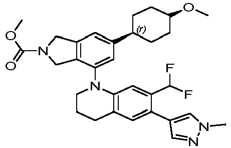 |
| M001028 | 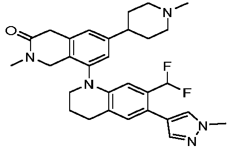 | M001206 | 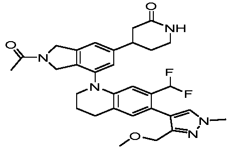 |
| M001030 | 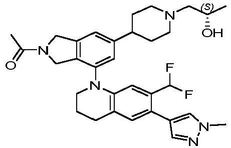 | M001207 | 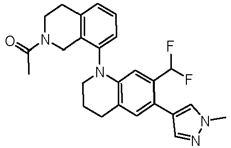 |
| M001032 | 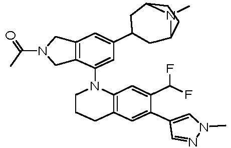 | M001208 | 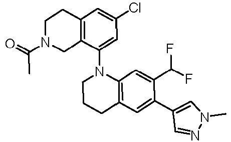 |
| M001033 | 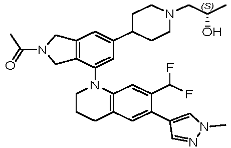 | M001209 | 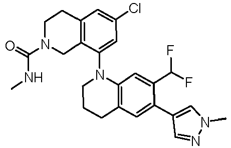 |
| M001035 | 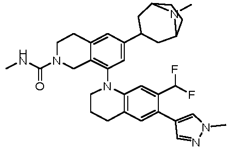 | M001210 | 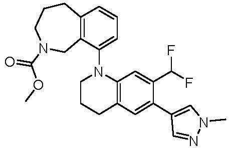 |
| M001040 | 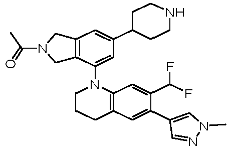 | M001211 | 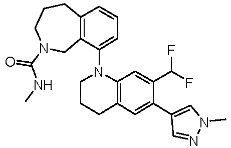 |
| M001041 | 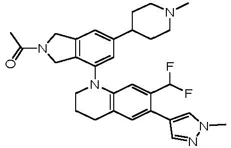 | M001212 | 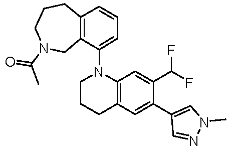 |
| M001042 | 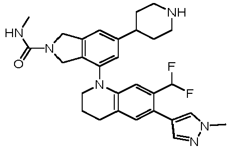 | M001213 | 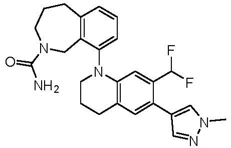 |
| M001043 | 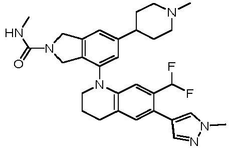 | M001214 | 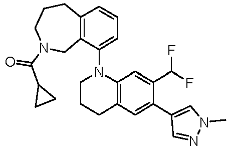 |
| M001044 | 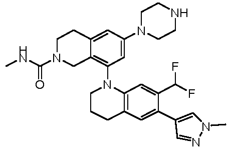 | M001215 | 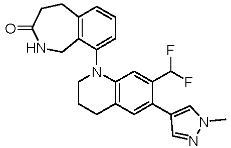 |
| M001045 | 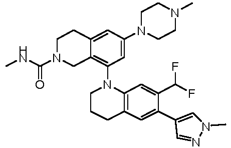 | M001217 | 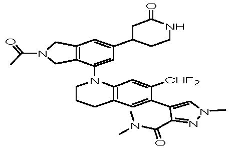 |
| M001046 | 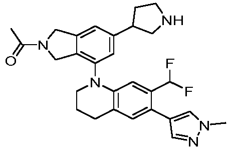 | M001218 | 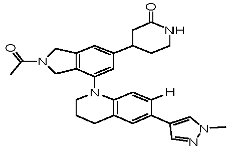 |
| M001047 | 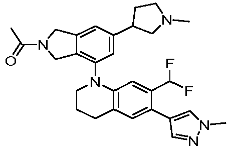 | M001219 | 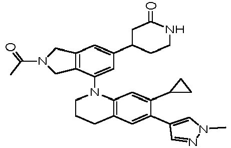 |
| M001048 | 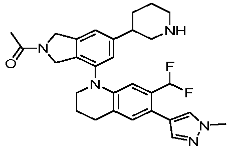 | M001221 | 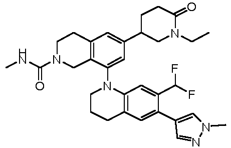 |
| M001049 | 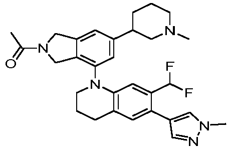 | M001222 | 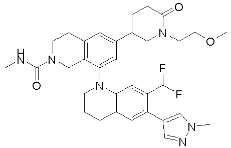 |
| M001050 | 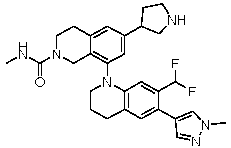 | M001223 | 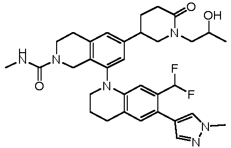 |
| M001051 | 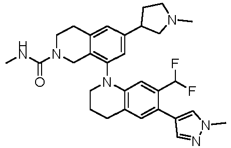 | M001224 | 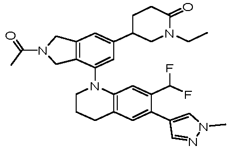 |
| M001052 | 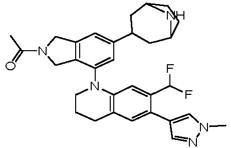 | M001225 | 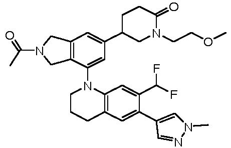 |
| M001053 | 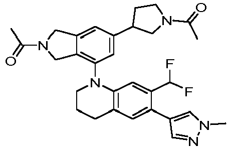 | M001226 | 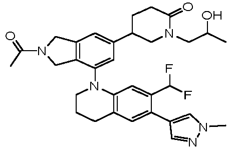 |
| M001054 | 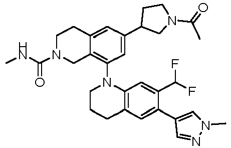 | M001227 | 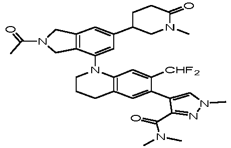 |
| M001055 | 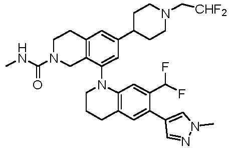 | M001228 | 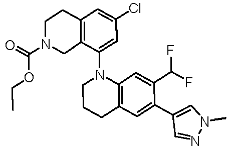 |
| M001056 | 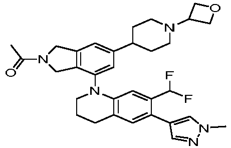 | M001229 | 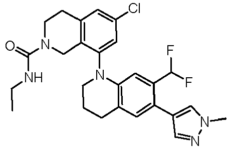 |
| M001057 | 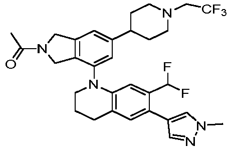 | M001230 | 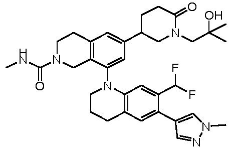 |
| M001058 | 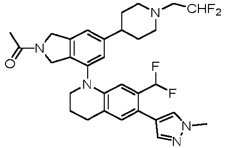 | M001231 | 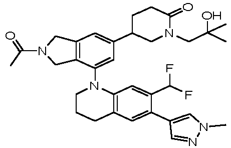 |
| M001059 | 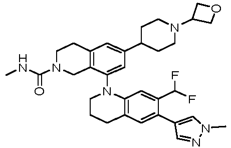 | M001232 | 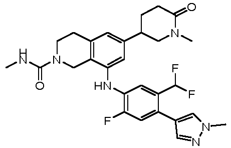 |
| M001060 | 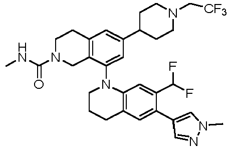 | M001233 | 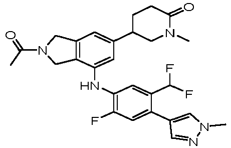 |
| M001061 | 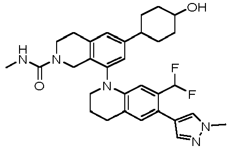 | M001234 | 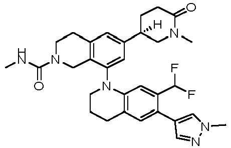 |
| M001062 | 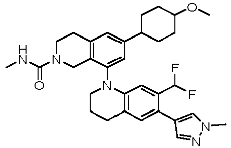 | M001235 | 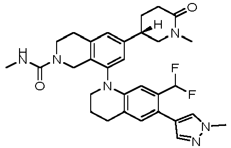 |
| M001063 | 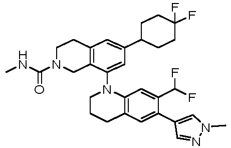 | M001236 | 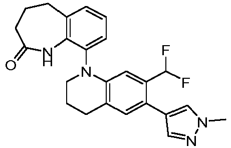 |
| M001064 | 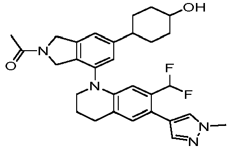 | M001237 | 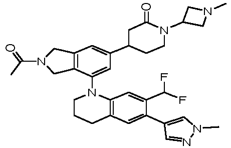 |
| M001065 | 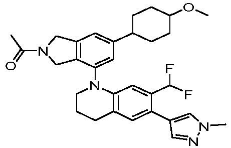 | M001238 | 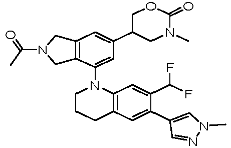 |
| M001066 | 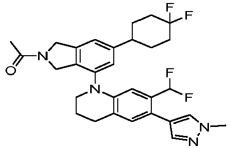 | M001239 | 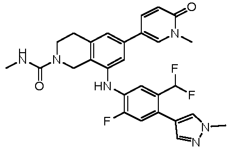 |
| M001067 | 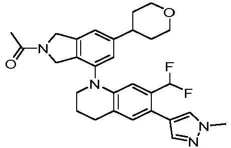 | M001240 | 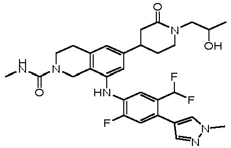 |
| M001068 | 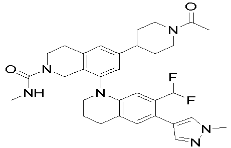 | M001241 | 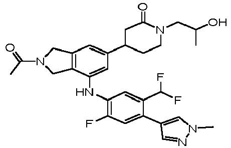 |
| M001069 | 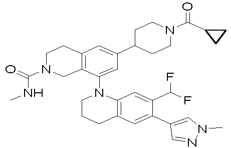 | M001242 | 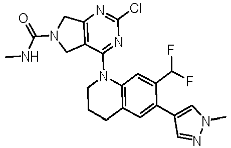 |
| M001070 | 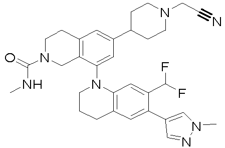 | M001243 | 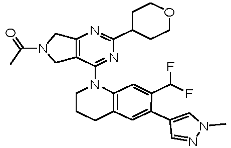 |
| M001071 | 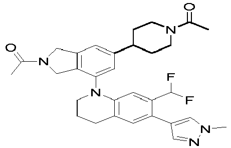 | M001244 | 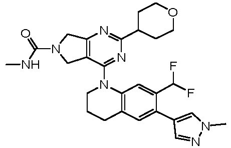 |
| M001072 | 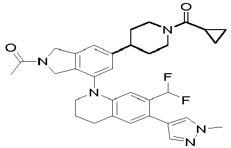 | M001245 | 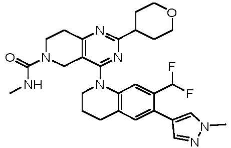 |
| M001073 | 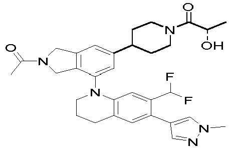 | M001246 | 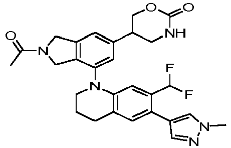 |
| M001074 | 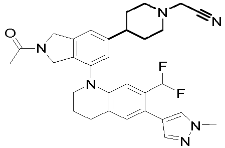 | M001247 | 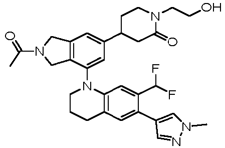 |
| M001075 | 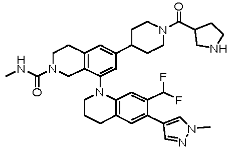 | M001248 | 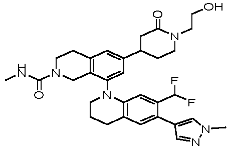 |
| M001076 | 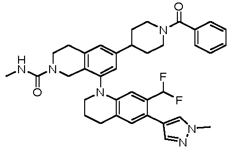 | M001249 | 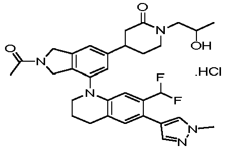 |
| M001079 | 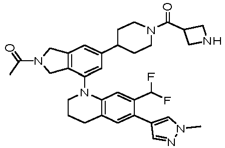 | M001250 | 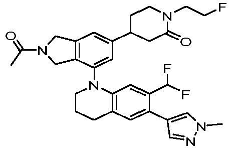 |
| M001080 | 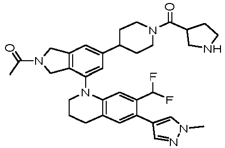 | M001251 | 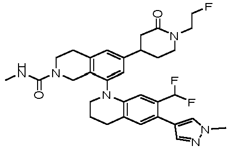 |
| M001081 | 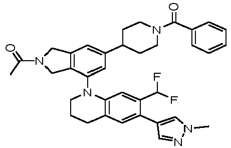 | M001252 | 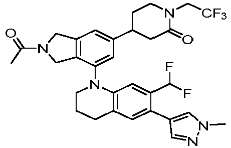 |
| M001082 | 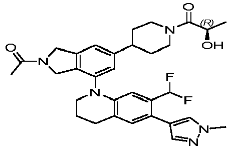 | M001253 | 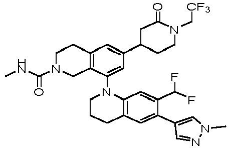 |
| M001083 | 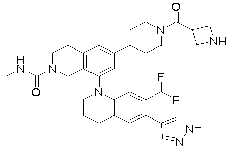 | M001254 | 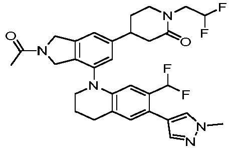 |
| M001084 | 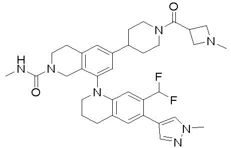 | M001255 | 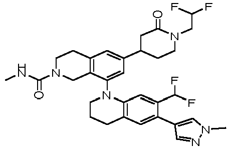 |
| M001085 | 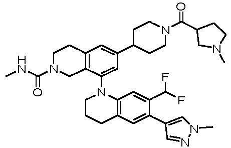 | M001256 | 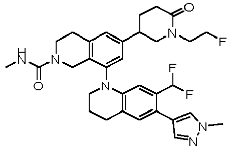 |
| M001086 | 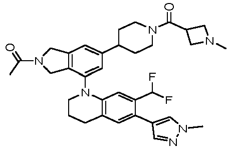 | M001257 | 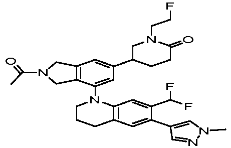 |
| M001087 | 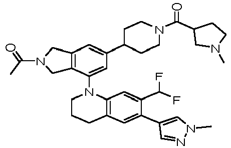 | M001258 | 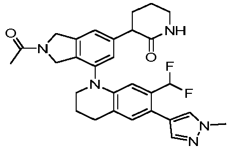 |
| M001088 | 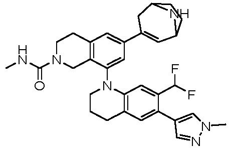 | M001260 | 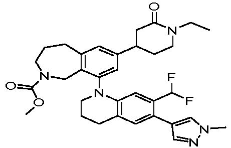 |
| M001089 | 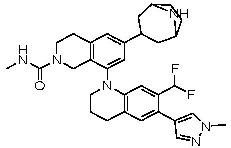 | M001261 | 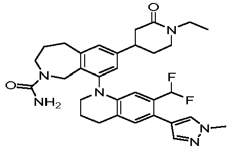 |
| M001090 | 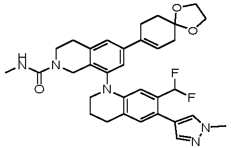 | M001262 | 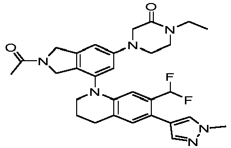 |
| M001092 | 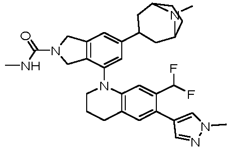 | M001263 | 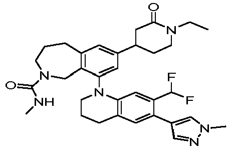 |
| M001093 | 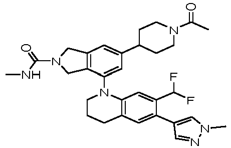 | M001264 | 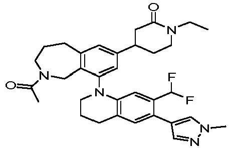 |
| M001094 | 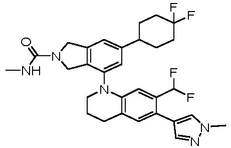 | M001268 | 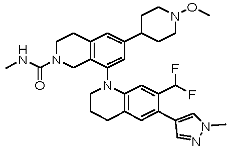 |
| M001095 | 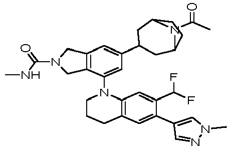 | M001269 | 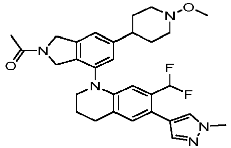 |
| M001096 | 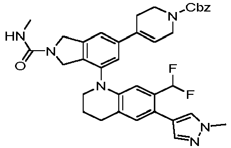 | M001270 | 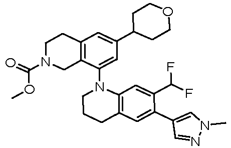 |
| M001097 | 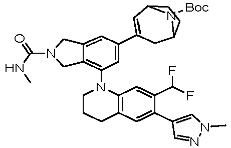 | M001271 | 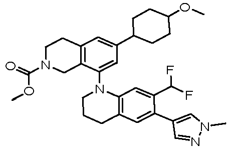 |
| M001098 | 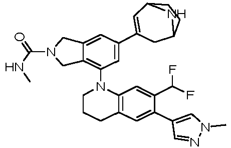 | M001272 | 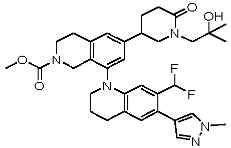 |
| M001099 | 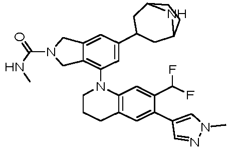 | M001274 | 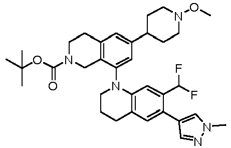 |
| M001101 | 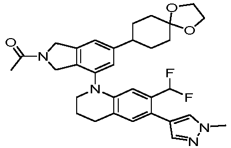 | M001275 | 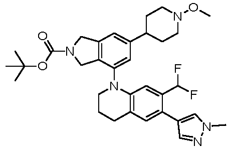 |
| M001102 | 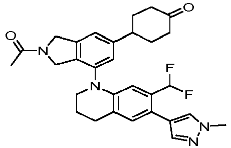 | M001276 | 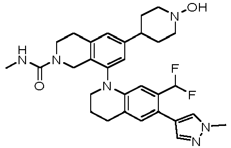 |
| M001103 | 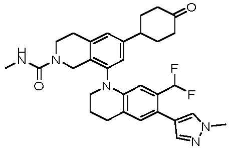 | M001277 | 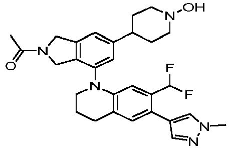 |
| M001116 | 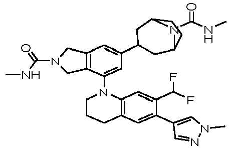 | M001280 | 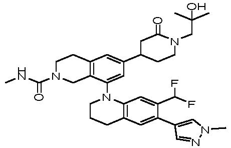 |
| M001117 | 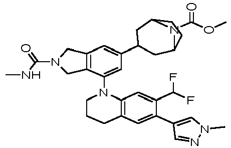 | M001281 | 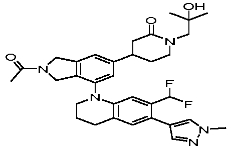 |
| M001118 | 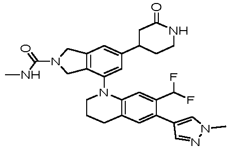 | M001287 | 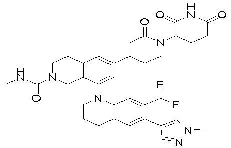 |
| M001119 | 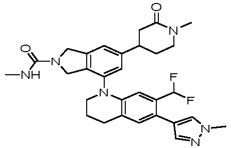 | M001288 | 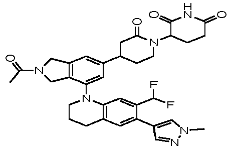 |
| M001120 | 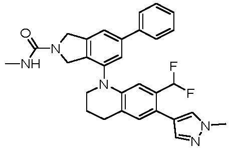 | M001289 | 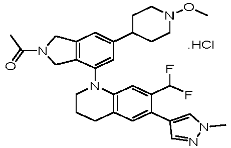 |
| M001121 | 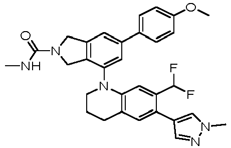 | M001296 | 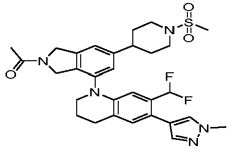 |
| M001122 | 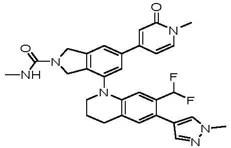 | M001297 | 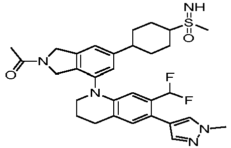 |
| M001123 | 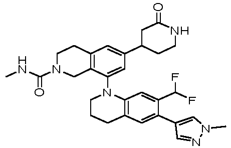 | M001298 | 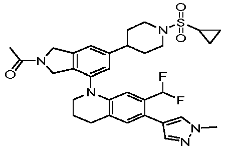 |
| M001124 | 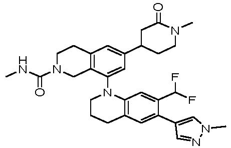 | M001299 | 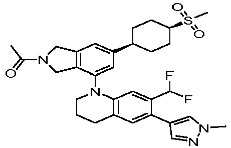 |
| M001125 | 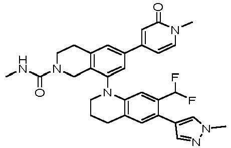 | M001300 | 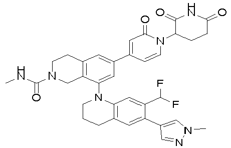 |
| M001126 | 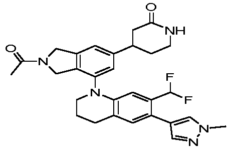 | M001301 | 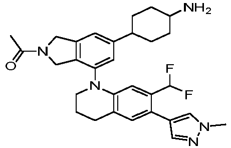 |
| M001127 | 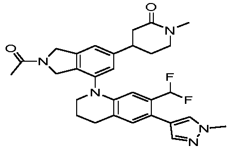 | M001302 | 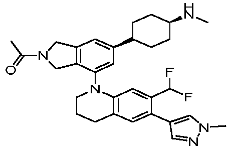 |
| M001128 | 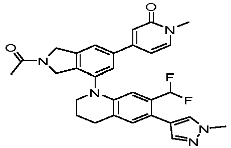 | M001303 | 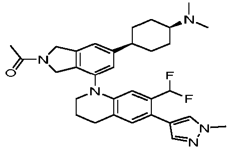 |
| M001129 | 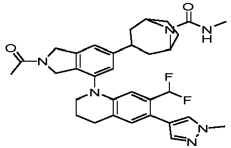 | M001304 | 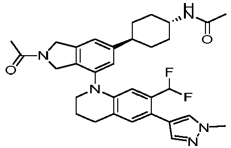 |
| M001132 | 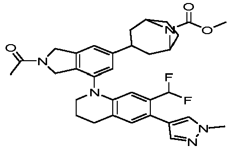 | M001305 | 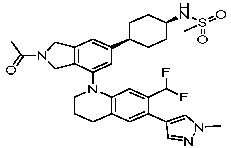 |
| M001137 | 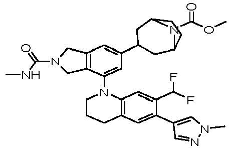 | M001306 | 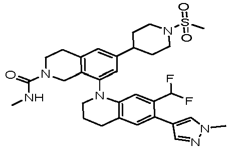 |
| M001142 | 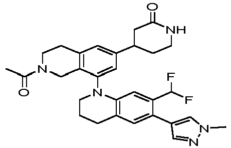 | M001307 | 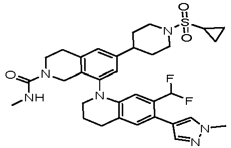 |
| M001143 | 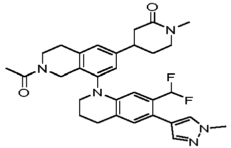 | M001308 | 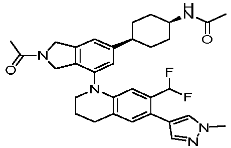 |
| M001144 | 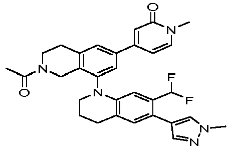 | M001309 | 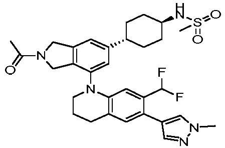 |
| M001153 | 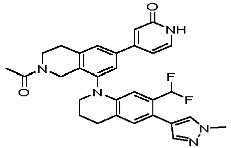 | M001310 | 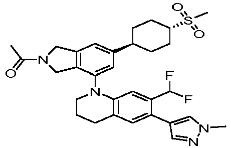 |
| M001154 | 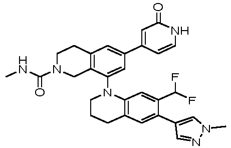 | M001311 | 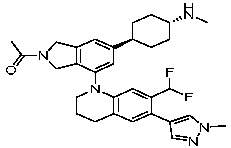 |
| M001155 | 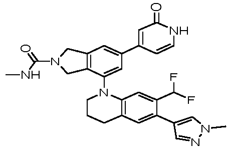 | M001312 | 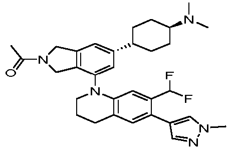 |
| M001156 | 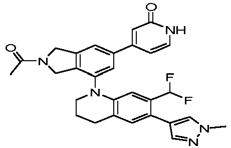 | M001313 | 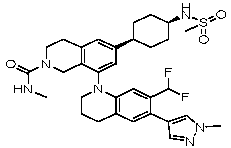 |
| M001161 | 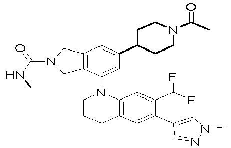 | M001314 | 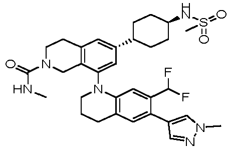 |
| M001162 | 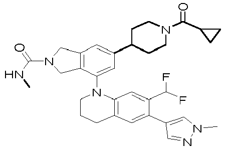 | M001315 | 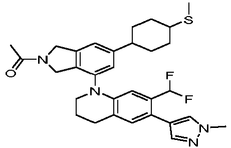 |
| M001163 | 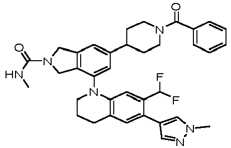 | M001316 | 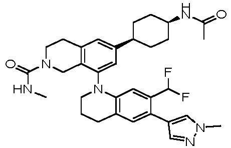 |
| M001164 | 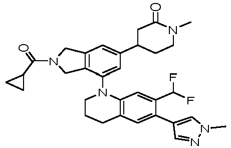 | M001317 | 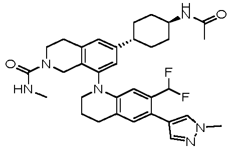 |
| M001166 | 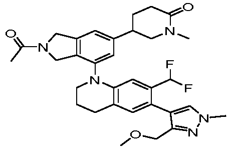 | M001318 | 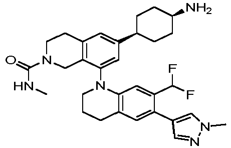 |
| M001167 | 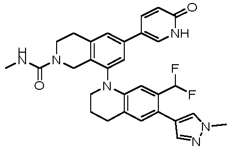 | M001319 | 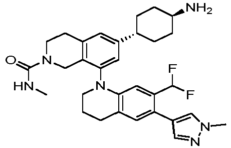 |
| M001168 | 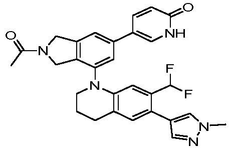 | M001320 | 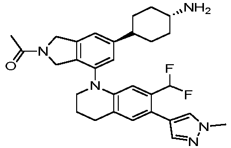 |
| M001169 | 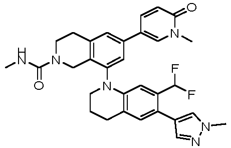 | M001321 | 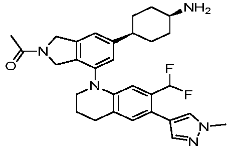 |
| M001170 | 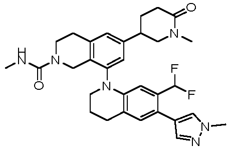 | M001322 | 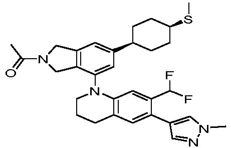 |
| M001171 | 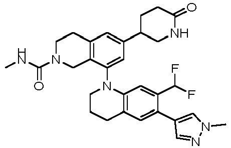 | M001323 | 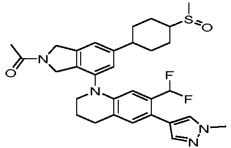 |
| M001172 | 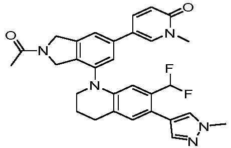 | M001324 | 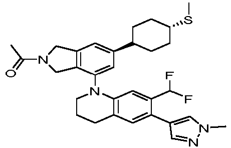 |
| M001173 | 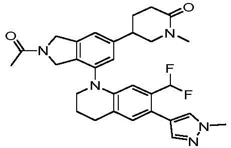 | M001327 | 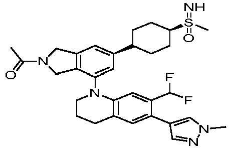 |
| M001174 | 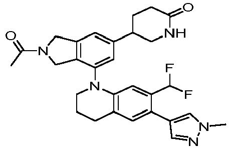 | M001328 | 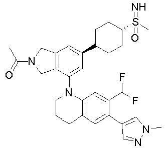 |
| M0011175 | 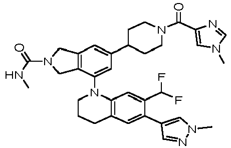 | M001329 | 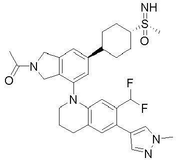 |
| M001176 | 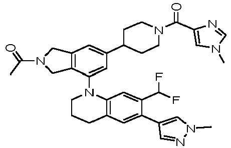 | M001330 | 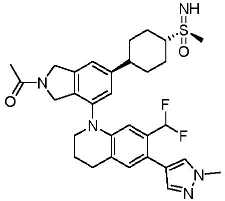 |
| M001178 | 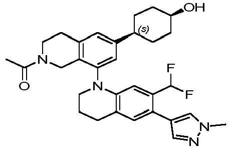 | M001331 | 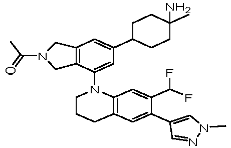 |
| M001179 | 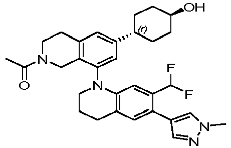 | M001332 | 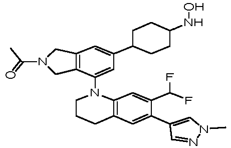 |
| M001180 | 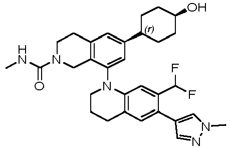 | M001333 | 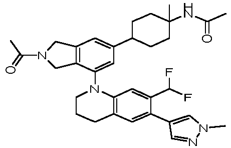 |
| M001181 | 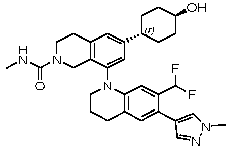 | M001334 | 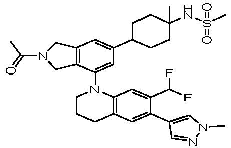 |
| M001184 | 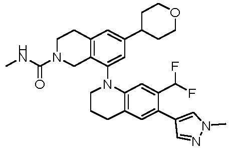 | M001335 | 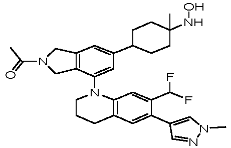 |
| M001186 | 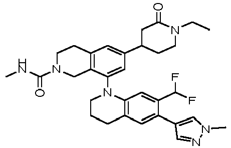 | M001336 | 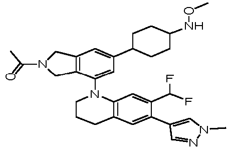 |
| M001187 | 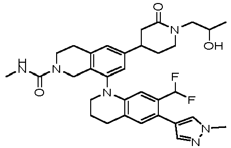 | M001337 | 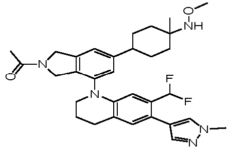 |
| M001188 | 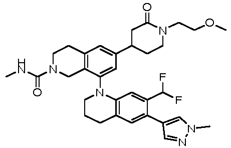 | M001340 | 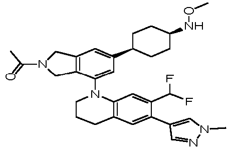 |
| M001189 | 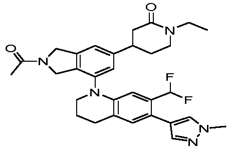 | M001341 | 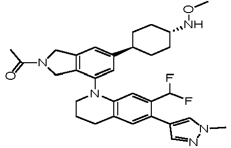 |
| M001190 | 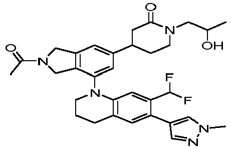 | | |

8. A pharmaceutical composition, wherein the pharmaceutical composition comprises the compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to any one of claims 1 to 7.

9. The pharmaceutical composition according to claim 8, wherein the pharmaceutical composition comprises a second therapeutic agent, and
the second therapeutic agent can be selected from drugs conventionally used for the treatment of cancer, cardiac disease, metabolic diseases, inflammatory diseases, fibrotic diseases and viral infections; preferably, categories of the second therapeutic agent can include androgen receptor antagonists such as enzalutamide, CYP17A1 inhibitors (17α-hydroxylase/C17,20 lyase) such as abiraterone, and cytotoxic chemotherapeutic agents such as docetaxel; categories of therapeutic agents for treating lung cancer include cytotoxic chemotherapeutic agents such as cisplatin, carboplatin and docetaxel; categories of therapeutic agents for treating bladder cancer include cytotoxic chemotherapeutic agents such as gemcitabine, cisplatin or immunotherapy such as the Bacillus Calmette-Guérin vaccine (BCG); the categories of the second therapeutic agent can also be selected from immune checkpoint inhibitors such as pembrolizumab, nivolumab, atezolizumab and ipilimumab, PARP (poly(ADP ribose)polymerase) inhibitors such as olaparib, and CDK4/6 (cyclin-dependent kinases 4 and 6) inhibitors; preferably, the second therapeutic agent can be selected from drugs for use in combination with KRAS inhibitors and the like.

10. Use of the compound represented by formula I and the racemate, the stereoisomer, the tautomer, the isotopic derivative, the nitrogen oxide, the solvate, the polymorph, the metabolite, the ester, the prodrug or the pharmaceutically acceptable salt thereof according to any one of claims 1 to 7 or the pharmaceutical composition according to claim 8 or 9 in the manufacture of a medicament for preventing and/or treating a CBP and/or EP300-mediated disease or condition.

11. The use according to claim 10, wherein the CBP and/or EP300-mediated disease or condition is selected from cancer, cardiac diseases, metabolic diseases, inflammatory diseases, fibrotic diseases, and viral infections; the cancer includes, but is not limited to, prostate cancer, breast cancer, bladder cancer, lung cancer, melanoma, colorectal cancer, gastric cancer, ovarian cancer, cervical cancer, bladder cancer, laryngeal cancer, multiple myeloma, liver cancer, lymphoma, leukemia, etc.; the prostate cancer may be, for example, castration-resistant prostate cancer (CRPC); the lung cancer may be, for example, non-small cell lung cancer or small cell lung cancer; the lymphoma may be selected from non-Hodgkin lymphoma, diffuse large B cell lymphoma, etc.
